# EUROPEAN PATENT APPLICATION

(11) **EP 3 733 695 A1**
(43) Date of publication of application: **04.11.2020**
(21) Application number: 18894804.6
(22) Date of filing: 26.12.2018
(51) Int. Cl.: C07K 14/705, C07K 16/28, C12N 15/62, C12N 15/63, C12N 5/0783, C12N 5/10, A61K 35/17, A61K 38/17, A61P 35/00

(54) **DUAL-ACTIVATING COSTIMULATORY MOLECULE RECEPTOR AND USE THEREOF**

(30) Priority: 28.12.2017 CN 201711462081
(71) Applicant: Shanghai Cell Therapy Group Co., Ltd, Jiading District Anting Town Shanghai 201805 (CN)
(72) Inventor: QIAN, Qijun, Shanghai 201805 (CN); JIN, Huajun, Shanghai 201805 (CN); XU, Huimin, Shanghai 201805 (CN); LIU, Xiangzhen, Shanghai 201805 (CN); LI, Linfang, Shanghai 201805 (CN); WANG, Chao, Shanghai 201805 (CN)
(74) Representative: Cabinet Beau de Loménie
(86) International application number: PCT/CN2018/123974
(87) International publication number: WO 2019/129086

(57) **Abstract**

Provided are a dual-activating costimulatory molecule receptor and the use thereof. The dual-activating costimulatory molecule receptor comprises the following elements from the N-terminus to the C-terminus: a selectable signal peptide, a costimulatory signal molecule-activating single-chain antibody, an extracellular hinge area, a transmembrane region and an intracellular costimulatory signal molecule. The dual-activating costimulatory molecule receptor can produce a strong clustering effect when co-modifying T-cells with first-generation CAR-T comprising a first signal, can kill tumor cells, and at the same time, does not trigger a strong T-cell immunity or cause potentially serious toxic side effects.

## Description

### Technical Field

The invention belongs to the field of cell biology and immunology, and relates to a dual-activating costimulatory molecule receptor and the use of treating malignant tumors by T cells modified with the receptor.

### Background

Adoptive cell therapy (ACT) is to reinject the treated autologous or allogeneic immune cells (mainly autologous cells) to a tumor patient to directly kill tumor cells, or kill tumor cells by stimulating the body's immune response to achieve the therapy purpose. At present, adoptive cell therapy for tumors is developing rapidly, and good results has been achieved in the clinical treatment of various malignant tumors (Nature.2016; Jun16; 534(7607):396-401); (Cell. 2016 Oct 6;167(2):405-418.e13). Tumor immune cell therapy is considered to be one of the most promising treatments for malignant tumors.

T cell activation requires the stimulation of two signals which are related to T cell activation. The TCR-CD3 complex on the surface of T cells binds to the antigen peptide-MHC molecule to provide the first signal for T cell activation and to determine the killing specificity of T cells; the costimulatory molecules (such as CD28) on the surface of T cells binds to the corresponding ligands (such as B7) to provide the second signal for T cell activation, and to promote T cell activation, proliferation and survival. However, the lack of or decreased expression of the first signal stimulus source (such as MHC molecules) and the second signal ligand (such as B7) of tumor cells cannot effectively provide signals related to T cell activation, and thus cannot activate T cell immune response. Extensive activation of T cell costimulatory molecules may bring strong toxic side effects.

The chimeric antigen receptor CAR (CAR) uses the extracellular single chain variable fragment (scFv) that specifically recognizes tumor antigens to activate the transmission of the intracellular signal CD3ζ or FcεRIγ ITAM (immunoreceptor tyrosine-based activation motifs). However, the first-generation CAR receptor lacks the co-stimulatory signal of T cells, which causes T cells to exert only an instant effect, with a short duration in the body and little secretion of cytokines.

The second-generation and third-generation CARs combine the two signals required for T cell activation, and link the second signal CD28 or/and 4-1BB intracellular signal region directly to the CD3ζ molecule, thus avoiding the barrier that prevents T cells from being activated due to the lack of the second signal, such as B7, in tumor cells. After the first signal and the second signal are combined, the activation, proliferation and killing effect of T cells is greatly improved, which greatly increases the therapy effect. Based on the current understanding on the mechanism of T cell activation, CD28 and 4-1BB molecules can provide the second activation signal and further strengthen the TCR/CD3 signal.

However, no matter what kind of CAR-T cells, they can only provide stimulus signals to the modified T cells, but lack bystander effect and cannot activate surrounding T cells. Activation of surrounding T cells would result in a stronger clustering effect and cause a series of cascade reactions that activate T cell function.

### SUMMARY

Based on in-depth research and creative work, the inventor has designed a Dual Costimulatory Activated Receptor (DCR), which transmits the second signal related to T cell activation by an extracellular agonistic antibody for a costimulatory signal molecule. The modified T cells can not only activate their own costimulatory signals through the extracellular CD137 agonistic antibody, but also activate the intracellular costimulatory signals of the surrounding unmodified (un-activated) T cells upon contacting with the unmodified T cells, thereby promoting T cell activation, proliferation and survival. In particular, when DCR modifies T cells together with the first-generation CAR-T containing the first signal, a strong clustering effect that kills tumor cells can be produced. In addition, the effect of this dual activation is only limited to the T cells in contact with each other, and will not induce strong T cell immunity or cause potentially serious toxic side effects like a CD137 agonistic antibody.

The followings are provided in the present disclosure:
One aspect of the present disclosure relates to an isolated polypeptide, comprising, from N-terminus to C-terminus, the following elements:
an optional signal peptide, a polypeptide that activates a costimulatory signal molecule (such as an agonistic single-chain antibody for the costimulatory signal molecule or a ligand of the costimulatory signal molecule), an extracellular hinge region, a transmembrane region, and a intracellular costimulatory signal molecule.

In one or more embodiments of the present disclosure, the polypeptide is characterized by any 1, 2, 3, 4 or 5 of the following items (1) to (5):
(1) the signal peptide is a membrane protein signal peptide; preferably, the signal peptide is one or more peptides selected from the group consisting of a CD8 signal peptide, a CD28 signal peptide, and a CD4 signal peptide; preferably, the signal peptide is a CD8 signal peptide; preferably, the amino acid sequence of the CD8 signal peptide is as shown in SEQ ID NO: 1;
(2) the agonistic single-chain antibody for the costimulatory signal molecule is any one or more of members selected from the group consisting of a CD137 agonistic single-chain antibody, a CD28 agonistic single-chain antibody and a CD40 agonistic single-chain antibody; the ligand of the costimulatory signal molecule is any one or more of members selected from the group consisting of a ligand of CD137, a ligand of CD28 and a ligand of CD40;
   preferably, the amino acid sequence of the CD137 agonistic single-chain antibody is as shown in SEQ ID NO: 2;
   preferably, the amino acid sequence of the CD28 agonistic single-chain antibody is as shown in SEQ ID NO: 31;
   preferably, the amino acid sequence of the CD40 agonistic single-chain antibody is as shown in SEQ ID NO: 55;
   preferably, the ligand of CD137 is 4-1BBL;
   preferably, the ligand of CD28 is CD80/CD86;
   preferably, the ligand of CD40 is CD40L;
(3) the extracellular hinge region is any one or more of members selected from the group consisting of a IgG4Fc CH2CH3 hinge region, a CD28 hinge region and a CD8 hinge region;
   preferably, the extracellular hinge region is a CD8 hinge region;
   preferably, the amino acid sequence of the CD8 hinge region is as shown in SEQ ID NO: 3;
   preferably, the extracellular hinge region is an IgG4Fc CH2CH3 hinge region;
   preferably, the amino acid sequence of the IgG4Fc CH2CH3 hinge region is as shown in SEQ ID NO: 56;
(4) the transmembrane region is any one or more of members selected from the group consisting of a CD28 transmembrane region, a CD8 transmembrane region, a CD3ζ transmembrane region, a CD134 transmembrane region, a CD137 transmembrane region, an ICOS transmembrane region and a DAP 10 transmembrane region; preferably, the transmembrane region is a CD28 transmembrane region; preferably, the amino acid sequence of the CD28 transmembrane region is as shown in SEQ ID NO: 4;
(5) the intracellular costimulatory signal molecule is any one or more of members selected from the group consisting of a CD28 intracellular domain, a CD134/OX40 intracellular domain, a CD137/4-1BB intracellular domain, a LCK intracellular domain, an ICOS intracellular domain and a DAP10 intracellular domains; preferably, the intracellular costimulatory signal molecule is a CD28 intracellular domain and/or a CD137 intracellular domain; preferably, the amino acid sequence of the CD28 intracellular domain is as shown in SEQ ID NO: 5; preferably, the amino acid sequence of the CD137 intracellular domain is as shown in SEQ ID NO: 6.

In one or more embodiments of the present disclosure, the polypeptide comprises, from N-terminus to C-terminus, the following elements:
an optional CD8 signal peptide, a CD137 agonistic single-chain antibody, a CD8 extracellular hinge region, a CD28 transmembrane region, a CD28 intracellular domain and/or a CD137 intracellular domain;
an optional CD8 signal peptide, a CD28 agonistic single-chain antibody, a CD8 extracellular hinge region, a CD28 transmembrane region, a CD28 intracellular domain and/or a CD137 intracellular domain; or
an optional CD8 signal peptide, a CD40 agonistic single-chain antibody, an IgG4Fc CH2CH3 hinge region, a CD28 transmembrane region, a CD28 intracellular domain and/or a CD137 intracellular domain;

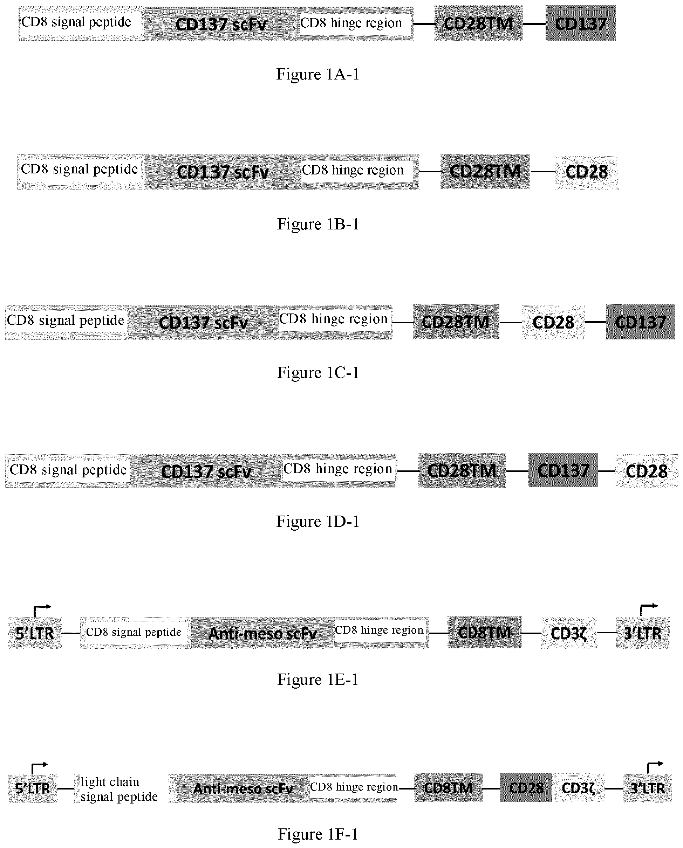

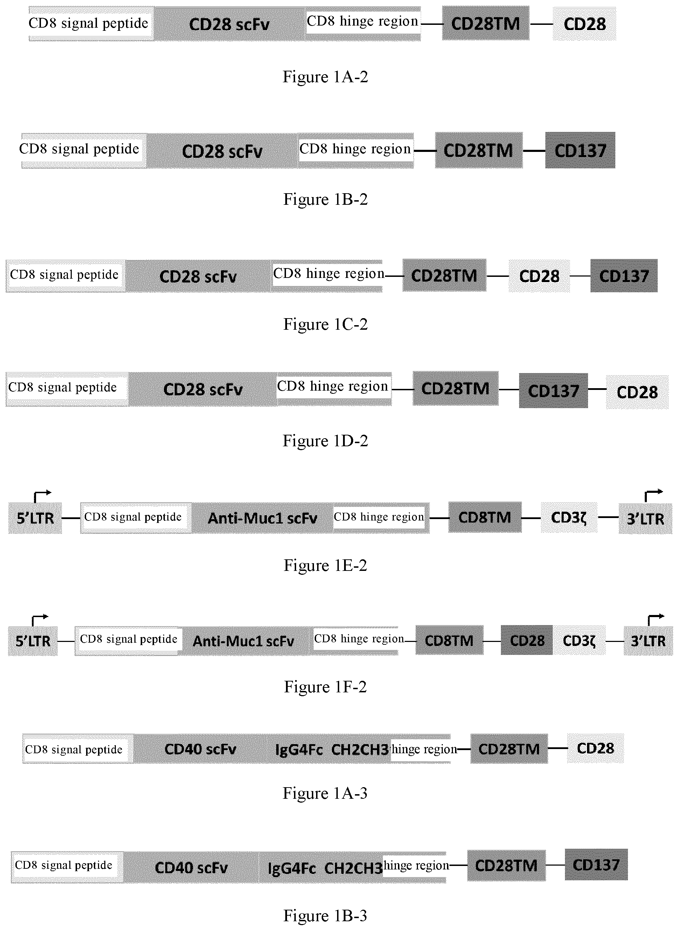

In one or more embodiments of the present disclosure, the polypeptide is as shown in Figure 1A-1 to Figure 1D-1.

In one or more embodiments of the present disclosure, the polypeptide is as shown in Figure 1A-2 to Figure 1D-2.

In one or more embodiments of the present disclosure, the polypeptide is as shown in Figure 1A-3 to Figure 1D-3.

In one or more embodiments of the present disclosure, the amino acid sequence of the polypeptide is as shown in any one of SEQ ID NOs: 7 to 14;
any one of SEQ ID NOs: 32 to 39; or
any one of SEQ ID NOs: 57 to 64.

Another aspect of the present disclosure relates to an isolated polynucleotide encoding the isolated polypeptide according to any embodiment of the present disclosure; preferably, the sequence of the isolated polynucleotide is as shown in any of SEQ ID NO: 15 or 22;
any one of SEQ ID NOs: 40 to 47; or
any one of SEQ ID NOs: 65 to 72.

Another aspect of the present disclosure relates to a nucleic acid construct comprising the polynucleotide of the present disclosure.

Another aspect of the present disclosure relates to a recombinant vector, comprising the polynucleotide of the present disclosure or the nucleic acid construct of the present disclosure; preferably, the recombinant vector is a recombinant cloning vector, a recombinant eukaryotic expression plasmid or a recombinant viral vector; preferably, the recombinant expression vector is a recombinant transposon vector; preferably, the transposon vector contains a transposition element selected from the group consisting of piggybac, sleeping beauty, frogprince, Tn5, and Ty; preferably, the recombinant expression vector is a recombinant vector obtained by recombining the polynucleotide of the present disclosure and a PS328b vector.

Another aspect of the present disclosure relates to a recombinant vector combination, comprising a first recombinant vector and a second recombinant vector, wherein:
The first recombinant vector is the recombinant vector of the present disclosure,

The second recombinant vector contains a coding sequence of a first-generation chimeric antigen receptor; preferably, the first-generation chimeric antigen receptor is one that targets mesothelin, Mucl or EGFR; preferably, the amino acid sequence of the first-generation chimeric antigen receptor is as shown in SEQ ID NO: 23, SEQ ID NO: 48 or SEQ ID NO: 73; preferably, the nucleic acid sequence of the first-generation chimeric antigen receptor is as shown in SEQ ID NO: 24, SEQ ID NO: 49 or SEQ ID NO: 74;
preferably, the second recombinant vector is a recombinant PNB328 vector.
wherein, the "first" and "second" in the above-mentioned "first recombinant vector" and "second recombinant vector" are only for the purpose of distinguishing, and do not mean the order.

Another aspect of the present disclosure relates to a recombinant host cell, wherein the cell contains the polynucleotide of the present disclosure, the nucleic acid construct of the present disclosure, the recombinant vector of the present disclosure, or the combination of the recombinant vectors of the present disclosure; preferably, the recombinant host cell is a recombinant mammalian cell; preferably, the recombinant host cell is a recombinant T cell; preferably, the recombinant T cell is a recombinant peripheral blood mononuclear cell.

Yet another aspect of the present disclosure relates to a T cell expressing the polypeptide according to any claim of the present disclosure and a first-generation chimeric antigen receptor; preferably, the recombinant T cell is a recombinant peripheral blood mononuclear cell; preferably, the first-generation chimeric antigen receptor is one that targets mesothelin, Mucl or EGFR; preferably, the amino acid sequence of the first-generation chimeric antigen receptor is as shown in SEQ ID NO: 23, SEQ ID NO: 48 or SEQ ID NO: 73.

Another aspect of the present disclosure relates to a pharmaceutical composition comprising any polypeptide of the present disclosure, the polynucleotide of the present disclosure, the nucleic acid construct of the present disclosure, the recombinant vector of the present disclosure, the combination of the recombinant vectors of the present disclosure, the recombinant host cell of the present disclosure or the T cell of the present disclosure; optionally, further comprising a pharmaceutically acceptable excipient.

Another aspect of the present disclosure relates to use of any polypeptide of the present disclosure, the polynucleotide of the present disclosure, the nucleic acid construct of the present disclosure, the recombinant vector of the present disclosure, the combination of the recombinant vectors of the present disclosure, the recombinant host cell of the present disclosure or the T cell of the present disclosure in the preparation of medicament for treating and/or preventing a cancer; preferably, the cancer is one that abnormally expresses mesothelin, Mucl or EGFR on the surface of its cancer cells; preferably, the cancer is selected from the group consisting of adenocarcinoma, lung cancer, colon cancer, colorectal cancer, breast cancer, ovarian cancer, cervical cancer, gastric cancer, bile duct cancer, gallbladder cancer, esophageal cancer, pancreatic cancer or prostate cancer.

Another aspect of the present disclosure relates to use of any polypeptide of the present disclosure, the polynucleotide of the present disclosure, the nucleic acid construct of the present disclosure, the recombinant vector of the present disclosure, the combination of the recombinant vectors of the present disclosure, the recombinant host cell of the present disclosure or the T cell of the present disclosure in the preparation of medicament for inhibiting a cancer cell; preferably, the cancer cell is one that abnormally expresses mesothelin, Mucl or EGFR on the cell surface; preferably, the cancer cell is selected from the group consisting of: cancer cells of adenocarcinoma, lung cancer, colon cancer, colorectal cancer, breast cancer, ovarian cancer, cervical cancer, gastric cancer, bile duct cancer, gallbladder cancer, esophageal cancer, pancreatic cancer or prostate cancer.

Another aspect of the present disclosure relates to a method of inhibiting a cancer cell *in vivo* or *in vitro*, comprising the step of administering to the cancer cell an effective amount of any polypeptide of the present disclosure, the polynucleotide of the present disclosure, the nucleic acid construct of the present disclosure, the recombinant vector of the present disclosure, the combination of the recombinant vectors of the present disclosure, the recombinant host cell of the present disclosure or the T cell of the present disclosure; preferably, the cancer cell is one that abnormally expresses mesothelin, Mucl or EGFR on the cell surface; preferably, the cancer cell is selected from the group consisting of: cancer cells of adenocarcinoma, lung cancer, colon cancer, colorectal cancer, breast cancer, ovarian cancer, cervical cancer, gastric cancer, bile duct cancer, gallbladder cancer, esophageal cancer, pancreatic cancer or prostate cancer.

Another aspect of the present disclosure relates to a method of treating and/or preventing cancer, comprising the step of administering to a subject in need thereof an effective amount of any polypeptide of the present disclosure, the polynucleotide of the present disclosure, the nucleic acid construct of the present disclosure, the recombinant vector of the present disclosure, the combination of the recombinant vectors of the present disclosure, the recombinant host cell of the present disclosure or the T cell of the present disclosure; preferably, the cancer is one that abnormally expresses mesothelin, Mucl or EGFR on the surface of its cancer cells; preferably, the cancer is selected from the group consisting of adenocarcinoma, lung cancer, colon cancer, colorectal cancer, breast cancer, ovarian cancer, cervical cancer, gastric cancer, bile duct cancer, gallbladder cancer, esophageal cancer, pancreatic cancer or prostate cancer.

Another aspect of the present disclosure relates to use of any polypeptide of the present disclosure, the polynucleotide of the present disclosure, the nucleic acid construct of the present disclosure, the recombinant vector of the present disclosure, the combination of the recombinant vectors of the present disclosure, the recombinant host cell of the present disclosure or the T cell of the present disclosure in the preparation of a medicament for promoting the secretion of a cytokine, wherein the cytokine is any one or more of members selected from the group consisting of IL-2, IL-4, IL-6, IL-10, TNF-α and IFN-γ.

Another aspect of the present disclosure relates to a method of promoting the secretion of a cytokine by T cells *in vivo* or *in vitro*, comprising the step of administering to the T cells an effective amount of any polypeptide of the present disclosure, the polynucleotide of the present disclosure, the nucleic acid construct of the present disclosure, the recombinant vector of the present disclosure, the combination of the recombinant vectors of the present disclosure, the recombinant host cell of the present disclosure or the T cell of the present disclosure, wherein the cytokine is any one or more of members selected from the group consisting of IL-2, IL-4, IL-6, IL-10, TNF-α and IFN-γ.

In the present disclosure, unless otherwise specified, the scientific and technical terms used herein have the meanings commonly understood by those skilled in the art. Moreover, the laboratory procedures of cell culture, molecular genetics, nucleic acid chemistry, and immunology used herein are all routine procedures widely used in the corresponding fields. Meanwhile, in order to better understand the present disclosure, definitions and explanations of related terms are provided below.

In the present disclosure, the term "isolated" or "be isolated" refers to substances obtained from the natural state by artificial means. If a certain "isolated" substance or component appears in nature, its natural environment may have changed, or the substance has been separated from the natural environment, or both. For example, if a certain non-isolated polynucleotide or polypeptide naturally exists in a living animal, the same polynucleotide or polypeptide with high purity isolated from its natural state is called "isolated". The term "isolated" or "be isolated" does not exclude the inclusion of artificial or synthetic substances, nor does it exclude the presence of other impure materials that do not affect the activity of the substance.

In the present disclosure, the term "vector" refers to a nucleic acid delivery vehicle into which polynucleotides can be inserted. When the vector can express the protein encoded by the inserted polynucleotide, the vector is called an expression vector. The vector can be introduced into a host cell by transformation, transduction or transfection, so that the genetic material elements carried by the vector can be expressed in the host cell. Vectors are well known to those skilled in the art, including but not limited to: plasmids; phagemids; cosmids; artificial chromosomes, such as yeast artificial chromosomes (YAC), bacterial artificial chromosomes (BAC) or PI derived artificial chromosomes (PAC); phages such as lambda phage or M13 phage and animal viruses, etc. Animal viruses that can be used as vectors include, but are not limited to, retroviruses (including lentiviruses), adenoviruses, adeno-associated viruses, herpes viruses (such as herpes simplex virus), poxviruses, baculoviruses, papillomaviruses, and papovaviruses, (e.g. SV40). A vector can contain a variety of elements that control expression, including but not limited to promoter sequences, transcription initiation sequences, enhancer sequences, selection elements and reporter genes. In addition, the vector may also contain an origin of replication.

In the present disclosure, the term "host cell" refers to a cell that can be used to introduce a vector, which includes, but is not limited to, prokaryotic cells such as *Escherichia coli* or *B. subtilis*, fungal cells such as yeast cells or *Aspergillus*, insect cells such as S2 *Drosophila melanogaster* cells or Sf9, or animal cells such as fibroblasts, CHO cells, COS cells, NSO cells, HeLa cells, BHK cells, HEK 293 cells or human cells.

In the present disclosure, the term "chimeric antigen receptor" (CAR) is an artificially modified receptor which can anchor the specific molecules (such as antibodies) recognizing tumor antigens to immune cells (such as T cells), so that the immune cells can recognize tumor antigens or virus antigens and kill tumor cells or virus-infected cells.

In the present disclosure, the term "CD137", the NCBI genebank Access ID of 3604, is expressed in T cells and can promote the proliferation and activation of T cells. In the second-generation chimeric antigen receptor-modified T cell therapy, it is often used as an intracellular costimulatory signal to enhance the activation and proliferation of T cells.

In the present disclosure, the term "CD28", the NCBI genebank Access ID of 940, is expressed in T cells and can promote the proliferation and activation of T cells. In the second-generation chimeric antigen receptor-modified T cell therapy, it is often used as an intracellular costimulatory signal to enhance the activation and proliferation of T cells.

In the present disclosure, the term "CD40", the NCBI genebank Access ID of 958, is expressed in T cells and can promote the proliferation and activation of T cells. In the second-generation chimeric antigen receptor-modified T cell therapy, it is often used as an intracellular costimulatory signal to enhance the activation and proliferation of T cells.

In the present disclosure, the term "single-chain antibody" or "single-chain antibody variable fragment (scFv)" refers to an antibody fragment formed by linking the amino acid sequences of V_{L} region and V_{H} region of an antibody via a Linker, which has the ability to bind to an antigen. Wherein the VL and VH domains pair to form a monovalent molecule by a connector that can produce a single polypeptide chain (see, for example, Bird et al., Science 242:423-426 (1988) and Huston et al., Proc. Natl. Acad. Sci. USA 85:5879-5883 (1988)) Such scFv molecules may have the general structure: NH₂-V_{L}-linker-V_{H}-COOH or NH₂-V_{H}-linker-V_{L}-COOH. A suitable linker in the art consists of repeated GGGGS amino acid sequence(s) or variants thereof. For example, a linker having an amino acid sequence of (GGGGS)₄ can be used, but variants thereof can also be used (Holliger et al. Proc. Natl. Acad. Sci. USA 90: 6444-6448). Other linkers that can be used in the present disclosure are described by Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56 and Roovers et al. (2001), Cancer Immunol.

In the present disclosure, the term "signal(s) related to T cell activation" refers to the two signals required for T cell activation, that is, the TCR-CD3 complex on the surface of T cells binding to the antigen peptide-MHC molecule to provide the first signal for T cell activation and determine the killing specificity of T cells; the costimulatory molecules (such as CD28) on the surface of T cells binding to the corresponding ligands (such as B7) to provide the second signal for T cell activation, promoting T cell activation, proliferation and survival.

In the present disclosure, the immunoreceptor tyrosine activation motif is a CD3ζ and/or FcεRIγ tyrosine activation motif; preferably, the immunoreceptor tyrosine activation motif is a CD3ζ tyrosine activation motif, amino acid sequence of which is as shown in SEQ ID NO: 25.

The term "co-stimulating molecule" or "costimulatory signal molecule" in the present disclosure refers to some adhesion molecules on the surface of immune cells, such as CD28, CD134/OX40, CD137/4-1BB, CD40, etc., which activate the second signal of the immune cells, enhance the proliferation and the cytokines secretion of the immune cells, and improve the survival of activated immune cells by binding to their ligands.

In the present disclosure, the term "PB" transposon is short for Piggybac. Transposon is a mobile genetic factor. A stretch of DNA sequence can be copied or cut separately from the original position, inserted into another site after circularization, which regulates the downstream genes. This process is called transposition. Due to the function of the transposon on the vector, meso G1 CAR or 137 DCR is integrated into the T cell genome.

Antibodies are divided into agonistic and inhibitory antibodies. In the present disclosure, the term "extracellular agonistic antibody" refers to a antibody anchored on the surface of the cell membrane and bound to a acting site of a cell surface molecule (i.e., the position where ligand and receptor bind to each other) to promote cell biological functions. CD137 extracellular agonistic antibody is considered to be a unique surface molecule of T cells, as CD137 molecule exists on the surface of most T cells. CD137 extracellular agonistic antibody can effectively recognize and activate the CD137 molecular signal and generate the second signal. CD137 can replace the second signal effect of APC.

In the present disclosure, the term "bystander effect" means that, for tumor cells or virus-infected cells, a single CAR-T cell can only activate its own second signal, but cannot further activate surrounding T cells, so that the surrounding T cells cannot provide a series of activated T cell functions.

In the present disclosure, the term "clustering effect" means that a single modified T cell can continuously recruit and activate surrounding un-activated T cells, and activate downstream signaling pathways of the surrounding T cells, leading to multiple T cells activation, proliferation and other functions.

In the present disclosure, the term "mesothelin" is also known as MSLN, meso, mesothelin, with the NCBI genebank Access ID of 10232. Initially, the synthesized mesothelin is a 69kDa cell surface protein. During the maturation, the synthesized mesothelin is lysed to two parts by furin, wherein, the 40-kDa fragment at the C-terminal is anchored on the membrane, and the 32-kDa fragment at the N-terminus is released in a dissolved form, called pegakaryocyte potentiation factor (MPF). The so-called mesothelin refers to the fragment anchored on the membrane. Mesothelin is overexpressed in various malignant tumors such as pancreatic cancer, mesothelioma, ovarian cancer and lung adenocarcinoma, and it is a promising target for cell therapy. The full-length mesothelin protein can be divided into three segments, Region I (296-390), II (391-486) and III (487-598).

In the present disclosure, the term "Mucl" is also known as mucin, with the NCBI genebank Access ID of 4582. Mucl is a type I transmembrane glycoprotein with a high molecular weight (>200kD), mostly linked to Ser/Thr on the polypeptide backbone by O-glycosidic bonds. Under normal circumstances, it is mainly expressed in a variety of tissues and organs near the lumen surface or glandular lumen surface of epithelial cells, with apical expression and polar distribution. When tumors occur, Mucl protein can be abnormally expressed on the surface of tumor cells, and its expression level can reach more than 100 times of normal. Moreover, its polar distribution on the cell surface is lost, and it can be evenly distributed on the entire cell surface. In addition, due to incomplete glycosylation, the structure of Mucl protein has also changed, with new sugar chains and peptide epitopes appeared.

In the present disclosure, the term "EGFR" is also known as epidermal growth factor receptor, ErbB-1 or HER1, with the NCBI genebank Access ID of 1956. EGFR is widely distributed on the cell surface of mammalian epithelial cells, fibroblasts, glial cells, keratinocytes, etc. EGFR signaling pathway plays an important role in physiological processes such as cell growth, proliferation and differentiation. EGFR is related to the inhibition of tumor cell proliferation, angiogenesis, tumor invasion, metastasis and apoptosis. The overexpression of EGFR plays an important role in the evolution of malignant tumors, such as glial cell carcinoma, kidney cancer, lung cancer, prostate cancer, pancreatic cancer, breast cancer and so on.

In the present disclosure, the term "a pharmaceutically acceptable carrier/excipient" refers to a carrier and/or an excipient that are pharmacologically and/or physiologically compatible with a subject and active ingredient(s), which is well known in the art (see, for example, Remington's Pharmaceutical Sciences, Gennaro AR Ed., 19th edition, Pennsylvania: Mack Publishing Company, 1995), including but not limited to, pH adjusting agent, surfactant, adjuvant, ion strength enhancer. For example, the pH adjusting agent includes, but is not limited to, phosphate buffer; the surfactant includes, but is not limited to, cationic, anionic or non-ionic surfactant, such as Tween-80; the ion strength enhancer includes, but is not limited to, sodium chloride.

In the present disclosure, the term "effective amount" refers to an amount sufficient to obtain or at least partially obtain the desired effect. For example, an effective amount for preventing a disease (such as a tumor) refers to an amount sufficient to prevent, prohibit, or delay the occurrence of a disease (such as a tumor); an effective amount for treating a disease refers to an amount sufficient to cure or at least partially prevent a disease and its complications in a patient having the disease. It is completely within the abilities of those skilled in the art to determine such an effective amount. For example, the effective amount for therapy will depend on the severity of the disease to be treated, the overall state of the patient's own immune system, the patient's general conditions such as age, weight and sex, the administration route of the drug, and other treatments administered simultaneously and so on.

In the present disclosure, the subject may be a mammal, such as a human.

The beneficial effects of the disclosure

When DCR modifies T cells together with the first-generation CAR-T containing the first signal, a strong clustering effect can be produced, which kills tumor cells. Meanwhile, the effect of this dual activation is only limited to the T cells in contact with each other, and will not induce strong T cell immunity or cause potentially serious toxic side effects like that from injecting a CD137 agonistic antibody. The T cells modified with CD137 dual costimulatory activated receptor combined with chimeric antigen receptor against mesothelin can specifically kill tumor cell lines with high mesothelin expression and is superior to the first-generation and second-generation CAR-T against mesothelin, with little or no killing effect on non-expressing tumor cell lines, having high efficiency and high specificity. While maintaining the efficacy of the first and second-generation CARs, the T cells activated by the CD137 dual costimulatory molecule activated receptor can activate their own second signal; the stronger the tumor-specific antigen, the stronger the first signal CD3ζ activation, and the stronger the second signal related to T cell activation transmitted by the CD137 extracellular agonistic antibody; gathering around the tumor, and continuously recruiting and activating the surrounding un-activated T cells, and activating the downstream signal pathways of T cells, causing T cell cascade activation, proliferation and survival.

When DCR modifies T cells together with the first-generation CAR-T containing the first signal, a strong clustering effect can be produced which kills tumor cells. Meanwhile, the effect of this dual activation is only limited to the T cells in contact with each other, and will not induce strong T cell immunity or cause potentially serious toxic side effects like that from injecting a CD28 agonistic antibody. The T cells modified with CD28 dual costimulatory activated receptor combined with chimeric antigen receptor against Mucl can specifically kill tumor cell lines with high Mucl expression and is superior to the first-generation and second-generation CAR-T against Mucl, with little or no killing effect on non-expressing tumor cell lines, having high efficiency and high specificity. While maintaining the efficacy of the first and second-generation CARs, the T cells activated by the CD28 dual costimulatory activated receptor can activate their own second signal; the stronger the tumor-specific antigen, the stronger the first signal CD3ζ activation, and the stronger the second signal related to T cell activation transmitted by the CD28 extracellular agonistic antibody; gathering around the tumor, and continuously recruiting and activating the surrounding un-activated T cells, and activating the downstream signal pathways of T cells, causing T cell cascade activation, proliferation and survival.

When DCR modifies T cells together with the first-generation CAR-T containing the first signal, a strong clustering effect can be produced, which kills tumor cells. Meanwhile, the effect of this dual activation is only limited to the T cells in contact with each other, and will not induce strong T cell immunity or cause potentially serious toxic side effects like that from injecting a CD40 agonistic antibody. The T cells modified with CD40 dual costimulatory activated receptor combined with chimeric antigen receptor against EGFR can specifically kill tumor cell lines with high EGFR expression and is superior to the first-generation and second-generation CAR-T against EGFR, with little or no killing effect on non-expressing tumor cell lines, having high efficiency and high specificity. While maintaining the efficacy of the first and second-generation CARs, the T cells activated by the CD40 dual costimulatory molecule activated receptor can activate their own second signal; the stronger the tumor-specific antigen, the stronger the first signal CD3ζ activation, and the stronger the second signal related to T cell activation transmitted by the CD40 extracellular agonistic antibody; gathering around the tumor, and continuously recruiting and activating the surrounding un-activated T cells, and activating the downstream signal pathways of T cells, causing T cell cascade activation, proliferation and survival.

### BRIEF DESCRIPTION OF DRAWINGS

Figure 1A-1: Schematic diagram of the structure of CD137 dual-activating costimulatory molecule receptor 137DCR1.
Figure 1B-1: Schematic diagram of the structure of CD137 dual-activating costimulatory molecule receptor 137DCR2.

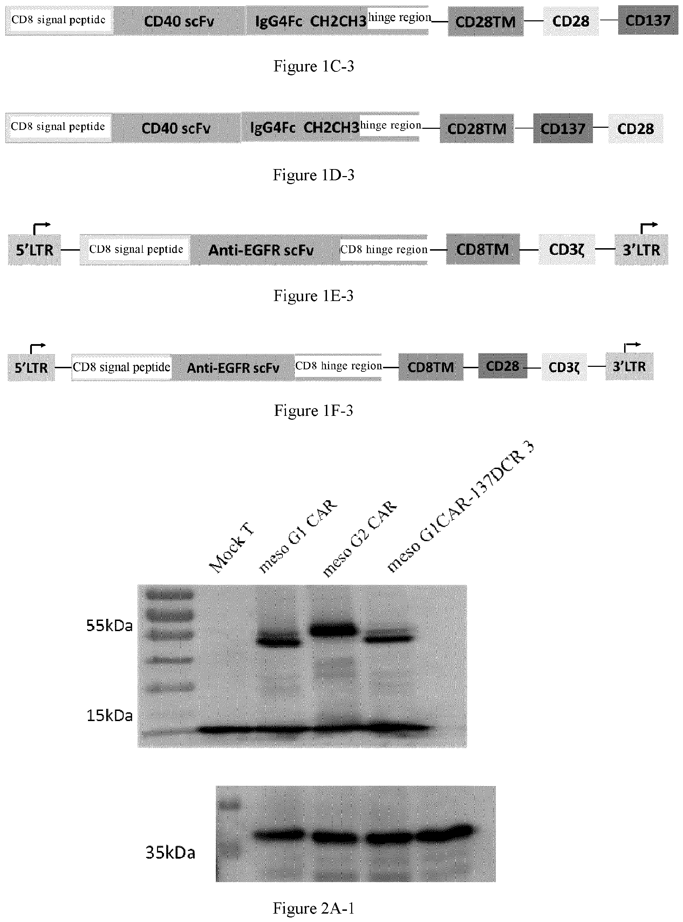

Figure 1C-1: Schematic diagram of the structure of CD137 dual-activating costimulatory molecule receptor 137DCR3.
Figure 1D-1: Schematic diagram of the structure of CD137 dual-activating costimulatory molecule receptor 137DCR4.
Figure 1E-1: Schematic diagram of the structure of meso G1 CAR
Figure 1F-1: Schematic diagram of the structure of meso G2 CAR
Figure 1A-2: Schematic diagram of the structure of CD28 dual-activating costimulatory molecule receptor 28DCR1.
Figure 1B-2: Schematic diagram of the structure of CD28 dual-activating costimulatory molecule receptor 28DCR2.
Figure 1C-2: Schematic diagram of the structure of CD28 dual-activating costimulatory molecule receptor 28DCR3.
Figure 1D-2: Schematic diagram of the structure of CD28 dual-activating costimulatory molecule receptor 28DCR4.
Figure 1E-2: Schematic diagram of the structure of Mucl G1 CAR
Figure 1F-2: Schematic diagram of the structure of Mucl G2 CAR
Figure 1A-3: Schematic diagram of the structure of CD40 dual-activating costimulatory molecule receptor 40DCR1.
Figure 1B-3: Schematic diagram of the structure of CD40 dual-activating costimulatory molecule receptor 40DCR2.
Figure 1C-3: Schematic diagram of the structure of CD40 dual-activating costimulatory molecule receptor 40DCR3.
Figure 1D-3: Schematic diagram of the structure of CD40 dual-activating costimulatory molecule receptor 40DCR4.
Figure 1E-3: Schematic diagram of the structure of EGFR G1 CAR
Figure 1F-3: Schematic diagram of the structure of EGFR G2 CAR.

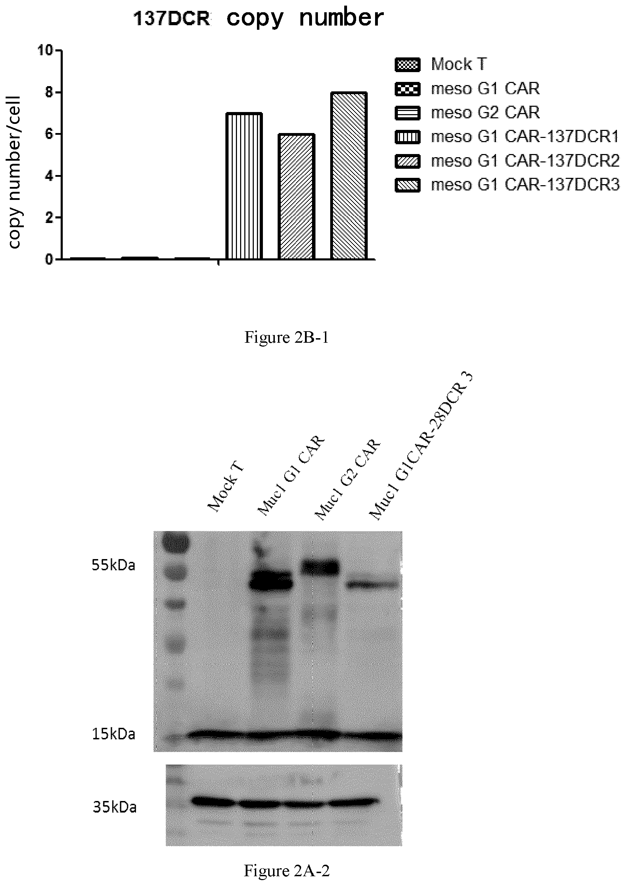

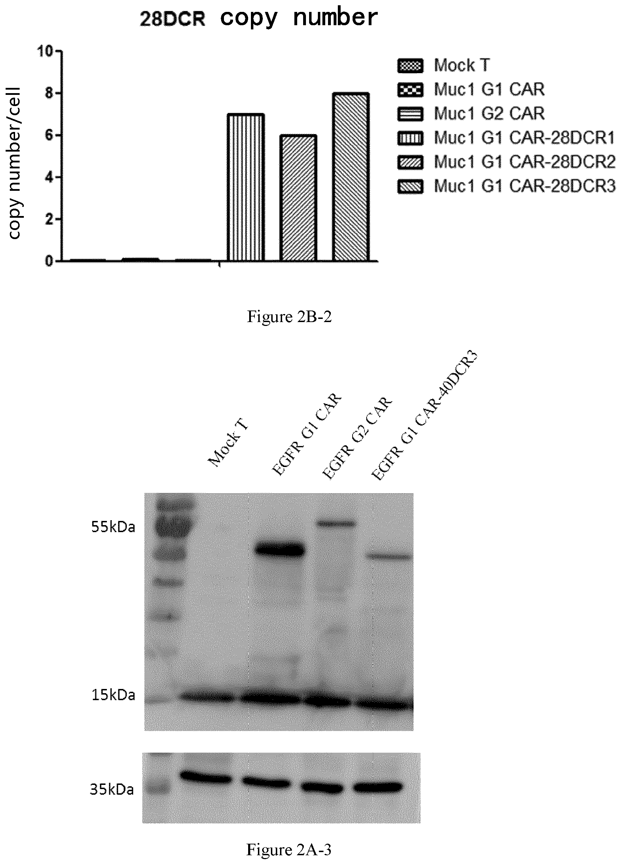

Figure 2A-1: CD3ζ expression profile in the dual-activating chimeric antigen receptor mesothelin CAR-T cells. The internal control is GADPH.

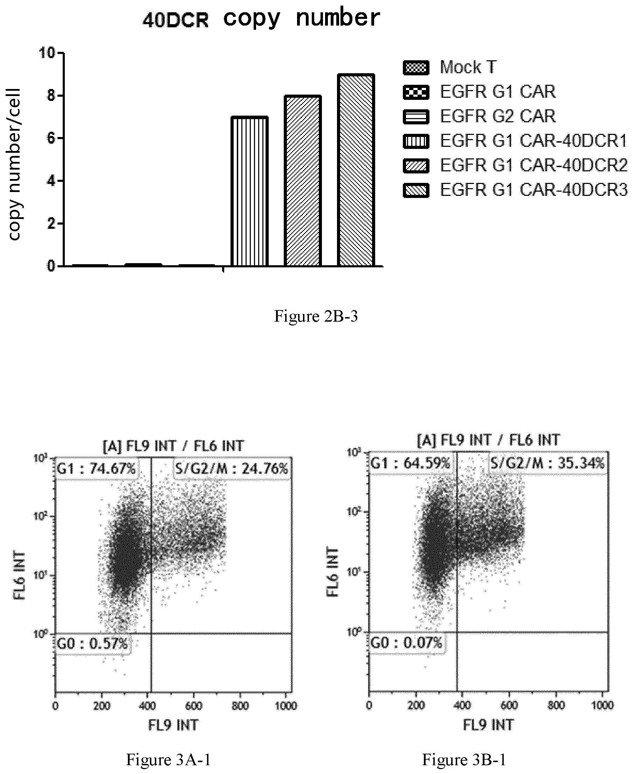

Figure 2B-1: copy numbers of 137DCR1, 137DCR2, 137DCR3 in the dual-activating chimeric antigen receptor mesothelin CAR-T cells.
Figure 2A-2: CD3ζ expression profile in the dual-activating chimeric antigen receptor Mucl CAR-T cells. The internal control is GADPH.
Figure 2B-2: Copy numbers of 28DCR1, 28DCR2, 28DCR3 in the dual-activating chimeric antigen receptor Mucl CAR-T cells.
Figure 2A-3: CD3ζ expression profile in the dual-activating chimeric antigen receptor EGFR CAR-T cells. The internal control is GADPH.
Figure 2B-3: Copy numbers of 40DCR1, 40DCR2, 40DCR3 in the dual-activating chimeric antigen receptor EGFR CAR-T cells.
Figure 3A-1: Function of electroporation of 137DCR, Mock T proliferation phenotype. The abscissa represents the fluorescence intensity of Hochest positive cells, and the ordinate represents the fluorescence intensity of Ki-67 positive cells. Ki-67 is channel 6, and Hochest is channel 9. The result of co-staining Ki67 and Hochest; after distinguishing between diploid and tetraploid, Ki67 is used to separate G0 phase resting cells and proliferating cells. The first quadrant is the cell undergoing DNA synthesis and division, that is, the cell in the S/G2/M phase; the second quadrant is the preparation phase for division, that is, the G1 phase.
Figure 3B-1: Function of electroporation of 137DCR1, proliferation phenotype of recombinant cell 137DCR1. The abscissa represents the fluorescence intensity of Hochest positive cells, and the ordinate represents the fluorescence intensity of Ki-67 positive cells. Ki-67 is channel 6, and Hochest is channel 9. The result of co-staining Ki67 and Hochest; after distinguishing between diploid and tetraploid, Ki67 is used to separate G0 phase resting cells and proliferating cells. The first quadrant is the cell undergoing DNA synthesis and division, that is, the cell in the S/G2/M phase; the second quadrant is the preparation phase for division, that is, the G1 phase.

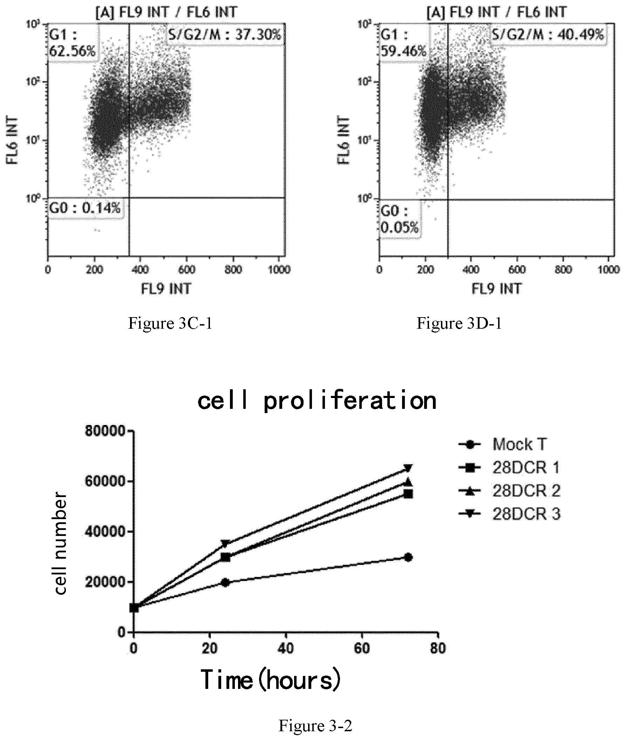

Figure 3C-1: Function of electroporation of 137DCR2, proliferation phenotype of recombinant cell 137DCR2. The abscissa represents the fluorescence intensity of Hochest positive cells, and the ordinate represents the fluorescence intensity of Ki-67 positive cells. Ki-67 is channel 6, and Hochest is channel 9. The result of co-staining Ki67 and Hochest; after distinguishing between diploid and tetraploid, Ki67 is used to separate G0 phase resting cells and proliferating cells. The first quadrant is the cell undergoing DNA synthesis and division, that is, the cell in the S/G2/M phase; the second quadrant is the preparation phase for division, that is, the G1 phase.
Figure 3D-1: Function of electroporation of 137DCR3, proliferation phenotype of recombinant cell 137DCR3. The abscissa represents the fluorescence intensity of Hochest positive cells, and the ordinate represents the fluorescence intensity of Ki-67 positive cells. Ki-67 is channel 6, and Hochest is channel 9. The result of co-staining Ki67 and Hochest; after distinguishing between diploid and tetraploid, Ki67 is used to separate G0 phase resting cells and proliferating cells. The first quadrant is the cell undergoing DNA synthesis and division, that is, the cell in the S/G2/M phase; the second quadrant is the preparation phase for division, that is, the G1 phase.
Figure 3-2: Function of electroporation of 28DCR, proliferation curve of recombinant cell 28DCR1/2/3 cells. The abscissa represents time (h), and the ordinate represents the number of cells.

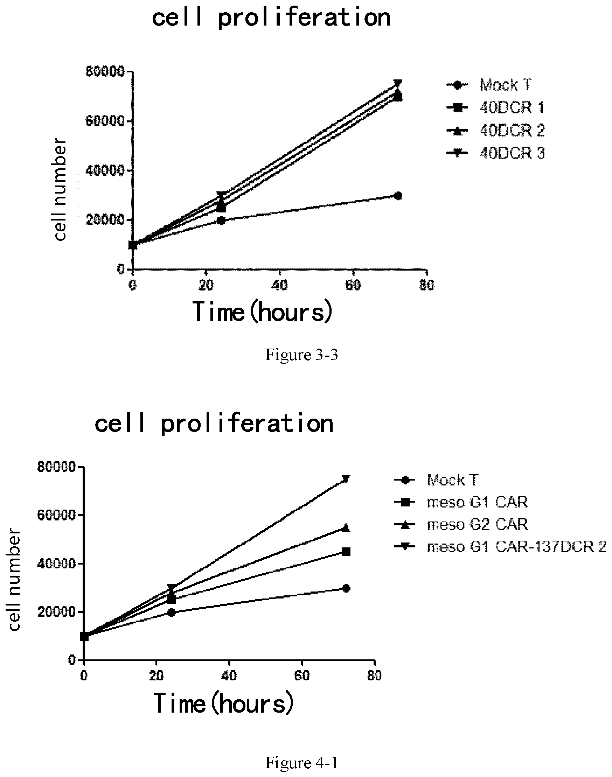

Figure 3-3: Function of electroporation of 40DCR, proliferation curve of recombinant cell 40DCR1/2/3 cells. The abscissa represents time (h), and the ordinate represents the number of cells.
Figure 4-1: Dual-activating chimeric antigen receptor mesothelin CAR-T cells, cell proliferation curve.

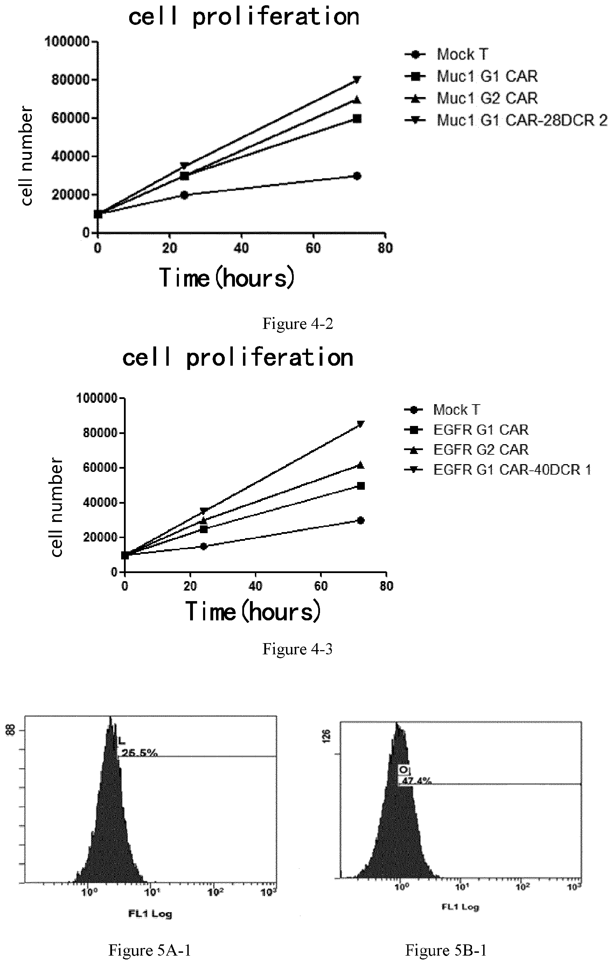

Figure 4-2: Dual-activating chimeric antigen receptor Mucl CAR-T cells, cell proliferation curve.
Figure 4-3: Dual-activating chimeric antigen receptor EGFR CAR-T cells, cell proliferation curve.

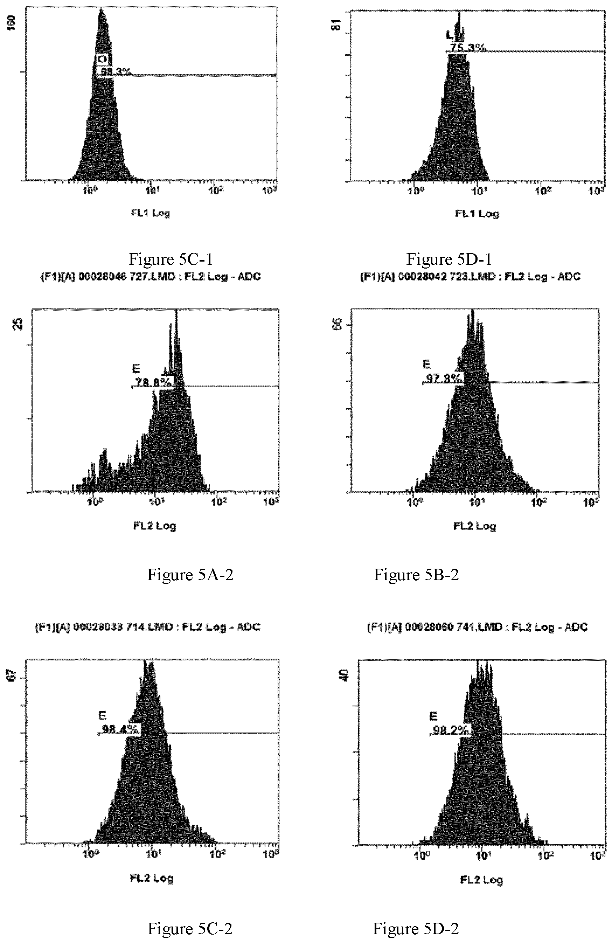

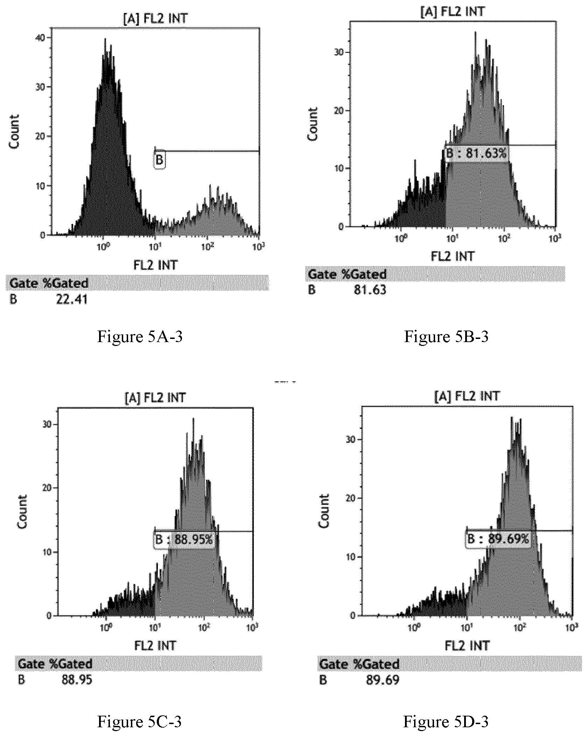

Figure 5A-1: Function of electroporation of 137DCR, CD137 phenotype of Mock T. Wherein the abscissa is the fluorescence intensity of a single CD137-positive cell, and the ordinate is the number of cells with different fluorescence intensities.
Figure 5B-1: Function of electroporation of 137DCR1, CD137 phenotype of recombinant cell 137DCR1. Wherein the abscissa is the fluorescence intensity of a single CD137-positive cell, and the ordinate is the number of cells with different fluorescence intensities.
Figure 5C-1: Function of electroporation of 137DCR2, CD137 phenotype of recombinant cell 137DCR2. Wherein the abscissa is the fluorescence intensity of a single CD137-positive cell, and the ordinate is the number of cells with different fluorescence intensities.
Figure 5D-1: Function of electroporation of 137DCR3, CD137 phenotype of recombinant cell 137DCR3. Wherein the abscissa is the fluorescence intensity of a single CD137-positive cell, and the ordinate is the number of cells with different fluorescence intensities.
Figure 5A-2: Function of electroporation of 28DCR, CD28 phenotype of Mock T. Wherein the abscissa is the fluorescence intensity of a single CD28-positive cell, and the ordinate is the number of cells with different fluorescence intensities.
Figure 5B-2: Function of electroporation of 28DCR1, CD28 phenotype of recombinant cell 28DCR1. Wherein the abscissa is the fluorescence intensity of a single CD28-positive cell, and the ordinate is the number of cells with different fluorescence intensities.
Figure 5C-2: Function of electroporation of 28DCR2, CD28 phenotype of recombinant cell 28DCR2. Wherein the abscissa is the fluorescence intensity of a single CD28-positive cell, and the ordinate is the number of cells with different fluorescence intensities.
Figure 5D-2: Function of electroporation of 28DCR3, CD28 phenotype of recombinant cell 28DCR3. Wherein the abscissa is the fluorescence intensity of a single CD28-positive cell, and the ordinate is the number of cells with different fluorescence intensities.
Figure 5A-3: Function of electroporation of 40DCR, CD40 phenotype of Mock T. Wherein the abscissa is the fluorescence intensity of a single CD40-positive cell, and the ordinate is the number of cells with different fluorescence intensities.
Figure 5B-3: Function of electroporation of 40DCR1, CD40 phenotype of recombinant cell 40DCR1. Wherein the abscissa is the fluorescence intensity of a single CD40-positive cell, and the ordinate is the number of cells with different fluorescence intensities.
Figure 5C-3: Function of electroporation of 40DCR2, CD40 phenotype of recombinant cell 40DCR2. Wherein the abscissa is the fluorescence intensity of a single CD40-positive cell, and the ordinate is the number of cells with different fluorescence intensities.
Figure 5D-3: Function of electroporation of 40DCR3, CD40 phenotype of recombinant cell 40DCR3. Wherein the abscissa is the fluorescence intensity of a single CD40-positive cell, and the ordinate is the number of cells with different fluorescence intensities.

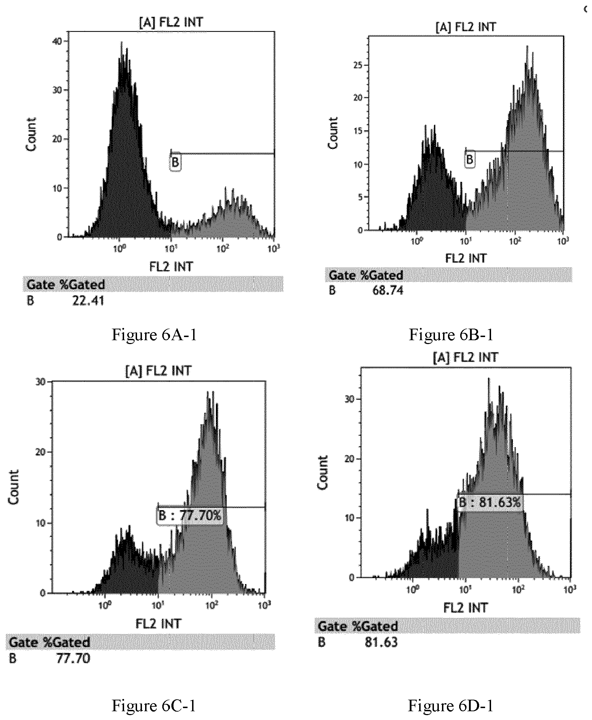

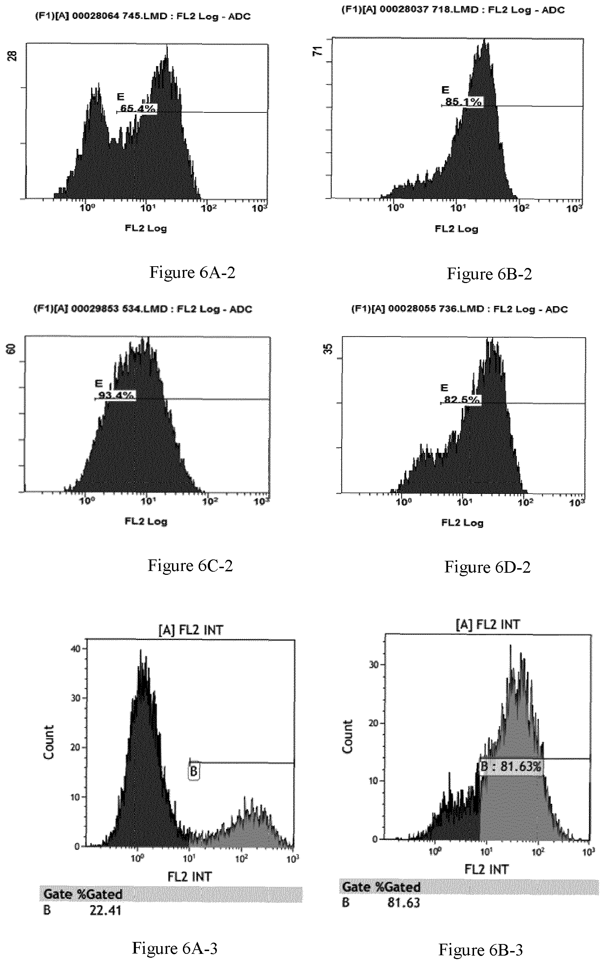

Figure 6A-1: Function of electroporation of 137DCR2 combined with meso G1 CAR, CD137 phenotype of Mock T. Wherein the abscissa is the fluorescence intensity of a single CD137-positive cell, and the ordinate is the number of cells with different fluorescence intensities.
Figure 6B-1: Function of electroporation of 137DCR2 combined with meso G1 CAR, CD137 phenotype of recombinant cell meso G1 CAR. Wherein the abscissa is the fluorescence intensity of a single CD137-positive cell, and the ordinate is the number of cells with different fluorescence intensities.

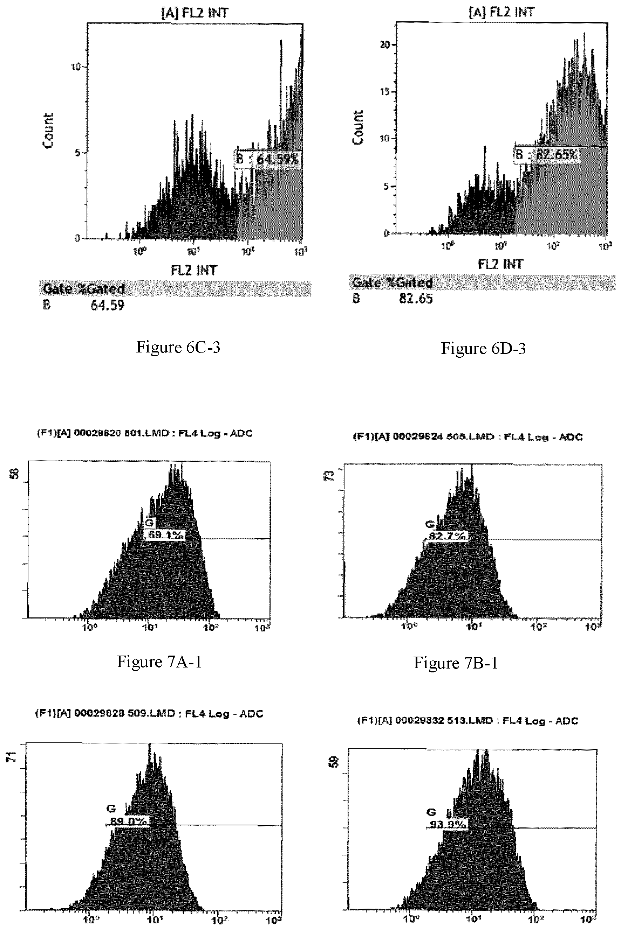

Figure 6C-1: Function of electroporation of 137DCR2 combined with meso G1 CAR, CD137 phenotype of recombinant cell meso G2 CAR. Wherein the abscissa is the fluorescence intensity of a single CD137-positive cell, and the ordinate is the number of cells with different fluorescence intensities.
Figure 6D-1: Function of electroporation of 137DCR combined with meso G1 CAR, CD137 phenotype of recombinant cell meso G1 CAR-137DCR1. Wherein the abscissa is the fluorescence intensity of a single CD137-positive cell, and the ordinate is the number of cells with different fluorescence intensities.
Figure 6A-2: Function of electroporation of 28DCR2 combined with Mucl G1 CAR, CD28 phenotype of Mock T. Wherein the abscissa is the fluorescence intensity of a single CD28-positive cell, and the ordinate is the number of cells with different fluorescence intensities.
Figure 6B-2: Function of electroporation of 28DCR2 combined with Mucl G1 CAR, CD28 phenotype of recombinant cell Mucl G1 CAR. Wherein the abscissa is the fluorescence intensity of a single CD28-positive cell, and the ordinate is the number of cells with different fluorescence intensities.
Figure 6C-2: Function of electroporation of 28DCR2 combined with Mucl G1 CAR, CD28 phenotype of recombinant cell Mucl G2 CAR. Wherein the abscissa is the fluorescence intensity of a single CD28-positive cell, and the ordinate is the number of cells with different fluorescence intensities.
Figure 6D-2: Function of electroporation of 28DCR2 combined with Mucl G1 CAR, CD28 phenotype of recombinant cell Mucl G1 CAR-28DCR1. Wherein the abscissa is the fluorescence intensity of a single CD28-positive cell, and the ordinate is the number of cells with different fluorescence intensities.
Figure 6A-3: Function of electroporation of 40DCR2 combined with EGFR G1 CAR, CD40 phenotype of Mock T. Wherein the abscissa is the fluorescence intensity of a single CD40-positive cell, and the ordinate is the number of cells with different fluorescence intensities.
Figure 6B-3: Function of electroporation of 40DCR2 combined with EGFR G1 CAR, CD40 phenotype of recombinant cell EGFR G1 CAR. Wherein the abscissa is the fluorescence intensity of a single CD40-positive cell, and the ordinate is the number of cells with different fluorescence intensities.
Figure 6C-3: Function of electroporation of 40DCR2 combined with EGFR G1 CAR, CD40 phenotype of recombinant cell EGFR G2 CAR. Wherein the abscissa is the fluorescence intensity of a single CD40-positive cell, and the ordinate is the number of cells with different fluorescence intensities.
Figure 6D-3: Function of electroporation of 40DCR2 combined with EGFR G1 CAR, CD40 phenotype of recombinant cell EGFR G1 CAR-40DCR1. Wherein the abscissa is the fluorescence intensity of a single CD40-positive cell, and the ordinate is the number of cells with different fluorescence intensities.

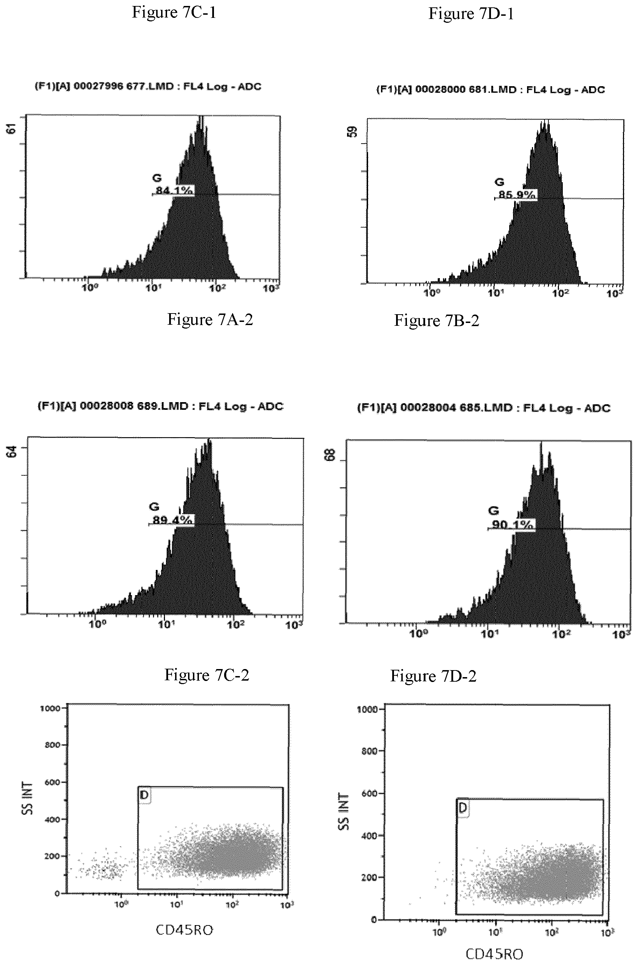

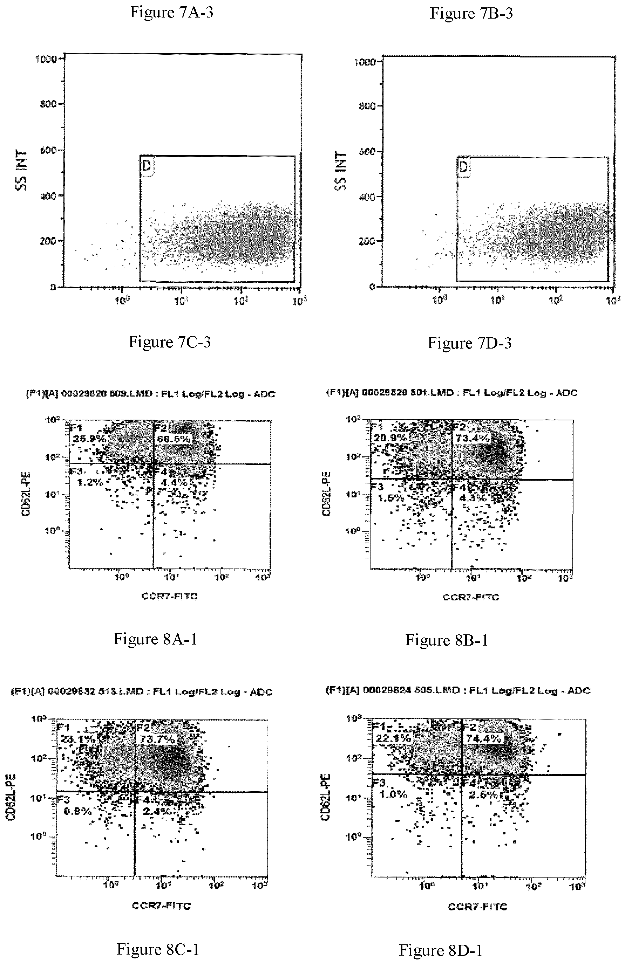

Figure 7A-1: Function of electroporation of 137DCR2 combined with meso G1 CAR, CD45RO phenotype of Mock T. Wherein the abscissa is the fluorescence intensity of a single CD45RO-positive cell, and the ordinate is the number of cells with different fluorescence intensities.
Figure 7B-1: Function of electroporation of 137DCR2 combined with meso G1 CAR, CD45RO phenotype of recombinant cell meso G1 CAR. Wherein the abscissa is the fluorescence intensity of a single CD45RO-positive cell, and the ordinate is the number of cells with different fluorescence intensities.
Figure 7C-1: Function of electroporation of 137DCR2 combined with meso G1 CAR, CD45RO phenotype of recombinant cell meso G2 CAR. Wherein the abscissa is the fluorescence intensity of a single CD45RO-positive cell, and the ordinate is the number of cells with different fluorescence intensities.
Figure 7D-1: Function of electroporation of 137DCR combined with meso G1 CAR, CD45RO phenotype of recombinant cell meso G1 CAR-137DCR1. Wherein the abscissa is the fluorescence intensity of a single CD45RO-positive cell, and the ordinate is the number of cells with different fluorescence intensities.
Figure 7A-2: Function of electroporation of 28DCR2 combined with Mucl G1 CAR, CD45RO phenotype of Mock T. Wherein the abscissa is the fluorescence intensity of a single CD45RO-positive cell, and the ordinate is the number of cells with different fluorescence intensities.
Figure 7B-2: Function of electroporation of 28DCR2 combined with Mucl G1 CAR, CD45RO phenotype of recombinant cell Mucl G1 CAR. Wherein the abscissa is the fluorescence intensity of a single CD45RO-positive cell, and the ordinate is the number of cells with different fluorescence intensities.
Figure 7C-2: Function of electroporation of 28DCR2 combined with Mucl G1 CAR, CD45RO phenotype of recombinant cell Mucl G2 CAR. Wherein the abscissa is the fluorescence intensity of a single CD45RO-positive cell, and the ordinate is the number of cells with different fluorescence intensities.
Figure 7D-2: Function of electroporation of 28DCR combined with Mucl G1 CAR, CD45RO phenotype of recombinant cell Mucl G1 CAR-28DCR1. Wherein the abscissa is the fluorescence intensity of a single CD45RO-positive cell, and the ordinate is the number of cells with different fluorescence intensities.
Figure 7A-3: Function of electroporation of 40DCR2 combined with EGFR G1 CAR, CD45RO phenotype of Mock T. Wherein the abscissa is the fluorescence intensity of a single CD45RO-positive cell, and the ordinate is the number of cells with different fluorescence intensities.
Figure 7B-3: Function of electroporation of 40DCR2 combined with EGFR G1 CAR, CD45RO phenotype of recombinant cell EGFR G1 CAR. Wherein the abscissa is the fluorescence intensity of a single CD45RO-positive cell, and the ordinate is the number of cells with different fluorescence intensities.
Figure 7C-3: Function of electroporation of 40DCR2 combined with EGFR G1 CAR, CD45RO phenotype of recombinant cell EGFR G2 CAR. Wherein the abscissa is the fluorescence intensity of a single CD45RO-positive cell, and the ordinate is the number of cells with different fluorescence intensities.
Figure 7D-3: Function of electroporation of 40DCR2 combined with EGFR G1 CAR, CD45RO phenotype of recombinant cell EGFR G1 CAR-40DCR1. Wherein the abscissa is the fluorescence intensity of a single CD45RO-positive cell, and the ordinate is the number of cells with different fluorescence intensities.

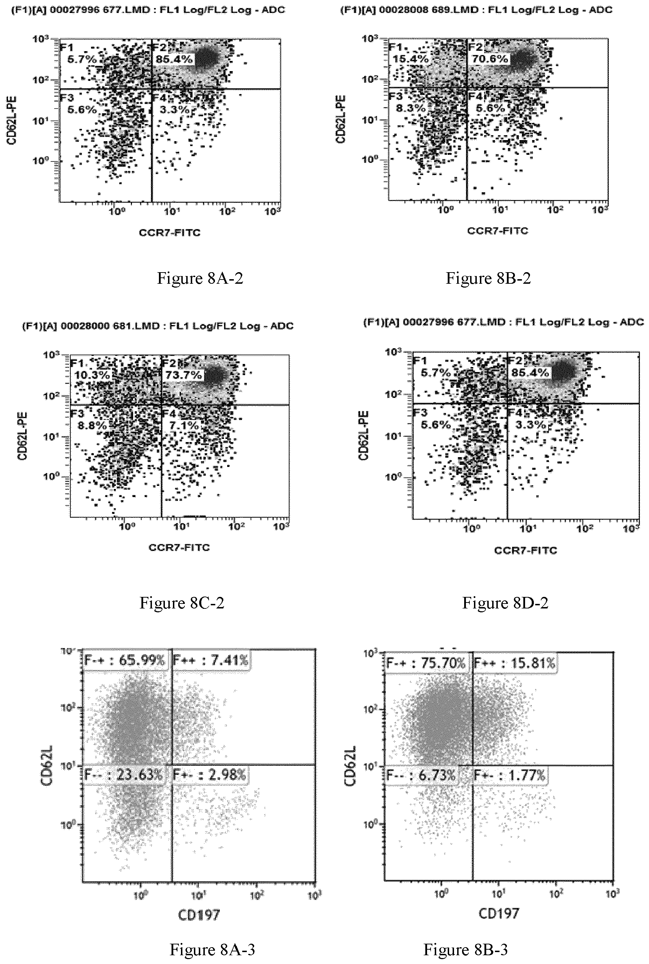

Figure 8A-1: Function of electroporation of 137DCR2 combined with meso G1 CAR, memory T phenotype of Mock T. Wherein the abscissa is the fluorescence intensity of a single CD62L-positive cell, and the ordinate is the fluorescence intensity of a single CCR7-positive cell.
Figure 8B-1: Function of electroporation of 137DCR2 combined with meso G1 CAR, memory T phenotype of recombinant cell meso G1 CAR. Wherein the abscissa is the fluorescence intensity of a single CD62L-positive cell, and the ordinate is the fluorescence intensity of a single CCR7-positive cell.

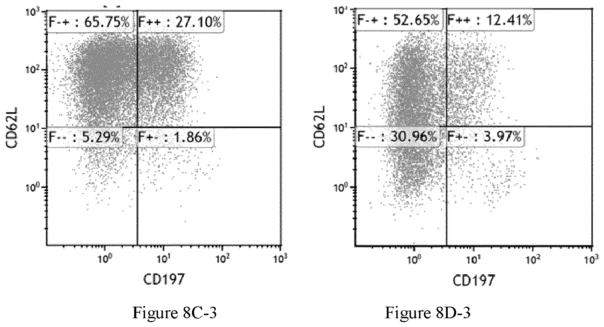

Figure 8C-1: Function of electroporation of 137DCR2 combined with meso G1 CAR, memory T phenotype of recombinant cell meso G2 CAR. Wherein the abscissa is the fluorescence intensity of a single CD62L-positive cell, and the ordinate is the fluorescence intensity of a single CCR7-positive cell.
Figure 8D-1: Function of electroporation of 137DCR combined with meso G1 CAR, memory T phenotype of recombinant cell meso G1 CAR-137DCR1. Wherein the abscissa is the fluorescence intensity of a single CD62L-positive cell, and the ordinate is the fluorescence intensity of a single CCR7-positive cell.
Figure 8A-2: Function of electroporation of 28DCR2 combined with Mucl G1 CAR, memory T phenotype of Mock T. Wherein the abscissa is the fluorescence intensity of a single CD62L-positive cell, and the ordinate is the fluorescence intensity of a single CCR7-positive cell.
Figure 8B-2: Function of electroporation of 28DCR2 combined with Mucl G1 CAR, memory T phenotype of recombinant cell Mucl G1 CAR. Wherein the abscissa is the fluorescence intensity of a single CD62L-positive cell, and the ordinate is the fluorescence intensity of a single CCR7-positive cell.
Figure 8C-2: Function of electroporation of 28DCR2 combined with Mucl G1 CAR, memory T phenotype of recombinant cell Mucl G2 CAR. Wherein the abscissa is the fluorescence intensity of a single CD62L-positive cell, and the ordinate is the fluorescence intensity of a single CCR7-positive cell.
Figure 8D-2: Function of electroporation of 28DCR combined with Mucl G1 CAR, memory T phenotype of recombinant cell Mucl G1 CAR-28DCR1. Wherein the abscissa is the fluorescence intensity of a single CD62L-positive cell, and the ordinate is the fluorescence intensity of a single CCR7-positive cell.
Figure 8A-3: Function of electroporation of 40DCR2 combined with EGFR G1 CAR, memory T phenotype of Mock T. Wherein the abscissa is the fluorescence intensity of a single CD62L-positive cell, and the ordinate is the fluorescence intensity of a single CCR7-positive cell.
Figure 8B-3: Function of electroporation of 40DCR2 combined with EGFR G1 CAR, memory T phenotype of recombinant cell EGFR G1 CAR. Wherein the abscissa is the fluorescence intensity of a single CD62L-positive cell, and the ordinate is the fluorescence intensity of a single CCR7-positive cell.
Figure 8C-3: Function of electroporation of 40DCR2 combined with EGFR G1 CAR, memory T phenotype of recombinant cell EGFR G2 CAR. Wherein the abscissa is the fluorescence intensity of a single CD62L-positive cell, and the ordinate is the fluorescence intensity of a single CCR7-positive cell.
Figure 8D-3: Function of electroporation of 40DCR combined with EGFR G1 CAR, memory T phenotype of recombinant cell EGFR G1 CAR-40DCR1. Wherein the abscissa is the fluorescence intensity of a single CD62L-positive cell, and the ordinate is the fluorescence intensity of a single CCR7-positive cell.

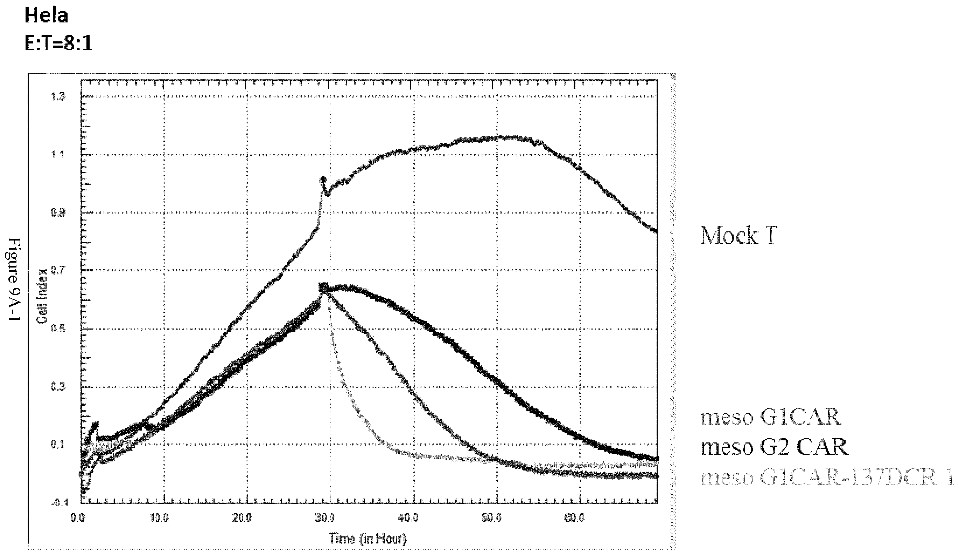

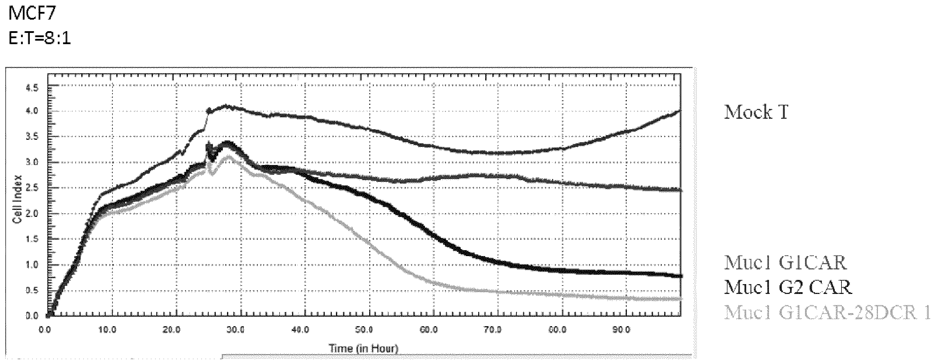

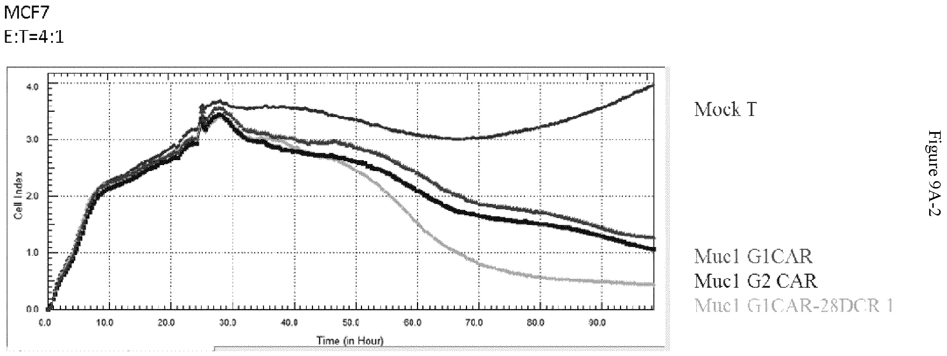

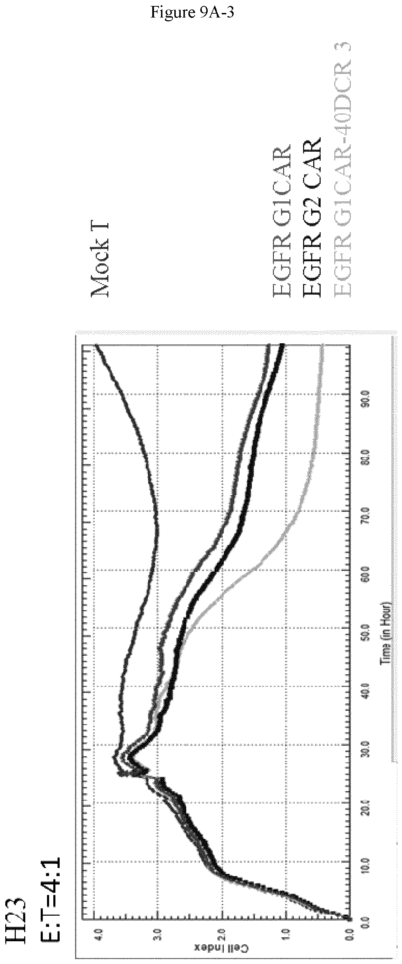

Figure 9A-1: Dual-activating chimeric antigen receptor mesothelin CAR-T cells kill Hela tumor cell lines *in vitro* with an effector target ratio of 8:1.

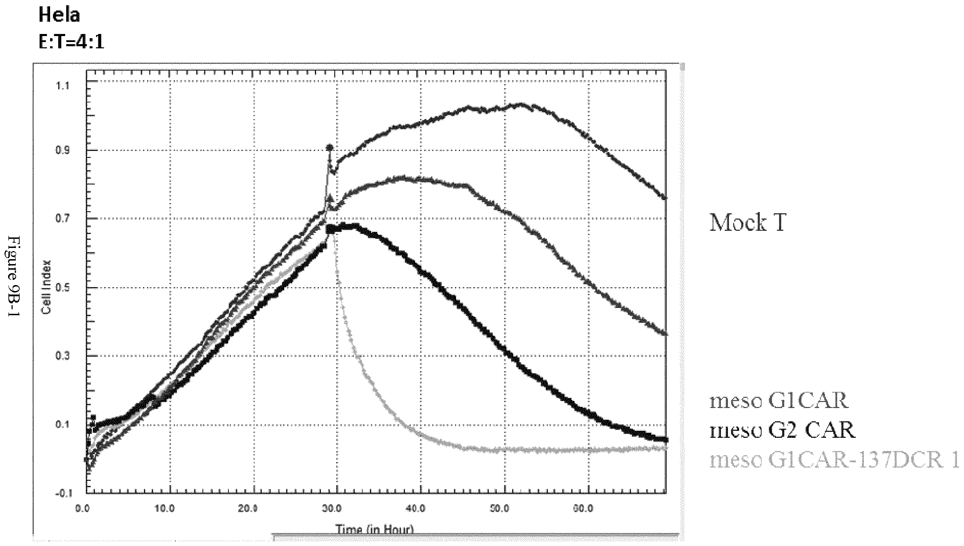

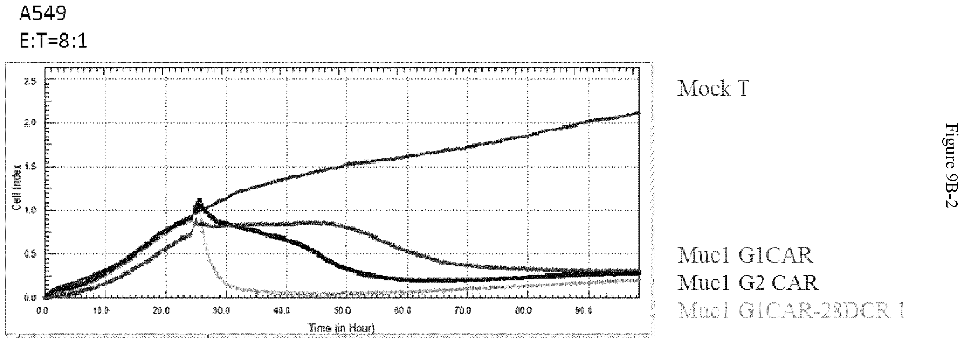

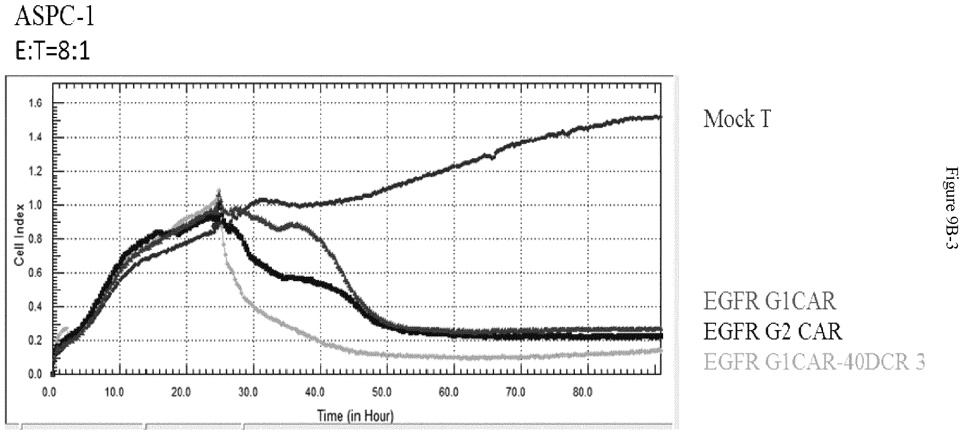

Figure 9B-1: Dual-activating chimeric antigen receptor mesothelin CAR-T cells kill Hela tumor cell lines *in vitro* with an effector target ratio of 4:1.

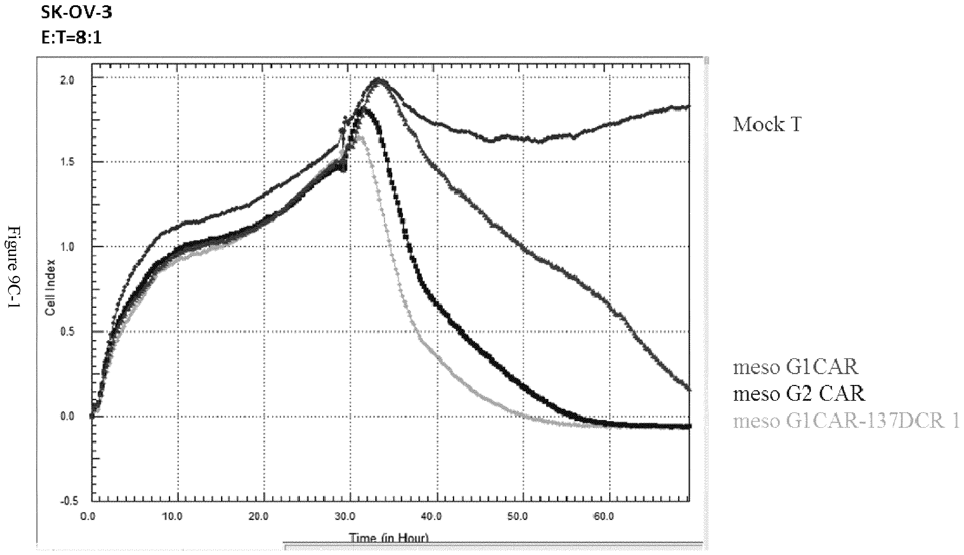

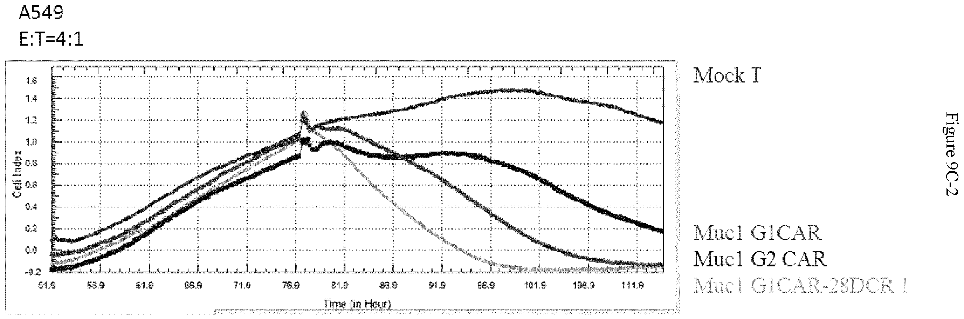

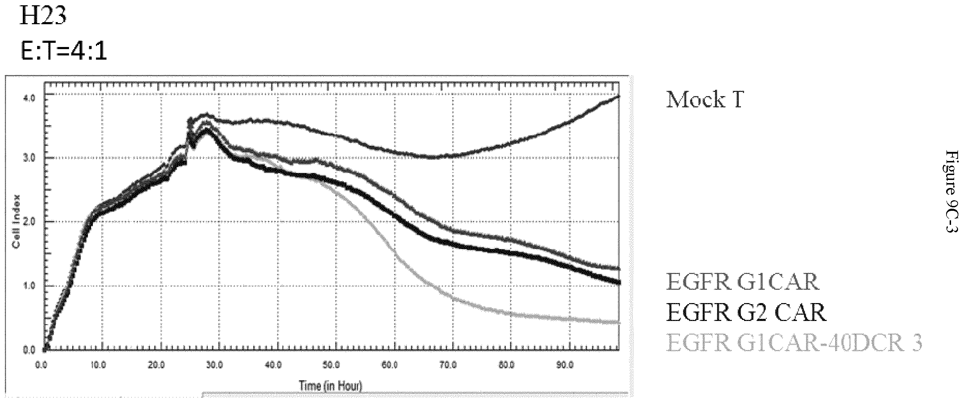

Figure 9C-1: Dual-activating chimeric antigen receptor mesothelin CAR-T cells kill SK-OV-3 tumor cell lines *in vitro* with an effector target ratio of 8:1.

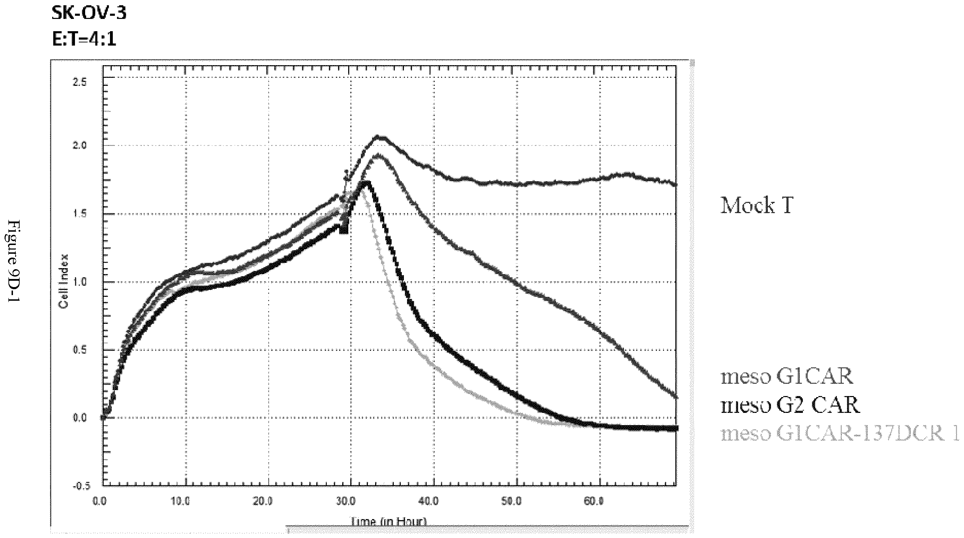

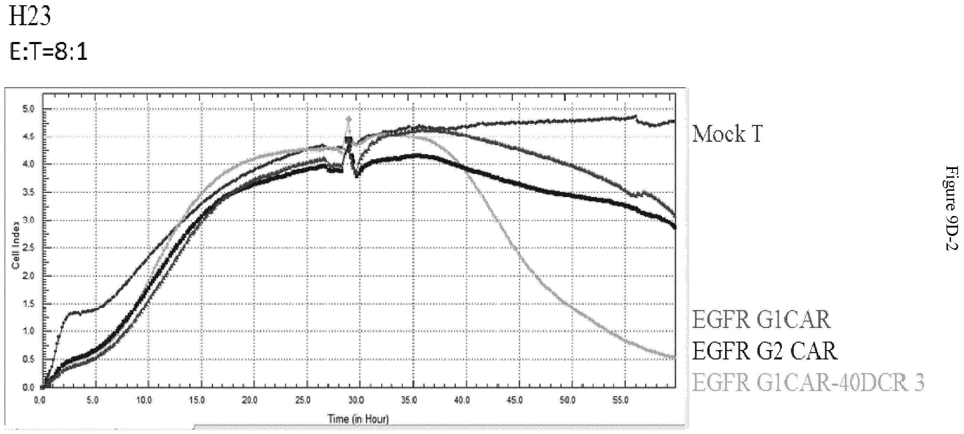

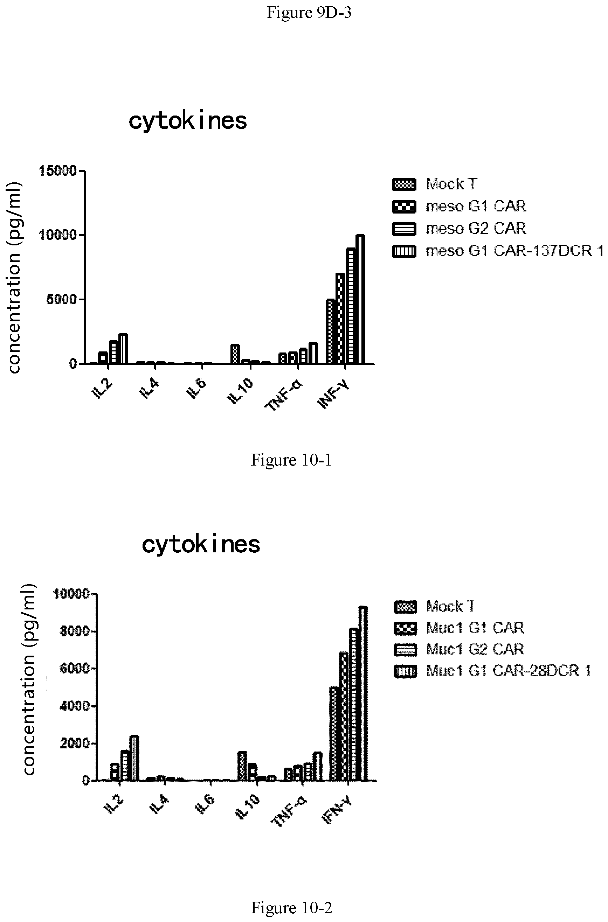

Figure 9D-1: Dual-activating chimeric antigen receptor mesothelin CAR-T cells kill SK-OV-3 tumor cell lines *in vitro* with an effector target ratio of 4:1.
Figure 9A-2: Dual-activating chimeric antigen receptor Mucl CAR-T cells kill MCF7 tumor cell lines *in vitro* with an effector target ratio of 8:1.
Figure 9B-2: Dual-activating chimeric antigen receptor Mucl CAR-T cells kill MCF7 tumor cell lines *in vitro* with an effector target ratio of 4:1.
Figure 9C-2: Dual-activating chimeric antigen receptor Mucl CAR-T cells kill A549 tumor cell lines *in vitro* with an effector target ratio of 8:1.
Figure 9D-2: Dual-activating chimeric antigen receptor Mucl CAR-T cells kill A549 tumor cell lines *in vitro* with an effector target ratio of 4:1.
Figure 9A-3: Dual-activating chimeric antigen receptor EGFR CAR-T cells kill H23 tumor cell lines *in vitro* with an effector target ratio of 8:1.
Figure 9B-3: Dual-activating chimeric antigen receptor EGFR CAR-T cells kill H23 tumor cell lines *in vitro* with an effector target ratio of 4:1.
Figure 9C-3: Dual-activating chimeric antigen receptor EGFR CAR-T cells kill ASPC-1 tumor cell lines *in vitro* with an effector target ratio of 8:1.
Figure 9D-3: Dual-activating chimeric antigen receptor EGFR CAR-T cells kill ASPC-1 tumor cell lines *in vitro* with an effector target ratio of 4:1.
Figure 10-1: Changes in cytokines IL-2, IL-4, IL-6, IL-10, TNF-α and IFN-γ of dual-activating chimeric antigen receptor mesothelin CAR-T cells under the stimulation with mesothelin antigen.
Figure 10-2: Changes in cytokines IL-2, IL-4, IL-6, IL-10, TNF-α and IFN-γ of dual-activating chimeric antigen receptor Mucl CAR-T cells under the stimulation with Mucl antigen.

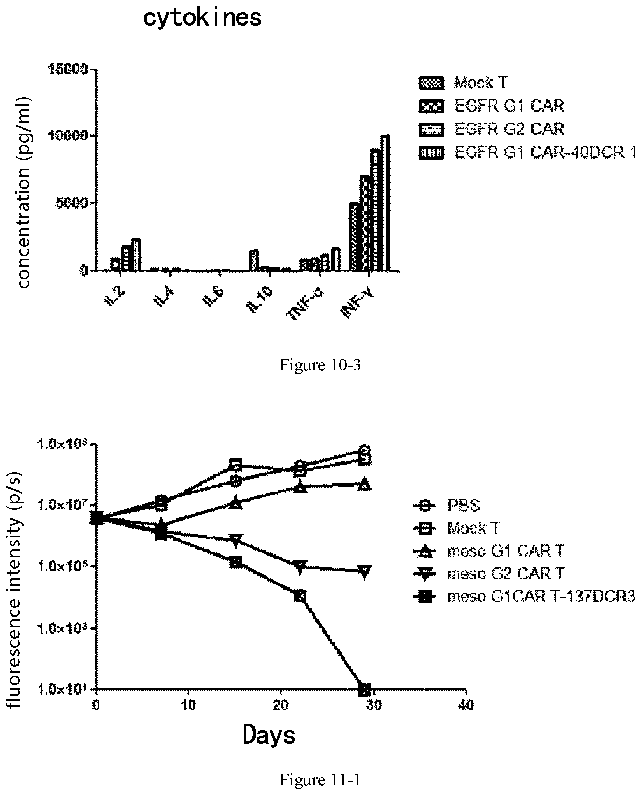

Figure 10-3: Changes in cytokines IL-2, IL-4, IL-6, IL-10, TNF-α and IFN-γ of dual-activating chimeric antigen receptor EGFR CAR-T cells under the stimulation with EGFR antigen.
Figure 11-1: The therapeutic effect of dual-activating chimeric antigen receptor mesothelin CAR-T cells on an ovarian cancer xenograft mouse model.

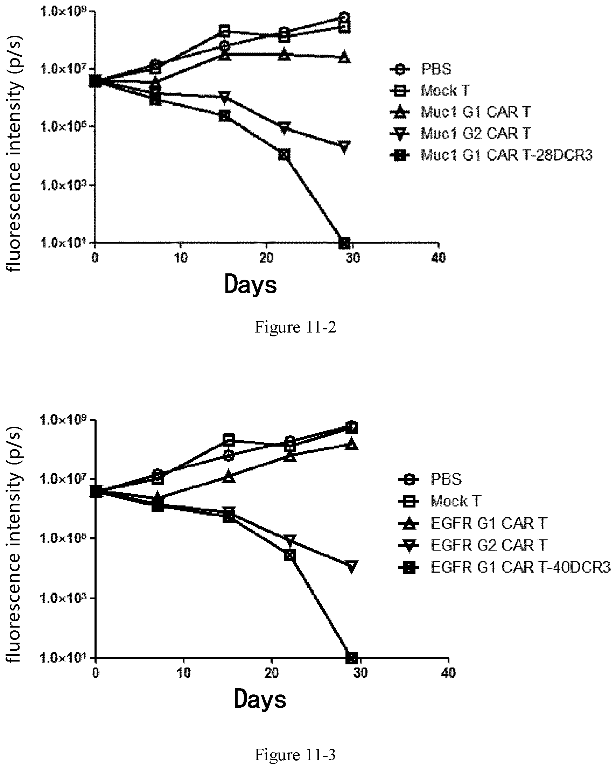

Figure 11-2: The therapeutic effect of dual-activating chimeric antigen receptor Mucl CAR-T cells on an ovarian cancer xenograft mouse model.
Figure 11-3: The therapeutic effect of dual-activating chimeric antigen receptor EGFR CAR-T cells on an ovarian cancer xenograft mouse model.

Some sequences involved in the present invention are as follows:
1. Amino acid sequence of CD8 signal peptide (SEQ ID NO:1)
   MGNSCYNIVATLLLVLNFERTRS
2. Amino acid sequence of CD137 extracellular agonistic single-chain antibody (SEQ ID NO: 2) 
QVQLQQWGAGLLKPSETLSLTCAVYGGSFSGYYWSWIRQSPEKGLEWIGEINHGGY
VTYNPSLESRVTISVDTSKNQFSLKLSSVTAADTAVYYCARDYGPGNYDWYFDLWGRGT
LVTVSSGGGGSGGGGSGGGGSEIVLTQSPATLSLSPGERATLSCRASQSVSSYLAWYQQK
 
PGQAPRLLIYDASNRATGIPARFSGSGSGTDFTLTISSLEPEDFAVYYCQQRSNWPPALTFG
GGTKVEIKE
3. Amino acid sequence of CD8α hinge region (SEQ ID NO:3)
   SKYGPPCPPCPIEVMYPPPYLDNEKSNGTIIHVKGKHLCPSPLFPGPSK
4. Amino acid sequence of CD28 transmembrane region (SEQ ID NO:4)
   PFWVLVVVGGVLACYSLLVTVAFIIFWV
5. Amino acid sequence of CD28 intracellular costimulatory signal domain (SEQ ID NO: 5)
   RSKRSRLLHSDYMNMTPRRPGPTRKHYQPYAPPRDFAAYRS
6. Amino acid sequence of CD137 intracellular costimulatory signal domain (SEQ ID NO: 6)
   KRGRKKLLYIFKQPFMRPVQTTQEEDGCSCRFPEEEEGGCEL
7. Amino acid sequence of 137DCR1 (including signal peptide) (SEQ ID NO:7) 
MGNSCYNIVATLLLVLNFERTRSQVQLQQWGAGLLKPSETLSLTCAVYGGSFSGYY
WSWIRQSPEKGLEWIGEINHGGYVTYNPSLESRVTISVDTSKNQFSLKLSSVTAADTAVY
YCARDYGPGNYDWYFDLWGRGTLVTVSSGGGGSGGGGSGGGGSEIVLTQSPATLSLSPG
ERATLSCRASQSVSSYLAWYQQKPGQAPRLLIYDASNRATGIPARFSGSGSGTDFTLTISSL
EPEDFAVYYCQQRSNWPPALTFGGGTKVEIKESKYGPPCPPCPIEVMYPPPYLDNEKSNGT
IIHVKGKHLCPSPLFPGPSKPFWVLVVVGGVLACYSLLVTVAFIIFWVRSKRSRLLHSDYM
NMTPRRPGPTRKHYQPYAPPRDFAAYRS
8. Amino acid sequence of 137DCR2 (including signal peptide) (SEQ ID NO:8) 
MGNSCYNIVATLLLVLNFERTRSQVQLQQWGAGLLKPSETLSLTCAVYGGSFSGYY
WSWIRQSPEKGLEWIGEINHGGYVTYNPSLESRVTISVDTSKNQFSLKLSSVTAADTAVY
YCARDYGPGNYDWYFDLWGRGTLVTVSSGGGGSGGGGSGGGGSEIVLTQSPATLSLSPG
ERATLSCRASQSVSSYLAWYQQKPGQAPRLLIYDASNRATGIPARFSGSGSGTDFTLTISSL
EPEDFAVYYCQQRSNWPPALTFGGGTKVEIKESKYGPPCPPCPIEVMYPPPYLDNEKSNGT
IIHVKGKHLCPSPLFPGPSKPFWVLVVVGGVLACYSLLVTVAFIIFWVKRGRKKLLYIFKQ
PFMRPVQTTQEEDGCSCRFPEEEEGGCEL
9. Amino acid sequence of 137DCR3 (including signal peptide) (SEQ ID NO:9) MGNSCYNIVATLLLVLNFERTRSQVQLQQWGAGLLKPSETLSLTCAVYGGSFSGYY 
WSWIRQSPEKGLEWIGEINHGGYVTYNPSLESRVTISVDTSKNQFSLKLSSVTAADTAVY
YCARDYGPGNYDWYFDLWGRGTLVTVSSGGGGSGGGGSGGGGSEIVLTQSPATLSLSPG
ERATLSCRASQSVSSYLAWYQQKPGQAPRLLIYDASNRATGIPARFSGSGSGTDFTLTISSL
EPEDFAVYYCQQRSNWPPALTFGGGTKVEIKESKYGPPCPPCPIEVMYPPPYLDNEKSNGT
IIHVKGKHLCPSPLFPGPSKPFWVLVVVGGVLACYSLLVTVAFIIFWVRSKRSRLLHSDYM
NMTPRRPGPTRKHYQPYAPPRDFAAYRSKRGRKKLLYIFKQPFMRPVQTTQEEDGCSCRF
PEEEEGGCEL
10. Amino acid sequence of 137DCR4 (including signal peptide) (SEQ ID NO:10) 
MGNSCYNIVATLLLVLNFERTRSQVQLQQWGAGLLKPSETLSLTCAVYGGSFSGYY
WSWIRQSPEKGLEWIGEINHGGYVTYNPSLESRVTISVDTSKNQFSLKLSSVTAADTAVY
YCARDYGPGNYDWYFDLWGRGTLVTVSSGGGGSGGGGSGGGGSEIVLTQSPATLSLSPG
ERATLSCRASQSVSSYLAWYQQKPGQAPRLLIYDASNRATGIPARFSGSGSGTDFTLTISSL
EPEDFAVYYCQQRSNWPPALTFGGGTKVEIKESKYGPPCPPCPIEVMYPPPYLDNEKSNGT
IIHVKGKHLCPSPLFPGPSKPFWVLVVVGGVLACYSLLVTVAFIIFWVKRGRKKLLYIFKQ
PFMRPVQTTQEEDGCSCRFPEEEEGGCELRSKRSRLLHSDYMNMTPRRPGPTRKHYQPYA
PPRDFAAYRS
11. Amino acid sequence of 137DCR1 (without signal peptide) (SEQ ID NO:11) 
QVQLQQWGAGLLKPSETLSLTCAVYGGSFSGYYWSWIRQSPEKGLEWIGEINHGGY
VTYNPSLESRVTISVDTSKNQFSLKLSSVTAADTAVYYCARDYGPGNYDWYFDLWGRGT
LVTVSSGGGGSGGGGSGGGGSEIVLTQSPATLSLSPGERATLSCRASQSVSSYLAWYQQK
PGQAPRLLIYDASNRATGIPARFSGSGSGTDFTLTISSLEPEDFAVYYCQQRSNWPPALTFG
GGTKVEIKESKYGPPCPPCPIEVMYPPPYLDNEKSNGTIIHVKGKHLCPSPLFPGPSKPFWV
LVVVGGVLACYSLLVTVAFIIFWVRSKRSRLLHSDYMNMTPRRPGPTRKHYQPYAPPRDF
AAYRS
12. Amino acid sequence of 137DCR2 (without signal peptide) (SEQ ID NO:12) 
QVQLQQWGAGLLKPSETLSLTCAVYGGSFSGYYWSWIRQSPEKGLEWIGEINHGGY
VTYNPSLESRVTISVDTSKNQFSLKLSSVTAADTAVYYCARDYGPGNYDWYFDLWGRGT
LVTVSSGGGGSGGGGSGGGGSEIVLTQSPATLSLSPGERATLSCRASQSVSSYLAWYQQK
PGQAPRLLIYDASNRATGIPARFSGSGSGTDFTLTISSLEPEDFAVYYCQQRSNWPPALTFG
GGTKVEIKESKYGPPCPPCPIEVMYPPPYLDNEKSNGTIIHVKGKHLCPSPLFPGPSKPFWV
LVVVGGVLACYSLLVTVAFIIFWVKRGRKKLLYIFKQPFMRPVQTTQEEDGCSCRFPEEEE
GGCEL
13. Amino acid sequence of 137DCR3 (without signal peptide) (SEQ ID NO:13) 
QVQLQQWGAGLLKPSETLSLTCAVYGGSFSGYYWSWIRQSPEKGLEWIGEINHGGY
VTYNPSLESRVTISVDTSKNQFSLKLSSVTAADTAVYYCARDYGPGNYDWYFDLWGRGT
LVTVSSGGGGSGGGGSGGGGSEIVLTQSPATLSLSPGERATLSCRASQSVSSYLAWYQQK
PGQAPRLLIYDASNRATGIPARFSGSGSGTDFTLTISSLEPEDFAVYYCQQRSNWPPALTFG
GGTKVEIKESKYGPPCPPCPIEVMYPPPYLDNEKSNGTIIHVKGKHLCPSPLFPGPSKPFWV
LVVVGGVLACYSLLVTVAFIIFWVRSKRSRLLHSDYMNMTPRRPGPTRKHYQPYAPPRDF
AAYRSKRGRKKLLYIFKQPFMRPVQTTQEEDGCSCRFPEEEEGGCEL
14. Amino acid sequence of 137DCR4 (without signal peptide) (SEQ ID NO:14) 
QVQLQQWGAGLLKPSETLSLTCAVYGGSFSGYYWSWIRQSPEKGLEWIGEINHGGY
VTYNPSLESRVTISVDTSKNQFSLKLSSVTAADTAVYYCARDYGPGNYDWYFDLWGRGT
LVTVSSGGGGSGGGGSGGGGSEIVLTQSPATLSLSPGERATLSCRASQSVSSYLAWYQQK
PGQAPRLLIYDASNRATGIPARFSGSGSGTDFTLTISSLEPEDFAVYYCQQRSNWPPALTFG
GGTKVEIKESKYGPPCPPCPIEVMYPPPYLDNEKSNGTIIHVKGKHLCPSPLFPGPSKPFWV
LVVVGGVLACYSLLVTVAFIIFWVKRGRKKLLYIFKQPFMRPVQTTQEEDGCSCRFPEEEE
GGCELRSKRSRLLHSDYMNMTPRRPGPTRKHYQPYAPPRDFAAYRS
15. Nucleic acid sequence of 137DCR1 (including signal peptide) (SEQ ID NO:15) 
ATGGGAAACAGCTGTTACAACATAGTAGCCACTCTGTTGCTGGTCCTCAACTTTG
AGAGGACAAGATCACAGGTGCAACTACAGCAGTGGGGCGCAGGACTGTTGAAGCCTT
CGGAGACCCTGTCCCTCACCTGCGCTGTCTATGGTGGGTCCTTCAGTGGTTACTACTG
GAGCTGGATACGCCAGTCCCCAGAGAAGGGGCTGGAGTGGATTGGGGAAATCAATCA
TGGTGGATACGTCACCTACAATCCGTCCCTCGAGAGTCGAGTCACCATATCAGTAGAC
ACGTCCAAGAACCAGTTCTCCCTGAAGCTGAGCTCTGTGACCGCCGCGGACACGGCT
GTATATTACTGTGCGAGGGACTATGGTCCGGGGAATTATGACTGGTACTTCGATCTCT
GGGGCCGTGGCACCCTGGTCACTGTCTCCTCAGGTGGAGGCGGTTCAGGCGGAGGTG
GCAGCGGCGGTGGCGGGTCGGAAATTGTGTTGACACAGTCTCCAGCCACCCTGTCTTT
GTCTCCAGGGGAAAGAGCCACCCTCTCCTGCAGGGCCAGTCAGAGTGTTAGCAGCTA
CTTAGCCTGGTACCAACAGAAACCTGGCCAGGCTCCCAGGCTCCTCATCTATGATGCA
TCCAACAGGGCCACTGGCATCCCAGCCAGGTTCAGTGGCAGTGGGTCTGGGACAGAC
TTCACTCTCACCATCAGCAGCCTAGAGCCTGAAGATTTTGCAGTTTATTACTGTCAGC
AGCGTAGCAACTGGCCTCCGGCGCTCACTTTCGGCGGAGGGACCAAGGTGGAGATCA
AAGAGTCCAAATATGGTCCCCCATGCCCACCATGCCCAATTGAAGTTATGTATCCTCC
 
TCCTTACCTAGACAATGAGAAGAGCAATGGAACCATTATCCATGTGAAAGGGAAACA
CCTTTGTCCAAGTCCCCTATTTCCCGGACCTTCTAAGCCCTTTTGGGTGCTGGTGGTGG
TTGGTGGAGTCCTGGCTTGCTATAGCTTGCTAGTAACAGTGGCCTTTATTATTTTCTGG
GTGAGGAGTAAGAGGAGCAGGCTCCTGCACAGTGACTACATGAACATGACTCCCCGC
CGCCCCGGGCCCACCCGCAAGCATTACCAGCCCTATGCCCCACCACGCGACTTCGCA
GCCTATCGCTCCTGATAA
16. Nucleic acid sequence of 137DCR2 (including signal peptide) (SEQ ID NO:16) 
ATGGGAAACAGCTGTTACAACATAGTAGCCACTCTGTTGCTGGTCCTCAACTTTG
AGAGGACAAGATCACAGGTGCAACTACAGCAGTGGGGCGCAGGACTGTTGAAGCCTT
CGGAGACCCTGTCCCTCACCTGCGCTGTCTATGGTGGGTCCTTCAGTGGTTACTACTG
GAGCTGGATACGCCAGTCCCCAGAGAAGGGGCTGGAGTGGATTGGGGAAATCAATCA
TGGTGGATACGTCACCTACAATCCGTCCCTCGAGAGTCGAGTCACCATATCAGTAGAC
ACGTCCAAGAACCAGTTCTCCCTGAAGCTGAGCTCTGTGACCGCCGCGGACACGGCT
GTATATTACTGTGCGAGGGACTATGGTCCGGGGAATTATGACTGGTACTTCGATCTCT
GGGGCCGTGGCACCCTGGTCACTGTCTCCTCAGGTGGAGGCGGTTCAGGCGGAGGTG
GCAGCGGCGGTGGCGGGTCGGAAATTGTGTTGACACAGTCTCCAGCCACCCTGTCTTT
GTCTCCAGGGGAAAGAGCCACCCTCTCCTGCAGGGCCAGTCAGAGTGTTAGCAGCTA
CTTAGCCTGGTACCAACAGAAACCTGGCCAGGCTCCCAGGCTCCTCATCTATGATGCA
TCCAACAGGGCCACTGGCATCCCAGCCAGGTTCAGTGGCAGTGGGTCTGGGACAGAC
TTCACTCTCACCATCAGCAGCCTAGAGCCTGAAGATTTTGCAGTTTATTACTGTCAGC
AGCGTAGCAACTGGCCTCCGGCGCTCACTTTCGGCGGAGGGACCAAGGTGGAGATCA
AAGAGTCCAAATATGGTCCCCCATGCCCACCATGCCCAATTGAAGTTATGTATCCTCC
TCCTTACCTAGACAATGAGAAGAGCAATGGAACCATTATCCATGTGAAAGGGAAACA
CCTTTGTCCAAGTCCCCTATTTCCCGGACCTTCTAAGCCCTTTTGGGTGCTGGTGGTG
TTGGTGGAGTCCTGGCTTGCTATAGCTTGCTAGTAACAGTGGCCTTTATTATTTTCTGG
GTGAAACGGGGCAGAAAGAAGCTCCTGTATATATTCAAACAACCATTTATGAGACCA
GTACAAACTACTCAAGAGGAAGATGGCTGTAGCTGCCGATTTCCAGAAGAAGAAGAA
GGAGGATGTGAACTGTGATAA
17. Nucleic acid sequence of 137DCR3 (including signal peptide) (SEQ ID NO:17) 
ATGGGAAACAGCTGTTACAACATAGTAGCCACTCTGTTGCTGGTCCTCAACTTTG
AGAGGACAAGATCACAGGTGCAACTACAGCAGTGGGGCGCAGGACTGTTGAAGCCTT
CGGAGACCCTGTCCCTCACCTGCGCTGTCTATGGTGGGTCCTTCAGTGGTTACTACTG
GAGCTGGATACGCCAGTCCCCAGAGAAGGGGCTGGAGTGGATTGGGGAAATCAATCA
 
TGGTGGATACGTCACCTACAATCCGTCCCTCGAGAGTCGAGTCACCATATCAGTAGAC
ACGTCCAAGAACCAGTTCTCCCTGAAGCTGAGCTCTGTGACCGCCGCGGACACGGCT
GTATATTACTGTGCGAGGGACTATGGTCCGGGGAATTATGACTGGTACTTCGATCTCT
GGGGCCGTGGCACCCTGGTCACTGTCTCCTCAGGTGGAGGCGGTTCAGGCGGAGGTG
GCAGCGGCGGTGGCGGGTCGGAAATTGTGTTGACACAGTCTCCAGCCACCCTGTCTTT
GTCTCCAGGGGAAAGAGCCACCCTCTCCTGCAGGGCCAGTCAGAGTGTTAGCAGCTA
CTTAGCCTGGTACCAACAGAAACCTGGCCAGGCTCCCAGGCTCCTCATCTATGATGCA
TCCAACAGGGCCACTGGCATCCCAGCCAGGTTCAGTGGCAGTGGGTCTGGGACAGAC
TTCACTCTCACCATCAGCAGCCTAGAGCCTGAAGATTTTGCAGTTTATTACTGTCAGC
AGCGTAGCAACTGGCCTCCGGCGCTCACTTTCGGCGGAGGGACCAAGGTGGAGATCA
AAGAGTCCAAATATGGTCCCCCATGCCCACCATGCCCAATTGAAGTTATGTATCCTCC
TCCTTACCTAGACAATGAGAAGAGCAATGGAACCATTATCCATGTGAAAGGGAAACA
CCTTTGTCCAAGTCCCCTATTTCCCGGACCTTCTAAGCCCTTTTGGGTGCTGGTGGTGG
TTGGTGGAGTCCTGGCTTGCTATAGCTTGCTAGTAACAGTGGCCTTTATTATTTTCTGG
GTGAGGAGTAAGAGGAGCAGGCTCCTGCACAGTGACTACATGAACATGACTCCCCGC
CGCCCCGGGCCCACCCGCAAGCATTACCAGCCCTATGCCCCACCACGCGACTTCGCA
GCCTATCGCTCCAAACGGGGCAGAAAGAAGCTCCTGTATATATTCAAACAACCATTT
ATGAGACCAGTACAAACTACTCAAGAGGAAGATGGCTGTAGCTGCCGATTTCCAGAA
GAAGAAGAAGGAGGATGTGAACTGTGATAA
18. Nucleic acid sequence of 137DCR4 (including signal peptide) (SEQ ID NO:18) 
ATGGGAAACAGCTGTTACAACATAGTAGCCACTCTGTTGCTGGTCCTCAACTTTG
AGAGGACAAGATCACAGGTGCAACTACAGCAGTGGGGCGCAGGACTGTTGAAGCCTT
CGGAGACCCTGTCCCTCACCTGCGCTGTCTATGGTGGGTCCTTCAGTGGTTACTACTG
GAGCTGGATACGCCAGTCCCCAGAGAAGGGGCTGGAGTGGATTGGGGAAATCAATCA
TGGTGGATACGTCACCTACAATCCGTCCCTCGAGAGTCGAGTCACCATATCAGTAGAC
ACGTCCAAGAACCAGTTCTCCCTGAAGCTGAGCTCTGTGACCGCCGCGGACACGGCT
GTATATTACTGTGCGAGGGACTATGGTCCGGGGAATTATGACTGGTACTTCGATCTCT
GGGGCCGTGGCACCCTGGTCACTGTCTCCTCAGGTGGAGGCGGTTCAGGCGGAGGTG
GCAGCGGCGGTGGCGGGTCGGAAATTGTGTTGACACAGTCTCCAGCCACCCTGTCTTT
GTCTCCAGGGGAAAGAGCCACCCTCTCCTGCAGGGCCAGTCAGAGTGTTAGCAGCTA
CTTAGCCTGGTACCAACAGAAACCTGGCCAGGCTCCCAGGCTCCTCATCTATGATGCA
TCCAACAGGGCCACTGGCATCCCAGCCAGGTTCAGTGGCAGTGGGTCTGGGACAGAC
TTCACTCTCACCATCAGCAGCCTAGAGCCTGAAGATTTTGCAGTTTATTACTGTCAGC
AGCGTAGCAACTGGCCTCCGGCGCTCACTTTCGGCGGAGGGACCAAGGTGGAGATCA
 
AAGAGTCCAAATATGGTCCCCCATGCCCACCATGCCCAATTGAAGTTATGTATCCTCC
TCCTTACCTAGACAATGAGAAGAGCAATGGAACCATTATCCATGTGAAAGGAAACA
CCTTTGTCCAAGTCCCCTATTTCCCGGACCTTCTAAGCCCTTTTGGGTGCTGGTGGTGG
TTGGTGGAGTCCTGGCTTGCTATAGCTTGCTAGTAACAGTGGCCTTTATTATTTTCTGG
GTGAAACGGGGCAGAAAGAAGCTCCTGTATATATTCAAACAACCATTTATGAGACCA
GTACAAACTACTCAAGAGGAAGATGGCTGTAGCTGCCGATTTCCAGAAGAAGAAGAA
GGAGGATGTGAACTGAGGAGTAAGAGGAGCAGGCTCCTGCACAGTGACTACATGAA
CATGACTCCCCGCCGCCCCGGGCCCACCCGCAAGCATTACCAGCCCTATGCCCCACCA
CGCGACTTCGCAGCCTATCGCTCCTGATAA
19. Nucleic acid sequence of 137DCR1 (without signal peptide) (SEQ ID NO:19) 
CAGGTGCAACTACAGCAGTGGGGCGCAGGACTGTTGAAGCCTTCGGAGACCCTG
TCCCTCACCTGCGCTGTCTATGGTGGGTCCTTCAGTGGTTACTACTGGAGCTGGATAC
GCCAGTCCCCAGAGAAGGGGCTGGAGTGGATTGGGGAAATCAATCATGGTGGATACG
TCACCTACAATCCGTCCCTCGAGAGTCGAGTCACCATATCAGTAGACACGTCCAAGA
ACCAGTTCTCCCTGAAGCTGAGCTCTGTGACCGCCGCGGACACGGCTGTATATTACTG
TGCGAGGGACTATGGTCCGGGGAATTATGACTGGTACTTCGATCTCTGGGGCCGTGGC
ACCCTGGTCACTGTCTCCTCAGGTGGAGGCGGTTCAGGCGGAGGTGGCAGCGGCGGT
GGCGGGTCGGAAATTGTGTTGACACAGTCTCCAGCCACCCTGTCTTTGTCTCCAGGGG
AAAGAGCCACCCTCTCCTGCAGGGCCAGTCAGAGTGTTAGCAGCTACTTAGCCTGGT
ACCAACAGAAACCTGGCCAGGCTCCCAGGCTCCTCATCTATGATGCATCCAACAGGG
CCACTGGCATCCCAGCCAGGTTCAGTGGCAGTGGGTCTGGGACAGACTTCACTCTCAC
CATCAGCAGCCTAGAGCCTGAAGATTTTGCAGTTTATTACTGTCAGCAGCGTAGCAAC
TGGCCTCCGGCGCTCACTTTCGGCGGAGGGACCAAGGTGGAGATCAAAGAGTCCAAA
TATGGTCCCCCATGCCCACCATGCCCAATTGAAGTTATGTATCCTCCTCCTTACCTAGA
CAATGAGAAGAGCAATGGAACCATTATCCATGTGAAAGGGAAACACCTTTGTCCAAG
TCCCCTATTTCCCGGACCTTCTAAGCCCTTTTGGGTGCTGGTGGTGGTTGGTGGAGTCC
TGGCTTGCTATAGCTTGCTAGTAACAGTGGCCTTTATTATTTTCTGGGTGAGGAGTAA
GAGGAGCAGGCTCCTGCACAGTGACTACATGAACATGACTCCCCGCCGCCCCGGGCC
CACCCGCAAGCATTACCAGCCCTATGCCCCACCACGCGACTTCGCAGCCTATCGCTCC
TGATAA
20. Nucleic acid sequence of 137DCR2 (without signal peptide) (SEQ ID NO:20) 
CAGGTGCAACTACAGCAGTGGGGCGCAGGACTGTTGAAGCCTTCGGAGACCCTG
TCCCTCACCTGCGCTGTCTATGGTGGGTCCTTCAGTGGTTACTACTGGAGCTGGATAC
 
GCCAGTCCCCAGAGAAGGGGCTGGAGTGGATTGGGAAATCAATCATGGTGGATACG
TCACCTACAATCCGTCCCTCGAGAGTCGAGTCACCATATCAGTAGACACGTCCAAGA
ACCAGTTCTCCCTGAAGCTGAGCTCTGTGACCGCCGCGGACACGGCTGTATATTACTG
TGCGAGGGACTATGGTCCGGGGAATTATGACTGGTACTTCGATCTCTGGGGCCGTGGC
ACCCTGGTCACTGTCTCCTCAGGTGGAGGCGGTTCAGGCGGAGGTGGCAGCGGCGGT
GGCGGGTCGGAAATTGTGTTGACACAGTCTCCAGCCACCCTGTCTTTGTCTCCAGGGG
AAAGAGCCACCCTCTCCTGCAGGGCCAGTCAGAGTGTTAGCAGCTACTTAGCCTGGT
ACCAACAGAAACCTGGCCAGGCTCCCAGGCTCCTCATCTATGATGCATCCAACAGGG
CCACTGGCATCCCAGCCAGGTTCAGTGGCAGTGGGTCTGGGACAGACTTCACTCTCAC
CATCAGCAGCCTAGAGCCTGAAGATTTTGCAGTTTATTACTGTCAGCAGCGTAGCAAC
TGGCCTCCGGCGCTCACTTTCGGCGGAGGGACCAAGGTGGAGATCAAAGAGTCCAAA
TATGGTCCCCCATGCCCACCATGCCCAATTGAAGTTATGTATCCTCCTCCTTACCTAGA
CAATGAGAAGAGCAATGGAACCATTATCCATGTGAAAGGGAAACACCTTTGTCCAAG
TCCCCTATTTCCCGGACCTTCTAAGCCCTTTTGGGTGCTGGTGGTGGTTGGTGGAGTCC
TGGCTTGCTATAGCTTGCTAGTAACAGTGGCCTTTATTATTTTCTGGGTGAAACGGGG
CAGAAAGAAGCTCCTGTATATATTCAAACAACCATTTATGAGACCAGTACAAACTAC
TCAAGAGGAAGATGGCTGTAGCTGCCGATTTCCAGAAGAAGAAGAAGGAGGATGTG
AACTGTGATAA
21. Nucleic acid sequence of 137DCR3 (without signal peptide) (SEQ ID NO:21) 
CAGGTGCAACTACAGCAGTGGGGCGCAGGACTGTTGAAGCCTTCGGAGACCCTG
TCCCTCACCTGCGCTGTCTATGGTGGGTCCTTCAGTGGTTACTACTGGAGCTGGATAC
GCCAGTCCCCAGAGAAGGGGCTGGAGTGGATTGGGGAAATCAATCATGGTGGATACG
TCACCTACAATCCGTCCCTCGAGAGTCGAGTCACCATATCAGTAGACACGTCCAAGA
ACCAGTTCTCCCTGAAGCTGAGCTCTGTGACCGCCGCGGACACGGCTGTATATTACTG
TGCGAGGGACTATGGTCCGGGGAATTATGACTGGTACTTCGATCTCTGGGGCCGTGGC
ACCCTGGTCACTGTCTCCTCAGGTGGAGGCGGTTCAGGCGGAGGTGGCAGCGGCGGT
GGCGGGTCGGAAATTGTGTTGACACAGTCTCCAGCCACCCTGTCTTTGTCTCCAGGGG
AAAGAGCCACCCTCTCCTGCAGGGCCAGTCAGAGTGTTAGCAGCTACTTAGCCTGGT
ACCAACAGAAACCTGGCCAGGCTCCCAGGCTCCTCATCTATGATGCATCCAACAGGG
CCACTGGCATCCCAGCCAGGTTCAGTGGCAGTGGGTCTGGGACAGACTTCACTCTCAC
CATCAGCAGCCTAGAGCCTGAAGATTTTGCAGTTTATTACTGTCAGCAGCGTAGCAAC
TGGCCTCCGGCGCTCACTTTCGGCGGAGGGACCAAGGTGGAGATCAAAGAGTCCAAA
TATGGTCCCCCATGCCCACCATGCCCAATTGAAGTTATGTATCCTCCTCCTTACCTAGA
CAATGAGAAGAGCAATGGAACCATTATCCATGTGAAAGGGAAACACCTTTGTCCAAG
 
TCCCCTATTTCCCGGACCTTCTAAGCCCTTTTGGGTGCTGGTGGTGGTTGGTGGAGTCC
TGGCTTGCTATAGCTTGCTAGTAACAGTGGCCTTTATTATTTCTGGGTGAGGAGTAA
GAGGAGCAGGCTCCTGCACAGTGACTACATGAACATGACTCCCCGCCGCCCCGGGCC
CACCCGCAAGCATTACCAGCCCTATGCCCCACCACGCGACTTCGCAGCCTATCGCTCC
AAACGGGGCAGAAAGAAGCTCCTGTATATATTCAAACAACCATTTATGAGACCAGTA
CAAACTACTCAAGAGGAAGATGGCTGTAGCTGCCGATTTCCAGAAGAAGAAGAAGG
AGGATGTGAACTGTGATAA
22. Nucleic acid sequence of 137DCR4 (without signal peptide) (SEQ ID NO:22) 
CAGGTGCAACTACAGCAGTGGGGCGCAGGACTGTTGAAGCCTTCGGAGACCCTG
TCCCTCACCTGCGCTGTCTATGGTGGGTCCTTCAGTGGTTACTACTGGAGCTGGATAC
GCCAGTCCCCAGAGAAGGGGCTGGAGTGGATTGGGGAAATCAATCATGGTGGATACG
TCACCTACAATCCGTCCCTCGAGAGTCGAGTCACCATATCAGTAGACACGTCCAAGA
ACCAGTTCTCCCTGAAGCTGAGCTCTGTGACCGCCGCGGACACGGCTGTATATTACTG
TGCGAGGGACTATGGTCCGGGGAATTATGACTGGTACTTCGATCTCTGGGGCCGTGGC
ACCCTGGTCACTGTCTCCTCAGGTGGAGGCGGTTCAGGCGGAGGTGGCAGCGGCGGT
GGCGGGTCGGAAATTGTGTTGACACAGTCTCCAGCCACCCTGTCTTTGTCTCCAGGGG
AAAGAGCCACCCTCTCCTGCAGGGCCAGTCAGAGTGTTAGCAGCTACTTAGCCTGGT
ACCAACAGAAACCTGGCCAGGCTCCCAGGCTCCTCATCTATGATGCATCCAACAGGG
CCACTGGCATCCCAGCCAGGTTCAGTGGCAGTGGGTCTGGGACAGACTTCACTCTCAC
CATCAGCAGCCTAGAGCCTGAAGATTTTGCAGTTTATTACTGTCAGCAGCGTAGCAAC
TGGCCTCCGGCGCTCACTTTCGGCGGAGGGACCAAGGTGGAGATCAAAGAGTCCAAA
TATGGTCCCCCATGCCCACCATGCCCAATTGAAGTTATGTATCCTCCTCCTTACCTAGA
CAATGAGAAGAGCAATGGAACCATTATCCATGTGAAAGGGAAACACCTTTGTCCAAG
TCCCCTATTTCCCGGACCTTCTAAGCCCTTTTGGGTGCTGGTGGTGGTTGGTGGAGTCC
TGGCTTGCTATAGCTTGCTAGTAACAGTGGCCTTTATTATTTTCTGGGTGAAACGGGG
CAGAAAGAAGCTCCTGTATATATTCAAACAACCATTTATGAGACCAGTACAAACTAC
TCAAGAGGAAGATGGCTGTAGCTGCCGATTTCCAGAAGAAGAAGAAGGAGGATGTG
AACTGAGGAGTAAGAGGAGCAGGCTCCTGCACAGTGACTACATGAACATGACTCCCC
GCCGCCCCGGGCCCACCCGCAAGCATTACCAGCCCTATGCCCCACCACGCGACTTCGC
AGCCTATCGCTCCTGATAA
23. Amino acid sequence of meso G1 CAR (SEQ ID NO:23) 
MALPVTALLLPLALLLHAARPSQVQLQQSGPELEKPGASVKISCKASGYSFTGYTMN
WVKQSHGKSLEWIGLITPYNGASSYNQKFRGKATLTVDKSSSTAYMDLLSLTSEDSAVYF
 
CARGGYDGRGFDYWGQGTTVTVSSGGGGSGGGGSGGGGSDIELTQSPAIMSASPGEKVT
MTCSASSSVSYMHWYQQKSGTSPKRWIYDTSKLASGVPGRFSGSGSGNSYSLTISSVEAE
DDATYYCQQWSKHPLTYGAGTKLEIKFVPVFLPAKPTTTPAPRPPTPAPTIASQPLSLRPEA
CRPAAGGAVHTRGLDFACDIYIWAPLAGTCGVLLLSLVITLYCNHRVKFSRSADAPAYQQ
GQNQLYNELNLGRREEYDVLDKRRGRDPEMGGKPRRKNPQEGLYNELQKDKMAEAYS
EIGMKGERRRGKGHDGLYQGLSTATKDTYDALHMQALPPR
24. Nucleic acid sequence of meso G1 CAR (SEQ ID NO:24) 
ATGGCCTTACCAGTGACCGCCTTGCTCCTGCCGCTGGCCTTGCTGCTCCACGCCG
CCAGGCCGAGCCAGGTACAACTGCAGCAGTCTGGGCCTGAGCTGGAGAAGCCTGGCG
CTTCAGTGAAGATATCCTGCAAGGCTTCTGGTTACTCATTCACTGGCTACACCATGAA
CTGGGTGAAGCAGAGCCATGGAAAGAGCCTTGAGTGGATTGGACTTATTACTCCTTA
CAATGGTGCTTCTAGCTACAACCAGAAGTTCAGGGGCAAGGCCACATTAACTGTAGA
CAAGTCATCCAGCACAGCCTACATGGACCTCCTCAGTCTGACATCTGAAGACTCTGCA
GTCTATTTCTGTGCAAGGGGGGGTTACGACGGGAGGGGTTTTGACTACTGGGGCCAA
GGGACCACGGTCACCGTCTCCTCAGGTGGAGGCGGTTCAGGCGGAGGTGGCAGCGGC
GGTGGCGGGTCGGACATCGAGCTCACTCAGTCTCCAGCAATCATGTCTGCATCTCCAG
GGGAGAAGGTCACCATGACCTGCAGTGCCAGCTCAAGTGTAAGTTACATGCACTGGT
ACCAGCAGAAGTCAGGCACCTCCCCCAAAAGATGGATTTATGACACATCCAAACTGG
CTTCTGGAGTCCCAGGTCGCTTCAGTGGCAGTGGGTCTGGAAACTCTTACTCTCTCAC
AATCAGCAGCGTGGAGGCTGAAGATGATGCAACTTATTACTGCCAGCAGTGGAGTAA
GCACCCTCTCACGTACGGTGCTGGGACAAAGTTGGAAATCAAATTCGTGCCGGTCTTC
CTGCCAGCGAAGCCCACCACGACGCCAGCGCCGCGACCACCAACACCGGCGCCCACC
ATCGCGTCGCAGCCCCTGTCCCTGCGCCCAGAGGCGTGCCGGCCAGCGGCGGGGGGC
GCAGTGCACACGAGGGGGCTGGACTTCGCCTGTGATATCTACATCTGGGCGCCCCTG
GCCGGGACTTGTGGGGTCCTTCTCCTGTCACTGGTTATCACCCTTTACTGCAACCACA
GAGTGAAGTTCAGCAGGAGCGCAGACGCCCCCGCGTACCAGCAGGGCCAGAACCAG
CTCTATAACGAGCTCAATCTAGGACGAAGAGAGGAGTACGATGTTTTGGACAAGAGA
CGTGGCCGGGACCCTGAGATGGGGGGAAAGCCGAGAAGGAAGAACCCTCAGGAAGG
CCTGTACAATGAACTGCAGAAAGATAAGATGGCGGAGGCCTACAGTGAGATTGGGAT
GAAAGGCGAGCGCCGGAGGGGCAAGGGGCACGATGGCCTTTACCAGGGTCTCAGTA
CAGCCACCAAGGACACCTACGACGCCCTTCACATGCAGGCCCTGCCCCCTCGCTGATA
A
25. Tyrosine activation motif of CD3ζ (SEQ ID NO: 25) 
RVKFSRSADAPAYQQGQNQLYNELNLGRREEYDVLDKRRGRDPEMGGKPRRKNPQ
EGLYNELQKDKMAEAYSEIGMKGERRRGKGHDGLYQGLSTATKDTYDALHMQALPPR
26. Nucleotide sequence of meso G2 CAR (SEQ ID NO:26) 
ATGGCCTTACCAGTGACCGCCTTGCTCCTGCCGCTGGCCTTGCTGCTCCACGCCG
CCAGGCCGAGCGAGGTGCAGCTGGTGGAGTCCGGGGGAGGCCTGGTCCAGCCTGGGG
GATCCCTGAGACTCTCCTGCGCAGCCTCTGGATTCGACCTCGGTTTCTACTTTTACGCC
TGTTGGGTCCGCCAGGCTCCAGGGAAGGGCCTGGAGTGGGTCTCATGCATTTATACTG
CTGGTAGTGGTAGCACGTACTACGCGAGCTGGGCGAAAGGCCGATTCACCATCTCCA
GAGACAATTCGAAGAACACGCTGTATCTGCAAATGAACAGTCTGAGAGCCGAGGACA
CGGCCGTGTATTACTGTGCGAGATCTACTGCTAATACTAGAAGTACTTATTATCTTAA
CTTGTGGGGCCAAGGCACCCTGGTCACCGTCTCCTCAGGCGGAGGCGGATCAGGTGG
TGGCGGATCTGGAGGTGGCGGAAGCGACATCCAGATGACCCAGTCTCCATCCTCCCT
GTCTGCATCTGTGGGAGACAGAGTCACCATCACTTGCCAGGCCAGTCAGAGGATTAG
TAGTTACTTATCCTGGTATCAGCAGAAACCAGGGAAAGTTCCCAAGCTCCTGATCTAT
GGTGCATCCACTCTGGCATCTGGGGTCCCCTCGCGGTTCAGTGGCAGTGGATCTGGGA
CAGATTTCACTCTCACCATCAGCAGCCTGCAGCCTGAAGATGTTGCCACTTACTACTG
TCAGAGTTATGCTTATTTTGATAGTAATAATTGGCATGCTTTCGGCGGAGGGACCAAG
GTGGAGATCAAAGAGTCCAAATATGGTCCCCCATGCCCACCATGCCCAGCACCTCCC
GTGGCCGGACCATCAGTCTTCCTGTTCCCCCCAAAACCCAAGGACACTCTCATGATCT
CCCGGACCCCTGAGGTCACGTGCGTGGTGGTGGACGTGAGCCAGGAAGACCCCGAGG
TCCAGTTCAACTGGTACGTGGATGGCGTGGAGGTGCATAATGCCAAGACAAAGCCGC
GGGAGGAGCAGTTCCAGAGCACGTACCGTGTGGTCAGCGTCCTCACCGTCCTGCACC
AGGACTGGCTGAACGGCAAGGAGTACAAGTGCAAGGTCTCCAACAAAGGCCTCCCGT
CCTCCATCGAGAAAACCATCTCCAAAGCCAAAGGGCAGCCCCGAGAGCCACAGGTGT
ACACCCTGCCCCCATCCCAGGAGGAGATGACCAAGAACCAGGTCAGCCTGACCTGCC
TGGTCAAAGGCTTCTACCCCAGCGACATCGCCGTGGAGTGGGAGAGCAATGGGCAGC
CGGAGAACAACTACAAGACCACGCCTCCCGTGCTGGACTCCGACGGCTCCTTCTTCCT
CTACAGCAGGCTAACCGTGGACAAGAGCAGGTGGCAGGAGGGGAATGTCTTCTCATG
CTCCGTGATGCATGAGGCTCTGCACAACCACTACACACAGAAGAGCCTCTCCCTGTCT
CTGGGTAAACCCTTTTGGGTGCTGGTGGTGGTTGGTGGAGTCCTGGCTTGCTATAGCT
TGCTAGTAACAGTGGCCTTTATTATTTTCTGGGTGAGGAGTAAGAGGAGCAGGCTCCT
GCACAGTGACTACATGAACATGACTCCCCGCCGCCCCGGGCCCACCCGCAAGCATTA
CCAGCCCTATGCCCCACCACGCGACTTCGCAGCCTATCGCTCCAGAGTGAAGTTCAGC
AGGAGCGCAGACGCCCCCGCGTACCAGCAGGGCCAGAACCAGCTCTATAACGAGCTC
 
AATCTAGGACGAAGAGAGGAGTACGATGTTTTGGACAAGAGACGTGGCCGGGACCCT
GAGATGGGGGGAAAGCCGAGAAGGAAGAACCCTCAGGAAGGCCTGTACAATGAACT
GCAGAAAGATAAGATGGCGGAGGCCTACAGTGAGATTGGGATGAAAGGCGAGCGCC
GGAGGGGCAAGGGGCACGATGGCCTTTACCAGGGTCTCAGTACAGCCACCAAGGACA
CCTACGACGCCCTTCACATGCAGGCCCTGCCCCCTCGC
27. Nucleic acid sequence of PS328b vector (SEQ ID NO:27) 
GGCGCGCCTTAACCCTAGAAAGATAATCATATTGTGACGTACGTTAAAGATAATC
ATGCGTAAAATTGACGCATGTCTAGAGGTGTGGAAAGTCCCCAGGCTCCCCAGCAGG
CAGAAGTATGCAAAGCATGCATCTCAATTAGTCAGCAACCAGGTGTGGAAAGTCCCC
AGGCTCCCCAGCAGGCAGAAGTATGCAAAGCATGCATCTCAATTAGTCAGCAACCAA
GGATCTGCGATCGCTCCGGTGCCCGTCAGTGGGCAGAGCGCACATCGCCCACAGTCC
CCGAGAAGTTGGGGGGAGGGGTCGGCAATTGAACGGGTGCCTAGAGAAGGTGGCGC
GGGGTAAACTGGGAAAGTGATGTCGTGTACTGGCTCCGCCTTTTTCCCGAGGGTGGG
GGAGAACCGTATATAAGTGCAGTAGTCGCCGTGAACGTTCTTTTTCGCAACGGGTTTG
CCGCCAGAACACAGCTGAAGCTTCGAGGGGCTCGCATCTCTCCTTCACGCGCCCGCCG
CCCTACCTGAGGCCGCCATCCACGCCGGTTGAGTCGCGTTCTGCCGCCTCCCGCCTGT
GGTGCCTCCTGAACTGCGTCCGCCGTCTAGGTAAGTTTAAAGCTCAGGTCGAGACCGG
GCCTTTGTCCGGCGCTCCCTTGGAGCCTACCTAGACTCAGCCGGCTCTCCACGCTTTG
CCTGACCCTGCTTGCTCAACTCTACGTCTTTGTTTCGTTTTCTGTTCTGCGCCGTTACA
GATCCAAGCTGTGACCGGCGCCTACGAATTCGGATCCTGCACTAGTGCTGTCGACCAG
ACATGATAAGATACATTGATGAGTTTGGACAAACCACAACTAGAATGCAGTGAAAAA
AATGCTTTATTTGTGAAATTTGTGATGCTATTGCTTTATTTGTAACCATTATAAGCTGC
AATAAACAAGTTAACAACAACAATTGCATTCATTTTATGTTTCAGGTTCAGGGGGAGG
TGTGGGAGGTTTTTTAAAGCAAGTAAAACCTCTACAAATGTGGTAGCATGCGTCAATT
TTACGCAGACTATCTTTCTAGGGTTAAATCGATCCCGCTAGCCCCGATATCCCCTTAA
TTAAGAGGGGGAGACCAAAGGGCGAGACGTTAAGGCCTCACGTGACATGTGAGCAA
AAGGCCAGCAAAAGGCCAGGAACCGTAAAAAGGCCGCGTTGCTGGCGTTTTTCCATA
GGCTCCGCCCCCCTGACGAGCATCACAAAAATCGACGCTCAAGTCAGAGGTGGCGAA
ACCCGACAGGACTATAAAGATACCAGGCGTTTCCCCCTGGAAGCTCCCTCGTGCGCTC
TCCTGTTCCGACCCTGCCGCTTACGGATACCTGTCCGCCTTTCTCCCTTCGGGAAGCG
TGGCGCTTTCTCATAGCTCACGCTGTAGGTATCTCAGTTCGGTGTAGGTCGTTCGCTCC
AAGCTGGGCTGTGTGCACGAACCCCCCGTTCAGCCCGACCGCTGCGCCTTATCCGGTA
ACTATCGTCTTGAGTCCAACCCGGTAAGACACGACTTATCGCCACTGGCAGCAGCCAC
TGGTAACAGGATTAGCAGAGCGAGGTATGTAGGCGGTGCTACAGAGTTCTTGAAGTG
 
GTGGCCTAACTACGGCTACACTAGAAGAACAGTATTTGGTATCTGCGCTCTGCTGAAG
CCAGTTACCTTCGGAAAAAGAGTTGGTAGCTCTTGATCCGGCAAACAAACCACCGCT
GGTAGCGGTGGTTTTTTTGTTTGCAAGCAGCAGATTACGCGCAGAAAAAAAGGATCT
CAAGAAGATCCTTTGATCTTTTCTACGGGGTCTGACGCTCAGTGGAACGAAAACTCAC
GTTAAGGGATTTTGGTCATGCCGTCTCAGAAGAACTCGTCAAGAAGGCGATAGAAGG
CGATGCGCTGCGAATCGGGAGCGGCGATACCGTAAAGCACGAGGAAGCGGTCAGCC
CATTCGCCGCCAAGCTCTTCAGCAATATCACGGGTAGCCAACGCTATGTCCTGATAGC
GGTCCGCCACACCCAGCCGGCCACAGTCGATGAATCCAGAAAAGCGGCCATTTTCCA
CCATGATATTCGGCAAGCAGGCATCGCCATGGGTCACGACGAGATCCTCGCCGTCGG
GCATGCTCGCCTTGAGCCTGGCGAACAGTTCGGCTGGCGCGAGCCCCTGATGCTCTTC
GTCCAGATCATCCTGATCGACAAGACCGGCTTCCATCCGAGTACGTGCTCTCTCGATG
CGATGTTTCGCTTGGTGGTCGAATGGGCAGGTAGCCGGATCAAGCGTATGCAGCCGC
CGCATTGCATCAGCCATGATGGATACTTTCTCGGCAGGAGCAAGGTGAGATGACAGG
AGATCCTGCCCCGGCACTTCGCCCAATAGCAGCCAGTCCCTTCCCGCTTCAGTGACAA
CGTCGAGTACAGCTGCGCAAGGAACGCCCGTCGTGGCCAGCCACGATAGCCGCGCTG
CCTCGTCTTGCAGTTCATTCAGGGCACCGGACAGGTCGGTCTTGACAAAAAGAACCG
GGCGCCCCTGCGCTGACAGCCGGAACACGGCGGCATCAGAGCAGCCGATTGTCTGTT
GTGCCCAGTCATAGCCGAATAGCCTCTCCACCCAAGCGGCCGGAGAACCTGCGTGCA
ATCCATCTTGTTCAATCATAATATTATTGAAGCATTTATCAGGGTTCGTCTCGTCCCGG
TCTCCTCCCATGCATG
28. Nucleotide sequence of primer CD137-F (SEQ ID NO: 28)
   CGAGTCACCATATCAGTA
29. Nucleotide sequence of primer CD137-R (SEQ ID NO: 29)
   CGAAGTACCAGTCATAATTC
30. Nucleotide sequence in probe Taqman (SEQ ID NO: 30)
   CCTCGCACAGTAATATACAGCCGT
31. Amino acid sequence of CD28 extracellular agonistic single-chain antibody (SEQ ID NO: 31) 
QVQLVQSGAEVKKPGASVKVSCKASGYTFTSYYIHWVRQAPGQGLEWIGCIYPGNV
NTNYNEKFKDRATLTVDTSISTAYMELSRLRSDDTAVYFCTRSHYGLDWNFDVWGQGTT
VTVSSGGGGSGGGGSGGGGSDIQMTQSPSSLSASVGDRVTITCHASQNIYVWLNWYQQK
 
PGKAPKLLIYKASNLHTGVPSRFSGSGSGTDFTLTISSLQPEDFATYYCQQGQTYPYTFGG
GTKVEIKE
32. Amino acid sequence of 28DCR1 (including signal peptide) (SEQ ID NO:32) 
MGNSCYNIVATLLLVLNFERTRSQVQLVQSGAEVKKPGASVKVSCKASGYTFTSYYI
HWVRQAPGQGLEWIGCIYPGNVNTNYNEKFKDRATLTVDTSISTAYMELSRLRSDDTAV
YFCTRSHYGLDWNFDVWGQGTTVTVSSGGGGSGGGGSGGGGSDIQMTQSPSSLSASVG
DRVTITCHASQNIYVWLNWYQQKPGKAPKLLIYKASNLHTGVPSRFSGSGSGTDFTLTISS
LQPEDFATYYCQQGQTYPYTFGGGTKVEIKESKYGPPCPPCPIEVMYPPPYLDNEKSNGTII
HVKGKHLCPSPLFPGPSKPFWVLVVVGGVLACYSLLVTVAFIIFWVRSKRSRLLHSDYMN
MTPRRPGPTRKHYQPYAPPRDFAAYRS
33. Amino acid sequence of 28DCR2 (including signal peptide) (SEQ ID NO:33) 
MGNSCYNIVATLLLVLNFERTRSQVQLVQSGAEVKKPGASVKVSCKASGYTFTSYYI
HWVRQAPGQGLEWIGCIYPGNVNTNYNEKFKDRATLTVDTSISTAYMELSRLRSDDTAV
YFCTRSHYGLDWNFDVWGQGTTVTVSSGGGGSGGGGSGGGGSDIQMTQSPSSLSASVG
DRVTITCHASQNIYVWLNWYQQKPGKAPKLLIYKASNLHTGVPSRFSGSGSGTDFTLTISS
LQPEDFATYYCQQGQTYPYTFGGGTKVEIKESKYGPPCPPCPIEVMYPPPYLDNEKSNGTII
HVKGKHLCPSPLFPGPSKPFWVLVVVGGVLACYSLLVTVAFIIFWVKRGRKKLLYIFKQP
FMRPVQTTQEEDGCSCRFPEEEEGGCEL
34. Amino acid sequence of 28DCR3 (including signal peptide) (SEQ ID NO:34) 
MGNSCYNIVATLLLVLNFERTRSQVQLVQSGAEVKKPGASVKVSCKASGYTFTSYYI
HWVRQAPGQGLEWIGCIYPGNVNTNYNEKFKDRATLTVDTSISTAYMELSRLRSDDTAV
YFCTRSHYGLDWNFDVWGQGTTVTVSSGGGGSGGGGSGGGGSDIQMTQSPSSLSASVG
DRVTITCHASQNIYVWLNWYQQKPGKAPKLLIYKASNLHTGVPSRFSGSGSGTDFTLTISS
LQPEDFATYYCQQGQTYPYTFGGGTKVEIKESKYGPPCPPCPIEVMYPPPYLDNEKSNGTII
HVKGKHLCPSPLFPGPSKPFWVLVVVGGVLACYSLLVTVAFIIFWVRSKRSRLLHSDYMN
MTPRRPGPTRKHYQPYAPPRDFAAYRSKRGRKKLLYIFKQPFMRPVQTTQEEDGCSCRFP
EEEEGGCEL
35. Amino acid sequence of 28DCR4 (including signal peptide) (SEQ ID NO:35) 
MGNSCYNIVATLLLVLNFERTRSQVQLVQSGAEVKKPGASVKVSCKASGYTFTSYYI
HWVRQAPGQGLEWIGCIYPGNVNTNYNEKFKDRATLTVDTSISTAYMELSRLRSDDTAV
YFCTRSHYGLDWNFDVWGQGTTVTVSSGGGGSGGGGSGGGGSDIQMTQSPSSLSASVG
 
DRVTITCHASQNIYVWLNWYQQKPGKAPKLLIYKASNLHTGVPSRFSGSGSGTDFTLTISS
LQPEDFATYYCQQGQTYPYTFGGGTKVEIKESKYGPPCPPCPIEVMYPPPYLDNEKSNGTII
HVKGKHLCPSPLFPGPSKPFWVLVVVGGVLACYSLLVTVAFIIFWVKRGRKKLLYIFKQP
FMRPVQTTQEEDGCSCRFPEEEEGGCELRSKRSRLLHSDYMNMTPRRPGPTRKHYQPYAP
PRDFAAYRS
36. Amino acid sequence of 28DCR1 (without signal peptide) (SEQ ID NO:36) 
QVQLVQSGAEVKKPGASVKVSCKASGYTFTSYYIHWVRQAPGQGLEWIGCIYPGNV
NTNYNEKFKDRATLTVDTSISTAYMELSRLRSDDTAVYFCTRSHYGLDWNFDVWGQGTT
VTVSSGGGGSGGGGSGGGGSDIQMTQSPSSLSASVGDRVTITCHASQNIYVWLNWYQQK
PGKAPKLLIYKASNLHTGVPSRFSGSGSGTDFTLTISSLQPEDFATYYCQQGQTYPYTFGG
GTKVEIKESKYGPPCPPCPIEVMYPPPYLDNEKSNGTIIHVKGKHLCPSPLFPGPSKPFWVL
VVVGGVLACYSLLVTVAFIIFWVRSKRSRLLHSDYMNMTPRRPGPTRKHYQPYAPPRDF
AAYRS
37. Amino acid sequence of 28DCR2 (without signal peptide) (SEQ ID NO:37) 
QVQLVQSGAEVKKPGASVKVSCKASGYTFTSYYIHWVRQAPGQGLEWIGCIYPGNV
NTNYNEKFKDRATLTVDTSISTAYMELSRLRSDDTAVYFCTRSHYGLDWNFDVWGQGTT
VTVSSGGGGSGGGGSGGGGSDIQMTQSPSSLSASVGDRVTITCHASQNIYVWLNWYQQK
PGKAPKLLIYKASNLHTGVPSRFSGSGSGTDFTLTISSLQPEDFATYYCQQGQTYPYTFGG
GTKVEIKESKYGPPCPPCPIEVMYPPPYLDNEKSNGTIIHVKGKHLCPSPLFPGPSKPFWVL
VVVGGVLACYSLLVTVAFIIFWVKRGRKKLLYIFKQPFMRPVQTTQEEDGCSCRFPEEEE
GGCEL
38. Amino acid sequence of 28DCR3 (without signal peptide) (SEQ ID NO:38) 
QVQLVQSGAEVKKPGASVKVSCKASGYTFTSYYIHWVRQAPGQGLEWIGCIYPGNV
NTNYNEKFKDRATLTVDTSISTAYMELSRLRSDDTAVYFCTRSHYGLDWNFDVWGQGTT
VTVSSGGGGSGGGGSGGGGSDIQMTQSPSSLSASVGDRVTITCHASQNIYVWLNWYQQK
PGKAPKLLIYKASNLHTGVPSRFSGSGSGTDFTLTISSLQPEDFATYYCQQGQTYPYTFGG
GTKVEIKESKYGPPCPPCPIEVMYPPPYLDNEKSNGTIIHVKGKHLCPSPLFPGPSKPFWVL
VVVGGVLACYSLLVTVAFIIFWVRSKRSRLLHSDYMNMTPRRPGPTRKHYQPYAPPRDF
AAYRSKRGRKKLLYIFKQPFMRPVQTTQEEDGCSCRFPEEEEGGCEL
39. Amino acid sequence of 28DCR4 (without signal peptide) (SEQ ID NO:39) QVQLVQSGAEVKKPGASVKVSCKASGYTFTSYYIHWVRQAPGQGLEWIGCIYPGNV 
NTNYNEKFKDRATLTVDTSISTAYMELSRLRSDDTAVYFCTRSHYGLDWNFDVWGQGTT
VTVSSGGGGSGGGGSGGGGSDIQMTQSPSSLSASVGDRVTITCHASQNIYVWLNWYQQK
PGKAPKLLIYKASNLHTGVPSRFSGSGSGTDFTLTISSLQPEDFATYYCQQGQTYPYTFGG
GTKVEIKESKYGPPCPPCPIEVMYPPPYLDNEKSNGTIIHVKGKHLCPSPLFPGPSKPFWVL
VVVGGVLACYSLLVTVAFIIFWVKRGRKKLLYIFKQPFMRPVQTTQEEDGCSCRFPEEEE
GGCELRSKRSRLLHSDYMNMTPRRPGPTRKHYQPYAPPRDFAAYRS
40. Nucleic acid sequence of 28DCR1 (including signal peptide) (SEQ ID NO:40) 
ATGGGAAACAGCTGTTACAACATAGTAGCCACTCTGTTGCTGGTCCTCAACTTTG
AGAGGACAAGATCACAGGTGCAGCTGGTGCAGTCTGGGGCTGAGGTGAAGAAGCCT
GGGGCCTCAGTGAAGGTCTCCTGCAAGGCTTCTGGATACACCTTCACCAGCTACTATA
TACACTGGGTGCGACAGGCCCCTGGACAAGGGCTTGAGTGGATTGGATGTATTTATCC
TGGAAATGTCAATACTAACTATAATGAGAAGTTCAAGGACAGGGCCACCCTGACCGT
AGACACGTCCATCAGCACAGCCTACATGGAGCTGAGCAGGCTGAGATCTGACGACAC
GGCCGTGTATTTCTGTACAAGATCACACTACGGCCTCGACTGGAACTTCGATGTCTGG
GGCCAAGGGACCACGGTCACCGTCTCCTCAGGTGGAGGCGGTTCAGGCGGAGGTGGC
AGCGGCGGTGGCGGGTCGGACATCCAGATGACCCAGTCTCCATCCTCCCTGTCTGCAT
CTGTAGGAGACAGAGTCACCATCACTTGCCATGCCAGTCAAAACATTTATGTTTGGTT
AAACTGGTATCAGCAGAAACCAGGGAAAGCCCCTAAGCTCCTGATCTATAAGGCTTC
CAACCTGCACACAGGGGTCCCATCAAGGTTCAGTGGCAGTGGATCTGGGACAGATTT
CACTCTCACCATCAGCAGTCTGCAACCTGAAGATTTTGCAACTTACTACTGTCAACAG
GGTCAAACTTATCCGTACACGTTCGGCGGAGGGACCAAGGTGGAGATCAAAGAGTCC
AAATATGGTCCCCCATGCCCACCATGCCCAATTGAAGTTATGTATCCTCCTCCTTACCT
AGACAATGAGAAGAGCAATGGAACCATTATCCATGTGAAAGGGAAACACCTTTGTCC
AAGTCCCCTATTTCCCGGACCTTCTAAGCCCTTTTGGGTGCTGGTGGTGGTTGGTGGA
GTCCTGGCTTGCTATAGCTTGCTAGTAACAGTGGCCTTTATTATTTTCTGGGTGAGGA
GTAAGAGGAGCAGGCTCCTGCACAGTGACTACATGAACATGACTCCCCGCCGCCCCG
GGCCCACCCGCAAGCATTACCAGCCCTATGCCCCACCACGCGACTTCGCAGCCTATCG
CTCCTGATAA
41. Nucleic acid sequence of 28DCR2 (including signal peptide) (SEQ ID NO:41) 
ATGGGAAACAGCTGTTACAACATAGTAGCCACTCTGTTGCTGGTCCTCAACTTTG
AGAGGACAAGATCACAGGTGCAGCTGGTGCAGTCTGGGGCTGAGGTGAAGAAGCCT
GGGGCCTCAGTGAAGGTCTCCTGCAAGGCTTCTGGATACACCTTCACCAGCTACTATA
TACACTGGGTGCGACAGGCCCCTGGACAAGGGCTTGAGTGGATTGGATGTATTTATCC
 
TGGAAATGTCAATACTAACTATAATGAGAAGTTCAAGGACAGGGCCACCCTGACCGT
AGACACGTCCATCAGCACAGCCTACATGGAGCTGAGCAGGCTGAGATCTGACGACAC
GGCCGTGTATTTCTGTACAAGATCACACTACGGCCTCGACTGGAACTTCGATGTCTGG
GGCCAAGGGACCACGGTCACCGTCTCCTCAGGTGGAGGCGGTTCAGGCGGAGGTGGC
AGCGGCGGTGGCGGGTCGGACATCCAGATGACCCAGTCTCCATCCTCCCTGTCTGCAT
CTGTAGGAGACAGAGTCACCATCACTTGCCATGCCAGTCAAAACATTTATGTTTGGTT
AAACTGGTATCAGCAGAAACCAGGGAAAGCCCCTAAGCTCCTGATCTATAAGGCTTC
CAACCTGCACACAGGGGTCCCATCAAGGTTCAGTGGCAGTGGATCTGGGACAGATTT
CACTCTCACCATCAGCAGTCTGCAACCTGAAGATTTTGCAACTTACTACTGTCAACAG
GGTCAAACTTATCCGTACACGTTCGGCGGAGGGACCAAGGTGGAGATCAAAGAGTCC
AAATATGGTCCCCCATGCCCACCATGCCCAATTGAAGTTATGTATCCTCCTCCTTACCT
AGACAATGAGAAGAGCAATGGAACCATTATCCATGTGAAAGGGAAACACCTTTGTCC
AAGTCCCCTATTTCCCGGACCTTCTAAGCCCTTTTGGGTGCTGGTGGTGGTTGGTGGA
GTCCTGGCTTGCTATAGCTTGCTAGTAACAGTGGCCTTTATTATTTTCTGGGTGAAACG
GGGCAGAAAGAAGCTCCTGTATATATTCAAACAACCATTTATGAGACCAGTACAAAC
TACTCAAGAGGAAGATGGCTGTAGCTGCCGATTTCCAGAAGAAGAAGAAGGAGGAT
GTGAACTGTGATAA
42. Nucleic acid sequence of 28DCR3 (including signal peptide) (SEQ ID NO:42) 
ATGGGAAACAGCTGTTACAACATAGTAGCCACTCTGTTGCTGGTCCTCAACTTTG
AGAGGACAAGATCACAGGTGCAGCTGGTGCAGTCTGGGGCTGAGGTGAAGAAGCCT
GGGGCCTCAGTGAAGGTCTCCTGCAAGGCTTCTGGATACACCTTCACCAGCTACTATA
TACACTGGGTGCGACAGGCCCCTGGACAAGGGCTTGAGTGGATTGGATGTATTTATCC
TGGAAATGTCAATACTAACTATAATGAGAAGTTCAAGGACAGGGCCACCCTGACCGT
AGACACGTCCATCAGCACAGCCTACATGGAGCTGAGCAGGCTGAGATCTGACGACAC
GGCCGTGTATTTCTGTACAAGATCACACTACGGCCTCGACTGGAACTTCGATGTCTGG
GGCCAAGGGACCACGGTCACCGTCTCCTCAGGTGGAGGCGGTTCAGGCGGAGGTGGC
AGCGGCGGTGGCGGGTCGGACATCCAGATGACCCAGTCTCCATCCTCCCTGTCTGCAT
CTGTAGGAGACAGAGTCACCATCACTTGCCATGCCAGTCAAAACATTTATGTTTGGTT
AAACTGGTATCAGCAGAAACCAGGGAAAGCCCCTAAGCTCCTGATCTATAAGGCTTC
CAACCTGCACACAGGGGTCCCATCAAGGTTCAGTGGCAGTGGATCTGGGACAGATTT
CACTCTCACCATCAGCAGTCTGCAACCTGAAGATTTTGCAACTTACTACTGTCAACAG
GGTCAAACTTATCCGTACACGTTCGGCGGAGGGACCAAGGTGGAGATCAAAGAGTCC
AAATATGGTCCCCCATGCCCACCATGCCCAATTGAAGTTATGTATCCTCCTCCTTACCT
AGACAATGAGAAGAGCAATGGAACCATTATCCATGTGAAAGGGAAACACCTTTGTCC
AAGTCCCCTATTTCCCGGACCTTCTAAGCCCTTTTGGGTGCTGGTGGTGGTTGGTGGA
 
GTCCTGGCTTGCTATAGCTTGCTAGTAACAGTGGCCTTTATTATTTCTGGGTGAGGA
GTAAGAGGAGCAGGCTCCTGCACAGTGACTACATGAACATGACTCCCCGCCGCCCCG
GGCCCACCCGCAAGCATTACCAGCCCTATGCCCCACCACGCGACTTCGCAGCCTATCG
CTCCAAACGGGGCAGAAAGAAGCTCCTGTATATATTCAAACAACCATTTATGAGACC
AGTACAAACTACTCAAGAGGAAGATGGCTGTAGCTGCCGATTTCCAGAAGAAGAAGA
AGGAGGATGTGAACTGTGATAA
43. Nucleic acid sequence of 28DCR4 (including signal peptide) (SEQ ID NO:43) 
ATGGGAAACAGCTGTTACAACATAGTAGCCACTCTGTTGCTGGTCCTCAACTTTG
AGAGGACAAGATCACAGGTGCAGCTGGTGCAGTCTGGGGCTGAGGTGAAGAAGCCT
GGGGCCTCAGTGAAGGTCTCCTGCAAGGCTTCTGGATACACCTTCACCAGCTACTATA
TACACTGGGTGCGACAGGCCCCTGGACAAGGGCTTGAGTGGATTGGATGTATTTATCC
TGGAAATGTCAATACTAACTATAATGAGAAGTTCAAGGACAGGGCCACCCTGACCGT
AGACACGTCCATCAGCACAGCCTACATGGAGCTGAGCAGGCTGAGATCTGACGACAC
GGCCGTGTATTTCTGTACAAGATCACACTACGGCCTCGACTGGAACTTCGATGTCTGG
GGCCAAGGGACCACGGTCACCGTCTCCTCAGGTGGAGGCGGTTCAGGCGGAGGTGGC
AGCGGCGGTGGCGGGTCGGACATCCAGATGACCCAGTCTCCATCCTCCCTGTCTGCAT
CTGTAGGAGACAGAGTCACCATCACTTGCCATGCCAGTCAAAACATTTATGTTTGGTT
AAACTGGTATCAGCAGAAACCAGGGAAAGCCCCTAAGCTCCTGATCTATAAGGCTTC
CAACCTGCACACAGGGGTCCCATCAAGGTTCAGTGGCAGTGGATCTGGGACAGATTT
CACTCTCACCATCAGCAGTCTGCAACCTGAAGATTTTGCAACTTACTACTGTCAACAG
GGTCAAACTTATCCGTACACGTTCGGCGGAGGGACCAAGGTGGAGATCAAAGAGTCC
AAATATGGTCCCCCATGCCCACCATGCCCAATTGAAGTTATGTATCCTCCTCCTTACCT
AGACAATGAGAAGAGCAATGGAACCATTATCCATGTGAAAGGGAAACACCTTTGTCC
AAGTCCCCTATTTCCCGGACCTTCTAAGCCCTTTTGGGTGCTGGTGGTGGTTGGTGGA
GTCCTGGCTTGCTATAGCTTGCTAGTAACAGTGGCCTTTATTATTTTCTGGGTGAAACG
GGGCAGAAAGAAGCTCCTGTATATATTCAAACAACCATTTATGAGACCAGTACAAAC
TACTCAAGAGGAAGATGGCTGTAGCTGCCGATTTCCAGAAGAAGAAGAAGGAGGAT
GTGAACTGAGGAGTAAGAGGAGCAGGCTCCTGCACAGTGACTACATGAACATGACTC
CCCGCCGCCCCGGGCCCACCCGCAAGCATTACCAGCCCTATGCCCCACCACGCGACTT
CGCAGCCTATCGCTCCTGATAA
44. Nucleic acid sequence of 28DCR1 (without signal peptide) (SEQ ID NO:44) 
CAGGTGCAGCTGGTGCAGTCTGGGGCTGAGGTGAAGAAGCCTGGGGCCTCAGTG
AAGGTCTCCTGCAAGGCTTCTGGATACACCTTCACCAGCTACTATATACACTGGGTGC
 
GACAGGCCCCTGGACAAGGGCTTGAGTGGATTGGATGTATTTATCCTGGAAATGTCA
ATACTAACTATAATGAGAAGTTCAAGGACAGGGCCACCCTGACCGTAGACACGTCCA
TCAGCACAGCCTACATGGAGCTGAGCAGGCTGAGATCTGACGACACGGCCGTGTATT
TCTGTACAAGATCACACTACGGCCTCGACTGGAACTTCGATGTCTGGGGCCAAGGGA
CCACGGTCACCGTCTCCTCAGGTGGAGGCGGTTCAGGCGGAGGTGGCAGCGGCGGTG
GCGGGTCGGACATCCAGATGACCCAGTCTCCATCCTCCCTGTCTGCATCTGTAGGAGA
CAGAGTCACCATCACTTGCCATGCCAGTCAAAACATTTATGTTTGGTTAAACTGGTAT
CAGCAGAAACCAGGGAAAGCCCCTAAGCTCCTGATCTATAAGGCTTCCAACCTGCAC
ACAGGGGTCCCATCAAGGTTCAGTGGCAGTGGATCTGGGACAGATTTCACTCTCACC
ATCAGCAGTCTGCAACCTGAAGATTTTGCAACTTACTACTGTCAACAGGGTCAAACTT
ATCCGTACACGTTCGGCGGAGGGACCAAGGTGGAGATCAAAGAGTCCAAATATGGTC
CCCCATGCCCACCATGCCCAATTGAAGTTATGTATCCTCCTCCTTACCTAGACAATGA
GAAGAGCAATGGAACCATTATCCATGTGAAAGGGAAACACCTTTGTCCAAGTCCCCT
ATTTCCCGGACCTTCTAAGCCCTTTTGGGTGCTGGTGGTGGTTGGTGGAGTCCTGGCTT
GCTATAGCTTGCTAGTAACAGTGGCCTTTATTATTTTCTGGGTGAGGAGTAAGAGGAG
CAGGCTCCTGCACAGTGACTACATGAACATGACTCCCCGCCGCCCCGGGCCCACCCG
CAAGCATTACCAGCCCTATGCCCCACCACGCGACTTCGCAGCCTATCGCTCCTGATAA
45. Nucleic acid sequence of 28DCR2 (without signal peptide) (SEQ ID NO:45) 
CAGGTGCAGCTGGTGCAGTCTGGGGCTGAGGTGAAGAAGCCTGGGGCCTCAGTG
AAGGTCTCCTGCAAGGCTTCTGGATACACCTTCACCAGCTACTATATACACTGGGTGC
GACAGGCCCCTGGACAAGGGCTTGAGTGGATTGGATGTATTTATCCTGGAAATGTCA
ATACTAACTATAATGAGAAGTTCAAGGACAGGGCCACCCTGACCGTAGACACGTCCA
TCAGCACAGCCTACATGGAGCTGAGCAGGCTGAGATCTGACGACACGGCCGTGTATT
TCTGTACAAGATCACACTACGGCCTCGACTGGAACTTCGATGTCTGGGGCCAAGGGA
CCACGGTCACCGTCTCCTCAGGTGGAGGCGGTTCAGGCGGAGGTGGCAGCGGCGGTG
GCGGGTCGGACATCCAGATGACCCAGTCTCCATCCTCCCTGTCTGCATCTGTAGGAGA
CAGAGTCACCATCACTTGCCATGCCAGTCAAAACATTTATGTTTGGTTAAACTGGTAT
CAGCAGAAACCAGGGAAAGCCCCTAAGCTCCTGATCTATAAGGCTTCCAACCTGCAC
ACAGGGGTCCCATCAAGGTTCAGTGGCAGTGGATCTGGGACAGATTTCACTCTCACC
ATCAGCAGTCTGCAACCTGAAGATTTTGCAACTTACTACTGTCAACAGGGTCAAACTT
ATCCGTACACGTTCGGCGGAGGGACCAAGGTGGAGATCAAAGAGTCCAAATATGGTC
CCCCATGCCCACCATGCCCAATTGAAGTTATGTATCCTCCTCCTTACCTAGACAATGA
GAAGAGCAATGGAACCATTATCCATGTGAAAGGGAAACACCTTTGTCCAAGTCCCCT
ATTTCCCGGACCTTCTAAGCCCTTTTGGGTGCTGGTGGTGGTTGGTGGAGTCCTGGCTT
 
GCTATAGCTTGCTAGTAACAGTGGCCTTTATTATTTTCTGGGTGAAACGGGGCAGAAA
GAAGCTCCTGTATATATTCAAACAACCATTTATGAGACCAGTACAAACTACTCAAGA
GGAAGATGGCTGTAGCTGCCGATTTCCAGAAGAAGAAGGAGGATGTGAACTGTG
ATAA
46. Nucleic acid sequence of 28DCR3 (without signal peptide) (SEQ ID NO:46) 
CAGGTGCAGCTGGTGCAGTCTGGGGCTGAGGTGAAGAAGCCTGGGGCCTCAGTG
AAGGTCTCCTGCAAGGCTTCTGGATACACCTTCACCAGCTACTATATACACTGGGTGC
GACAGGCCCCTGGACAAGGGCTTGAGTGGATTGGATGTATTTATCCTGGAAATGTCA
ATACTAACTATAATGAGAAGTTCAAGGACAGGGCCACCCTGACCGTAGACACGTCCA
TCAGCACAGCCTACATGGAGCTGAGCAGGCTGAGATCTGACGACACGGCCGTGTATT
TCTGTACAAGATCACACTACGGCCTCGACTGGAACTTCGATGTCTGGGGCCAAGGGA
CCACGGTCACCGTCTCCTCAGGTGGAGGCGGTTCAGGCGGAGGTGGCAGCGGCGGTG
GCGGGTCGGACATCCAGATGACCCAGTCTCCATCCTCCCTGTCTGCATCTGTAGGAGA
CAGAGTCACCATCACTTGCCATGCCAGTCAAAACATTTATGTTTGGTTAAACTGGTAT
CAGCAGAAACCAGGGAAAGCCCCTAAGCTCCTGATCTATAAGGCTTCCAACCTGCAC
ACAGGGGTCCCATCAAGGTTCAGTGGCAGTGGATCTGGGACAGATTTCACTCTCACC
ATCAGCAGTCTGCAACCTGAAGATTTTGCAACTTACTACTGTCAACAGGGTCAAACTT
ATCCGTACACGTTCGGCGGAGGGACCAAGGTGGAGATCAAAGAGTCCAAATATGGTC
CCCCATGCCCACCATGCCCAATTGAAGTTATGTATCCTCCTCCTTACCTAGACAATGA
GAAGAGCAATGGAACCATTATCCATGTGAAAGGGAAACACCTTTGTCCAAGTCCCCT
ATTTCCCGGACCTTCTAAGCCCTTTTGGGTGCTGGTGGTGGTTGGTGGAGTCCTGGCTT
GCTATAGCTTGCTAGTAACAGTGGCCTTTATTATTTTCTGGGTGAGGAGTAAGAGGAG
CAGGCTCCTGCACAGTGACTACATGAACATGACTCCCCGCCGCCCCGGGCCCACCCG
CAAGCATTACCAGCCCTATGCCCCACCACGCGACTTCGCAGCCTATCGCTCCAAACGG
GGCAGAAAGAAGCTCCTGTATATATTCAAACAACCATTTATGAGACCAGTACAAACT
ACTCAAGAGGAAGATGGCTGTAGCTGCCGATTTCCAGAAGAAGAAGAAGGAGGATG
TGAACTGTGATAA
47. Nucleic acid sequence of 28DCR4 (without signal peptide) (SEQ ID NO:47) 
CAGGTGCAGCTGGTGCAGTCTGGGGCTGAGGTGAAGAAGCCTGGGGCCTCAGTG
AAGGTCTCCTGCAAGGCTTCTGGATACACCTTCACCAGCTACTATATACACTGGGTGC
GACAGGCCCCTGGACAAGGGCTTGAGTGGATTGGATGTATTTATCCTGGAAATGTCA
ATACTAACTATAATGAGAAGTTCAAGGACAGGGCCACCCTGACCGTAGACACGTCCA
TCAGCACAGCCTACATGGAGCTGAGCAGGCTGAGATCTGACGACACGGCCGTGTATT
 
TCTGTACAAGATCACACTACGGCCTCGACTGGAACTTCGATGTCTGGGGCCAAGGGA
CCACGGTCACCGTCTCCTCAGGTGGAGGCGGTTCAGGCGGAGGTGGCAGCGGCGGTG
GCGGGTCGGACATCCAGATGACCCAGTCTCCATCCTCCCTGTCTGCATCTGTAGGAGA
CAGAGTCACCATCACTTGCCATGCCAGTCAAAACATTTATGTTTGGTTAAACTGGTAT
CAGCAGAAACCAGGGAAAGCCCCTAAGCTCCTGATCTATAAGGCTTCCAACCTGCAC
ACAGGGGTCCCATCAAGGTTCAGTGGCAGTGGATCTGGGACAGATTTCACTCTCACC
ATCAGCAGTCTGCAACCTGAAGATTTTGCAACTTACTACTGTCAACAGGGTCAAACTT
ATCCGTACACGTTCGGCGGAGGGACCAAGGTGGAGATCAAAGAGTCCAAATATGGTC
CCCCATGCCCACCATGCCCAATTGAAGTTATGTATCCTCCTCCTTACCTAGACAATGA
GAAGAGCAATGGAACCATTATCCATGTGAAAGGGAAACACCTTTGTCCAAGTCCCCT
ATTTCCCGGACCTTCTAAGCCCTTTTGGGTGCTGGTGGTTGGTGGAGTCCTGGCTT
GCTATAGCTTGCTAGTAACAGTGGCCTTTATTATTTTCTGGGTGAAACGGGGCAGAAA
GAAGCTCCTGTATATATTCAAACAACCATTTATGAGACCAGTACAAACTACTCAAGA
GGAAGATGGCTGTAGCTGCCGATTTCCAGAAGAAGAAGGAGGATGTGAACTGA
GGAGTAAGAGGAGCAGGCTCCTGCACAGTGACTACATGAACATGACTCCCCGCCGCC
CCGGGCCCACCCGCAAGCATTACCAGCCCTATGCCCCACCACGCGACTTCGCAGCCTA
TCGCTCCTGATAA
48. Amino acid sequence of Mucl G1 CAR (SEQ ID NO:48) 
MALPVTALLLPLALLLHAARPSEVQLQQSGGGLVQPGGSMKLSCVASGFTFSNYWM
NWVRQSPEKGLEWVAEIRLKSNNYATHYAESVKGRFTISRDDSKSSVYLQMNNLRAEDT
GIYYCTFGNSFAYWGQGTTVTVSSGGSGSGGSGSGGSGSDIVVTQESALTTSPGETVTLTC
RSSTGAVTTSNYANWVQEKPDHLFTGLIGGTNNRAPGVPARFSGSLIGDKAALTITGAQT
EDEAIYFCALWYSNHWVFGGGTKLTVLGSEESKYGPPCPPCPAPPVAGPSVFLFPPKPKDT
LMISRTPEVTCVVVDVSQEDPEVQFNWYVDGVEVHNAKTKPREEQFQSTYRVVSVLTVL
HQDWLNGKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVFSCSVM
HEALHNHYTQKSLSLSLGKFVPVFLPAKPTTTPAPRPPTPAPTIASQPLSLRPEACRPAAGG
AVHTRGLDFACDIYIWAPLAGTCGVLLLSLVITLYCNHKRGRKKLLYIFKQPFMRPVQTT
QEEDGCSCRFPEEEEGGCELRVKFSRSADAPAYQQGQNQLYNELNLGRREEYDVLDKRR
GRDPEMGGKPRRKNPQEGLYNELQKDKMAEAYSEIGMKGERRRGKGHDGLYQGLSTAT
KDTYDALHMQALPPR
49. Nucleic acid sequence of Mucl G1 CAR (SEQ ID NO:49) CCATGCCCACCATGCCCAGCACCTCCCGTGGCCGGACCATCAGTCTTCCTGTTCC 
CCCCAAAACCCAAGGACACTCTCATGATCTCCCGGACCCCTGAGGTCACGTGCGTGGT
GGTGGACGTGAGCCAGGAAGACCCCGAGGTCCAGTTCAACTGGTACGTGGATGGCGT
GGAGGTGCATAATGCCAAGACAAAGCCGCGGGAGGAGCAGTTCCAGAGCACGTACC
GTGTGGTCAGCGTCCTCACCGTCCTGCACCAGGACTGGCTGAACGGCAAGGAGTACA
AGTGCAAGGTCTCCAACAAAGGCCTCCCGTCCTCCATCGAGAAAACCATCTCCAAAG
CCAAAGGGCAGCCCCGAGAGCCACAGGTGTACACCCTGCCCCCATCCCAGGAGGAGA
TGACCAAGAACCAGGTCAGCCTGACCTGCCTGGTCAAAGGCTTCTACCCCAGCGACA
TCGCCGTGGAGTGGGAGAGCAATGGGCAGCCGGAGAACAACTACAAGACCACGCCT
CCCGTGCTGGACTCCGACGGCTCCTTCTTCCTCTACAGCAGGCTAACCGTGGACAAGA
GCAGGTGGCAGGAGGGGAATGTCTTCTCATGCTCCGTGATGCATGAGGCTCTGCACA
ACCACTACACACAGAAGAGCCTCTCCCTGTCTCTGGGTAAATTCGTGCCGGTCTTCCT
GCCAGCGAAGCCCACCACGACGCCAGCGCCGCGACCACCAACACCGGCGCCCACCAT
CGCGTCGCAGCCCCTGTCCCTGCGCCCAGAGGCGTGCCGGCCAGCGGCGGGGGGCGC
AGTGCACACGAGGGGGCTGGACTTCGCCTGTGATATCTACATCTGGGCGCCCCTGGCC
GGGACTTGTGGGGTCCTTCTCCTGTCACTGGTTATCACCCTTTACTGCAACCACAAAC
GGGGCAGAAAGAAGCTCCTGTATATATTCAAACAACCATTTATGAGACCAGTACAAA
CTACTCAAGAGGAAGATGGCTGTAGCTGCCGATTTCCAGAAGAAGAAGAAGGAGGAT
GTGAACTGAGAGTGAAGTTCAGCAGGAGCGCAGACGCCCCCGCGTACCAGCAGGGCC
AGAACCAGCTCTATAACGAGCTCAATCTAGGACGAAGAGAGGAGTACGATGTTTTGG
ACAAGAGACGTGGCCGGGACCCTGAGATGGGGGGAAAGCCGAGAAGGAAGAACCCT
CAGGAAGGCCTGTACAATGAACTGCAGAAAGATAAGATGGCGGAGGCCTACAGTGA
GATTGGGATGAAAGGCGAGCGCCGGAGGGGCAAGGGGCACGATGGCCTTTACCAGG
GTCTCAGTACAGCCACCAAGGACACCTACGACGCCCTTCACATGCAGGCCCTGCCCCC
TCGC
50. Nucleotide sequence of Mucl G2 CAR (SEQ ID NO:50) 
ATGGCCTTACCAGTGACCGCCTTGCTCCTGCCGCTGGCCTTGCTGCTCCACGCCG
CCAGGCCGAGCGAGGTCCAGCTGCAGCAGTCAGGAGGAGGCTTGGTGCAACCTGGAG
GATCCATGAAACTCTCCTGTGTTGCCTCTGGATTCACTTTCAGTAACTACTGGATGAA
CTGGGTCCGCCAGTCTCCAGAGAAGGGGCTTGAGTGGGTTGCTGAAATTAGATTGAA
ATCTAATAATTATGCAACACATTATGCGGAGTCTGTGAAAGGGAGGTTCACCATCTCA
AGAGATGATTCCAAAAGTAGTGTCTACCTGCAAATGAACAACTTAAGAGCTGAAGAC
ACTGGCATTTATTACTGTACCTTTGGTAACTCCTTTGCTTACTGGGGCCAAGGGACCA
CGGTCACCGTCTCCTCAGGTGGTTCTGGTTCTGGCGGCTCCGGTTCCGGTGGATCCGG
CTCTGATATCGTTGTGACTCAGGAATCTGCACTCACCACATCACCTGGTGAAACAGTC
 
ACACTCACTTGTCGCTCAAGTACTGGGGCTGTTACAACTAGTAACTATGCCAACTGGG
TCCAAGAAAAACCAGATCATTTATTCACTGGTCTAATAGGTGGTACCAACAACCGAG
CACCAGGTGTTCCTGCCAGATTCTCAGGCTCCCTGATTGGAGACAAGGCTGCCCTCAC
CATCACAGGGGCACAGACTGAGGATGAGGCAATATATTTCTGTGCTCTATGGTACAG
CAACCATTGGGTGTTCGGTGGAGGAACCAAACTGACTGTCCTAGGATCCGAGGAGTC
CAAATATGGTCCCCCATGCCCACCATGCCCAGCACCTCCCGTGGCCGGACCATCAGTC
TTCCTGTTCCCCCCAAAACCCAAGGACACTCTCATGATCTCCCGGACCCCTGAGGTCA
CGTGCGTGGTGGTGGACGTGAGCCAGGAAGACCCCGAGGTCCAGTTCAACTGGTACG
TGGATGGCGTGGAGGTGCATAATGCCAAGACAAAGCCGCGGGAGGAGCAGTTCCAG
AGCACGTACCGTGTGGTCAGCGTCCTCACCGTCCTGCACCAGGACTGGCTGAACGGC
AAGGAGTACAAGTGCAAGGTCTCCAACAAAGGCCTCCCGTCCTCCATCGAGAAAACC
ATCTCCAAAGCCAAAGGGCAGCCCCGAGAGCCACAGGTGTACACCCTGCCCCCATCC
CAGGAGGAGATGACCAAGAACCAGGTCAGCCTGACCTGCCTGGTCAAAGGCTTCTAC
CCCAGCGACATCGCCGTGGAGTGGGAGAGCAATGGGCAGCCGGAGAACAACTACAA
GACCACGCCTCCCGTGCTGGACTCCGACGGCTCCTTCTTCCTCTACAGCAGGCTAACC
GTGGACAAGAGCAGGTGGCAGGAGGGGAATGTCTTCTCATGCTCCGTGATGCATGAG
GCTCTGCACAACCACTACACACAGAAGAGCCTCTCCCTGTCTCTGGGTAAATTCGTGC
CGGTCTTCCTGCCAGCGAAGCCCACCACGACGCCAGCGCCGCGACCACCAACACCGG
CGCCCACCATCGCGTCGCAGCCCCTGTCCCTGCGCCCAGAGGCGTGCCGGCCAGCGG
CGGGGGGCGCAGTGCACACGAGGGGGCTGGACTTCGCCTGTGATATCTACATCTGGG
CGCCCCTGGCCGGGACTTGTGGGGTCCTTCTCCTGTCACTGGTTATCACCCTTTACTGC
AACCACAAACGGGGCAGAAAGAAGCTCCTGTATATATTCAAACAACCATTTATGAGA
CCAGTACAAACTACTCAAGAGGAAGATGGCTGTAGCTGCCGATTTCCAGAAGAAGAA
GAAGGAGGATGTGAACTGAGAGTGAAGTTCAGCAGGAGCGCAGACGCCCCCGCGTA
CCAGCAGGGCCAGAACCAGCTCTATAACGAGCTCAATCTAGGACGAAGAGAGGAGT
ACGATGTTTTGGACAAGAGACGTGGCCGGGACCCTGAGATGGGGGGAAAGCCGAGA
AGGAAGAACCCTCAGGAAGGCCTGTACAATGAACTGCAGAAAGATAAGATGGCGGA
GGCCTACAGTGAGATTGGGATGAAAGGCGAGCGCCGGAGGGGCAAGGGGCACGATG
GCCTTTACCAGGGTCTCAGTACAGCCACCAAGGACACCTACGACGCCCTTCACATGCA
GGCCCTGCCCCCTCGC
51. Nucleotide sequence of primer CD28-F (SEQ ID NO: 51)
   GCTTCTGGATACACCTTC
52. Nucleotide sequence of primer CD28-R (SEQ ID NO: 52)
   CCTTGAACTTCTCATTATAGTTAG
53. Nucleotide sequence in probe Taqman (SEQ ID NO: 53)
   AATACATCCAATCCACTCAAGCC
54. Amino acid sequence of CD8 signal peptide (SEQ ID NO:54)
   MALPVTALLLPLALLLHAARPS
55. Amino acid sequence of CD40 extracellular agonistic single-chain antibody (SEQ ID NO: 55) 
QVQLVQSGAEVKKPGASVKVSCKASGYTFTGYYMHWVRQAPGQGLEWMGWINPD
SGGTNYAQKFQGRVTMTRDTSISTAYMELNRLRSDDTAVYYCARDQPLGYCTNGVCSYF
DYWGQGTLVTVSSGGGGSGGGGSGGGGSDIQMTQSPSSVSASVGDRVTITCRASQGIYS
WLAWYQQKPGKAPNLLIYTASTLQSGVPSRFSGSGSGTDFTLTISSLQPEDFATYYCQQA
NIFPLTFGGGTKVEIK
56. Amino acid sequence of IgG4Fc CH2CH3 hinge region (SEQ ID NO:56) 
ESKYGPPCPPCPAPEFLGGPSVFLFPPKPKDTLMISRTPEVTCVVVDVSQEDPEVQFN
WYVDGVEVHNAKTKPREEQFNSTYRVVSVLTVLHQDWLNGKEYKCKVSNKGLPSSIEK
TISKAKGQPREPQVYTLPPSQEEMTKNQVSLTCLVKGFYPSDIAVEWESNGQPENNYKTT
PPVLDSDGSFFLYSRLTVDKSRWQEGNVFSCSVMHEALHNHYTQKSLSLSLGK
57. Amino acid sequence of 40DCR1 (including signal peptide) (SEQ ID NO:57) 
MALPVTALLLPLALLLHAARPSQVQLVQSGAEVKKPGASVKVSCKASGYTFTGYYM
HWVRQAPGQGLEWMGWINPDSGGTNYAQKFQGRVTMTRDTSISTAYMELNRLRSDDT
AVYYCARDQPLGYCTNGVCSYFDYWGQGTLVTVSSGGGGSGGGGSGGGGSDIQMTQSP
SSVSASVGDRVTITCRASQGIYSWLAWYQQKPGKAPNLLIYTASTLQSGVPSRFSGSGSGT
DFTLTISSLQPEDFATYYCQQANIFPLTFGGGTKVEIKESKYGPPCPPCPAPEFLGGPSVFLF
PPKPKDTLMISRTPEVTCVVVDVSQEDPEVQFNWYVDGVEVHNAKTKPREEQFNSTYRV
VSVLTVLHQDWLNGKEYKCKVSNKGLPSSIEKTISKAKGQPREPQVYTLPPSQEEMTKNQ
VSLTCLVKGFYPSDIAVEWESNGQPENNYKTTPPVLDSDGSFFLYSRLTVDKSRWQEGNV
FSCSVMHEALHNHYTQKSLSLSLGKPFWVLVVVGGVLACYSLLVTVAFIIFWVRSKRSRL
LHSDYMNMTPRRPGPTRKHYQPYAPPRDFAAYRS
58. Amino acid sequence of 40DCR2 (including signal peptide) (SEQ ID NO:58) 
MALPVTALLLPLALLLHAARPSQVQLVQSGAEVKKPGASVKVSCKASGYTFTGYYM
HWVRQAPGQGLEWMGWINPDSGGTNYAQKFQGRVTMTRDTSISTAYMELNRLRSDDT
AVYYCARDQPLGYCTNGVCSYFDYWGQGTLVTVSSGGGGSGGGGSGGGGSDIQMTQSP
SSVSASVGDRVTITCRASQGIYSWLAWYQQKPGKAPNLLIYTASTLQSGVPSRFSGSGSGT
DFTLTISSLQPEDFATYYCQQANIFPLTFGGGTKVEIKESKYGPPCPPCPAPEFLGGPSVFLF
PPKPKDTLMISRTPEVTCVVVDVSQEDPEVQFNWYVDGVEVHNAKTKPREEQFNSTYRV
VSVLTVLHQDWLNGKEYKCKVSNKGLPSSIEKTISKAKGQPREPQVYTLPPSQEEMTKNQ
VSLTCLVKGFYPSDIAVEWESNGQPENNYKTTPPVLDSDGSFFLYSRLTVDKSRWQEGNV
FSCSVMHEALHNHYTQKSLSLSLGKPFWVLVVVGGVLACYSLLVTVAFIIFWVKRGRKK
LLYIFKQPFMRPVQTTQEEDGCSCRFPEEEEGGCEL
59. Amino acid sequence of 40DCR3 (including signal peptide) (SEQ ID NO:59) 
MALPVTALLLPLALLLHAARPSQVQLVQSGAEVKKPGASVKVSCKASGYTFTGYYM
HWVRQAPGQGLEWMGWINPDSGGTNYAQKFQGRVTMTRDTSISTAYMELNRLRSDDT
AVYYCARDQPLGYCTNGVCSYFDYWGQGTLVTVSSGGGGSGGGGSGGGGSDIQMTQSP
SSVSASVGDRVTITCRASQGIYSWLAWYQQKPGKAPNLLIYTASTLQSGVPSRFSGSGSGT
DFTLTISSLQPEDFATYYCQQANIFPLTFGGGTKVEIKESKYGPPCPPCPAPEFLGGPSVFLF
PPKPKDTLMISRTPEVTCVVVDVSQEDPEVQFNWYVDGVEVHNAKTKPREEQFNSTYRV
VSVLTVLHQDWLNGKEYKCKVSNKGLPSSIEKTISKAKGQPREPQVYTLPPSQEEMTKNQ
VSLTCLVKGFYPSDIAVEWESNGQPENNYKTTPPVLDSDGSFFLYSRLTVDKSRWQEGNV
FSCSVMHEALHNHYTQKSLSLSLGKPFWVLVVVGGVLACYSLLVTVAFIIFWVRSKRSRL
LHSDYMNMTPRRPGPTRKHYQPYAPPRDFAAYRSKRGRKKLLYIFKQPFMRPVQTTQEE
DGCSCRFPEEEEGGCEL
60. Amino acid sequence of 40DCR4 (including signal peptide) (SEQ ID NO:60) 
MALPVTALLLPLALLLHAARPSQVQLVQSGAEVKKPGASVKVSCKASGYTFTGYYM
HWVRQAPGQGLEWMGWINPDSGGTNYAQKFQGRVTMTRDTSISTAYMELNRLRSDDT
AVYYCARDQPLGYCTNGVCSYFDYWGQGTLVTVSSGGGGSGGGGSGGGGSDIQMTQSP
SSVSASVGDRVTITCRASQGIYSWLAWYQQKPGKAPNLLIYTASTLQSGVPSRFSGSGSGT
DFTLTISSLQPEDFATYYCQQANIFPLTFGGGTKVEIKESKYGPPCPPCPAPEFLGGPSVFLF
PPKPKDTLMISRTPEVTCVVVDVSQEDPEVQFNWYVDGVEVHNAKTKPREEQFNSTYRV
VSVLTVLHQDWLNGKEYKCKVSNKGLPSSIEKTISKAKGQPREPQVYTLPPSQEEMTKNQ
VSLTCLVKGFYPSDIAVEWESNGQPENNYKTTPPVLDSDGSFFLYSRLTVDKSRWQEGNV
FSCSVMHEALHNHYTQKSLSLSLGKPFWVLVVVGGVLACYSLLVTVAFIIFWVKRGRKK
LLYIFKQPFMRPVQTTQEEDGCSCRFPEEEEGGCELRSKRSRLLHSDYMNMTPRRPGPTR
 KHYQPYAPPRDFAAYRS
61. Amino acid sequence of 40DCR1 (without signal peptide) (SEQ ID NO:61) 
QVQLVQSGAEVKKPGASVKVSCKASGYTFTGYYMHWVRQAPGQGLEWMGWINPD
SGGTNYAQKFQGRVTMTRDTSISTAYMELNRLRSDDTAVYYCARDQPLGYCTNGVCSYF
DYWGQGTLVTVSSGGGGSGGGGSGGGGSDIQMTQSPSSVSASVGDRVTITCRASQGIYS
WLAWYQQKPGKAPNLLIYTASTLQSGVPSRFSGSGSGTDFTLTISSLQPEDFATYYCQQA
NIFPLTFGGGTKVEIKESKYGPPCPPCPAPEFLGGPSVFLFPPKPKDTLMISRTPEVTCVVVD
VSQEDPEVQFNWYVDGVEVHNAKTKPREEQFNSTYRVVSVLTVLHQDWLNGKEYKCK
VSNKGLPSSIEKTISKAKGQPREPQVYTLPPSQEEMTKNQVSLTCLVKGFYPSDIAVEWES
NGQPENNYKTTPPVLDSDGSFFLYSRLTVDKSRWQEGNVFSCSVMHEALHNHYTQKSLS
LSLGKPFWVLVVVGGVLACYSLLVTVAFIIFWVRSKRSRLLHSDYMNMTPRRPGPTRKH
YQPYAPPRDFAAYRS
62. Amino acid sequence of 40DCR2 (without signal peptide) (SEQ ID NO:62) 
QVQLVQSGAEVKKPGASVKVSCKASGYTFTGYYMHWVRQAPGQGLEWMGWINPD
SGGTNYAQKFQGRVTMTRDTSISTAYMELNRLRSDDTAVYYCARDQPLGYCTNGVCSYF
DYWGQGTLVTVSSGGGGSGGGGSGGGGSDIQMTQSPSSVSASVGDRVTITCRASQGIYS
WLAWYQQKPGKAPNLLIYTASTLQSGVPSRFSGSGSGTDFTLTISSLQPEDFATYYCQQA
NIFPLTFGGGTKVEIKESKYGPPCPPCPAPEFLGGPSVFLFPPKPKDTLMISRTPEVTCVVVD
VSQEDPEVQFNWYVDGVEVHNAKTKPREEQFNSTYRVVSVLTVLHQDWLNGKEYKCK
VSNKGLPSSIEKTISKAKGQPREPQVYTLPPSQEEMTKNQVSLTCLVKGFYPSDIAVEWES
NGQPENNYKTTPPVLDSDGSFFLYSRLTVDKSRWQEGNVFSCSVMHEALHNHYTQKSLS
LSLGKPFWVLVVVGGVLACYSLLVTVAFIIFWVKRGRKKLLYIFKQPFMRPVQTTQEEDG
CSCRFPEEEEGGCEL
63. Amino acid sequence of 40DCR3 (without signal peptide) (SEQ ID NO:63) 
QVQLVQSGAEVKKPGASVKVSCKASGYTFTGYYMHWVRQAPGQGLEWMGWINPD
SGGTNYAQKFQGRVTMTRDTSISTAYMELNRLRSDDTAVYYCARDQPLGYCTNGVCSYF
DYWGQGTLVTVSSGGGGSGGGGSGGGGSDIQMTQSPSSVSASVGDRVTITCRASQGIYS
WLAWYQQKPGKAPNLLIYTASTLQSGVPSRFSGSGSGTDFTLTISSLQPEDFATYYCQQA
NIFPLTFGGGTKVEIKESKYGPPCPPCPAPEFLGGPSVFLFPPKPKDTLMISRTPEVTCVVVD
VSQEDPEVQFNWYVDGVEVHNAKTKPREEQFNSTYRVVSVLTVLHQDWLNGKEYKCK
VSNKGLPSSIEKTISKAKGQPREPQVYTLPPSQEEMTKNQVSLTCLVKGFYPSDIAVEWES
NGQPENNYKTTPPVLDSDGSFFLYSRLTVDKSRWQEGNVFSCSVMHEALHNHYTQKSLS
 
LSLGKPFWVLVVVGGVLACYSLLVTVAFIIFWVRSKRSRLLHSDYMNMTPRRPGPTRKH
YQPYAPPRDFAAYRSKRGRKKLLYIFKQPFMRPVQTTQEEDGCSCRFPEEEEGGCEL
64. Amino acid sequence of 40DCR4 (without signal peptide) (SEQ ID NO:64) 
QVQLVQSGAEVKKPGASVKVSCKASGYTFTGYYMHWVRQAPGQGLEWMGWINPD
SGGTNYAQKFQGRVTMTRDTSISTAYMELNRLRSDDTAVYYCARDQPLGYCTNGVCSYF
DYWGQGTLVTVSSGGGGSGGGGSGGGGSDIQMTQSPSSVSASVGDRVTITCRASQGIYS
WLAWYQQKPGKAPNLLIYTASTLQSGVPSRFSGSGSGTDFTLTISSLQPEDFATYYCQQA
NIFPLTFGGGTKVEIKESKYGPPCPPCPAPEFLGGPSVFLFPPKPKDTLMISRTPEVTCVVVD
VSQEDPEVQFNWYVDGVEVHNAKTKPREEQFNSTYRVVSVLTVLHQDWLNGKEYKCK
VSNKGLPSSIEKTISKAKGQPREPQVYTLPPSQEEMTKNQVSLTCLVKGFYPSDIAVEWES
NGQPENNYKTTPPVLDSDGSFFLYSRLTVDKSRWQEGNVFSCSVMHEALHNHYTQKSLS
LSLGKPFWVLVVVGGVLACYSLLVTVAFIIFWVKRGRKKLLYIFKQPFMRPVQTTQEEDG
CSCRFPEEEEGGCELRSKRSRLLHSDYMNMTPRRPGPTRKHYQPYAPPRDFAAYRS
65. Nucleic acid sequence of 40DCR1 (including signal peptide) (SEQ ID NO:65) 
ATGGCCTTACCAGTGACCGCCTTGCTCCTGCCGCTGGCCTTGCTGCTCCACGCCG
CCAGGCCGAGCCAGGTGCAGCTGGTGCAGTCTGGGGCTGAGGTGAAGAAGCCTGGGG
CCTCAGTGAAGGTCTCCTGCAAGGCTTCTGGATACACCTTCACCGGCTACTATATGCA
CTGGGTGCGACAGGCCCCTGGACAAGGGCTTGAGTGGATGGGATGGATCAACCCTGA
CAGTGGTGGCACAAACTATGCACAGAAGTTTCAGGGCAGGGTCACCATGACCAGGGA
CACGTCCATCAGCACAGCCTACATGGAGCTGAACAGGCTGAGATCTGACGACACGGC
CGTGTATTACTGTGCGAGAGATCAGCCCCTAGGATATTGTACTAATGGTGTATGCTCC
TACTTTGACTACTGGGGCCAGGGAACCCTGGTCACCGTCTCCTCAGGTGGAGGCGGTT
CAGGCGGAGGTGGCAGCGGCGGTGGCGGGTCGGACATCCAGATGACCCAGTCTCCAT
CTTCCGTGTCTGCATCTGTAGGAGACAGAGTCACCATCACTTGTCGGGCGAGTCAGGG
TATTTACAGCTGGTTAGCCTGGTATCAGCAGAAACCAGGGAAAGCCCCTAACCTCCTG
ATCTATACTGCATCCACTTTACAAAGTGGGGTCCCATCAAGGTTCAGCGGCAGTGGAT
CTGGGACAGATTTCACTCTCACCATCAGCAGCCTGCAACCTGAAGATTTTGCAACTTA
CTATTGTCAACAGGCTAACATTTTCCCGCTCACTTTCGGCGGAGGGACCAAGGTGGAG
ATCAAAGAGTCCAAATATGGTCCCCCATGCCCACCATGCCCAGCACCTGAGTTCCTGG
GGGGACCATCAGTCTTCCTGTTCCCCCCAAAACCCAAGGACACTCTCATGATCTCCCG
GACCCCTGAGGTCACGTGCGTGGTGGTGGACGTGAGCCAGGAAGACCCCGAGGTCCA
GTTCAACTGGTACGTGGATGGCGTGGAGGTGCATAATGCCAAGACAAAGCCGCGGGA
GGAGCAGTTCAACAGCACGTACCGTGTGGTCAGCGTCCTCACCGTCCTGCACCAGGA
 
CTGGCTGAACGGCAAGGAGTACAAGTGCAAGGTCTCCAACAAAGGCCTCCCGTCCTC
CATCGAGAAAACCATCTCCAAAGCCAAAGGGCAGCCCCGAGAGCCACAGGTGTACAC
CCTGCCCCCATCCCAGGAGGAGATGACCAAGAACCAGGTCAGCCTGACCTGCCTGGT
CAAAGGCTTCTACCCCAGCGACATCGCCGTGGAGTGGGAGAGCAATGGGCAGCCGGA
GAACAACTACAAGACCACGCCTCCCGTGCTGGACTCCGACGGCTCCTTCTTCCTCTAC
AGCAGGCTAACCGTGGACAAGAGCAGGTGGCAGGAGGGGAATGTCTTCTCATGCTCC
GTGATGCATGAGGCTCTGCACAACCACTACACACAGAAGAGCCTCTCCCTGTCTCTGG
GTAAACCCTTTTGGGTGCTGGTGGTGGTTGGTGGAGTCCTGGCTTGCTATAGCTTGCT
AGTAACAGTGGCCTTTATTATTTTCTGGGTGAGGAGTAAGAGGAGCAGGCTCCTGCAC
AGTGACTACATGAACATGACTCCCCGCCGCCCCGGGCCCACCCGCAAGCATTACCAG
CCCTATGCCCCACCACGCGACTTCGCAGCCTATCGCTCC
66. Nucleic acid sequence of 40DCR2 (including signal peptide) (SEQ ID NO:66) 
ATGGCCTTACCAGTGACCGCCTTGCTCCTGCCGCTGGCCTTGCTGCTCCACGCCG
CCAGGCCGAGCCAGGTGCAGCTGGTGCAGTCTGGGGCTGAGGTGAAGAAGCCTGGGG
CCTCAGTGAAGGTCTCCTGCAAGGCTTCTGGATACACCTTCACCGGCTACTATATGCA
CTGGGTGCGACAGGCCCCTGGACAAGGGCTTGAGTGGATGGGATGGATCAACCCTGA
CAGTGGTGGCACAAACTATGCACAGAAGTTTCAGGGCAGGGTCACCATGACCAGGGA
CACGTCCATCAGCACAGCCTACATGGAGCTGAACAGGCTGAGATCTGACGACACGGC
CGTGTATTACTGTGCGAGAGATCAGCCCCTAGGATATTGTACTAATGGTGTATGCTCC
TACTTTGACTACTGGGGCCAGGGAACCCTGGTCACCGTCTCCTCAGGTGGAGGCGGTT
CAGGCGGAGGTGGCAGCGGCGGTGGCGGGTCGGACATCCAGATGACCCAGTCTCCAT
CTTCCGTGTCTGCATCTGTAGGAGACAGAGTCACCATCACTTGTCGGGCGAGTCAGGG
TATTTACAGCTGGTTAGCCTGGTATCAGCAGAAACCAGGGAAAGCCCCTAACCTCCTG
ATCTATACTGCATCCACTTTACAAAGTGGGGTCCCATCAAGGTTCAGCGGCAGTGGAT
CTGGGACAGATTTCACTCTCACCATCAGCAGCCTGCAACCTGAAGATTTTGCAACTTA
CTATTGTCAACAGGCTAACATTTTCCCGCTCACTTTCGGCGGAGGGACCAAGGTGGAG
ATCAAAGAGTCCAAATATGGTCCCCCATGCCCACCATGCCCAGCACCTGAGTTCCTGG
GGGGACCATCAGTCTTCCTGTTCCCCCCAAAACCCAAGGACACTCTCATGATCTCCCG
GACCCCTGAGGTCACGTGCGTGGTGGTGGACGTGAGCCAGGAAGACCCCGAGGTCCA
GTTCAACTGGTACGTGGATGGCGTGGAGGTGCATAATGCCAAGACAAAGCCGCGGGA
GGAGCAGTTCAACAGCACGTACCGTGTGGTCAGCGTCCTCACCGTCCTGCACCAGGA
CTGGCTGAACGGCAAGGAGTACAAGTGCAAGGTCTCCAACAAAGGCCTCCCGTCCTC
CATCGAGAAAACCATCTCCAAAGCCAAAGGGCAGCCCCGAGAGCCACAGGTGTACAC
CCTGCCCCCATCCCAGGAGGAGATGACCAAGAACCAGGTCAGCCTGACCTGCCTGGT
 
CAAAGGCTTCTACCCCAGCGACATCGCCGTGGAGTGGGAGAGCAATGGGCAGCCGGA
GAACAACTACAAGACCACGCCTCCCGTGCTGGACTCCGACGGCTCCTTCTTCCTCTAC
AGCAGGCTAACCGTGGACAAGAGCAGGTGGCAGGAGGGGAATGTCTTCTCATGCTCC
GTGATGCATGAGGCTCTGCACAACCACTACACACAGAAGAGCCTCTCCCTGTCTCTGG
GTAAACCCTTTTGGGTGCTGGTGGTGGTTGGTGGAGTCCTGGCTTGCTATAGCTTGCT
AGTAACAGTGGCCTTTATTATTTTCTGGGTGAGGAGTAAGAGGAGCAGGCTCCTGCAC
AGTGACTACATGAACATGACTCCCCGCCGCCCCGGGCCCACCCGCAAGCATTACCAG
CCCTATGCCCCACCACGCGACTTCGCAGCCTATCGCTCC
67. Nucleic acid sequence of 40DCR3 (including signal peptide) (SEQ ID NO:67) 
ATGGCCTTACCAGTGACCGCCTTGCTCCTGCCGCTGGCCTTGCTGCTCCACGCCG
CCAGGCCGAGCCAGGTGCAGCTGGTGCAGTCTGGGGCTGAGGTGAAGAAGCCTGGGG
CCTCAGTGAAGGTCTCCTGCAAGGCTTCTGGATACACCTTCACCGGCTACTATATGCA
CTGGGTGCGACAGGCCCCTGGACAAGGGCTTGAGTGGATGGGATGGATCAACCCTGA
CAGTGGTGGCACAAACTATGCACAGAAGTTTCAGGGCAGGGTCACCATGACCAGGGA
CACGTCCATCAGCACAGCCTACATGGAGCTGAACAGGCTGAGATCTGACGACACGGC
CGTGTATTACTGTGCGAGAGATCAGCCCCTAGGATATTGTACTAATGGTGTATGCTCC
TACTTTGACTACTGGGGCCAGGGAACCCTGGTCACCGTCTCCTCAGGTGGAGGCGGTT
CAGGCGGAGGTGGCAGCGGCGGTGGCGGGTCGGACATCCAGATGACCCAGTCTCCAT
CTTCCGTGTCTGCATCTGTAGGAGACAGAGTCACCATCACTTGTCGGGCGAGTCAGGG
TATTTACAGCTGGTTAGCCTGGTATCAGCAGAAACCAGGGAAAGCCCCTAACCTCCTG
ATCTATACTGCATCCACTTTACAAAGTGGGGTCCCATCAAGGTTCAGCGGCAGTGGAT
CTGGGACAGATTTCACTCTCACCATCAGCAGCCTGCAACCTGAAGATTTTGCAACTTA
CTATTGTCAACAGGCTAACATTTTCCCGCTCACTTTCGGCGGAGGGACCAAGGTGGAG
ATCAAAGAGTCCAAATATGGTCCCCCATGCCCACCATGCCCAGCACCTGAGTTCCTGG
GGGGACCATCAGTCTTCCTGTTCCCCCCAAAACCCAAGGACACTCTCATGATCTCCCG
GACCCCTGAGGTCACGTGCGTGGTGGTGGACGTGAGCCAGGAAGACCCCGAGGTCCA
GTTCAACTGGTACGTGGATGGCGTGGAGGTGCATAATGCCAAGACAAAGCCGCGGGA
GGAGCAGTTCAACAGCACGTACCGTGTGGTCAGCGTCCTCACCGTCCTGCACCAGGA
CTGGCTGAACGGCAAGGAGTACAAGTGCAAGGTCTCCAACAAAGGCCTCCCGTCCTC
CATCGAGAAAACCATCTCCAAAGCCAAAGGGCAGCCCCGAGAGCCACAGGTGTACAC
CCTGCCCCCATCCCAGGAGGAGATGACCAAGAACCAGGTCAGCCTGACCTGCCTGGT
CAAAGGCTTCTACCCCAGCGACATCGCCGTGGAGTGGGAGAGCAATGGGCAGCCGGA
GAACAACTACAAGACCACGCCTCCCGTGCTGGACTCCGACGGCTCCTTCTTCCTCTAC
AGCAGGCTAACCGTGGACAAGAGCAGGTGGCAGGAGGGGAATGTCTTCTCATGCTCC
 
GTGATGCATGAGGCTCTGCACAACCACTACACACAGAAGAGCCTCTCCCTGTCTCTGG
GTAAACCCTTTTGGGTGCTGGTGGTGGTTGGTGGAGTCCTGGCTTGCTATAGCTTGCT
AGTAACAGTGGCCTTTATTATTTTCTGGGTGAGGAGTAAGAGGAGCAGGCTCCTGCAC
AGTGACTACATGAACATGACTCCCCGCCGCCCCGGGCCCACCCGCAAGCATTACCAG
CCCTATGCCCCACCACGCGACTTCGCAGCCTATCGCTCCAAACGGGGCAGAAAGAAG
CTCCTGTATATATTCAAACAACCATTTATGAGACCAGTACAAACTACTCAAGAGGAA
GATGGCTGTAGCTGCCGATTTCCAGAAGAAGAAGAAGGAGGATGTGAACTG
68. Nucleic acid sequence of 40DCR4 (including signal peptide) (SEQ ID NO:68) 
ATGGCCTTACCAGTGACCGCCTTGCTCCTGCCGCTGGCCTTGCTGCTCCACGCCG
CCAGGCCGAGCCAGGTGCAGCTGGTGCAGTCTGGGGCTGAGGTGAAGAAGCCTGGGG
CCTCAGTGAAGGTCTCCTGCAAGGCTTCTGGATACACCTTCACCGGCTACTATATGCA
CTGGGTGCGACAGGCCCCTGGACAAGGGCTTGAGTGGATGGGATGGATCAACCCTGA
CAGTGGTGGCACAAACTATGCACAGAAGTTTCAGGGCAGGGTCACCATGACCAGGGA
CACGTCCATCAGCACAGCCTACATGGAGCTGAACAGGCTGAGATCTGACGACACGGC
CGTGTATTACTGTGCGAGAGATCAGCCCCTAGGATATTGTACTAATGGTGTATGCTCC
TACTTTGACTACTGGGGCCAGGGAACCCTGGTCACCGTCTCCTCAGGTGGAGGCGGTT
CAGGCGGAGGTGGCAGCGGCGGTGGCGGGTCGGACATCCAGATGACCCAGTCTCCAT
CTTCCGTGTCTGCATCTGTAGGAGACAGAGTCACCATCACTTGTCGGGCGAGTCAGGG
TATTTACAGCTGGTTAGCCTGGTATCAGCAGAAACCAGGGAAAGCCCCTAACCTCCTG
ATCTATACTGCATCCACTTTACAAAGTGGGGTCCCATCAAGGTTCAGCGGCAGTGGAT
CTGGGACAGATTTCACTCTCACCATCAGCAGCCTGCAACCTGAAGATTTTGCAACTTA
CTATTGTCAACAGGCTAACATTTTCCCGCTCACTTTCGGCGGAGGGACCAAGGTGGAG
ATCAAAGAGTCCAAATATGGTCCCCCATGCCCACCATGCCCAGCACCTGAGTTCCTGG
GGGGACCATCAGTCTTCCTGTTCCCCCCAAAACCCAAGGACACTCTCATGATCTCCCG
GACCCCTGAGGTCACGTGCGTGGTGGTGGACGTGAGCCAGGAAGACCCCGAGGTCCA
GTTCAACTGGTACGTGGATGGCGTGGAGGTGCATAATGCCAAGACAAAGCCGCGGGA
GGAGCAGTTCAACAGCACGTACCGTGTGGTCAGCGTCCTCACCGTCCTGCACCAGGA
CTGGCTGAACGGCAAGGAGTACAAGTGCAAGGTCTCCAACAAAGGCCTCCCGTCCTC
CATCGAGAAAACCATCTCCAAAGCCAAAGGGCAGCCCCGAGAGCCACAGGTGTACAC
CCTGCCCCCATCCCAGGAGGAGATGACCAAGAACCAGGTCAGCCTGACCTGCCTGGT
CAAAGGCTTCTACCCCAGCGACATCGCCGTGGAGTGGGAGAGCAATGGGCAGCCGGA
GAACAACTACAAGACCACGCCTCCCGTGCTGGACTCCGACGGCTCCTTCTTCCTCTAC
AGCAGGCTAACCGTGGACAAGAGCAGGTGGCAGGAGGGGAATGTCTTCTCATGCTCC
GTGATGCATGAGGCTCTGCACAACCACTACACACAGAAGAGCCTCTCCCTGTCTCTGG
 
GTAAACCCTTTTGGGTGCTGGTGGTGGTTGGTGGAGTCCTGGCTTGCTATAGCTTGCT
AGTAACAGTGGCCTTTATTATTTCTGGGTGAAACGGGGCAGAAAGAAGCTCCTGTAT
ATATTCAAACAACCATTTATGAGACCAGTACAAACTACTCAAGAGGAAGATGGCTGT
AGCTGCCGATTTCCAGAAGAAGAAGAAGGAGGATGTGAACTGAGGAGTAAGAGGAG
CAGGCTCCTGCACAGTGACTACATGAACATGACTCCCCGCCGCCCCGGGCCCACCCG
CAAGCATTACCAGCCCTATGCCCCACCACGCGACTTCGCAGCCTATCGCTCC
69. Nucleic acid sequence of 40DCR1 (without signal peptide) (SEQ ID NO:69) 
CAGGTGCAGCTGGTGCAGTCTGGGGCTGAGGTGAAGAAGCCTGGGGCCTCAGTG
AAGGTCTCCTGCAAGGCTTCTGGATACACCTTCACCGGCTACTATATGCACTGGGTGC
GACAGGCCCCTGGACAAGGGCTTGAGTGGATGGGATGGATCAACCCTGACAGTGGTG
GCACAAACTATGCACAGAAGTTTCAGGGCAGGGTCACCATGACCAGGGACACGTCCA
TCAGCACAGCCTACATGGAGCTGAACAGGCTGAGATCTGACGACACGGCCGTGTATT
ACTGTGCGAGAGATCAGCCCCTAGGATATTGTACTAATGGTGTATGCTCCTACTTTGA
CTACTGGGGCCAGGGAACCCTGGTCACCGTCTCCTCAGGTGGAGGCGGTTCAGGCGG
AGGTGGCAGCGGCGGTGGCGGGTCGGACATCCAGATGACCCAGTCTCCATCTTCCGT
GTCTGCATCTGTAGGAGACAGAGTCACCATCACTTGTCGGGCGAGTCAGGGTATTTAC
AGCTGGTTAGCCTGGTATCAGCAGAAACCAGGGAAAGCCCCTAACCTCCTGATCTAT
ACTGCATCCACTTTACAAAGTGGGGTCCCATCAAGGTTCAGCGGCAGTGGATCTGGG
ACAGATTTCACTCTCACCATCAGCAGCCTGCAACCTGAAGATTTTGCAACTTACTATT
GTCAACAGGCTAACATTTTCCCGCTCACTTTCGGCGGAGGGACCAAGGTGGAGATCA
AAGAGTCCAAATATGGTCCCCCATGCCCACCATGCCCAGCACCTGAGTTCCTGGGGG
GACCATCAGTCTTCCTGTTCCCCCCAAAACCCAAGGACACTCTCATGATCTCCCGGAC
CCCTGAGGTCACGTGCGTGGTGGTGGACGTGAGCCAGGAAGACCCCGAGGTCCAGTT
CAACTGGTACGTGGATGGCGTGGAGGTGCATAATGCCAAGACAAAGCCGCGGGAGG
AGCAGTTCAACAGCACGTACCGTGTGGTCAGCGTCCTCACCGTCCTGCACCAGGACTG
GCTGAACGGCAAGGAGTACAAGTGCAAGGTCTCCAACAAAGGCCTCCCGTCCTCCAT
CGAGAAAACCATCTCCAAAGCCAAAGGGCAGCCCCGAGAGCCACAGGTGTACACCCT
GCCCCCATCCCAGGAGGAGATGACCAAGAACCAGGTCAGCCTGACCTGCCTGGTCAA
AGGCTTCTACCCCAGCGACATCGCCGTGGAGTGGGAGAGCAATGGGCAGCCGGAGAA
CAACTACAAGACCACGCCTCCCGTGCTGGACTCCGACGGCTCCTTCTTCCTCTACAGC
AGGCTAACCGTGGACAAGAGCAGGTGGCAGGAGGGGAATGTCTTCTCATGCTCCGTG
ATGCATGAGGCTCTGCACAACCACTACACACAGAAGAGCCTCTCCCTGTCTCTGGGTA
AACCCTTTTGGGTGCTGGTGGTGGTTGGTGGAGTCCTGGCTTGCTATAGCTTGCTAGT
AACAGTGGCCTTTATTATTTTCTGGGTGAGGAGTAAGAGGAGCAGGCTCCTGCACAGT
 
GACTACATGAACATGACTCCCCGCCGCCCCGGGCCCACCCGCAAGCATTACCAGCCC
TATGCCCCACCACGCGACTTCGCAGCCTATCGCTCC
70. Nucleic acid sequence of 28DCR2 (without signal peptide) (SEQ ID NO:70 
CAGGTGCAGCTGGTGCAGTCTGGGGCTGAGGTGAAGAAGCCTGGGGCCTCAGTG
AAGGTCTCCTGCAAGGCTTCTGGATACACCTTCACCGGCTACTATATGCACTGGGTGC
GACAGGCCCCTGGACAAGGGCTTGAGTGGATGGGATGGATCAACCCTGACAGTGGTG
GCACAAACTATGCACAGAAGTTTCAGGGCAGGGTCACCATGACCAGGGACACGTCCA
TCAGCACAGCCTACATGGAGCTGAACAGGCTGAGATCTGACGACACGGCCGTGTATT
ACTGTGCGAGAGATCAGCCCCTAGGATATTGTACTAATGGTGTATGCTCCTACTTTGA
CTACTGGGGCCAGGGAACCCTGGTCACCGTCTCCTCAGGTGGAGGCGGTTCAGGCGG
AGGTGGCAGCGGCGGTGGCGGGTCGGACATCCAGATGACCCAGTCTCCATCTTCCGT
GTCTGCATCTGTAGGAGACAGAGTCACCATCACTTGTCGGGCGAGTCAGGGTATTTAC
AGCTGGTTAGCCTGGTATCAGCAGAAACCAGGGAAAGCCCCTAACCTCCTGATCTAT
ACTGCATCCACTTTACAAAGTGGGGTCCCATCAAGGTTCAGCGGCAGTGGATCTGGG
ACAGATTTCACTCTCACCATCAGCAGCCTGCAACCTGAAGATTTTGCAACTTACTATT
GTCAACAGGCTAACATTTTCCCGCTCACTTTCGGCGGAGGGACCAAGGTGGAGATCA
AAGAGTCCAAATATGGTCCCCCATGCCCACCATGCCCAGCACCTGAGTTCCTGGGGG
GACCATCAGTCTTCCTGTTCCCCCCAAAACCCAAGGACACTCTCATGATCTCCCGGAC
CCCTGAGGTCACGTGCGTGGTGGTGGACGTGAGCCAGGAAGACCCCGAGGTCCAGTT
CAACTGGTACGTGGATGGCGTGGAGGTGCATAATGCCAAGACAAAGCCGCGGGAGG
AGCAGTTCAACAGCACGTACCGTGTGGTCAGCGTCCTCACCGTCCTGCACCAGGACTG
GCTGAACGGCAAGGAGTACAAGTGCAAGGTCTCCAACAAAGGCCTCCCGTCCTCCAT
CGAGAAAACCATCTCCAAAGCCAAAGGGCAGCCCCGAGAGCCACAGGTGTACACCCT
GCCCCCATCCCAGGAGGAGATGACCAAGAACCAGGTCAGCCTGACCTGCCTGGTCAA
AGGCTTCTACCCCAGCGACATCGCCGTGGAGTGGGAGAGCAATGGGCAGCCGGAGAA
CAACTACAAGACCACGCCTCCCGTGCTGGACTCCGACGGCTCCTTCTTCCTCTACAGC
AGGCTAACCGTGGACAAGAGCAGGTGGCAGGAGGGGAATGTCTTCTCATGCTCCGTG
ATGCATGAGGCTCTGCACAACCACTACACACAGAAGAGCCTCTCCCTGTCTCTGGGTA
AACCCTTTTGGGTGCTGGTGGTGGTTGGTGGAGTCCTGGCTTGCTATAGCTTGCTAGT
AACAGTGGCCTTTATTATTTTCTGGGTGAGGAGTAAGAGGAGCAGGCTCCTGCACAGT
GACTACATGAACATGACTCCCCGCCGCCCCGGGCCCACCCGCAAGCATTACCAGCCC
TATGCCCCACCACGCGACTTCGCAGCCTATCGCTCC
71. Nucleic acid sequence of 40DCR3 (without signal peptide) (SEQ ID NO:71) 
CAGGTGCAGCTGGTGCAGTCTGGGGCTGAGGTGAAGAAGCCTGGGGCCTCAGTG
AAGGTCTCCTGCAAGGCTTCTGGATACACCTTCACCGGCTACTATATGCACTGGGTGC
GACAGGCCCCTGGACAAGGGCTTGAGTGGATGGGATGGATCAACCCTGACAGTGGTG
GCACAAACTATGCACAGAAGTTTCAGGGCAGGGTCACCATGACCAGGGACACGTCCA
TCAGCACAGCCTACATGGAGCTGAACAGGCTGAGATCTGACGACACGGCCGTGTATT
ACTGTGCGAGAGATCAGCCCCTAGGATATTGTACTAATGGTGTATGCTCCTACTTTGA
CTACTGGGGCCAGGGAACCCTGGTCACCGTCTCCTCAGGTGGAGGCGGTTCAGGCGG
AGGTGGCAGCGGCGGTGGCGGGTCGGACATCCAGATGACCCAGTCTCCATCTTCCGT
GTCTGCATCTGTAGGAGACAGAGTCACCATCACTTGTCGGGCGAGTCAGGGTATTTAC
AGCTGGTTAGCCTGGTATCAGCAGAAACCAGGGAAAGCCCCTAACCTCCTGATCTAT
ACTGCATCCACTTTACAAAGTGGGGTCCCATCAAGGTTCAGCGGCAGTGGATCTGGG
ACAGATTTCACTCTCACCATCAGCAGCCTGCAACCTGAAGATTTTGCAACTTACTATT
GTCAACAGGCTAACATTTTCCCGCTCACTTTCGGCGGAGGGACCAAGGTGGAGATCA
AAGAGTCCAAATATGGTCCCCCATGCCCACCATGCCCAGCACCTGAGTTCCTGGGGG
GACCATCAGTCTTCCTGTTCCCCCCAAAACCCAAGGACACTCTCATGATCTCCCGGAC
CCCTGAGGTCACGTGCGTGGTGGTGGACGTGAGCCAGGAAGACCCCGAGGTCCAGTT
CAACTGGTACGTGGATGGCGTGGAGGTGCATAATGCCAAGACAAAGCCGCGGGAGG
AGCAGTTCAACAGCACGTACCGTGTGGTCAGCGTCCTCACCGTCCTGCACCAGGACTG
GCTGAACGGCAAGGAGTACAAGTGCAAGGTCTCCAACAAAGGCCTCCCGTCCTCCAT
CGAGAAAACCATCTCCAAAGCCAAAGGGCAGCCCCGAGAGCCACAGGTGTACACCCT
GCCCCCATCCCAGGAGGAGATGACCAAGAACCAGGTCAGCCTGACCTGCCTGGTCAA
AGGCTTCTACCCCAGCGACATCGCCGTGGAGTGGGAGAGCAATGGGCAGCCGGAGAA
CAACTACAAGACCACGCCTCCCGTGCTGGACTCCGACGGCTCCTTCTTCCTCTACAGC
AGGCTAACCGTGGACAAGAGCAGGTGGCAGGAGGGGAATGTCTTCTCATGCTCCGTG
ATGCATGAGGCTCTGCACAACCACTACACACAGAAGAGCCTCTCCCTGTCTCTGGGTA
AACCCTTTTGGGTGCTGGTGGTGGTTGGTGGAGTCCTGGCTTGCTATAGCTTGCTAGT
AACAGTGGCCTTTATTATTTTCTGGGTGAGGAGTAAGAGGAGCAGGCTCCTGCACAGT
GACTACATGAACATGACTCCCCGCCGCCCCGGGCCCACCCGCAAGCATTACCAGCCC
TATGCCCCACCACGCGACTTCGCAGCCTATCGCTCCAAACGGGGCAGAAAGAAGCTC
CTGTATATATTCAAACAACCATTTATGAGACCAGTACAAACTACTCAAGAGGAAGAT
GGCTGTAGCTGCCGATTTCCAGAAGAAGAAGAAGGAGGATGTGAACTG
72. Nucleic acid sequence of 40DCR4 (without signal peptide) (SEQ ID NO:72) 
CAGGTGCAGCTGGTGCAGTCTGGGGCTGAGGTGAAGAAGCCTGGGGCCTCAGTG
AAGGTCTCCTGCAAGGCTTCTGGATACACCTTCACCGGCTACTATATGCACTGGGTGC
 
GACAGGCCCCTGGACAAGGGCTTGAGTGGATGGATGGATCAACCCTGACAGTGGTG
GCACAAACTATGCACAGAAGTTTCAGGGCAGGGTCACCATGACCAGGGACACGTCCA
TCAGCACAGCCTACATGGAGCTGAACAGGCTGAGATCTGACGACACGGCCGTGTATT
ACTGTGCGAGAGATCAGCCCCTAGGATATTGTACTAATGGTGTATGCTCCTACTTTGA
CTACTGGGGCCAGGGAACCCTGGTCACCGTCTCCTCAGGTGGAGGCGGTTCAGGCGG
AGGTGGCAGCGGCGGTGGCGGGTCGGACATCCAGATGACCCAGTCTCCATCTTCCGT
GTCTGCATCTGTAGGAGACAGAGTCACCATCACTTGTCGGGCGAGTCAGGGTATTTAC
AGCTGGTTAGCCTGGTATCAGCAGAAACCAGGGAAAGCCCCTAACCTCCTGATCTAT
ACTGCATCCACTTTACAAAGTGGGGTCCCATCAAGGTTCAGCGGCAGTGGATCTGGG
ACAGATTTCACTCTCACCATCAGCAGCCTGCAACCTGAAGATTTTGCAACTTACTATT
GTCAACAGGCTAACATTTTCCCGCTCACTTTCGGCGGAGGGACCAAGGTGGAGATCA
AAGAGTCCAAATATGGTCCCCCATGCCCACCATGCCCAGCACCTGAGTTCCTGGGGG
GACCATCAGTCTTCCTGTTCCCCCCAAAACCCAAGGACACTCTCATGATCTCCCGGAC
CCCTGAGGTCACGTGCGTGGTGGTGGACGTGAGCCAGGAAGACCCCGAGGTCCAGTT
CAACTGGTACGTGGATGGCGTGGAGGTGCATAATGCCAAGACAAAGCCGCGGGAGG
AGCAGTTCAACAGCACGTACCGTGTGGTCAGCGTCCTCACCGTCCTGCACCAGGACTG
GCTGAACGGCAAGGAGTACAAGTGCAAGGTCTCCAACAAAGGCCTCCCGTCCTCCAT
CGAGAAAACCATCTCCAAAGCCAAAGGGCAGCCCCGAGAGCCACAGGTGTACACCCT
GCCCCCATCCCAGGAGGAGATGACCAAGAACCAGGTCAGCCTGACCTGCCTGGTCAA
AGGCTTCTACCCCAGCGACATCGCCGTGGAGTGGGAGAGCAATGGGCAGCCGGAGAA
CAACTACAAGACCACGCCTCCCGTGCTGGACTCCGACGGCTCCTTCTTCCTCTACAGC
AGGCTAACCGTGGACAAGAGCAGGTGGCAGGAGGGGAATGTCTTCTCATGCTCCGTG
ATGCATGAGGCTCTGCACAACCACTACACACAGAAGAGCCTCTCCCTGTCTCTGGGTA
AACCCTTTTGGGTGCTGGTGGTGGTTGGTGGAGTCCTGGCTTGCTATAGCTTGCTAGT
AACAGTGGCCTTTATTATTTTCTGGGTGAAACGGGGCAGAAAGAAGCTCCTGTATATA
TTCAAACAACCATTTATGAGACCAGTACAAACTACTCAAGAGGAAGATGGCTGTAGC
TGCCGATTTCCAGAAGAAGAAGAAGGAGGATGTGAACTGAGGAGTAAGAGGAGCAG
GCTCCTGCACAGTGACTACATGAACATGACTCCCCGCCGCCCCGGGCCCACCCGCAA
GCATTACCAGCCCTATGCCCCACCACGCGACTTCGCAGCCTATCGCTCC
73. Amino acid sequence of EGFR G1 CAR (SEQ ID NO:73) 
ATMALPVTALLLPLALLLHAARPSDILLTQSPVILSVSPGERVSFSCRASQSIGTNIHW
YQQRTNGSPRLLIKYASESISGIPSRFSGSGSGTDFTLSINSVESEDIADYYCQQNNNWPTTF
GAGTKLELKGGGGSGGGGSGGGGSQVQLKQSGPGLVQPSQSLSITCTVSGFSLTNYGVH
WVRQSPGKGLEWLGVIWSGGNTDYNTPFTSRLSINKDNSKSQVFFKMNSLQSNDTAIYY
 
CARALTYYDYEFAYWGQGTLVTVSSFVPVFLPAKPTTTPAPRPPTPAPTIASQPLSLRPEA
CRPAAGGAVHTRGLDFACDIYIWAPLAGTCGVLLLSLVITLYCNHRSKRSRLLHSDYMN
MTPRRPGPTRKHYQPYAPPRDFAAYRSRVKFSRSADAPAYQQGQNQLYNELNLGRREEY
DVLDKRRGRDPEMGGKPRRKNPQEGLYNELQKDKMAEAYSEIGMKGERRRGKGHDGL
YQGLSTATKDTYDALHMQALPPR
74. Nucleic acid sequence of EGFR G1 CAR (SEQ ID NO:74) 
GCCACCATGGCCTTACCAGTGACCGCCTTGCTCCTGCCGCTGGCCTTGCTGCTCC
ACGCCGCCAGGCCGAGCGACATCTTGCTGACTCAGTCTCCAGTCATCCTGTCTGTGAG
TCCAGGAGAAAGAGTCAGTTTCTCCTGCAGGGCCAGTCAGAGTATTGGCACAAACAT
ACACTGGTATCAGCAAAGAACAAATGGTTCTCCAAGGCTTCTCATAAAGTATGCTTCT
GAGTCTATCTCTGGGATCCCTTCCAGGTTTAGTGGCAGTGGATCAGGGACAGATTTTA
CTCTTAGCATCAACAGTGTGGAGTCTGAAGATATTGCAGATTATTACTGTCAACAAAA
TAATAACTGGCCAACCACGTTCGGTGCTGGGACCAAGCTGGAGCTGAAAGGTGGAGG
CGGTTCAGGCGGAGGTGGCAGCGGCGGTGGCGGGTCGCAGGTGCAGCTGAAGCAGTC
AGGACCTGGCCTAGTGCAGCCCTCACAGAGCCTGTCCATCACCTGCACAGTCTCTGGT
TTCTCATTAACTAACTATGGTGTACACTGGGTTCGCCAGTCTCCAGGAAAGGGTCTGG
AGTGGCTGGGAGTGATATGGAGTGGTGGAAACACAGACTATAATACACCTTTCACAT
CCAGACTGAGCATCAACAAGGACAATTCCAAGAGCCAAGTTTTCTTTAAAATGAACA
GTCTGCAATCTAATGACACAGCCATATATTACTGTGCCAGAGCCCTCACCTACTATGA
TTACGAGTTTGCTTACTGGGGCCAAGGGACTCTGGTCACTGTCTCTTCGTTCGTGCCG
GTCTTCCTGCCAGCGAAGCCCACCACGACGCCAGCGCCGCGACCACCAACACCGGCG
CCCACCATCGCGTCGCAGCCCCTGTCCCTGCGCCCAGAGGCGTGCCGGCCAGCGGCG
GGGGGCGCAGTGCACACGAGGGGGCTGGACTTCGCCTGTGATATCTACATCTGGGCG
CCCCTGGCCGGGACTTGTGGGGTCCTTCTCCTGTCACTGGTTATCACCCTTTACTGCAA
CCACAGGAGTAAGAGGAGCAGGCTCCTGCACAGTGACTACATGAACATGACTCCCCG
CCGCCCCGGGCCCACCCGCAAGCATTACCAGCCCTATGCCCCACCACGCGACTTCGCA
GCCTATCGCTCCAGAGTGAAGTTCAGCAGGAGCGCAGACGCCCCCGCGTACCAGCAG
GGCCAGAACCAGCTCTATAACGAGCTCAATCTAGGACGAAGAGAGGAGTACGATGTT
TTGGACAAGAGACGTGGCCGGGACCCTGAGATGGGGGGAAAGCCGAGAAGGAAGAA
CCCTCAGGAAGGCCTGTACAATGAACTGCAGAAAGATAAGATGGCGGAGGCCTACAG
TGAGATTGGGATGAAAGGCGAGCGCCGGAGGGGCAAGGGGCACGATGGCCTTTACC
AGGGTCTCAGTACAGCCACCAAGGACACCTACGACGCCCTTCACATGCAGGCCCTGC
CCCCTCGCTGATAA
75. Nucleotide sequence of EGFR G2 CAR (SEQ ID NO:75) 
GCCACCATGGCCTTACCAGTGACCGCCTTGCTCCTGCCGCTGGCCTTGCTGCTCC
ACGCCGCCAGGCCGAGCGACATCTTGCTGACTCAGTCTCCAGTCATCCTGTCTGTGAG
TCCAGGAGAAAGAGTCAGTTTCTCCTGCAGGGCCAGTCAGAGTATTGGCACAAACAT
ACACTGGTATCAGCAAAGAACAAATGGTTCTCCAAGGCTTCTCATAAAGTATGCTTCT
GAGTCTATCTCTGGGATCCCTTCCAGGTTTAGTGGCAGTGGATCAGGGACAGATTTTA
CTCTTAGCATCAACAGTGTGGAGTCTGAAGATATTGCAGATTATTACTGTCAACAAAA
TAATAACTGGCCAACCACGTTCGGTGCTGGGACCAAGCTGGAGCTGAAAGGTGGAGG
CGGTTCAGGCGGAGGTGGCAGCGGCGGTGGCGGGTCGCAGGTGCAGCTGAAGCAGTC
AGGACCTGGCCTAGTGCAGCCCTCACAGAGCCTGTCCATCACCTGCACAGTCTCTGGT
TTCTCATTAACTAACTATGGTGTACACTGGGTTCGCCAGTCTCCAGGAAAGGGTCTGG
AGTGGCTGGGAGTGATATGGAGTGGTGGAAACACAGACTATAATACACCTTTCACAT
CCAGACTGAGCATCAACAAGGACAATTCCAAGAGCCAAGTTTTCTTTAAAATGAACA
GTCTGCAATCTAATGACACAGCCATATATTACTGTGCCAGAGCCCTCACCTACTATGA
TTACGAGTTTGCTTACTGGGGCCAAGGGACTCTGGTCACTGTCTCTTCGTTCGTGCCG
GTCTTCCTGCCAGCGAAGCCCACCACGACGCCAGCGCCGCGACCACCAACACCGGCG
CCCACCATCGCGTCGCAGCCCCTGTCCCTGCGCCCAGAGGCGTGCCGGCCAGCGGCG
GGGGGCGCAGTGCACACGAGGGGGCTGGACTTCGCCTGTGATATCTACATCTGGGCG
CCCCTGGCCGGGACTTGTGGGGTCCTTCTCCTGTCACTGGTTATCACCCTTTACTGCAA
CCACAGGAGTAAGAGGAGCAGGCTCCTGCACAGTGACTACATGAACATGACTCCCCG
CCGCCCCGGGCCCACCCGCAAGCATTACCAGCCCTATGCCCCACCACGCGACTTCGCA
GCCTATCGCTCCAGAGTGAAGTTCAGCAGGAGCGCAGACGCCCCCGCGTACCAGCAG
GGCCAGAACCAGCTCTATAACGAGCTCAATCTAGGACGAAGAGAGGAGTACGATGTT
TTGGACAAGAGACGTGGCCGGGACCCTGAGATGGGGGGAAAGCCGAGAAGGAAGAA
CCCTCAGGAAGGCCTGTACAATGAACTGCAGAAAGATAAGATGGCGGAGGCCTACAG
TGAGATTGGGATGAAAGGCGAGCGCCGGAGGGGCAAGGGGCACGATGGCCTTTACC
AGGGTCTCAGTACAGCCACCAAGGACACCTACGACGCCCTTCACATGCAGGCCCTGC
CCCCTCGCTGATAA
76. Nucleotide sequence of primer CD40-F (SEQ ID NO: 76)
   ACCTCCTGATCTATACTG
77. Nucleotide sequence of primer CD40-R (SEQ ID NO: 77)
   GATGGTGAGAGTGAAATC
78. Nucleotide sequence in probe Taqman (SEQ ID NO: 78)
   CACTGCCGCTGAACCTTGATG

### DETAILED DESCRIPTION

The embodiments of the present disclosure illustrated by way of specific examples below. Those skilled in the art will understand that these examples are merely exemplary and should not be considered as limiting the scope of the present disclosure. The experimental methods without specifying the specific technology or conditions in the following examples generally used the conventional conditions, such as those described in Sambrook. J., Molecular Cloning: A Laboratory Manual (3rd ed.), translated by Huang Peitang, Science Press*,* or followed the manufacturer's recommendation. The used reagents or instruments without specifying the manufacturer are all conventional products that are commercially available.

### Example 1-(1): Construction of 5 recombinant plasmids: pNB328-meso CAR G1, pNB328-meso G2 CAR, PS328b 137 DCR1, PS328b 137 DCR2 and PS328b 137 DCR3

1. 137DCR1 gene (SEQ ID NO: 15), 137DCR2 gene (SEQ ID NO: 16), 137DCR3 gene (SEQ ID NO: 17), meso G1 CAR gene (SEQ ID NO: 24) and meso G2 CAR Gene (SEQ ID NO: 26) were synthesized artificially, and diagrams of their structures are shown in Figure 1A-1, Figure 1B-1, Figure 1C-1, Figure 1E-1, and Figure 1F-1, respectively. The synthesized 5 genes were inserted into PNB328 vector and PS328b vector, between EcoRI and SalI restriction sites.

pNB328 vector contains EF1α promoter, PB transposon and other elements, which is constructed according to Example 2 of WO2017054647A1. PS328b is an artificially synthesized sequence, synthesized by Shanghai Generay Biological Engineering Co., Ltd., with sequence shown in SEQ ID NO:27.

The constructed recombinant plasmids were named pNB328-meso G1 CAR plasmid, pNB328-meso G2 CAR plasmid, PS328b 137DCR1 plasmid, PS328b 137DCR2 plasmid and PS328b 137DCR3 plasmid. The constructed recombinant plasmids can carry foreign genes and be integrated into the host cell genome.

### Example 1-(2): Construction of 5 recombinant plasmids: pNB328-Muc1 G1 CAR, pNB328-Muc1 G2 CAR, PS328b 28DCR1, PS328b 28DCR2 and PS328b 28DCR3

1. 28DCR1 gene (SEQ ID NO: 40), 28DCR2 gene (SEQ ID NO: 41), 28DCR3 gene (SEQ ID NO: 42), Mucl G1 CAR gene (SEQ ID NO: 49) and Mucl G2 CAR Gene (SEQ ID NO: 50) were synthesized artificially, and diagrams of their structures are shown in Figure 1A-2, Figure 1B-2, Figure 1C-2, Figure 1E-2, and Figure 1F-2, respectively. The synthesized 5 genes were inserted into PNB328 vector and PS328b vector, between EcoRI and SalI restriction sites.

pNB328 vector contains EF1α promoter, PB transposon and other elements, which is constructed according to Example 2 of WO2017054647A1. PS328b is an artificially synthesized sequence, synthesized by Shanghai Generay Biological Engineering Co., Ltd., with sequence shown in SEQ ID NO:27.

The constructed recombinant plasmids were named pNB328-Muc1 G1 CAR plasmid, pNB328-Muc1 G2 CAR plasmid, PS328b 28DCR1 plasmid, PS328b 28DCR2 plasmid and PS328b 28DCR3 plasmid. The constructed recombinant plasmids can carry foreign genes and be integrated into the host cell genome.

### Example 1-(3): Construction of 5 recombinant plasmids: pNB328-EGFR G1 CAR, pNB328-EGFR G2 CAR, PS328b 40DCR1, PS328b 40DCR2 and PS328b 40DCR3

1. 40DCR1gene (SEQ ID NO: 65), 40DCR2 gene (SEQ ID NO: 66), 40DCR3 gene (SEQ ID NO: 67), EGFR G1 CAR gene (SEQ ID NO: 74) and EGFR G2 CAR Gene (SEQ ID NO: 75) were synthesized artificially, and diagrams of their structures are shown in Figure 1A-3, Figure 1B-3, Figure 1C-3, Figure 1F-3, and Figure 1D-3, respectively. The synthesized 5 genes were inserted into pNB328 vector and PS328b vector, between EcoRI and SalI restriction sites.

pNB328 vector contains EF1α promoter, PB transposon and other elements, which is constructed according to Example 2 of WO2017054647A1. PS328b is an artificially synthesized sequence, synthesized by Shanghai Generay Biological Engineering Co., Ltd., with sequence shown in SEQ ID NO:27.

The constructed recombinant plasmids were named pNB328-EGFR G1 CAR plasmid, pNB328-EGFR G2 CAR plasmid, PS328b 40DCR1 plasmid, PS328b 40DCR2 plasmid and PS328b 40DCR3 plasmid. The constructed recombinant plasmids can carry foreign genes and be integrated into the host cell genome.

### Example 2-(1): Construction and identification of 9 chimeric antigen receptor modified T cells: recombinant cells meso G1 CAR, meso G2 CAR, meso G1 CAR-137DCR1, meso G1 CAR-137DCR2, meso G1 CAR-137DCR3, 137DCR1, 137DCR2, 137DCR3 and Mock T

### (1) Construction of 9 recombinant cells

Peripheral blood mononuclear cells (PBMCs) were adherently cultured for 2-4 hours, and the non-adherent suspension cells were the naive T cells. The suspension cells were collected in a 15ml centrifuge tube, centrifuged at 1200rmp for 3min, and the supernatant was discarded; normal saline was added, centrifuged at 1200rmp for 3min, and the normal saline was discarded, and the steps of "normal saline was added, centrifuged at 1200rmp for 3min, and the normal saline was discarded" were repeat for three times.

5×10⁶ of the above cells were added to each of eight 1.5ml centrifuge tubes, numbered a, b, c, d, e, f, g, h, centrifuged at 1200rmp for 3min, the supernatant was discarded and 100µl electroporation reagent of the Electroporation Kit (Lonza) was added to each tube in proportion, wherein:
tube a: add 8µg of pNB328-meso G1 CAR plasmid,
tube b: add 8µg of pNB328-meso G2 CAR plasmid,
tube c: add 4 µg of each of PS328b 137DCR1 plasmid and pNB328-meso G1 CAR plasmid,
tube d: add 4 µg of each of PS328b 137DCR2 plasmid and pNB328-meso G1 CAR plasmid,
tube e: add 4 µg of each of PS328b 137DCR3 plasmid and pNB328-meso G1 CAR plasmid,
tube f: add 8µg of PS328b 137DCR1 plasmid,
tube g: add 8µg of PS328b 137DCR2 plasmid,
tube h: add 8µg of PS328b 137DCR3 plasmid,
Each of the above 8 tubes was re-suspended and mixed to obtain mixtures.

Each of the mixture was transferred to an electroporation cup, which was put into the electroporation instrument, the required program was selected for electrical shock; the micro pipette in the kit was used to transfer the electroporated cell suspension to a six-well plate with the medium (AIM-V medium containing 2% FBS), which was mixed well and cultured in a 37°C, 5% CO2 incubator for 6 hours; then stimulating factor IL-2 and meso/anti-CD28 was added, cultured at 37°C, 5% CO2 for 3 to 4 days, the growth of T cells was observed. The recombinant T cells expressing pNB328-meso G1 CAR, pNB328-meso G2 CAR, pNB328-meso G1 CAR-137DCR1, pNB328-meso G1 CAR-137DCR2, pNB328-meso G1 CAR-137DCR3, PS328b 137DCR1, PS328b 137DCR2 and PS328 137DCR3 gene are obtained and named recombinant cell meso G1 CAR, recombinant cell meso G2 CAR, recombinant cell meso G1 CAR-137DCR1, recombinant cell meso G1 CAR-137DCR2 and recombinant cell meso G1 CAR-137DCR3, recombinant cell 137DCR1, recombinant cell 137DCR2 and recombinant cell 137DCR3, respectively.

In addition, 5×10⁶ cells were added in another centrifuge tube (tube i), centrifuged at 1200rmp for 3min, the supernatant was discarded, the electroporation kit (purchased from Lonza) was taken, 100µl electroporation reagent of the Electroporation Kit (Lonza) in proportion was added, and 8µg control plasmid (PNB328) was added, the control T cell, Mock T, was constructed and obtained according to the method described above.

### (2) Identification of positive recombinant cells

### ①Detection of meso G1 CAR or meso G2 CAR gene expression by Western blot method

The above-mentioned recombinant cells meso G1 CAR, meso G2 CAR, meso G1 CAR-137DCR1, meso G1CAR-137DCR2, meso G1 CAR-137DCR3 and Mock-T cells were collected, and washed twice with normal saline,
160µl of cell lysate was added and placed on ice for 10min; after the cells were fully lysed, centrifuged at 12000rpm at 4°C for 10min, and the supernatant was collected. 40µl of 5×loading Buffer was added, incubated at 100°C for 10min, then placed on ice for 5min.
The expression of CD3ζ of the previously constructed recombinant cells was detected by Western blot etc., using CD3ζ antibody (Abcam), GAPDH antibody (Beyotime), HRP goat anti-mouse secondary antibody (Jackson). The results are shown in Figure 2A-1.
The results show that CD3ζ is highly expressed in all of these constructed recombinant T cells.

### ②Detection of 137DCR1-3 gene expression by RT-PCR

The genomic DNA of recombinant cells meso G1 CAR, meso G2 CAR, meso G1 CAR-137DCR1, meso G1 CAR-137DCR2, meso G1 CAR-137DCR3 and Mock-T was extracted (kit method) using the experimental procedure based on the attached instruction in the kit.

The DNA concentration of each recombinant cells was determined, and the expression level of 137DCR gene was detected by fluorescence real-time quantitative PCR. The reaction was: 95°C, 15s; 95°C, 5s; 60°C, 15s; for 40 cycles.

The PCR reaction system (20µl) was as follows:
Taqman: 10µl
CD137-F: 0.4µl
CD137-R: 0.4µl
Cd137-probe: 0.2µl
Actin mix: 1µl
H₂O:7µl

The primer sequences were as follows:
CD137-F: CGAGTCACCATATCAGTA (SEQ ID NO: 28)
CD137-R: CGAAGTACCAGTCATAATTC (SEQ ID NO: 29)
Taqman: 5'FAM-CCTCGCACAGTAATATACAGCCGT-Trama (SEQ ID NO: 30)

The results are shown in Figure2B-1. The results show that the expression levels of 137DCR1, 137DCR2 or 137DCR3 genes in the recombinant cells meso G1 CAR-137DCR1, meso G1 CAR-137DCR2, meso G1 CAR-137DCR3 are very high.

### Example 2-(2): Construction and identification of 9 chimeric antigen receptor modified T cells: recombinant cells Mucl G1 CAR, Mucl G2 CAR, Mucl G1 CAR-28DCR1, Mucl G1 CAR-28DCR2, Muc1 G1 CAR-28DCR3, 28DCR1, 28DCR2, 28DCR3 and Mock T

### (1) Construction of 9 recombinant cells

Peripheral blood mononuclear cells (PBMCs) were adherently cultured for 2-4 hours, and the non-adherent suspension cells were the naive T cells. The suspension cells were collected in a 15ml centrifuge tube, centrifuged at 1200rmp for 3min, and the supernatant was discarded; normal saline was added, centrifuged at 1200rmp for 3min, and the normal saline was discarded, and the steps of "normal saline was added, centrifuged at 1200rmp for 3min, and the normal saline was discarded" were repeated for three times.

5×10⁶ of the above cells was added to each of eight 1.5ml centrifuge tubes, numbered a, b, c, d, e, f, g, h, centrifuged at 1200rmp for 3min, the supernatant was discarded and 100µl electroporation reagent of the Electroporation Kit (Lonza) was added to each tube in proportion, wherein:
tube a: add 8µg of pNB328-Muc1 G1 CAR plasmid,
tube b: add 8µg of pNB328-Muc1 G2 CAR plasmid,
tube c: add 4 µg of each of PS328b 28DCR1 plasmid and pNB328-Muc1 G1 CAR plasmid,
tube d: add 4 µg of each of PS328b 28DCR2 plasmid and pNB328-Muc1 G1 CAR plasmid,
tube e: add 4 µg of each of PS328b 28DCR3 plasmid and pNB328-Muc1 G1 CAR plasmid,
tube f: add 8µg of PS328b 28DCR1 plasmid,
tube g: add 8µg of PS328b 28DCR2 plasmid,
tube h: add 8µg of PS328b 28DCR3 plasmid,
Each of the above 8 tubes was re-suspended and mixed to obtain mixtures.

The mixture was transferred to an electroporation cup which was put into the electroporation instrument, the required program was selected for electrical shock; the micro pipette in the kit was used to transfer the electroporated cell suspension to a six-well plate with the medium (AIM-V medium containing 2% FBS), mixed well and cultured in a 37°C, 5% CO2 incubator for 6 hours; then stimulating factor IL-2 and Muc1/anti-CD28 was added, cultured at 37°C, 5% CO2 for 3 to 4 days, the growth of T cells was observed. The recombinant T cells expressing pNB328-Muc1 G1 CAR, pNB328-Muc1 G2 CAR, pNB328-Muc1 G1 CAR-28DCR1, pNB328-Muc1 G1 CAR-28DCR2, pNB328-Muc1 G1 CAR-28DCR3, PS328b 28DCR1, PS328b 28DCR2 and PS328b 28DCR3 gene were obtained and named recombinant cell Mucl G1 CAR, recombinant cell Mucl G2 CAR, recombinant cell Mucl G1 CAR-28DCR1, recombinant cell Mucl G1 CAR-28DCR2 and recombinant cell Mucl G1 CAR-28DCR3, recombinant cell 28DCR1, recombinant cell 28DCR2 and recombinant cell 28DCR3, respectively.

In addition, 5×10⁶ cells were added in another centrifuge tube (tube i), centrifuged at 1200rmp for 3min, the supernatant was discarded, the electroporation kit (purchased from Lonza) was taken, 100µl electroporation reagent of the Electroporation Kit (Lonza) in proportion was added and 8µg control plasmid (PNB328) was added, the control T cell, Mock T was obtained according to the method described above.

### (2) Identification of positive recombinant cells

### ①Detection of Muc1 G1 CAR or Muc1 G2 CAR gene expression by Western blot method

The above-mentioned recombinant cells Mucl G1 CAR, Mucl G2 CAR, Mucl G1 CAR-28DCR1, Mucl G1CAR-28DCR2, Mucl G1 CAR-28DCR3 and Mock-T cells were collected, washed twice with normal saline,
160µl of cell lysate was added and placed on ice for 10min; after the cells were fully lysed, centrifuged at 12000rpm at 4°C for 10min, and the supernatant was collected. 40µl of 5×loading Buffer was added, incubated at 100°C for 10min, then placed on ice for 5min.

The expression of CD3ζ of the constructed recombinant cells was detected by Western blot etc., using CD3ζ antibody (Abcam), GAPDH antibody (Beyotime), HRP goat anti-mouse secondary antibody (Jackson). The results are shown in Figure 2A-2.

The results show that CD3ζ is highly expressed in all of these constructed recombinant T cells.

### ②Detection of 28DCR1-3 gene expression by RT-PCR

The genomic DNA of recombinant cells Mucl G1 CAR, Mucl G2 CAR, Mucl G1 CAR-28DCR1, Mucl G1 CAR-28DCR2, Mucl G1 CAR-28DCR3 and Mock-T was extracted (kit method) using the experimental procedure based on the attached instruction in the kit.

The DNA concentration of each recombinant cells was determined, and the expression level of 28DCR gene was detected by fluorescence real-time quantitative PCR. The reaction was: 95°C, 15s; 95°C, 5s; 60°C, 15s; for 40 cycles.

The PCR reaction system (20µl) was as follows:
Taqman: 10µl
CD28-F: 0.4µl
CD28-R: 0.4µl
CD28-probe: 0.2µl
Actin mix: 1µl
H₂O:7µl

The primer sequences were as follows:
CD28-F: GCTTCTGGATACACCTTC (SEQ ID NO: 51)
CD28-R: CCTTGAACTTCTCATTATAGTTAG (SEQ ID NO: 52)
Taqman: 5'FAM-AATACATCCAATCCACTCAAGCC-Trama (SEQ ID NO: 53)

The results are shown in Figure 2B-2. The results show that the expression levels of 28DCR1, 28DCR2 or 28DCR3 genes in the recombinant cells Muc1 G1 CAR-28DCR1, Mucl G1 CAR-28DCR2, Mucl G1 CAR-28DCR3 are very high.

### Example 2-(3): Construction and identification of 9 chimeric antigen receptor modified T cells: recombinant cells EGFR G1 CAR, EGFR G2 CAR, EGFR G1 CAR-40DCR1, EGFR G1 CAR-40DCR2, EGFR G1 CAR-40DCR3, 40DCR1, 40DCR2, 40DCR3 and Mock T

### (1) Construction of 9 recombinant cells

Peripheral blood mononuclear cells (PBMCs) were adherently cultured for 2-4 hours, and the non-adherent suspension cells were the naive T cells. The suspension cells were collected in a 15ml centrifuge tube, centrifuged at 1200rmp for 3min, and the supernatant was discarded; normal saline was added, centrifuged at 1200rmp for 3min, and the normal saline was discarded, and the steps of "normal saline was added, centrifuged at 1200rmp for 3min, and the normal saline was discarded" was repeated for three times.

5×10⁶ of the above cells were added to each of eight 1.5ml centrifuge tubes, numbered a, b, c, d, e, f, g, h, centrifuged at 1200rmp for 3min, the supernatant was discarded and 100µl electroporation reagent of the Electroporation Kit (Lonza) was added to each tube in proportion, wherein:
tube a: add 8µg of pNB328-EGFR G1 CAR plasmid,
tube b: add 8µg of pNB328-EGFR G2 CAR plasmid,
tube c: add 4 µg of each of PS328b 40DCR1 plasmid and pNB328-EGFR G1 CAR plasmid,
tube d: add 4 µg of each of PS328b 40DCR2 plasmid and pNB328-EGFR G1 CAR plasmid,
tube e: add 4 µg of each of PS328b 40DCR3 plasmid and pNB328-EGFR G1 CAR plasmid,
tube f: add 8µg of PS328b 40DCR1 plasmid,
tube g: add 8µg of PS328b 40DCR2 plasmid,
tube h: add 8µg of PS328b 40DCR3 plasmid,
Each of the above 8 tubes was re-suspended and mixed to obtain mixtures.

The mixture was transferred to an electroporation cup which was put into the electroporation instrument, the required program was selected for electrical shock; the micro pipette in the kit was used to transfer the electroporated cell suspension to a six-well plate with the medium (AIM-V medium containing 2% FBS), mixed well and cultured in a 37°C, 5% CO2 incubator for 6 hours; then stimulating factor IL-2 and EGFR/anti-CD28 was added, cultured at 37°C, 5% CO2 for 3 to 4 days, the growth of T cells was observed. The recombinant T cells expressing pNB328-EGFR G1 CAR, pNB328-EGFR G2 CAR, pNB328-EGFR G1 CAR-40DCR1, pNB328-EGFR G1 CAR-40DCR2, pNB328-EGFR G1 CAR-40DCR3, PS328b 40DCR1, PS328b 40DCR2 and PS328 40DCR3 gene were obtained and named as recombinant cell EGFR G1 CAR, recombinant cell EGFR G2 CAR, recombinant cell EGFR G1 CAR-40DCR1, recombinant cell EGFR G1 CAR-40DCR2 and recombinant cell EGFR G1 CAR-40DCR3, recombinant cell 40DCR1, recombinant cell 40DCR2 and recombinant cell 40DCR3, respectively.

In addition, 5×10⁶ cells were added in another centrifuge tube (tube j), centrifuged at 1200rmp for 3min, the supernatant was discarded, the electroporation kit (purchased from Lonza) was taken, 100µl electroporation reagent of the Electroporation Kit (Lonza) in proportion was added and 8µg control plasmid (PNB328) was added, the control T cell, Mock T was obtained according to the method described above.

### (2) Identification of positive recombinant cells

### ①Detection of EGFR G1 CAR or EGFR G2 CAR gene expression by Western blot method

The above-mentioned recombinant cells EGFR G1 CAR, EGFR G2 CAR, EGFR G1 CAR-40DCR1, EGFR G1CAR-40DCR2, EGFR G1 CAR-40DCR3 and Mock-T cells were collected, washed twice with normal saline,
160µl of cell lysate was added and placed on ice for 10min; after the cells were fully lysed, centrifuged at 12000rpm at 4°C for 10min, and the supernatant was collected. 40µl of 5×loading Buffer was added, incubated at 100°C for 10min, then placed on ice for 5min.

The expression of CD3ζ of the 5 constructed recombinant cells was detected by Western blot etc., using CD3ζ antibody (Abcam), GAPDH antibody (Beyotime), HRP goat anti-mouse secondary antibody (Jackson). The results are shown in Figure 2A-3.

The results show that CD3ζ is highly expressed in all of these 5 constructed recombinant T cells.

### ②Detection of 40DCR gene expression by RT-PCR

The genomic DNA of recombinant cells EGFR G1 CAR, EGFR G2 CAR, EGFR G1 CAR-40DCR1, EGFR G1 CAR-40DCR2, EGFR G1 CAR-40DCR3 and Mock-T was extracted (kit method) using the experimental procedure based on the attached instruction in the kit.

The DNA concentration of each recombinant cell was determined, and the expression level of 40DCR gene was detected by fluorescence real-time quantitative PCR. The reaction was: 95°C, 15s; 95°C, 5s; 60°C, 15s; for 40 cycles.

The PCR reaction system (20µl) was as follows:
Taqman: 10µl
CD40-F: 0.4µl
CD40-R: 0.4µl
CD40-probe: 0.2µl
Actin mix: 1µl
H₂O:7µl

The primer sequences were as follows:
CD40-F: ACCTCCTGATCTATACTG (SEQ ID NO: 76)
CD40-R: GATGGTGAGAGTGAAATC (SEQ ID NO: 77)
Taqman: 5'FAM-CACTGCCGCTGAACCTTGATG-Trama (SEQ ID NO: 78)

The results are shown in Figure 2B-3. The results show that the expression levels of 40DCR genes in the recombinant cells EGFR G1 CAR-137DCR1, EGFR G1 CAR-137DCR2, and EGFR G1 CAR-137DCR3 are very high.

### Example 3-(1): Detection of cell proliferation activity by Flow cytometry

### 1. Experimental samples, reagents and instruments

The recombinant cells 137DCR1, 137DCR2, 137DCR3 and MockT prepared according to Example 2-(1) .

2% FBS-containing PBS (1ml Hyclone FBS+49ml PBS) was formulated in a 50ml centrifuge tube; 10× BD Perm/Wash™ buffer was diluted by 10-folds with ddH₂O, and placed on ice; Hoechst 33342 stock solution was diluted into working solution at 1:100 with ddH₂O (1µl/test).

Low temperature centrifuge (4°C pre-cooling).

### 2. Experimental method

All reagents were kept on ice during the experiment.

Steps were as follows:
(1) 1×10⁶-2×10⁶ of each of the above cells was collected in a 1.5ml centrifuge tube, an appropriate amount of 2% FBS-containing PBS was added, centrifuged at 5000rpm, 4°C for 5min, and the supernatant was discarded;
(2) the cells were re-suspended with 100µl Fixation/Permeabilization Solution; the resuspension should be sufficient and gentle; the cells were fixed and permeabilized at 4°C for 20min;
(3) 1ml of Perm/Wash™ buffer of the working concentration was added, mixed well, centrifuged at 7000rpm, 4°C for 5min, the supernatant was discarded, and washed again;
(4) the cells were re-suspended with 100µl of Perm/Wash™ buffer of the working concentration. 2-3µl of Ki-67-APC antibody was added, and incubated at 4°C in dark for 30min;
(5) 1ml of Perm/Wash™ buffer of working concentration was added, mixed well, centrifuged at 7000rpm, 4°C for 5min, the supernatant was discarded, and washed again;
(6) the cells were re-suspended with 100µl of Perm/Wash™ buffer of the working concentration. 1µl Hoechst 33342 working solution was added and incubated on ice for 15min;
(7) 400µl of Perm/Wash™ buffer of the working concentration was further added, mixed well, and detected on the machine.

### 3. Experimental results

The results are shown in Figures 3A-1 to 3D-1.

The results show that MockT has the slowest proliferation rate, 137DCR1 has a slower proliferation rate, 137DCR2 has a faster proliferation rate, and 137DCR3 has the fastest proliferation rate.

### Example 4-(1): Detection of cell proliferation activity by Cell Proliferation Activity Kit

### 1. Experimental samples and reagents

The recombinant cells meso G1 CAR, meso G2 CAR, meso G1 CAR-137DCR2 and MockT prepared according to Example 2-(1).

CellTiter-Glo® Luminescent Cell Viability Assay, Promega, Cat.# G7570.

### 2. Experimental method

(1) The above cells that have been cultured for 8 days were added in a 96-well white plate in 100 µL of AIM-V medium (containing cells) for each well.
(2) A blank control without cells was used to obtain the background fluorescence value.
(3) the complexes to be tested were added to the plate and incubated for 30 min in an incubator.
(4) 100 µL of CellTiter-Glo reagent was added, mixed well on an oscillator for 2 minutes, and incubated at room temperature for 10 minutes, then readings were obtained.
(5) Detections were made according to the above steps on the 8th, 9th, and 10th day of the culture.

### 3. Experimental results

The results are shown in Figure 4-1.

The results show that MockT has the slowest proliferation rate, meso G1 CAR has a slower proliferation rate, meso G2 CAR has a faster proliferation rate, and meso G1 CAR-137DCR2 has the fastest proliferation rate.

### Example 4-(2): Detection of cell proliferation activity by Cell Proliferation Activity Kit

### 1. Experimental samples and reagents

The recombinant cells Mucl G1 CAR, Mucl G2 CAR, Mucl G1 CAR-28DCR2, 28DCR1, 28DCR2, 28DCR3 and Mock T prepared according to Example 2-(2).

CellTiter-Glo® Luminescent Cell Viability Assay, Promega, Cat.# G7570.

### 2. Experimental method

(1) The above cells that have been cultured for 8 days were added in a 96-well white plate in 100 µL of AIM-V medium (containing cells) for each well.
(2) A blank control without cells was used to obtain the background fluorescence value.
(3) the complexes to be tested were added to the plate and incubated for 30 min in an incubator.
(4) 100 µL of CellTiter-Glo reagent was added, mixed well on an oscillator for 2 minutes, and incubated at room temperature for 10 minutes, then readings were obtained.
(5) Detections were made according to the above steps on the 8th, 9th, and 10th day of the culture.

### 3. Experimental results

The results are shown in Figures 3-2 and 4-2.

Figure 3-2 shows that MockT has the slowest proliferation rate, and 28DCR has a faster proliferation rate.

Figure 4-2 shows that MockT has the slowest proliferation rate, Mucl G1 CAR has a slower proliferation rate, Mucl G2 CAR haw a faster proliferation rate, and Mucl G1 CAR-28DCR2 has the fastest proliferation rate.

### Example 4-(3): Detection of cell proliferation activity by Cell Proliferation Activity Kit

### 1. Experimental samples and reagents

The recombinant cells EGFR G1 CAR, EGFR G2 CAR, EGFR G1 CAR-40DCR2, 40DCR1, 40DCR2, 40DCR3 and Mock T prepared according to Example 2-(3).

CellTiter-Glo® Luminescent Cell Viability Assay, Promega, Cat.# G7570.

### 2. Experimental method

(1) The above cells that have been cultured for 8 days were added in a 96-well white plate in 100 µL of AIM-V medium (containing cells) for each well.
(2) A blank control without cells was used to obtain the background fluorescence value.
(3) the complexes to be tested was added to the plate and incubated for 30 min in an incubator.
(4) 100 µL of CellTiter-Glo reagent was added, mixed well on an oscillator for 2 minutes, and incubated at room temperature for 10 minutes, then readings were obtained.
(5) Detections were made according to the above steps on the 8th, 9th, and 10th day of the culture.

### 3. Experimental results

The results are shown in Figures 3-3 and 4-3.

Figure 3-3 shows that MockT has the slowest proliferation rate, and 40DCR has a faster proliferation rate.

Figure 4-3 shows that MockT has the slowest proliferation rate, EGFR G1 CAR has a slower proliferation rate, EGFR G2 CAR haw a faster proliferation rate, and EGFR G1 CAR-40DCR2 has the fastest proliferation rate.

### Example 5-(1): Detection of phenotype of dual costimulatory molecule activated receptor 137DCR combined with meso G1 CAR-T cell by flow cytometry under stimulation with mesothelin antigen

### 1. Experimental samples

The recombinant cells 137DCR1, 137DCR2, 137DCR 3, meso G1 CAR, meso G2 CAR, meso G1 CAR-137DCR3 and Mock T prepared according to Example 2-(1).

### 2. Experimental method

The above cells were each collected and added to a 1.5ml EP tube at 1×10⁶ cells/tube after counting, washed twice with PBS, centrifuged at 1200rpm for 5min, 2µl of isotype control antibody IgG1-PE, fluorescence flow antibody anti-CD137, isotype control (IgG1FITC+IgG1PC5+IgG1PE), (anti-CD45RO-PC5, anti-CD62L-FITC, anti-CCR7-PE) were added, the precipitate was flicked to make it mix evenly, incubated at room temperature in dark for 30 min, washed with PBS once, 400 µl PBS was added, the cells were transferred to a flow tube, and detected on the machine.3. Experimental results

The results are shown in Figures 5A-1 to 5D-1, Figures 6A-1 to 6D-1, Figures 7A-1 to 7D-1 and Figures 8A-1 to 8D-1. Wherein:
Figures 5A-1 to 5D-1 show 3 single-transformed cells: 137DCR1, 137DCR2, and 137DCR3, with the CD137 phenotype greatly improved compared to Mock T.

Figures 6A-1 to 6D-1 show the CD137 phenotype of Mock T, meso G1 CAR, meso G2 CAR, and meso G1 CAR-137DCR2. Compared with the other three groups, meso G1 CAR-137DCR2 is greatly improved.

Figures 7A-1 to 7D-1 show the CD45RO phenotype of Mock T, meso G1 CAR, meso G2 CAR, meso G1 CAR-137DCR2, indicating the degree of cell activation. Cells have been activated in large amounts.

Figures 8A-1 to 8D-1 show the memory T phenotypes of Mock T, meso G1 CAR, meso G2 CAR, and meso G1 CAR-137DCR2. Compared with the other three groups, meso G1 CAR-137DCR2 promotes the formation of memory T.

### Example 5-(2): Detection of phenotype of dual costimulatory molecule activated receptor 28DCR combined with Mucl G1 CAR-T cell by flow cytometry under stimulation with Mucl antigen

### 1. Experimental samples

The recombinant cells Mucl G1 CAR, Mucl G2 CAR, Mucl G1 CAR-28DCR3, 28DCR1, 28DCR2, 28DCR3 and Mock T prepared according to Example 2-(2).

### 2. Experimental method

The above cells were each collected and added to a 1.5ml EP tube at 1×10⁶ cells/tube after counting, washed twice with PBS, centrifuged at 1200rpm for 5min, 2µl of isotype control antibody IgG1-PE, fluorescence flow antibody anti-CD 137, isotype control (IgG1FITC+IgG1PC5+IgG1PE), (anti-CD45RO-PC5, anti-CD62L-FITC, anti-CCR7-PE) were added, the precipitate was flicked to make it mix evenly, incubated at room temperature in dark for 30 min, washed with PBS once, 400 µl PBS was added, the cells were transferred to a flow tube, and detected on the machine.

### 3. Experimental results

The results are shown in Figures 5A-2 to 5D-2, Figures 6A-2 to 6D-2, Figures 7A-2 to 7D-2 and Figures 8A-2 to 8D-2. Wherein:
Figures 5A-2 to 5D-2 show 3 single-transformed cells: 28DCR1, 28DCR2, and 28DCR3, with the CD137 phenotype greatly improved compared to Mock T.

Figures 6A-2 to 6D-2 show the CD137 phenotype of Mock T, Mucl G1 CAR, Mucl G2 CAR, and Mucl G1 CAR-28DCR2. Compared with the other three groups, Mucl G1 CAR-28DCR2 is greatly improved.

Figures 7A-2 to 7D-2 show the CD45RO phenotype of Mock T, Mucl G1 CAR, Mucl G2 CAR, Mucl G1 CAR-28DCR2, indicating the degree of cell activation. Cells have been activated in large amounts.

Figures 8A-2 to 8D-2 show the memory T phenotype of Mock T, Mucl G1 CAR, Mucl G2 CAR, and Mucl G1 CAR-28DCR2. Compared with the other three groups, Mucl G1 CAR-28DCR2 promotes the formation of memory T.

### Example 5-(3): Detection of phenotype of dual costimulatory molecule activated receptor 40DCR combined with EGFR G1 CAR-T cell by flow cytometry under stimulation with EGFR antigen

### 1. Experimental samples

The recombinant cells EGFR G1 CAR, EGFR G2 CAR, EGFR G1 CAR-40DCR3, 40DCR1, 40DCR2, 40DCR3 and Mock T prepared according to Example 2-(3).

### 2. Experimental method

The above cells were each collected and added to a 1.5ml EP tube at 1×10⁶ cells/tube after counting, washed twice with PBS, centrifuged at 1200rpm for 5min, 2µl of isotype control antibody IgG1-PE, fluorescence flow antibody anti-CD 137, isotype control (IgG1FITC+IgG1PC5+IgG1PE), (anti-CD45RO-PC5, anti-CD62L-FITC, anti-CCR7-PE) were added, the precipitate was flicked to make it mix evenly, incubated at room temperature in dark for 30 min, washed with PBS once, 400 µl PBS was added, the cells were transferred to a flow tube, and detected on the machine.

### 3. Experimental results

The results are shown in Figures 5A-3 to 5D-3, Figures 6A-3 to 6D-3, Figures 7A-3 to 7D-3 and Figures 8A-3 to 8D-3. Wherein:
Figures 5A-3 to 5D-3 show 3 single-transformed cells 40DCR1, 40DCR2, and 40DCR3, with the CD137 phenotype greatly improved compared to Mock T;
Figures 6A-3 to 6D-3 show the CD137 phenotype of Mock T, EGFR G1 CAR, EGFR G2 CAR, and EGFR G1 CAR-40DCR2. Compared with the other three groups, EGFR G1 CAR-40DCR2 is greatly improved;
Figures 7A-3 to 7D-3 show the CD45RO phenotype of Mock T, EGFR G1 CAR, EGFR G2 CAR, EGFR G1 CAR-40DCR2, indicating the degree of cell activation. Cells have been activated in large amounts;
Figures 8A-3 to 8D-3 show the memory T phenotype of Mock T, EGFR G1 CAR, EGFR G2 CAR, and EGFR G1 CAR-40DCR2. Compared with the other three groups, EGFR G1 CAR-40DCR2 promotes the formation of memory T.

### Example 6-(1): Detection of the killing effect in vitro of dual costimulatory molecule activated receptor 137DCR combined with meso G1 CAR-T cells on tumor cells by Real-time label-free cell analysis system

### 1. Experimental samples

Effector cells: the recombinant cells meso G1 CAR, meso G2 CAR, meso G1 CAR-137DCR1, meso G1 CAR-137DCR3 and Mock T prepared according to Example 2-(1).

Target cells: cervical cancer cell Hela, ovarian cancer cell SK-OV-3 (both from ATCC, American Type Culture Collection).

### 2. Experimental method

Target cells and effector cells that matched in MHC class I typing were selected, and Real-time label-free cell analysis system (RTCA) was used to detect the killing effect of the above cells *in vitro.* The steps were as follows:
(1) Zero adjustment: 50µl DMEM or 1640 culture medium was added to each well, put into the instrument, step 1 was selected for zero adjustment;
(2) Target cells plating: cervical cancer cell Hela and ovarian cancer cell SK-OV-3 were each plated at 10⁴ cells/50µl per well on a plate containing detection electrodes, placed for a few minutes to stabilize the cells, then put into the instrument, step 2 was started to culture the cells;
(3) Adding effector cells: After 24h culture of target cells, step 2 was paused and effector cells were added at 50µl per well, with the effector target ratio of 8:1 or 4:1 (both 10⁴ tumor cells) and non-transferred Mock T cells were used as a control, step3 was started to continue co-cultivation for 24h, then the cell proliferation curve was observed.

### 2. Experimental results

The results are shown in Figures 9A-1 to 9D-1.

The results show that Mock T has the weakest killing effect on tumor cells, meso G1 CAR has a weaker killing effect on tumor cells, meso G2 CAR has a stronger killing effect on tumor cells, meso G1 CAR-137DCR1 and meso G1 CAR-137DCR3 have the strongest killing effect on tumor cells.

### Example 6-(2): Detection of the killing effect in vitro of dual costimulatory molecule activated receptor 28DCR combined with Mucl G1 CAR-T cells on tumor cells by Real-time label-free cell analysis system

### 1. Experimental samples

Effector cells: the recombinant cells Mucl G1 CAR, Mucl G2 CAR, Mucl G1 CAR-28DCR1, Mucl G1 CAR-28DCR3, 28DCR1, 28DCR2, 28DCR3 and Mock T prepared according to Example 2-(2).

Target cells: cervical cancer cell Hela, ovarian cancer cell SK-OV-3 (both from ATCC, American Type Culture Collection).

### 2. Experimental method

Target cells and effector cells that matched in MHC class I typing were selected, and Real-time label-free cell analysis system (RTCA) was used to detect the killing effect of the above cells *in vitro.* The steps were as follows:
(1) Zero adjustment: 50µl DMEM or 1640 culture medium was added to each well, put into the instrument, step 1 was selected for zero adjustment;
(2) Target cells plating: cervical cancer cell Hela and ovarian cancer cell SK-OV-3 were each plated at 10⁴ cells/50µl per well on a plate containing detection electrodes, placed for a few minutes to stabilize the cells, then put into the instrument, step 2 was started to culture the cells;
(3) Adding effector cells: After 24h culture of target cells, step 2 was paused and effector cells were added at 50µl per well, with the effector target ratio of 8:1 or 4:1 (both 10⁴ tumor cells) and non-transferred Mock T cells were used as a control, step3 was started to continue co-cultivation for 24h, then the cell proliferation curve was observed.

### 2. Experimental results

The results are shown in Figures 9A-2 to 9D-2.

The results show that Mock T has the weakest killing effect on tumor cells, Mucl G1 CAR has a weaker killing effect on tumor cells, Mucl G2 CAR has a stronger killing effect on tumor cells, Mucl G1 CAR-28DCR1 and Mucl G1 CAR-28DCR3 have the strongest killing effect on tumor cells.

### Example 6-(3): Detection of the killing effect in vitro of dual costimulatory molecule activated receptor 40DCR combined with EGFR G1 CAR-T cells on tumor cells by Real-time label-free cell analysis system

### 1. Experimental samples

Effector cells: the recombinant cells EGFR G1 CAR, EGFR G2 CAR, EGFR G1 CAR-40DCR1, EGFR G1 CAR-40DCR3, 40DCR1, 40DCR2, 40DCR3 and Mock T prepared according to Example 2-(3).

Target cells: cervical cancer cell Hela, ovarian cancer cell SK-OV-3 (both from ATCC, American Type Culture Collection).

### 2. Experimental method

Target cells and effector cells that matched in MHC class I typing were selected, and Real-time label-free cell analysis system (RTCA) was used to detect the killing effect of the above cells *in vitro.* The steps were as follows:
(1) Zero adjustment: 50µl DMEM or 1640 culture medium was added to each well, put into the instrument, step 1 was selected for zero adjustment;
(2) Target cell plating: cervical cancer cell Hela and ovarian cancer cell SK-OV-3 were each plated at 10⁴ cells/50µl per well on a plate containing detection electrodes, placed for a few minutes to stabilize the cells, then put into the instrument, step 2 was started to culture the cells;
(3) Adding effector cells: After 24h culture of target cells, step 2 was paused and effector cells were added at 50µl per well, with the effector target ratio of 8:1 or 4:1 (both 10⁴ tumor cells) and non-transferred Mock T cells were used as a control, step3 was started to continue co-cultivation for 24h, then the cell proliferation curve was observed.

### 2. Experimental results

The results are shown in Figures 9A-3 to 9D-3.

The results show that Mock T has the weakest killing effect on tumor cells, EGFR G1 CAR has a weaker killing effect on tumor cells, EGFR G2 CAR has a stronger killing effect on tumor cells, EGFR G1 CAR-40DCR1 and EGFR G1 CAR-40DCR3 have the strongest killing effect on tumor cells.

### Example 7-(1): Detection by flow cytometry of cytokines secretion of meso-CAR-T upon stimulation with mesothelin antigen

### 1. Experimental samples

The recombinant cells meso G1 CAR, meso G2 CAR, meso G1 CAR-137DCR1 and Mock T prepared according to Example 2-(1).

### 2. Experimental method

1. A 96-well plate was coated with 5µg/ml mesothelin antigen overnight at 4°C, washed 3 times with PBS, and added with 1×10⁵ cells of each sample separately, and the cell supernatant was collected after 24 hours of culture. BD™CBA Human Th1/Th2 Cytokine Kit II was used to detect the cytokines secretion of meso CAR-T cells upon stimulation with mesothelin antigen.

Steps were as follows:
(1) Human IL-2, IL-4, IL-6, IL-10, TNF, IFN-γ capture magnetic beads were mixed by vortex, 50µl of mixed beads was added to each tube;
(2) 50µl of human Th1/Th2 cytokine standard (doubling diluted to 5000pg/ml, 2500pg/ml, 1250pg/ml, 625pg/ml, 312.5pg/ml, 156pg/ml, 80pg/ml, 40pg/ml, 20pg/ ml, or Opg/ml) and 50µl of the sample to be tested (diluted by 2-fold with the diluent) were added;
(3) 50µl of human Th1/Th2-II-PE detection antibody was added to each tube;
(4) Incubated at room temperature in dark for 3h;
(5) 1ml of Wash Buffer was added to each tube, centrifuged at 200 for 5min, and the supernatant was discarded;
(6) 300µl of Wash Buffer was added to each tube to resuspend the cells, and transferred to a flow cytometry tube for detecting the fluorescence value by a flow cytometer.

### 3. Experimental results

The results are shown in Figure 10-1.

The results show that compared with Mock T cells, the secretion amounts of various cytokines (IL-2, IL-4, IL-6, IL-10, TNF-α and IFN-γ) by other cells are greatly improved. Mock T has the lowest secretion amounts of various cytokines, meso G1 CAR has lower secretion amounts of various cytokines, meso G2 CAR has higher secretion amounts of various cytokines, meso G1 CAR-137DCR1 has the highest secretion amounts of various cytokines.

### Example 7-(2): Detection by flow cytometry of secretion amounts of cytokines by Muc1-CAR-T upon stimulation with Mucl antigen

### 1. Experimental samples

The recombinant cells Mucl G1 CAR, Mucl G2 CAR, Mucl G1 CAR-28DCR1 and Mock T prepared according to Example 2-(2).

### 2. Experimental method

1. A 96-well plate was coated with 5µg/ml Mucl antigen overnight at 4°C, washed 3 times with PBS, and added with 1×10⁵ cells of each sample separately, and the cell supernatant was collected after 24 hours of culture. BD™CBA Human Th1/Th2 Cytokine Kit II was used to detect the cytokines secretion of Mucl CAR-T cells upon stimulation with Mucl antigen.

Steps are as follows:
(1) Human IL-2, IL-4, IL-6, IL-10, TNF, IFN-γ capture magnetic beads were mixed by vortex, 50µl of mixed beads was added to each tube;
(2) 50µl of human Th1/Th2 cytokine standard (doubling diluted to 5000pg/ml, 2500pg/ml, 1250pg/ml, 625pg/ml, 312.5pg/ml, 156pg/ml, 80pg/ml, 40pg/ml, 20pg/ ml, or Opg/ml) and 50µl of the sample to be tested (diluted by 2-fold with the diluent) were added;
(3) 50µl of human Th1/Th2-II-PE detection antibody was added to each tube;
(4) Incubated at room temperature in dark for 3h;
(5) 1ml of Wash Buffer was added to each tube, centrifuged at 200 for 5min, and the supernatant was discarded;
(6) 300µl of Wash Buffer was added to each tube to resuspend the cells, and transferred to a flow cytometry tube for detecting the fluorescence value by a flow cytometer.

### 3. Experimental results

The results are shown in Figure 10-2.

The results show that compared with Mock T cells, the secretion amounts of various cytokines (IL-2, IL-4, IL-6, IL-10, TNF-α and IFN-γ) by other cells are greatly improved. Mock T has the lowest secretion amounts of various cytokines, Mucl G1 CAR has lower secretion amounts of various cytokines, Mucl G2 CAR has higher secretion amounts of various cytokines, Mucl G1 CAR-28DCR1 has the highest secretion amounts of various cytokines.

### Example 7-(3): Detection by flow cytometry of secretion amounts of cytokines by EGFR-CAR-T upon stimulation with EGFR antigen

### 1. Experimental samples

The recombinant cells EGFR G1 CAR, EGFR G2 CAR, EGFR G1 CAR-40DCR1 and Mock T prepared according to Example 2-(3).

### 2. Experimental method

1. A 96-well plate was coated with 5µg/ml EGFR antigen overnight at 4°C, washed 3 times with PBS, and added with 1×10⁵ cells of each sample separately, and the cell supernatant was collected after 24 hours of culture. BD™CBA Human Th1/Th2 Cytokine Kit II was used to detect the cytokines secretion of EGFR CAR-T cells upon stimulation with EGFR antigen.

Steps are as follows:
(1) Human IL-2, IL-4, IL-6, IL-10, TNF, IFN-γ capture magnetic beads were mixed by vortex, 50µl of mixed beads was added to each tube;
(2) 50µl of human Th1/Th2 cytokine standard (doubling diluted to 5000pg/ml, 2500pg/ml, 1250pg/ml, 625pg/ml, 312.5pg/ml, 156pg/ml, 80pg/ml, 40pg/ml, 20pg/ ml, or Opg/ml) and 50µl of the sample to be tested (diluted by 2-fold with the diluent) were added;
(3) 50µl of human Th1/Th2-II-PE detection antibody was added to each tube;
(4) Incubated at room temperature in dark for 3h;
(5) 1ml of Wash Buffer was added to each tube, centrifuged at 200 for 5min, and the supernatant was discarded;
(6) 300µl of Wash Buffer was added to each tube to resuspend the cells, and transferred to a flow cytometry tube for detecting the fluorescence value by a flow cytometer.

### 3. Experimental results

The results are shown in Figure 10-3.

The results show that compared with Mock T cells, the secretion amounts of various cytokines (IL-2, IL-4, IL-6, IL-10, TNF-α and IFN-γ) by other cells are greatly improved. Mock T has the lowest secretion amounts of various cytokines, EGFR G1 CAR has lower secretion amounts of various cytokines, EGFR G2 CAR has higher secretion amounts of various cytokines, EGFR G1 CAR-40DCR1 has the highest secretion amounts of various cytokines.

### Example 8-(1): Functional assay in vivo of dual costimulatory molecule activated receptor 137 DCR combined with meso G1 CAR-T cells

### 1. Experimental samples and animals

20 NSG immunodeficiency mice of 4-6 weeks old, with an average weight of 22-27g, were provided by Beijing Vitalstar Biotech Co., Ltd., and raised by a SPF animal laboratory.

The recombinant cells meso G1 CAR, meso G2 CAR, meso G1 CAR-137DCR3 and Mock T prepared according to Example 2-(1).

Ovarian cancer cell SK-OV-3-luc (Shanghai Huiying Biotechnology Co., Ltd.).

### 2. Experimental method

(1) Adhered human ovarian cancer cells SK-OV-3-luc in logarithmic growth phase cultured *in vitro* were digested with 0.25% trypsin, centrifuged, collected and re-suspended in PBS solution. The cells were centrifuged at 1000rmp for 2 minutes at room temperature, the supernatant was discarded and the cells were suspended in PBS solution, centrifuged to collect the cells, and the cell concentration of the cell suspension was adjusted to 5×10⁷ cells/ml.
(2) The SK-OV-3-luc cells were inoculated subcutaneously in the right dorsum of the mouse at 0.1 ml/mouse. 10 days after the inoculation, the sizes of the tumors were observed by a living body imager.
(4) the NSG immunodeficient mice were randomly divided into 4 groups (five mice each group). The administration was conducted through the tail vein injection at 0.1ml per mouse (5×10⁶ positive cells) with PBS as the solvent. The administration was conducted only once.
(4) the living states of mice were observed every day and the change of the tumor in each mouse was observed by a living body imager every 10 days.

### 3. Experimental results

The results are shown in Figure 11-1.

The results show the following: for the control group Mock T, the tumor cells have a strong fluorescence intensity; for the first generation mesothelin-targeting CAR, meso G1 CAR group, the tumor cells have a weakened fluorescence intensity, showing a certain therapeutic effect; for meso G2 CAR, the tumor cells have a weaker fluorescence intensity, showing a better therapeutic effect; for meso G1 CAR-137DCR3, the tumor cells have the weakest fluorescence intensity, showing the best therapeutic effect.

### Example 8-(2): Functional experiment in vivo of dual costimulatory molecule activated receptor 28DCR combined with Mucl G1 CAR-T cells

### 1. Experimental samples and animals

20 NSG immunodeficiency mice of 4-6 weeks old, with an average weight of 22-27g, were provided by Beijing Vitalstar Biotech Co., Ltd., and raised by a SPF animal laboratory.

The recombinant cells Mucl G1 CAR, Mucl G2 CAR, Mucl G1 CAR-28DCR3 and Mock T prepared according to Example 2-(2).

Ovarian cancer cell SK-OV-3-luc (Shanghai Huiying Biotechnology Co., Ltd.).

### 2. Experimental method

(1) Adhered human ovarian cancer cells SK-OV-3-luc in logarithmic growth phase cultured *in vitro* were digested with 0.25% trypsin, centrifuged, collected and re-suspended in PBS solution. The cells were centrifuged at 1000rmp for 2 minutes at room temperature, the supernatant was discarded and the cells were suspended in PBS solution, centrifuged to collect the cells, and the cell concentration of the cell suspension was adjusted to 5×10⁷ cells/ml.
(2) The SK-OV-3-luc cells were inoculated subcutaneously in the right dorsum of the mouse at 0.1 ml/mouse. 10 days after the inoculation, the sizes of the tumors were observed by a living body imager.
(3) the NSG immunodeficient mice were randomly divided into 4 groups (five mice each group). The administration was conducted through the tail vein injection at 0.1ml per mouse (5×10⁶ positive cells) with PBS as the solvent. The administration was conducted only once.
(4) the living states of mice were observed every day and the change of the tumor in each mouse was observed by the living body imager every 10 days.

### 3. Experimental results

The results are shown in Figure 11-2.

The results show the following: for the control group Mock T, the tumor cells have strong fluorescence intensity; for the first generation Mucl-targeting CAR, Mucl G1 CAR, the tumor cells have a weakened fluorescence intensity, showing a certain therapeutic effect; for Mucl G2 CAR, the tumor cells have a weaker fluorescence intensity, showing a better therapeutic effect; for Mucl G1 CAR-28DCR3, the tumor cells have the weakest fluorescence intensity, showing the best therapeutic effect.

### Example 8-(3): Functional experiment in vivo of dual costimulatory molecule activated receptor 40DCR combined with EGFR G1 CAR-T cells

### 1. Experimental samples and animals

20 NSG immunodeficiency mice of 4-6 weeks old, with an average weight of 22-27g, were provided by Beijing Vitalstar Biotech Co., Ltd., and raised by a SPF animal laboratory.

The recombinant cells EGFR G1 CAR, EGFR G2 CAR, EGFR G1 CAR-40DCR3 and Mock T prepared according to Example 2-(3).

Ovarian cancer cell SK-OV-3-luc (Shanghai Huiying Biotechnology Co., Ltd.).

### 2. Experimental method

(1) Adhered human ovarian cancer cells SK-OV-3-luc in logarithmic growth phase cultured *in vitro* were digested with 0.25% trypsin, centrifuged, collected and re-suspended in PBS solution. The cells were centrifuged at 1000rmp for 2 minutes at room temperature, the supernatant was discarded and the cells were suspended in PBS solution, centrifuged to collect the cells, and the cell concentration of the cell suspension was adjusted to 5×10⁷ cells/ml.
(2) The SK-OV-3-luc cells were inoculated subcutaneously in the right dorsum of the mouse at 0.1 ml/mouse. 10 days after the inoculation, the sizes of the tumors were observed by a living body imager.
(3) the NSG immunodeficient mice were randomly divided into 4 groups (five mice each group). The administration was conducted through the tail vein injection at 0.1ml per mouse (5×10⁶ positive cells) with PBS as the solvent. The administration was conducted only once.
(4) the living states of mice were observed every day and the change of the tumor in each mouse was observed by the living body imager every 10 days.

### 3. Experimental results

The results are shown in Figure 11-3.

The results show the following: for the control group Mock T, the tumor cells have a strong fluorescence intensity; for the first generation EGFR-targeting CAR, EGFR G1 CAR, the tumor cells have a weakened fluorescence intensity, showing a certain therapeutic effect; for EGFR G2 CAR, the tumor cells have a weaker fluorescence intensity, showing a better therapeutic effect; for EGFR G1 CAR-28DCR3, the tumor cells have the weakest fluorescence intensity, showing the best therapeutic effect.

Although specific embodiments of the present disclosure have been described in detail, those skilled in the art will understand, based on the disclosed teachings, various modifications and substitutions can be made to those details, and these changes are all within the protection scope of the present disclosure. The scope of the invention is provided by the appended claims and any equivalents thereof.

## Claims

1. An isolated polypeptide, comprising, from N-terminus to C-terminus, the following elements:
an optional signal peptide, a polypeptide that activates a costimulatory signal molecule (such as an agonistic single-chain antibody of the costimulatory signal molecule or a ligand of the costimulatory signal molecule), an extracellular hinge region, a transmembrane region, and an intracellular costimulatory signal molecule.

2. The polypeptide according to claim 1, wherein the polypeptide is **characterized by** any 1, 2, 3, 4 or 5 of the following items (1) to (5):
(1) the signal peptide is a membrane protein signal peptide; preferably, the signal peptide is one or more peptides selected from the group consisting of a CD8 signal peptide, a CD28 signal peptide, and a CD4 signal peptide; preferably, the signal peptide is a CD8 signal peptide; preferably, the amino acid sequence of the CD8 signal peptide is as shown in SEQ ID NO: 1;
(2) the agonistic single-chain antibody of the costimulatory signal molecule is any one or more members selected from the group consisting of a CD137 agonistic single-chain antibody, a CD28 agonistic single-chain antibody and a CD40 agonistic single-chain antibody; the ligand of the costimulatory signal molecule is any one or more members selected from the group consisting of a ligand of CD137, a ligand of CD28 and a ligand of CD40;
preferably, the amino acid sequence of the CD137 agonistic single-chain antibody is shown in SEQ ID NO: 2;
preferably, the amino acid sequence of the CD28 agonistic single-chain antibody is shown in SEQ ID NO: 31;
preferably, the amino acid sequence of the CD40 agonistic single-chain antibody is as shown in SEQ ID NO: 55;
preferably, the ligand of CD137 is 4-1BBL;
preferably, the ligand of CD28 is CD80/CD86;
preferably, the ligand of CD40 is CD40L;
(3) the extracellular hinge region is one or more members selected from the group consisting of an IgG4Fc CH2CH3 hinge region, a CD28 hinge region and a CD8 hinge region;
preferably, the extracellular hinge region is a CD8 hinge region;
preferably, the amino acid sequence of the CD8 hinge region is as shown in SEQ ID NO: 3;
preferably, the extracellular hinge region is an IgG4Fc CH2CH3 hinge region; preferably, the amino acid sequence of the IgG4Fc CH2CH3 hinge region is as shown in SEQ ID NO: 56;
(4) the transmembrane region is one or more members selected from the group consisting of a CD28 transmembrane region, a CD8 transmembrane region, a CD3ζ transmembrane region, a CD134 transmembrane region, a CD137 transmembrane region, an ICOS transmembrane region and a DAP10 transmembrane region; preferably, the transmembrane region is a CD28 transmembrane region; preferably, the amino acid sequence of the CD28 transmembrane region is as shown in SEQ ID NO: 4;
(5) the intracellular costimulatory signal molecule is any one or more of members selected from the group consisting of a CD28 intracellular domain, a CD134/OX40 intracellular domain, a CD137/4-1BB intracellular domain, an LCK intracellular domain, an ICOS intracellular domain and a DAP10 intracellular domain; preferably, the intracellular costimulatory signal molecule is a CD28 intracellular domain and/or a CD137 intracellular domain; preferably, the amino acid sequence of the CD28 intracellular domain is as shown in SEQ ID NO: 5; preferably, the amino acid sequence of the CD137 intracellular domain is as shown in SEQ ID NO: 6.

3. The polypeptide according to claim 1 or 2, comprising, from N-terminus to C-terminus, the following elements:
an optional CD8 signal peptide, a CD137 agonistic single-chain antibody, a CD8 extracellular hinge region, a CD28 transmembrane region, and a CD28 intracellular domain and/or a CD137 intracellular domain;
an optional CD8 signal peptide, a CD28 agonistic single-chain antibody, a CD8 extracellular hinge region, a CD28 transmembrane region, and a CD28 intracellular domain and/or a CD137 intracellular domain; or
an optional CD8 signal peptide, a CD40 agonistic single-chain antibody, a IgG4Fc CH2CH3 hinge region, a CD28 transmembrane region, and a CD28 intracellular domain and/or a CD137 intracellular domain.

4. The polypeptide according to any one of claims 1 to 3, wherein the amino acid sequence of the polypeptide is as shown in any one of SEQ ID NOs: 7 to 14;
any one of SEQ ID NOs: 32 to 39; or
any one of SEQ ID NOs: 57 to 64.

5. An isolated polynucleotide, wherein the isolated polynucleotide encodes the isolated polypeptide according to any one of claims 1 to 4; preferably, the sequence of the isolated polynucleotide is as shown in any one of SEQ ID NO: 15 or 22;
any one of SEQ ID NOs: 40 to 47; or
any one of SEQ ID NOs: 65 to 72.

6. A nucleic acid construct comprising the polynucleotide according to claim 5.

7. A recombinant vector, wherein the recombinant vector comprises the polynucleotide according to claim 5 or the nucleic acid construct according to claim 6; preferably, the recombinant vector is a recombinant cloning vector, a recombinant eukaryotic expression plasmid or a recombinant viral vector; preferably, the recombinant expression vector is a recombinant transposon vector; preferably, the transposon vector contains a transposition element selected from the group consisting of piggybac, sleeping beauty, frog prince, Tn5, and Ty; preferably, the recombinant expression vector is a recombinant vector obtained by recombining the polynucleotide according to claim 5 and PS328b vector.

8. A combination of recombinant vectors, comprising a first recombinant vector and a second recombinant vector, wherein:
the first recombinant vector is the recombinant vector according to claim 7,
the second recombinant vector contains a coding sequence of a first-generation chimeric antigen receptor; preferably, the first-generation chimeric antigen receptor is a first-generation chimeric antigen receptor that targets mesothelin, Mucl or EGFR; preferably, the amino acid sequence of the first-generation chimeric antigen receptor is as shown in SEQ ID NO: 23, SEQ ID NO: 48 or SEQ ID NO: 73; preferably, the nucleic acid sequence of the first-generation chimeric antigen receptor is as shown in SEQ ID NO: 24, SEQ ID NO: 49 or SEQ ID NO: 74;
preferably, the second recombinant vector is a recombinant PNB328 vector.

9. A recombinant host cell, wherein the cell contains the polynucleotide according to claim 5, the nucleic acid construct according to claim 6, the recombinant vector according to claim 7, or the combination of recombinant vectors according to claim 8; preferably, the recombinant host cell is a recombinant mammalian cell; preferably, the recombinant host cell is a recombinant T cell; preferably, the recombinant T cell is a recombinant peripheral blood mononuclear cell.

10. A T cell, wherein the T cell expresses the polypeptide according to any one of claims 1-4 and a first-generation chimeric antigen receptor; preferably, the recombinant T cell is a recombinant peripheral blood mononuclear cell; preferably, the first-generation chimeric antigen receptor is a first-generation chimeric antigen receptor that targets mesothelin, Mucl or EGFR; preferably, the amino acid sequence of the first-generation chimeric antigen receptor is as shown in SEQ ID NO: 23, SEQ ID NO: 48 or SEQ ID NO: 73.

11. A pharmaceutical composition comprising the polypeptide according to any one of claims 1-4, the polynucleotide according to claim 5, the nucleic acid construct according to claim 6, the recombinant vector according to claim 7, the combination of recombinant vectors according to claim 8, the recombinant host cell according to claim 9 or the T cell according to claim 10; optionally, further comprising a pharmaceutically acceptable excipient.

12. Use of the polypeptide according to any one of claims 1-4, the polynucleotide according to claim 5, the nucleic acid construct according to claim 6, the recombinant vector according to claim 7, the combination of recombinant vectors according to claim 8, the recombinant host cell according to claim 9 or the T cell according to claim 10 in the preparation of medicament for treating and/or preventing a cancer; preferably, the cancer being one that abnormally expresses mesothelin, Mucl or EGFR on the surface of its cancer cells; preferably, the cancer being selected from the group consisting of adenocarcinoma, lung cancer, colon cancer, colorectal cancer, breast cancer, ovarian cancer, cervical cancer, gastric cancer, bile duct cancer, gallbladder cancer, esophageal cancer, pancreatic cancer or prostate cancer.

13. Use of the polypeptide according to any one of claims 1-4, the polynucleotide according to claim 5, the nucleic acid construct according to claim 6, the recombinant vector according to claim 7, the combination of recombinant vectors according to claim 8, the recombinant host cell according to claim 9 or the T cell according to claim 10 in the preparation of medicament for inhibiting a cancer cell; preferably, the cancer cell being one that abnormally expresses mesothelin, Mucl or EGFR on the cell surface; preferably, the cancer cell being selected from the group consisting of: cancer cells of adenocarcinoma, lung cancer, colon cancer, colorectal cancer, breast cancer, ovarian cancer, cervical cancer, gastric cancer, bile duct cancer, gallbladder cancer, esophageal cancer, pancreatic cancer or prostate cancer.

14. Use of the polypeptide according to any one of claims 1-4, the polynucleotide according to claim 5, the nucleic acid construct according to claim 6, the recombinant vector according to claim 7, the combination of recombinant vectors according to claim 8, the recombinant host cell according to claim 9 or the T cell according to claim 10 in the preparation of a medicament for promoting secretion of a cytokine, wherein the cytokine is one or more members selected from the group consisting of IL-2, IL-4, IL-6, IL-10, TNF-α and IFN-γ.

15. The polypeptide according to any one of claims 1-4, the polynucleotide according to claim 5, the nucleic acid construct according to claim 6, the recombinant vector according to claim 7, the combination of recombinant vectors according to claim 8, the recombinant host cell according to claim 9 or the T cell according to claim 10, for use in treating and/or preventing a cancer; preferably, the cancer being one that abnormally expresses mesothelin, Mucl or EGFR on the surface of its cancer cells; preferably, the cancer being selected from the group consisting of adenocarcinoma, lung cancer, colon cancer, colorectal cancer, breast cancer, ovarian cancer, cervical cancer, gastric cancer, bile duct cancer, gallbladder cancer, esophageal cancer, pancreatic cancer or prostate cancer.

16. The polypeptide according to any one of claims 1-4, the polynucleotide according to claim 5, the nucleic acid construct according to claim 6, the recombinant vector according to claim 7, the combination of recombinant vectors according to claim 8, the recombinant host cell according to claim 9 or the T cell according to claim 10, for use in inhibiting a cancer cell; preferably, the cancer cell being one that abnormally expresses mesothelin, Mucl or EGFR on the cell surface; preferably, the cancer cell being selected from the group consisting of: cancer cells of adenocarcinoma, lung cancer, colon cancer, colorectal cancer, breast cancer, ovarian cancer, cervical cancer, gastric cancer, bile duct cancer, gallbladder cancer, esophageal cancer, pancreatic cancer or prostate cancer.

17. The polypeptide according to any one of claims 1-4, the polynucleotide according to claim 5, the nucleic acid construct according to claim 6, the recombinant vector according to claim 7, the combination of recombinant vectors according to claim 8, the recombinant host cell according to claim 9 or the T cell according to claim 10, for use in promoting the secretion of a cytokine, wherein the cytokine is one or more members selected from the group consisting of IL-2, IL-4, IL-6, IL-10, TNF-α and IFN-γ.

18. A method of treating and/or preventing cancer, comprising the step of administering to a subject in need thereof an effective amount of the polypeptide according to any one of claims 1-4, the polynucleotide according to claim 5, the nucleic acid construct according to claim 6, the recombinant vector according to claim 7, the combination of recombinant vectors according to claim 8, the recombinant host cell according to claim 9 or the T cell according to claim 10; preferably, the cancer being one that abnormally expresses mesothelin, Mucl or EGFR on the surface of its cancer cells; preferably, the cancer being selected from the group consisting of adenocarcinoma, lung cancer, colon cancer, colorectal cancer, breast cancer, ovarian cancer, cervical cancer, gastric cancer, bile duct cancer, gallbladder cancer, esophageal cancer, pancreatic cancer or prostate cancer.

19. A method of inhibiting a cancer cell *in vivo* or *in vitro,* comprising the step of administering to the cancer cell an effective amount of the polypeptide according to any one of claims 1-4, the polynucleotide according to claim 5, the nucleic acid construct according to claim 6, the recombinant vector according to claim 7, the combination of recombinant vectors according to claim 8, the recombinant host cell according to claim 9 or the T cell according to claim 10; preferably, the cancer cell being one that abnormally expresses mesothelin, Mucl or EGFR on the cell surface; preferably, the cancer cell being selected from the group consisting of: cancer cells of adenocarcinoma, lung cancer, colon cancer, colorectal cancer, breast cancer, ovarian cancer, cervical cancer, gastric cancer, bile duct cancer, gallbladder cancer, esophageal cancer, pancreatic cancer or prostate cancer.

20. A method of promoting the secretion of a cytokine by T cells *in vivo* or *in vitro,* comprising the step of administering to the T cells an effective amount of the polypeptide according to any one of claims 1-4, the polynucleotide according to claim 5, the nucleic acid construct according to claim 6, the recombinant vector according to claim 7, the combination of recombinant vectors according to claim 8, the recombinant host cell according to claim 9 or the T cell according to claim 10, wherein the cytokine is one or more members selected from the group consisting of IL-2, IL-4, IL-6, IL-10, TNF-α and IFN-γ.


SEQUENCE LISTING

<110>  SHANGHAI CELL THERAPY RESEARCH INSTITUTE
       SHANGHAI CELL THERAPY GROUP CO., LTD

<120>  DUAL-ACTIVATING COSTIMULATORY MOLECULE RECEPTOR AND USE THEREOF

<130>  IEC180209PCT

<150>  201711462081.7
<151>  2017-12-28

<160>  78

<170>  PatentIn version 3.5

<210>  1
<211>  23
<212>  PRT
<213>  Artificial Sequence

<220>
<223>  Amino acid sequence of CD8 signal peptide

<400>  1

Met Gly Asn Ser Cys Tyr Asn Ile Val Ala Thr Leu Leu Leu Val Leu
1               5                   10                  15

Asn Phe Glu Arg Thr Arg Ser
            20

<210>  2
<211>  246
<212>  PRT
<213>  Artificial Sequence

<220>
<223>  Amino acid sequence of CD137 extracellular agonistic single-chain
antibody

<400>  2

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Ser Pro Glu Lys Gly Leu Glu Trp Ile
        35                  40                  45

Gly Glu Ile Asn His Gly Gly Tyr Val Thr Tyr Asn Pro Ser Leu Glu
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Asp Tyr Gly Pro Gly Asn Tyr Asp Trp Tyr Phe Asp Leu Trp Gly
            100                 105                 110

Arg Gly Thr Leu Val Thr Val Ser Ser Gly Gly Gly Gly Ser Gly Gly
        115                 120                 125

Gly Gly Ser Gly Gly Gly Gly Ser Glu Ile Val Leu Thr Gln Ser Pro
    130                 135                 140

Ala Thr Leu Ser Leu Ser Pro Gly Glu Arg Ala Thr Leu Ser Cys Arg
145                 150                 155                 160

Ala Ser Gln Ser Val Ser Ser Tyr Leu Ala Trp Tyr Gln Gln Lys Pro
                165                 170                 175

Gly Gln Ala Pro Arg Leu Leu Ile Tyr Asp Ala Ser Asn Arg Ala Thr
            180                 185                 190

Gly Ile Pro Ala Arg Phe Ser Gly Ser Gly Ser Gly Thr Asp Phe Thr
        195                 200                 205

Leu Thr Ile Ser Ser Leu Glu Pro Glu Asp Phe Ala Val Tyr Tyr Cys
    210                 215                 220

Gln Gln Arg Ser Asn Trp Pro Pro Ala Leu Thr Phe Gly Gly Gly Thr
225                 230                 235                 240

Lys Val Glu Ile Lys Glu
                245

<210>  3
<211>  49
<212>  PRT
<213>  Artificial Sequence

<220>
<223>  Amino acid sequence of CD8¦Á hinge region

<400>  3

Ser Lys Tyr Gly Pro Pro Cys Pro Pro Cys Pro Ile Glu Val Met Tyr
1               5                   10                  15

Pro Pro Pro Tyr Leu Asp Asn Glu Lys Ser Asn Gly Thr Ile Ile His
            20                  25                  30

Val Lys Gly Lys His Leu Cys Pro Ser Pro Leu Phe Pro Gly Pro Ser
        35                  40                  45

Lys

<210>  4
<211>  28
<212>  PRT
<213>  Artificial Sequence

<220>
<223>  Amino acid sequence of CD28 transmembrane region

<400>  4

Pro Phe Trp Val Leu Val Val Val Gly Gly Val Leu Ala Cys Tyr Ser
1               5                   10                  15

Leu Leu Val Thr Val Ala Phe Ile Ile Phe Trp Val
            20                  25

<210>  5
<211>  41
<212>  PRT
<213>  Artificial Sequence

<220>
<223>  Amino acid sequence of CD28 intracellular costimulatory signal domain

<400>  5

Arg Ser Lys Arg Ser Arg Leu Leu His Ser Asp Tyr Met Asn Met Thr
1               5                   10                  15

Pro Arg Arg Pro Gly Pro Thr Arg Lys His Tyr Gln Pro Tyr Ala Pro
            20                  25                  30

Pro Arg Asp Phe Ala Ala Tyr Arg Ser
        35                  40

<210>  6
<211>  42
<212>  PRT
<213>  Artificial Sequence

<220>
<223>  Amino acid sequence of CD137 intracellular costimulatory signal domain

<400>  6

Lys Arg Gly Arg Lys Lys Leu Leu Tyr Ile Phe Lys Gln Pro Phe Met
1               5                   10                  15

Arg Pro Val Gln Thr Thr Gln Glu Glu Asp Gly Cys Ser Cys Arg Phe
            20                  25                  30

Pro Glu Glu Glu Glu Gly Gly Cys Glu Leu
        35                  40

<210>  7
<211>  387
<212>  PRT
<213>  Artificial Sequence

<220>
<223>  Amino acid sequence of 137DCR1 (including signal peptide)

<400>  7

Met Gly Asn Ser Cys Tyr Asn Ile Val Ala Thr Leu Leu Leu Val Leu
1               5                   10                  15

Asn Phe Glu Arg Thr Arg Ser Gln Val Gln Leu Gln Gln Trp Gly Ala
            20                  25                  30

Gly Leu Leu Lys Pro Ser Glu Thr Leu Ser Leu Thr Cys Ala Val Tyr
        35                  40                  45

Gly Gly Ser Phe Ser Gly Tyr Tyr Trp Ser Trp Ile Arg Gln Ser Pro
    50                  55                  60

Glu Lys Gly Leu Glu Trp Ile Gly Glu Ile Asn His Gly Gly Tyr Val
65                  70                  75                  80

Thr Tyr Asn Pro Ser Leu Glu Ser Arg Val Thr Ile Ser Val Asp Thr
                85                  90                  95

Ser Lys Asn Gln Phe Ser Leu Lys Leu Ser Ser Val Thr Ala Ala Asp
            100                 105                 110

Thr Ala Val Tyr Tyr Cys Ala Arg Asp Tyr Gly Pro Gly Asn Tyr Asp
        115                 120                 125

Trp Tyr Phe Asp Leu Trp Gly Arg Gly Thr Leu Val Thr Val Ser Ser
    130                 135                 140

Gly Gly Gly Gly Ser Gly Gly Gly Gly Ser Gly Gly Gly Gly Ser Glu
145                 150                 155                 160

Ile Val Leu Thr Gln Ser Pro Ala Thr Leu Ser Leu Ser Pro Gly Glu
                165                 170                 175

Arg Ala Thr Leu Ser Cys Arg Ala Ser Gln Ser Val Ser Ser Tyr Leu

180                 185                 190

Ala Trp Tyr Gln Gln Lys Pro Gly Gln Ala Pro Arg Leu Leu Ile Tyr
        195                 200                 205

Asp Ala Ser Asn Arg Ala Thr Gly Ile Pro Ala Arg Phe Ser Gly Ser
    210                 215                 220

Gly Ser Gly Thr Asp Phe Thr Leu Thr Ile Ser Ser Leu Glu Pro Glu
225                 230                 235                 240

Asp Phe Ala Val Tyr Tyr Cys Gln Gln Arg Ser Asn Trp Pro Pro Ala
                245                 250                 255

Leu Thr Phe Gly Gly Gly Thr Lys Val Glu Ile Lys Glu Ser Lys Tyr
            260                 265                 270

Gly Pro Pro Cys Pro Pro Cys Pro Ile Glu Val Met Tyr Pro Pro Pro
        275                 280                 285

Tyr Leu Asp Asn Glu Lys Ser Asn Gly Thr Ile Ile His Val Lys Gly
    290                 295                 300

Lys His Leu Cys Pro Ser Pro Leu Phe Pro Gly Pro Ser Lys Pro Phe
305                 310                 315                 320

Trp Val Leu Val Val Val Gly Gly Val Leu Ala Cys Tyr Ser Leu Leu
                325                 330                 335

Val Thr Val Ala Phe Ile Ile Phe Trp Val Arg Ser Lys Arg Ser Arg
            340                 345                 350

Leu Leu His Ser Asp Tyr Met Asn Met Thr Pro Arg Arg Pro Gly Pro
        355                 360                 365

Thr Arg Lys His Tyr Gln Pro Tyr Ala Pro Pro Arg Asp Phe Ala Ala
    370                 375                 380

Tyr Arg Ser
385

<210>  8
<211>  388
<212>  PRT
<213>  Artificial Sequence

<220>
<223>  Amino acid sequence of 137DCR2 (including signal peptide)

<400>  8

Met Gly Asn Ser Cys Tyr Asn Ile Val Ala Thr Leu Leu Leu Val Leu
1               5                   10                  15

Asn Phe Glu Arg Thr Arg Ser Gln Val Gln Leu Gln Gln Trp Gly Ala
            20                  25                  30

Gly Leu Leu Lys Pro Ser Glu Thr Leu Ser Leu Thr Cys Ala Val Tyr
        35                  40                  45

Gly Gly Ser Phe Ser Gly Tyr Tyr Trp Ser Trp Ile Arg Gln Ser Pro
    50                  55                  60

Glu Lys Gly Leu Glu Trp Ile Gly Glu Ile Asn His Gly Gly Tyr Val
65                  70                  75                  80

Thr Tyr Asn Pro Ser Leu Glu Ser Arg Val Thr Ile Ser Val Asp Thr
                85                  90                  95

Ser Lys Asn Gln Phe Ser Leu Lys Leu Ser Ser Val Thr Ala Ala Asp
            100                 105                 110

Thr Ala Val Tyr Tyr Cys Ala Arg Asp Tyr Gly Pro Gly Asn Tyr Asp
        115                 120                 125

Trp Tyr Phe Asp Leu Trp Gly Arg Gly Thr Leu Val Thr Val Ser Ser
    130                 135                 140

Gly Gly Gly Gly Ser Gly Gly Gly Gly Ser Gly Gly Gly Gly Ser Glu
145                 150                 155                 160

Ile Val Leu Thr Gln Ser Pro Ala Thr Leu Ser Leu Ser Pro Gly Glu
                165                 170                 175

Arg Ala Thr Leu Ser Cys Arg Ala Ser Gln Ser Val Ser Ser Tyr Leu
            180                 185                 190

Ala Trp Tyr Gln Gln Lys Pro Gly Gln Ala Pro Arg Leu Leu Ile Tyr
        195                 200                 205

Asp Ala Ser Asn Arg Ala Thr Gly Ile Pro Ala Arg Phe Ser Gly Ser
    210                 215                 220

Gly Ser Gly Thr Asp Phe Thr Leu Thr Ile Ser Ser Leu Glu Pro Glu
225                 230                 235                 240

Asp Phe Ala Val Tyr Tyr Cys Gln Gln Arg Ser Asn Trp Pro Pro Ala

                245                 250                 255

Leu Thr Phe Gly Gly Gly Thr Lys Val Glu Ile Lys Glu Ser Lys Tyr
            260                 265                 270

Gly Pro Pro Cys Pro Pro Cys Pro Ile Glu Val Met Tyr Pro Pro Pro
        275                 280                 285

Tyr Leu Asp Asn Glu Lys Ser Asn Gly Thr Ile Ile His Val Lys Gly
    290                 295                 300

Lys His Leu Cys Pro Ser Pro Leu Phe Pro Gly Pro Ser Lys Pro Phe
305                 310                 315                 320

Trp Val Leu Val Val Val Gly Gly Val Leu Ala Cys Tyr Ser Leu Leu
                325                 330                 335

Val Thr Val Ala Phe Ile Ile Phe Trp Val Lys Arg Gly Arg Lys Lys
            340                 345                 350

Leu Leu Tyr Ile Phe Lys Gln Pro Phe Met Arg Pro Val Gln Thr Thr
        355                 360                 365

Gln Glu Glu Asp Gly Cys Ser Cys Arg Phe Pro Glu Glu Glu Glu Gly
    370                 375                 380

Gly Cys Glu Leu
385

<210> 9
<211> 429
<212> PRT
<213> Artificial Sequence

<220>
<223> Amino acid sequence of 137DCR3 (including signal peptide)

<400> 9

Met Gly Asn Ser Cys Tyr Asn Ile Val Ala Thr Leu Leu Leu Val Leu
1               5                   10                  15

Asn Phe Glu Arg Thr Arg Ser Gln Val Gln Leu Gln Gln Trp Gly Ala
            20                  25                  30

Gly Leu Leu Lys Pro Ser Glu Thr Leu Ser Leu Thr Cys Ala Val Tyr
        35                  40                  45

Gly Gly Ser Phe Ser Gly Tyr Tyr Trp Ser Trp Ile Arg Gln Ser Pro
    50                  55                  60

Glu Lys Gly Leu Glu Trp Ile Gly Glu Ile Asn His Gly Gly Tyr Val
65                  70                  75                  80

Thr Tyr Asn Pro Ser Leu Glu Ser Arg Val Thr Ile Ser Val Asp Thr
                85                  90                  95

Ser Lys Asn Gln Phe Ser Leu Lys Leu Ser Ser Val Thr Ala Ala Asp
            100                 105                 110

Thr Ala Val Tyr Tyr Cys Ala Arg Asp Tyr Gly Pro Gly Asn Tyr Asp
        115                 120                 125

Trp Tyr Phe Asp Leu Trp Gly Arg Gly Thr Leu Val Thr Val Ser Ser
    130                 135                 140

Gly Gly Gly Gly Ser Gly Gly Gly Gly Ser Gly Gly Gly Gly Ser Glu
145                 150                 155                 160

Ile Val Leu Thr Gln Ser Pro Ala Thr Leu Ser Leu Ser Pro Gly Glu
                165                 170                 175

Arg Ala Thr Leu Ser Cys Arg Ala Ser Gln Ser Val Ser Ser Tyr Leu
            180                 185                 190

Ala Trp Tyr Gln Gln Lys Pro Gly Gln Ala Pro Arg Leu Leu Ile Tyr
        195                 200                 205

Asp Ala Ser Asn Arg Ala Thr Gly Ile Pro Ala Arg Phe Ser Gly Ser
    210                 215                 220

Gly Ser Gly Thr Asp Phe Thr Leu Thr Ile Ser Ser Leu Glu Pro Glu
225                 230                 235                 240

Asp Phe Ala Val Tyr Tyr Cys Gln Gln Arg Ser Asn Trp Pro Pro Ala
                245                 250                 255

Leu Thr Phe Gly Gly Gly Thr Lys Val Glu Ile Lys Glu Ser Lys Tyr
            260                 265                 270

Gly Pro Pro Cys Pro Pro Cys Pro Ile Glu Val Met Tyr Pro Pro Pro
        275                 280                 285

Tyr Leu Asp Asn Glu Lys Ser Asn Gly Thr Ile Ile His Val Lys Gly
    290                 295                 300

Lys His Leu Cys Pro Ser Pro Leu Phe Pro Gly Pro Ser Lys Pro Phe

305                 310                 315                 320

Trp Val Leu Val Val Val Gly Gly Val Leu Ala Cys Tyr Ser Leu Leu
                325                 330                 335

Val Thr Val Ala Phe Ile Ile Phe Trp Val Arg Ser Lys Arg Ser Arg
            340                 345                 350

Leu Leu His Ser Asp Tyr Met Asn Met Thr Pro Arg Arg Pro Gly Pro
        355                 360                 365

Thr Arg Lys His Tyr Gln Pro Tyr Ala Pro Pro Arg Asp Phe Ala Ala
    370                 375                 380

Tyr Arg Ser Lys Arg Gly Arg Lys Lys Leu Leu Tyr Ile Phe Lys Gln
385                 390                 395                 400

Pro Phe Met Arg Pro Val Gln Thr Thr Gln Glu Glu Asp Gly Cys Ser
                405                 410                 415

Cys Arg Phe Pro Glu Glu Glu Glu Gly Gly Cys Glu Leu
            420                 425

<210>  10
<211>  429
<212>  PRT
<213>  Artificial Sequence

<220>
<223>  Amino acid sequence of 137DCR4 (including signal peptide)

<400>  10

Met Gly Asn Ser Cys Tyr Asn Ile Val Ala Thr Leu Leu Leu Val Leu
1               5                   10                  15

Asn Phe Glu Arg Thr Arg Ser Gln Val Gln Leu Gln Gln Trp Gly Ala
            20                  25                  30

Gly Leu Leu Lys Pro Ser Glu Thr Leu Ser Leu Thr Cys Ala Val Tyr
        35                  40                  45

Gly Gly Ser Phe Ser Gly Tyr Tyr Trp Ser Trp Ile Arg Gln Ser Pro
    50                  55                  60

Glu Lys Gly Leu Glu Trp Ile Gly Glu Ile Asn His Gly Gly Tyr Val
65                  70                  75                  80

Thr Tyr Asn Pro Ser Leu Glu Ser Arg Val Thr Ile Ser Val Asp Thr
                85                  90                  95

Ser Lys Asn Gln Phe Ser Leu Lys Leu Ser Ser Val Thr Ala Ala Asp
        100                 105                 110

Thr Ala Val Tyr Tyr Cys Ala Arg Asp Tyr Gly Pro Gly Asn Tyr Asp
    115                 120                 125

Trp Tyr Phe Asp Leu Trp Gly Arg Gly Thr Leu Val Thr Val Ser Ser
    130                 135                 140

Gly Gly Gly Gly Ser Gly Gly Gly Gly Ser Gly Gly Gly Gly Ser Glu
145                 150                 155                 160

Ile Val Leu Thr Gln Ser Pro Ala Thr Leu Ser Leu Ser Pro Gly Glu
                165                 170                 175

Arg Ala Thr Leu Ser Cys Arg Ala Ser Gln Ser Val Ser Ser Tyr Leu
        180                 185                 190

Ala Trp Tyr Gln Gln Lys Pro Gly Gln Ala Pro Arg Leu Leu Ile Tyr
    195                 200                 205

Asp Ala Ser Asn Arg Ala Thr Gly Ile Pro Ala Arg Phe Ser Gly Ser
    210                 215                 220

Gly Ser Gly Thr Asp Phe Thr Leu Thr Ile Ser Ser Leu Glu Pro Glu
225                 230                 235                 240

Asp Phe Ala Val Tyr Tyr Cys Gln Gln Arg Ser Asn Trp Pro Pro Ala
                245                 250                 255

Leu Thr Phe Gly Gly Gly Thr Lys Val Glu Ile Lys Glu Ser Lys Tyr
        260                 265                 270

Gly Pro Pro Cys Pro Pro Cys Pro Ile Glu Val Met Tyr Pro Pro Pro
    275                 280                 285

Tyr Leu Asp Asn Glu Lys Ser Asn Gly Thr Ile Ile His Val Lys Gly
    290                 295                 300

Lys His Leu Cys Pro Ser Pro Leu Phe Pro Gly Pro Ser Lys Pro Phe
305                 310                 315                 320

Trp Val Leu Val Val Val Gly Gly Val Leu Ala Cys Tyr Ser Leu Leu
                325                 330                 335

Val Thr Val Ala Phe Ile Ile Phe Trp Val Lys Arg Gly Arg Lys Lys

340                 345                 350

Leu Leu Tyr Ile Phe Lys Gln Pro Phe Met Arg Pro Val Gln Thr Thr
        355                 360                 365

Gln Glu Glu Asp Gly Cys Ser Cys Arg Phe Pro Glu Glu Glu Glu Gly
    370                 375                 380

Gly Cys Glu Leu Arg Ser Lys Arg Ser Arg Leu Leu His Ser Asp Tyr
385                 390                 395                 400

Met Asn Met Thr Pro Arg Arg Pro Gly Pro Thr Arg Lys His Tyr Gln
                405                 410                 415

Pro Tyr Ala Pro Pro Arg Asp Phe Ala Ala Tyr Arg Ser
            420                 425

<210>  11
<211>  364
<212>  PRT
<213>  Artificial Sequence

<220>
<223>  Amino acid sequence of 137DCR1 (without signal peptide)

<400>  11

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Ser Pro Glu Lys Gly Leu Glu Trp Ile
        35                  40                  45

Gly Glu Ile Asn His Gly Gly Tyr Val Thr Tyr Asn Pro Ser Leu Glu
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Asp Tyr Gly Pro Gly Asn Tyr Asp Trp Tyr Phe Asp Leu Trp Gly
            100                 105                 110

Arg Gly Thr Leu Val Thr Val Ser Ser Gly Gly Gly Gly Ser Gly Gly
        115                 120                 125

Gly Gly Ser Gly Gly Gly Gly Ser Glu Ile Val Leu Thr Gln Ser Pro
    130                 135                 140

Ala Thr Leu Ser Leu Ser Pro Gly Glu Arg Ala Thr Leu Ser Cys Arg
145                 150                 155                 160

Ala Ser Gln Ser Val Ser Ser Tyr Leu Ala Trp Tyr Gln Gln Lys Pro
                165                 170                 175

Gly Gln Ala Pro Arg Leu Leu Ile Tyr Asp Ala Ser Asn Arg Ala Thr
            180                 185                 190

Gly Ile Pro Ala Arg Phe Ser Gly Ser Gly Ser Gly Thr Asp Phe Thr
        195                 200                 205

Leu Thr Ile Ser Ser Leu Glu Pro Glu Asp Phe Ala Val Tyr Tyr Cys
    210                 215                 220

Gln Gln Arg Ser Asn Trp Pro Pro Ala Leu Thr Phe Gly Gly Gly Thr
225                 230                 235                 240

Lys Val Glu Ile Lys Glu Ser Lys Tyr Gly Pro Pro Cys Pro Pro Cys
                245                 250                 255

Pro Ile Glu Val Met Tyr Pro Pro Pro Tyr Leu Asp Asn Glu Lys Ser
            260                 265                 270

Asn Gly Thr Ile Ile His Val Lys Gly Lys His Leu Cys Pro Ser Pro
        275                 280                 285

Leu Phe Pro Gly Pro Ser Lys Pro Phe Trp Val Leu Val Val Val Gly
    290                 295                 300

Gly Val Leu Ala Cys Tyr Ser Leu Leu Val Thr Val Ala Phe Ile Ile
305                 310                 315                 320

Phe Trp Val Arg Ser Lys Arg Ser Arg Leu Leu His Ser Asp Tyr Met
                325                 330                 335

Asn Met Thr Pro Arg Arg Pro Gly Pro Thr Arg Lys His Tyr Gln Pro
            340                 345                 350

Tyr Ala Pro Pro Arg Asp Phe Ala Ala Tyr Arg Ser
        355                 360

<210>  12

<211>  365
<212>  PRT
<213>  Artificial Sequence

<220>
<223>  Amino acid sequence of 137DCR2 (without signal peptide)

<400>  12

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Ser Pro Glu Lys Gly Leu Glu Trp Ile
        35                  40                  45

Gly Glu Ile Asn His Gly Gly Tyr Val Thr Tyr Asn Pro Ser Leu Glu
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Asp Tyr Gly Pro Gly Asn Tyr Asp Trp Tyr Phe Asp Leu Trp Gly
            100                 105                 110

Arg Gly Thr Leu Val Thr Val Ser Ser Gly Gly Gly Gly Ser Gly Gly
        115                 120                 125

Gly Gly Ser Gly Gly Gly Gly Ser Glu Ile Val Leu Thr Gln Ser Pro
    130                 135                 140

Ala Thr Leu Ser Leu Ser Pro Gly Glu Arg Ala Thr Leu Ser Cys Arg
145                 150                 155                 160

Ala Ser Gln Ser Val Ser Ser Tyr Leu Ala Trp Tyr Gln Gln Lys Pro
                165                 170                 175

Gly Gln Ala Pro Arg Leu Leu Ile Tyr Asp Ala Ser Asn Arg Ala Thr
            180                 185                 190

Gly Ile Pro Ala Arg Phe Ser Gly Ser Gly Ser Gly Thr Asp Phe Thr
        195                 200                 205

Leu Thr Ile Ser Ser Leu Glu Pro Glu Asp Phe Ala Val Tyr Tyr Cys
    210                 215                 220

Gln Gln Arg Ser Asn Trp Pro Pro Ala Leu Thr Phe Gly Gly Gly Thr
225                 230                 235                 240

Lys Val Glu Ile Lys Glu Ser Lys Tyr Gly Pro Pro Cys Pro Pro Cys
                245                 250                 255

Pro Ile Glu Val Met Tyr Pro Pro Pro Tyr Leu Asp Asn Glu Lys Ser
            260                 265                 270

Asn Gly Thr Ile Ile His Val Lys Gly Lys His Leu Cys Pro Ser Pro
        275                 280                 285

Leu Phe Pro Gly Pro Ser Lys Pro Phe Trp Val Leu Val Val Val Gly
    290                 295                 300

Gly Val Leu Ala Cys Tyr Ser Leu Leu Val Thr Val Ala Phe Ile Ile
305                 310                 315                 320

Phe Trp Val Lys Arg Gly Arg Lys Lys Leu Leu Tyr Ile Phe Lys Gln
                325                 330                 335

Pro Phe Met Arg Pro Val Gln Thr Thr Gln Glu Glu Asp Gly Cys Ser
            340                 345                 350

Cys Arg Phe Pro Glu Glu Glu Glu Gly Gly Cys Glu Leu
        355                 360                 365

<210>  13
<211>  406
<212>  PRT
<213>  Artificial Sequence

<220>
<223>  Amino acid sequence of 137DCR3 (without signal peptide)

<400>  13

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Ser Pro Glu Lys Gly Leu Glu Trp Ile
        35                  40                  45

Gly Glu Ile Asn His Gly Gly Tyr Val Thr Tyr Asn Pro Ser Leu Glu
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Asp Tyr Gly Pro Gly Asn Tyr Asp Trp Tyr Phe Asp Leu Trp Gly
            100                 105                 110

Arg Gly Thr Leu Val Thr Val Ser Ser Gly Gly Gly Gly Ser Gly Gly
        115                 120                 125

Gly Gly Ser Gly Gly Gly Gly Ser Glu Ile Val Leu Thr Gln Ser Pro
    130                 135                 140

Ala Thr Leu Ser Leu Ser Pro Gly Glu Arg Ala Thr Leu Ser Cys Arg
145                 150                 155                 160

Ala Ser Gln Ser Val Ser Ser Tyr Leu Ala Trp Tyr Gln Gln Lys Pro
                165                 170                 175

Gly Gln Ala Pro Arg Leu Leu Ile Tyr Asp Ala Ser Asn Arg Ala Thr
            180                 185                 190

Gly Ile Pro Ala Arg Phe Ser Gly Ser Gly Ser Gly Thr Asp Phe Thr
        195                 200                 205

Leu Thr Ile Ser Ser Leu Glu Pro Glu Asp Phe Ala Val Tyr Tyr Cys
    210                 215                 220

Gln Gln Arg Ser Asn Trp Pro Pro Ala Leu Thr Phe Gly Gly Gly Thr
225                 230                 235                 240

Lys Val Glu Ile Lys Glu Ser Lys Tyr Gly Pro Pro Cys Pro Pro Cys
                245                 250                 255

Pro Ile Glu Val Met Tyr Pro Pro Pro Tyr Leu Asp Asn Glu Lys Ser
            260                 265                 270

Asn Gly Thr Ile Ile His Val Lys Gly Lys His Leu Cys Pro Ser Pro
        275                 280                 285

Leu Phe Pro Gly Pro Ser Lys Pro Phe Trp Val Leu Val Val Val Gly
    290                 295                 300

Gly Val Leu Ala Cys Tyr Ser Leu Leu Val Thr Val Ala Phe Ile Ile
305                 310                 315                 320

Phe Trp Val Arg Ser Lys Arg Ser Arg Leu Leu His Ser Asp Tyr Met
                325                 330                 335

Asn Met Thr Pro Arg Arg Pro Gly Pro Thr Arg Lys His Tyr Gln Pro
            340                 345                 350

Tyr Ala Pro Pro Arg Asp Phe Ala Ala Tyr Arg Ser Lys Arg Gly Arg
        355                 360                 365

Lys Lys Leu Leu Tyr Ile Phe Lys Gln Pro Phe Met Arg Pro Val Gln
    370                 375                 380

Thr Thr Gln Glu Glu Asp Gly Cys Ser Cys Arg Phe Pro Glu Glu Glu
385                 390                 395                 400

Glu Gly Gly Cys Glu Leu
                405

<210> 14
<211> 406
<212> PRT
<213> Artificial Sequence

<220>
<223> Amino acid sequence of 137DCR4 (without signal peptide)

<400> 14

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Ser Pro Glu Lys Gly Leu Glu Trp Ile
        35                  40                  45

Gly Glu Ile Asn His Gly Gly Tyr Val Thr Tyr Asn Pro Ser Leu Glu
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Asp Tyr Gly Pro Gly Asn Tyr Asp Trp Tyr Phe Asp Leu Trp Gly
            100                 105                 110

Arg Gly Thr Leu Val Thr Val Ser Ser Gly Gly Gly Gly Ser Gly Gly
        115                 120                 125

Gly Gly Ser Gly Gly Gly Gly Ser Glu Ile Val Leu Thr Gln Ser Pro
    130                 135                 140

Ala Thr Leu Ser Leu Ser Pro Gly Glu Arg Ala Thr Leu Ser Cys Arg
145                 150                 155                 160

Ala Ser Gln Ser Val Ser Ser Tyr Leu Ala Trp Tyr Gln Gln Lys Pro
                165                 170                 175

Gly Gln Ala Pro Arg Leu Leu Ile Tyr Asp Ala Ser Asn Arg Ala Thr
            180                 185                 190

Gly Ile Pro Ala Arg Phe Ser Gly Ser Gly Ser Gly Thr Asp Phe Thr
        195                 200                 205

Leu Thr Ile Ser Ser Leu Glu Pro Glu Asp Phe Ala Val Tyr Tyr Cys
    210                 215                 220

Gln Gln Arg Ser Asn Trp Pro Pro Ala Leu Thr Phe Gly Gly Gly Thr
225                 230                 235                 240

Lys Val Glu Ile Lys Glu Ser Lys Tyr Gly Pro Pro Cys Pro Pro Cys
                245                 250                 255

Pro Ile Glu Val Met Tyr Pro Pro Pro Tyr Leu Asp Asn Glu Lys Ser
            260                 265                 270

Asn Gly Thr Ile Ile His Val Lys Gly Lys His Leu Cys Pro Ser Pro
        275                 280                 285

Leu Phe Pro Gly Pro Ser Lys Pro Phe Trp Val Leu Val Val Val Gly
    290                 295                 300

Gly Val Leu Ala Cys Tyr Ser Leu Leu Val Thr Val Ala Phe Ile Ile
305                 310                 315                 320

Phe Trp Val Lys Arg Gly Arg Lys Lys Leu Leu Tyr Ile Phe Lys Gln
                325                 330                 335

Pro Phe Met Arg Pro Val Gln Thr Thr Gln Glu Glu Asp Gly Cys Ser
            340                 345                 350

Cys Arg Phe Pro Glu Glu Glu Glu Gly Gly Cys Glu Leu Arg Ser Lys
        355                 360                 365

Arg Ser Arg Leu Leu His Ser Asp Tyr Met Asn Met Thr Pro Arg Arg
    370                 375                 380

Pro Gly Pro Thr Arg Lys His Tyr Gln Pro Tyr Ala Pro Pro Arg Asp
385                 390                 395                 400

Phe Ala Ala Tyr Arg Ser
                405

<210>  15
<211>  1167
<212>  DNA
<213>  Artificial Sequence

<220>
<223>  Nucleic acid sequence of 137DCR1 (including signal peptide)

<400>  15
atgggaaaca gctgttacaa catagtagcc actctgttgc tggtcctcaa ctttgagagg      60

acaagatcac aggtgcaact acagcagtgg ggcgcaggac tgttgaagcc ttcggagacc     120

ctgtccctca cctgcgctgt ctatggtggg tccttcagtg gttactactg gagctggata     180

cgccagtccc cagagaaggg gctggagtgg attggggaaa tcaatcatgg tggatacgtc     240

acctacaatc cgtccctcga gagtcgagtc accatatcag tagacacgtc caagaaccag     300

ttctccctga agctgagctc tgtgaccgcc gcggacacgg ctgtatatta ctgtgcgagg     360

gactatggtc cggggaatta tgactggtac ttcgatctct ggggccgtgg caccctggtc     420

actgtctcct caggtggagg cggttcaggc ggaggtggca gcggcggtgg cgggtcggaa     480

attgtgttga cacagtctcc agccaccctg tctttgtctc caggggaaag agccaccctc     540

tcctgcaggg ccagtcagag tgttagcagc tacttagcct ggtaccaaca gaaacctggc     600

caggctccca ggctcctcat ctatgatgca tccaacaggg ccactggcat cccagccagg     660

ttcagtggca gtgggtctgg gacagacttc actctcacca tcagcagcct agagcctgaa     720

gattttgcag tttattactg tcagcagcgt agcaactggc ctccggcgct cactttcggc     780

ggagggacca aggtggagat caaagagtcc aaatatggtc ccccatgccc accatgccca     840

attgaagtta tgtatcctcc tccttaccta gacaatgaga agagcaatgg aaccattatc     900

catgtgaaag ggaaacacct ttgtccaagt cccctatttc ccggaccttc taagcccttt     960

tgggtgctgg tggtggttgg tggagtcctg gcttgctata gcttgctagt aacagtggcc    1020

tttattattt tctgggtgag gagtaagagg agcaggctcc tgcacagtga ctacatgaac    1080

atgactcccc gccgccccgg gcccacccgc aagcattacc agccctatgc cccaccacgc    1140

gacttcgcag cctatcgctc ctgataa                                        1167

<210>  16
<211>  1170
<212>  DNA
<213>  Artificial Sequence

<220>
<223>  Nucleic acid sequence of 137DCR2 (including signal peptide)

<400>  16
atgggaaaca gctgttacaa catagtagcc actctgttgc tggtcctcaa ctttgagagg      60

acaagatcac aggtgcaact acagcagtgg ggcgcaggac tgttgaagcc ttcggagacc     120

ctgtccctca cctgcgctgt ctatggtggg tccttcagtg gttactactg gagctggata     180

cgccagtccc cagagaaggg gctggagtgg attggggaaa tcaatcatgg tggatacgtc     240

acctacaatc cgtccctcga gagtcgagtc accatatcag tagacacgtc caagaaccag     300

ttctccctga agctgagctc tgtgaccgcc gcggacacgg ctgtatatta ctgtgcgagg     360

gactatggtc cggggaatta tgactggtac ttcgatctct ggggccgtgg caccctggtc     420

actgtctcct caggtggagg cggttcaggc ggaggtggca gcggcggtgg cgggtcggaa     480

attgtgttga cacagtctcc agccaccctg tctttgtctc caggggaaag agccaccctc     540

tcctgcaggg ccagtcagag tgttagcagc tacttagcct ggtaccaaca gaaacctggc     600

caggctccca ggctcctcat ctatgatgca tccaacaggg ccactggcat cccagccagg     660

ttcagtggca gtgggtctgg gacagacttc actctcacca tcagcagcct agagcctgaa     720

gattttgcag tttattactg tcagcagcgt agcaactggc ctccggcgct cactttcggc     780

ggagggacca aggtggagat caaagagtcc aaatatggtc ccccatgccc accatgccca     840

attgaagtta tgtatcctcc tccttaccta gacaatgaga agagcaatgg aaccattatc     900

catgtgaaag ggaaacacct ttgtccaagt cccctatttc ccggaccttc taagcccttt     960

tgggtgctgg tggtggttgg tggagtcctg gcttgctata gcttgctagt aacagtggcc    1020

tttattattt tctgggtgaa acggggcaga aagaagctcc tgtatatatt caaacaacca    1080

tttatgagac cagtacaaac tactcaagag gaagatggct gtagctgccg atttccagaa    1140

gaagaagaag gaggatgtga actgtgataa                                      1170

<210>  17
<211>  1293
<212>  DNA
<213>  Artificial Sequence

<220>
<223>  Nucleic acid sequence of 137DCR3 (including signal peptide)

<400>  17
atgggaaaca gctgttacaa catagtagcc actctgttgc tggtcctcaa ctttgagagg      60

acaagatcac aggtgcaact acagcagtgg ggcgcaggac tgttgaagcc ttcggagacc     120

ctgtccctca cctgcgctgt ctatggtggg tccttcagtg gttactactg gagctggata 180

cgccagtccc cagagaaggg gctggagtgg attggggaaa tcaatcatgg tggatacgtc 240

acctacaatc cgtccctcga gagtcgagtc accatatcag tagacacgtc caagaaccag 300

ttctccctga agctgagctc tgtgaccgcc gcggacacgg ctgtatatta ctgtgcgagg 360

gactatggtc cggggaatta tgactggtac ttcgatctct ggggccgtgg caccctggtc 420

actgtctcct caggtggagg cggttcaggc ggaggtggca gcggcggtgg cgggtcggaa 480

attgtgttga cacagtctcc agccaccctg tctttgtctc caggggaaag agccaccctc 540

tcctgcaggg ccagtcagag tgttagcagc tacttagcct ggtaccaaca gaaacctggc 600

caggctccca ggctcctcat ctatgatgca tccaacaggg ccactggcat cccagccagg 660

ttcagtggca gtgggtctgg gacagacttc actctcacca tcagcagcct agagcctgaa 720

gattttgcag tttattactg tcagcagcgt agcaactggc ctccggcgct cactttcggc 780

ggagggacca aggtggagat caaagagtcc aaatatggtc ccccatgccc accatgccca 840

attgaagtta tgtatcctcc tccttaccta gacaatgaga agagcaatgg aaccattatc 900

catgtgaaag ggaaacacct ttgtccaagt cccctatttc ccggaccttc taagcccttt 960

tgggtgctgg tggtggttgg tggagtcctg gcttgctata gcttgctagt aacagtggcc 1020

tttattattt tctgggtgag gagtaagagg agcaggctcc tgcacagtga ctacatgaac 1080

atgactcccc gccgccccgg gcccacccgc aagcattacc agccctatgc cccaccacgc 1140

gacttcgcag cctatcgctc caaacggggc agaaagaagc tcctgtatat attcaaacaa 1200

ccatttatga gaccagtaca aactactcaa gaggaagatg gctgtagctg ccgatttcca 1260

gaagaagaag aaggaggatg tgaactgtga taa 1293

<210> 18
<211> 1293
<212> DNA
<213> Artificial Sequence

<220>
<223> Nucleic acid sequence of 137DCR4 (including signal peptide)

<400> 18
atgggaaaca gctgttacaa catagtagcc actctgttgc tggtcctcaa ctttgagagg 60

acaagatcac aggtgcaact acagcagtgg ggcgcaggac tgttgaagcc ttcggagacc 120

ctgtccctca cctgcgctgt ctatggtggg tccttcagtg gttactactg gagctggata 180

cgccagtccc cagagaaggg gctggagtgg attggggaaa tcaatcatgg tggatacgtc 240

acctacaatc cgtccctcga gagtcgagtc accatatcag tagacacgtc caagaaccag 300

ttctccctga agctgagctc tgtgaccgcc gcggacacgg ctgtatatta ctgtgcgagg 360

gactatggtc cggggaatta tgactggtac ttcgatctct ggggccgtgg caccctggtc 420

actgtctcct caggtggagg cggttcaggc ggaggtggca gcggcggtgg cgggtcggaa 480

attgtgttga cacagtctcc agccaccctg tctttgtctc caggggaaag agccaccctc 540

tcctgcaggg ccagtcagag tgttagcagc tacttagcct ggtaccaaca gaaacctggc 600

caggctccca ggctcctcat ctatgatgca tccaacaggg ccactggcat cccagccagg 660

ttcagtggca gtgggtctgg gacagacttc actctcacca tcagcagcct agagcctgaa 720

gattttgcag tttattactg tcagcagcgt agcaactggc ctccggcgct cactttcggc 780

ggagggacca aggtggagat caaagagtcc aaatatggtc ccccatgccc accatgccca 840

attgaagtta tgtatcctcc tccttaccta gacaatgaga agagcaatgg aaccattatc 900

catgtgaaag ggaaacacct ttgtccaagt cccctatttc ccggaccttc taagcccttt 960

tgggtgctgg tggtggttgg tggagtcctg gcttgctata gcttgctagt aacagtggcc 1020

tttattattt tctgggtgaa acggggcaga aagaagctcc tgtatatatt caaacaacca 1080

tttatgagac cagtacaaac tactcaagag gaagatggct gtagctgccg atttccagaa 1140

gaagaagaag gaggatgtga actgaggagt aagaggagca ggctcctgca cagtgactac 1200

atgaacatga ctccccgccg ccccgggccc acccgcaagc attaccagcc ctatgcccca 1260

ccacgcgact tcgcagccta tcgctcctga taa 1293

<210> 19
<211> 1098
<212> DNA
<213> Artificial Sequence

<220>
<223> Nucleic acid sequence of 137DCR1 (without signal peptide)

<400> 19
caggtgcaac tacagcagtg gggcgcagga ctgttgaagc cttcggagac cctgtccctc 60

acctgcgctg tctatggtgg gtccttcagt ggttactact ggagctggat acgccagtcc 120

ccagagaagg ggctggagtg gattggggaa atcaatcatg gtggatacgt cacctacaat 180

ccgtccctcg agagtcgagt caccatatca gtagacacgt ccaagaacca gttctccctg 240

aagctgagct ctgtgaccgc cgcggacacg gctgtatatt actgtgcgag ggactatggt 300

ccggggaatt atgactggta cttcgatctc tggggccgtg gcaccctggt cactgtctcc 360

tcaggtggag gcggttcagg cggaggtggc agcggcggtg gcgggtcgga aattgtgttg 420

acacagtctc cagccaccct gtctttgtct ccaggggaaa gagccaccct ctcctgcagg 480

gccagtcaga gtgttagcag ctacttagcc tggtaccaac agaaacctgg ccaggctccc 540

aggctcctca tctatgatgc atccaacagg gccactggca tcccagccag gttcagtggc 600

agtgggtctg ggacagactt cactctcacc atcagcagcc tagagcctga agattttgca 660

gtttattact gtcagcagcg tagcaactgg cctccggcgc tcactttcgg cggagggacc 720

aaggtggaga tcaaagagtc caaatatggt cccccatgcc caccatgccc aattgaagtt 780

atgtatcctc ctccttacct agacaatgag aagagcaatg gaaccattat ccatgtgaaa 840

gggaaacacc tttgtccaag tcccctattt cccggacctt ctaagccctt ttgggtgctg 900

gtggtggttg gtggagtcct ggcttgctat agcttgctag taacagtggc ctttattatt 960

ttctgggtga ggagtaagag gagcaggctc ctgcacagtg actacatgaa catgactccc 1020

cgccgccccg ggcccacccg caagcattac cagccctatg ccccaccacg cgacttcgca 1080

gcctatcgct cctgataa 1098

<210> 20
<211> 1101
<212> DNA
<213> Artificial Sequence

<220>
<223> Nucleic acid sequence of 137DCR2 (without signal peptide)

<400> 20
caggtgcaac tacagcagtg gggcgcagga ctgttgaagc cttcggagac cctgtccctc 60

acctgcgctg tctatggtgg gtccttcagt ggttactact ggagctggat acgccagtcc 120

ccagagaagg ggctggagtg gattggggaa atcaatcatg gtggatacgt cacctacaat 180

ccgtccctcg agagtcgagt caccatatca gtagacacgt ccaagaacca gttctccctg 240

aagctgagct ctgtgaccgc cgcggacacg gctgtatatt actgtgcgag ggactatggt 300

ccggggaatt atgactggta cttcgatctc tggggccgtg gcaccctggt cactgtctcc 360

tcaggtggag gcggttcagg cggaggtggc agcggcggtg gcgggtcgga aattgtgttg 420

acacagtctc cagccaccct gtctttgtct ccaggggaaa gagccaccct ctcctgcagg 480

gccagtcaga gtgttagcag ctacttagcc tggtaccaac agaaacctgg ccaggctccc 540

aggctcctca tctatgatgc atccaacagg gccactggca tcccagccag gttcagtggc 600

agtgggtctg ggacagactt cactctcacc atcagcagcc tagagcctga agattttgca 660

gtttattact gtcagcagcg tagcaactgg cctccggcgc tcactttcgg cggagggacc 720

aaggtggaga tcaaagagtc caaatatggt cccccatgcc caccatgccc aattgaagtt 780

atgtatcctc ctccttacct agacaatgag aagagcaatg gaaccattat ccatgtgaaa 840

gggaaacacc tttgtccaag tcccctattt cccggacctt ctaagccctt ttgggtgctg 900

gtggtggttg gtggagtcct ggcttgctat agcttgctag taacagtggc ctttattatt 960

ttctgggtga aacggggcag aaagaagctc ctgtatatat tcaaacaacc atttatgaga 1020

ccagtacaaa ctactcaaga ggaagatggc tgtagctgcc gatttccaga agaagaagaa 1080

ggaggatgtg aactgtgata a                                              1101

<210>  21
<211>  1224
<212>  DNA
<213>  Artificial Sequence

<220>
<223>  Nucleic acid sequence of 137DCR3 (without signal peptide)

<400>  21
caggtgcaac tacagcagtg gggcgcagga ctgttgaagc cttcggagac cctgtccctc      60

acctgcgctg tctatggtgg gtccttcagt ggttactact ggagctggat acgccagtcc     120

ccagagaagg ggctggagtg gattggggaa atcaatcatg gtggatacgt cacctacaat     180

ccgtccctcg agagtcgagt caccatatca gtagacacgt ccaagaacca gttctccctg     240

aagctgagct ctgtgaccgc cgcggacacg gctgtatatt actgtgcgag ggactatggt     300

ccggggaatt atgactggta cttcgatctc tggggccgtg gcaccctggt cactgtctcc     360

tcaggtggag gcggttcagg cggaggtggc agcggcggtg gcgggtcgga aattgtgttg     420

acacagtctc cagccaccct gtctttgtct ccaggggaaa gagccaccct ctcctgcagg     480

gccagtcaga gtgttagcag ctacttagcc tggtaccaac agaaacctgg ccaggctccc     540

aggctcctca tctatgatgc atccaacagg gccactggca tcccagccag gttcagtggc     600

agtgggtctg ggacagactt cactctcacc atcagcagcc tagagcctga agattttgca     660

gtttattact gtcagcagcg tagcaactgg cctccggcgc tcactttcgg cggagggacc     720

aaggtggaga tcaaagagtc caaatatggt cccccatgcc caccatgccc aattgaagtt     780

atgtatcctc ctccttacct agacaatgag aagagcaatg gaaccattat ccatgtgaaa     840

gggaaacacc tttgtccaag tcccctattt cccggacctt ctaagccctt ttgggtgctg     900

gtggtggttg gtggagtcct ggcttgctat agcttgctag taacagtggc ctttattatt     960

ttctgggtga ggagtaagag gagcaggctc ctgcacagtg actacatgaa catgactccc    1020

cgccgccccg ggcccacccg caagcattac cagccctatg ccccaccacg cgacttcgca    1080

gcctatcgct ccaaacgggg cagaaagaag ctcctgtata tattcaaaca accatttatg    1140

agaccagtac aaactactca agaggaagat ggctgtagct gccgatttcc agaagaagaa    1200

gaaggaggat gtgaactgtg ataa                                           1224

<210>  22
<211>  1224
<212>  DNA
<213>  Artificial Sequence

<220>
<223>  Nucleic acid sequence of 137DCR4 (without signal peptide)

<400>  22
caggtgcaac tacagcagtg gggcgcagga ctgttgaagc cttcggagac cctgtccctc      60

acctgcgctg tctatggtgg gtccttcagt ggttactact ggagctggat acgccagtcc     120

ccagagaagg ggctggagtg gattggggaa atcaatcatg gtggatacgt cacctacaat     180

ccgtccctcg agagtcgagt caccatatca gtagacacgt ccaagaacca gttctccctg     240

aagctgagct ctgtgaccgc cgcggacacg gctgtatatt actgtgcgag ggactatggt     300

ccggggaatt atgactggta cttcgatctc tggggccgtg gcaccctggt cactgtctcc     360

tcaggtggag gcggttcagg cggaggtggc agcggcggtg gcgggtcgga aattgtgttg     420

acacagtctc cagccaccct gtctttgtct ccaggggaaa gagccaccct ctcctgcagg     480

gccagtcaga gtgttagcag ctacttagcc tggtaccaac agaaacctgg ccaggctccc     540

aggctcctca tctatgatgc atccaacagg gccactggca tcccagccag gttcagtggc     600

agtgggtctg ggacagactt cactctcacc atcagcagcc tagagcctga agattttgca     660

gtttattact gtcagcagcg tagcaactgg cctccggcgc tcactttcgg cggagggacc     720

aaggtggaga tcaaagagtc caaatatggt cccccatgcc caccatgccc aattgaagtt     780

atgtatcctc ctccttacct agacaatgag aagagcaatg gaaccattat ccatgtgaaa     840

gggaaacacc tttgtccaag tcccctattt cccggacctt ctaagccctt ttgggtgctg     900

gtggtggttg gtggagtcct ggcttgctat agcttgctag taacagtggc ctttattatt     960

ttctgggtga aacggggcag aaagaagctc ctgtatatat tcaaacaacc atttatgaga    1020

ccagtacaaa ctactcaaga ggaagatggc tgtagctgcc gatttccaga agaagaagaa    1080

ggaggatgtg aactgaggag taagaggagc aggctcctgc acagtgacta catgaacatg    1140

actccccgcc gccccgggcc cacccgcaag cattaccagc cctatgcccc accacgcgac    1200

ttcgcagcct atcgctcctg ataa                                           1224

<210>  23
<211>  455
<212>  PRT
<213>  Artificial Sequence

<220>
<223>  Amino acid sequence of meso G1 CAR

<400>  23

Met Ala Leu Pro Val Thr Ala Leu Leu Leu Pro Leu Ala Leu Leu Leu
1               5                   10                  15

His Ala Ala Arg Pro Ser Gln Val Gln Leu Gln Gln Ser Gly Pro Glu
            20                  25                  30

Leu Glu Lys Pro Gly Ala Ser Val Lys Ile Ser Cys Lys Ala Ser Gly

35                  40                  45

Tyr Ser Phe Thr Gly Tyr Thr Met Asn Trp Val Lys Gln Ser His Gly
    50                  55                  60

Lys Ser Leu Glu Trp Ile Gly Leu Ile Thr Pro Tyr Asn Gly Ala Ser
65                  70                  75                  80

Ser Tyr Asn Gln Lys Phe Arg Gly Lys Ala Thr Leu Thr Val Asp Lys
                85                  90                  95

Ser Ser Ser Thr Ala Tyr Met Asp Leu Leu Ser Leu Thr Ser Glu Asp
            100                 105                 110

Ser Ala Val Tyr Phe Cys Ala Arg Gly Gly Tyr Asp Gly Arg Gly Phe
        115                 120                 125

Asp Tyr Trp Gly Gln Gly Thr Thr Val Thr Val Ser Ser Gly Gly Gly
    130                 135                 140

Gly Ser Gly Gly Gly Gly Ser Gly Gly Gly Gly Ser Asp Ile Glu Leu
145                 150                 155                 160

Thr Gln Ser Pro Ala Ile Met Ser Ala Ser Pro Gly Glu Lys Val Thr
                165                 170                 175

Met Thr Cys Ser Ala Ser Ser Ser Val Ser Tyr Met His Trp Tyr Gln
            180                 185                 190

Gln Lys Ser Gly Thr Ser Pro Lys Arg Trp Ile Tyr Asp Thr Ser Lys
        195                 200                 205

Leu Ala Ser Gly Val Pro Gly Arg Phe Ser Gly Ser Gly Ser Gly Asn
    210                 215                 220

Ser Tyr Ser Leu Thr Ile Ser Ser Val Glu Ala Glu Asp Asp Ala Thr
225                 230                 235                 240

Tyr Tyr Cys Gln Gln Trp Ser Lys His Pro Leu Thr Tyr Gly Ala Gly
                245                 250                 255

Thr Lys Leu Glu Ile Lys Phe Val Pro Val Phe Leu Pro Ala Lys Pro
            260                 265                 270

Thr Thr Thr Pro Ala Pro Arg Pro Pro Thr Pro Ala Pro Thr Ile Ala
        275                 280                 285

Ser Gln Pro Leu Ser Leu Arg Pro Glu Ala Cys Arg Pro Ala Ala Gly
    290                 295                 300

Gly Ala Val His Thr Arg Gly Leu Asp Phe Ala Cys Asp Ile Tyr Ile
305                 310                 315                 320

Trp Ala Pro Leu Ala Gly Thr Cys Gly Val Leu Leu Leu Ser Leu Val
                325                 330                 335

Ile Thr Leu Tyr Cys Asn His Arg Val Lys Phe Ser Arg Ser Ala Asp
            340                 345                 350

Ala Pro Ala Tyr Gln Gln Gly Gln Asn Gln Leu Tyr Asn Glu Leu Asn
        355                 360                 365

Leu Gly Arg Arg Glu Glu Tyr Asp Val Leu Asp Lys Arg Arg Gly Arg
    370                 375                 380

Asp Pro Glu Met Gly Gly Lys Pro Arg Arg Lys Asn Pro Gln Glu Gly
385                 390                 395                 400

Leu Tyr Asn Glu Leu Gln Lys Asp Lys Met Ala Glu Ala Tyr Ser Glu
                405                 410                 415

Ile Gly Met Lys Gly Glu Arg Arg Arg Gly Lys Gly His Asp Gly Leu
            420                 425                 430

Tyr Gln Gly Leu Ser Thr Ala Thr Lys Asp Thr Tyr Asp Ala Leu His
        435                 440                 445

Met Gln Ala Leu Pro Pro Arg
    450                 455

<210>  24
<211>  1371
<212>  DNA
<213>  Artificial Sequence

<220>
<223>  Nucleic acid sequence of meso G1 CAR

<400>  24
atggccttac cagtgaccgc cttgctcctg ccgctggcct tgctgctcca cgccgccagg      60

ccgagccagg tacaactgca gcagtctggg cctgagctgg agaagcctgg cgcttcagtg     120

aagatatcct gcaaggcttc tggttactca ttcactggct acaccatgaa ctgggtgaag     180

cagagccatg gaaagagcct tgagtggatt ggacttatta ctccttacaa tggtgcttct     240

agctacaacc agaagttcag gggcaaggcc acattaactg tagacaagtc atccagcaca     300

gcctacatgg acctcctcag tctgacatct gaagactctg cagtctattt ctgtgcaagg 360

gggggttacg acgggagggg ttttgactac tggggccaag ggaccacggt caccgtctcc 420

tcaggtggag gcggttcagg cggaggtggc agcggcggtg gcgggtcgga catcgagctc 480

actcagtctc cagcaatcat gtctgcatct ccaggggaga aggtcaccat gacctgcagt 540

gccagctcaa gtgtaagtta catgcactgg taccagcaga agtcaggcac ctcccccaaa 600

agatggattt atgacacatc caaactggct tctggagtcc caggtcgctt cagtggcagt 660

gggtctggaa actcttactc tctcacaatc agcagcgtgg aggctgaaga tgatgcaact 720

tattactgcc agcagtggag taagcaccct ctcacgtacg gtgctgggac aaagttggaa 780

atcaaattcg tgccggtctt cctgccagcg aagcccacca cgacgccagc gccgcgacca 840

ccaacaccgg cgcccaccat cgcgtcgcag cccctgtccc tgcgcccaga ggcgtgccgg 900

ccagcggcgg ggggcgcagt gcacacgagg gggctggact tcgcctgtga tatctacatc 960

tgggcgcccc tggccgggac ttgtggggtc cttctcctgt cactggttat caccctttac 1020

tgcaaccaca gagtgaagtt cagcaggagc gcagacgccc ccgcgtacca gcagggccag 1080

aaccagctct ataacgagct caatctagga cgaagagagg agtacgatgt tttggacaag 1140

agacgtggcc gggaccctga gatgggggga aagccgagaa ggaagaaccc tcaggaaggc 1200

ctgtacaatg aactgcagaa agataagatg gcggaggcct acagtgagat tgggatgaaa 1260

ggcgagcgcc ggaggggcaa ggggcacgat ggcctttacc agggtctcag tacagccacc 1320

aaggacacct acgacgccct tcacatgcag gccctgcccc ctcgctgata a 1371

<210> 25
<211> 112
<212> PRT
<213> Artificial Sequence

<220>
<223> Tyrosine activation motif of CD3¦Æ

<400> 25

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  26
<211>  2043
<212>  DNA
<213>  Artificial Sequence

<220>
<223>  Nucleotide sequence of meso G2 CAR

<400>  26
atggccttac cagtgaccgc cttgctcctg ccgctggcct tgctgctcca cgccgccagg      60

ccgagcgagg tgcagctggt ggagtccggg ggaggcctgg tccagcctgg gggatccctg     120

agactctcct gcgcagcctc tggattcgac ctcggtttct acttttacgc ctgttgggtc     180

cgccaggctc cagggaaggg cctggagtgg gtctcatgca tttatactgc tggtagtggt     240

agcacgtact acgcgagctg ggcgaaaggc cgattcacca tctccagaga caattcgaag     300

aacacgctgt atctgcaaat gaacagtctg agagccgagg acacggccgt gtattactgt     360

gcgagatcta ctgctaatac tagaagtact tattatctta acttgtgggg ccaaggcacc     420

ctggtcaccg tctcctcagg cggaggcgga tcaggtggtg gcggatctgg aggtggcgga     480

agcgacatcc agatgaccca gtctccatcc tccctgtctg catctgtggg agacagagtc     540

accatcactt gccaggccag tcagaggatt agtagttact tatcctggta tcagcagaaa     600

ccagggaaag ttcccaagct cctgatctat ggtgcatcca ctctggcatc tggggtcccc     660

tcgcggttca gtggcagtgg atctgggaca gatttcactc tcaccatcag cagcctgcag     720

cctgaagatg ttgccactta ctactgtcag agttatgctt attttgatag taataattgg     780

catgctttcg gcggagggac caaggtggag atcaaagagt ccaaatatgg tcccccatgc     840

ccaccatgcc cagcacctcc cgtggccgga ccatcagtct tcctgttccc cccaaaaccc     900

aaggacactc tcatgatctc ccggacccct gaggtcacgt gcgtggtggt ggacgtgagc     960

caggaagacc ccgaggtcca gttcaactgg tacgtggatg gcgtggaggt gcataatgcc    1020

aagacaaagc cgcgggagga gcagttccag agcacgtacc gtgtggtcag cgtcctcacc    1080

gtcctgcacc aggactggct gaacggcaag gagtacaagt gcaaggtctc caacaaaggc    1140

ctcccgtcct ccatcgagaa aaccatctcc aaagccaaag ggcagccccg agagccacag    1200

gtgtacaccc tgcccccatc ccaggaggag atgaccaaga accaggtcag cctgacctgc    1260

ctggtcaaag gcttctaccc cagcgacatc gccgtggagt gggagagcaa tgggcagccg 1320

gagaacaact acaagaccac gcctcccgtg ctggactccg acggctcctt cttcctctac 1380

agcaggctaa ccgtggacaa gagcaggtgg caggagggga atgtcttctc atgctccgtg 1440

atgcatgagg ctctgcacaa ccactacaca cagaagagcc tctccctgtc tctgggtaaa 1500

ccctttgggg tgctggtggt ggttggtgga gtcctggctt gctatagctt gctagtaaca 1560

gtggccttta ttattttctg ggtgaggagt aagaggagca ggctcctgca cagtgactac 1620

atgaacatga ctccccgccg ccccgggccc acccgcaagc attaccagcc ctatgcccca 1680

ccacgcgact tcgcagccta tcgctccaga gtgaagttca gcaggagcgc agacgccccc 1740

gcgtaccagc agggccagaa ccagctctat aacgagctca atctaggacg aagagaggag 1800

tacgatgttt tggacaagag acgtggccgg gaccctgaga tggggggaaa gccgagaagg 1860

aagaaccctc aggaaggcct gtacaatgaa ctgcagaaag ataagatggc ggaggcctac 1920

agtgagattg ggatgaaagg cgagcgccgg aggggcaagg ggcacgatgg cctttaccag 1980

ggtctcagta cagccaccaa ggacacctac gacgcccttc acatgcaggc cctgccccct 2040

cgc 2043

<210> 27
<211> 2718
<212> DNA
<213> Artificial Sequence

<220>
<223> Nucleic acid sequence of PS328b vector

<400> 27
ggcgcgcctt aaccctagaa agataatcat attgtgacgt acgttaaaga taatcatgcg 60

taaaattgac gcatgtctag aggtgtggaa agtccccagg ctccccagca ggcagaagta 120

tgcaaagcat gcatctcaat tagtcagcaa ccaggtgtgg aaagtcccca ggctccccag 180

caggcagaag tatgcaaagc atgcatctca attagtcagc aaccaaggat ctgcgatcgc 240

tccggtgccc gtcagtgggc agagcgcaca tcgcccacag tccccgagaa gttgggggga 300

ggggtcggca attgaacggg tgcctagaga aggtggcgcg gggtaaactg ggaaagtgat 360

gtcgtgtact ggctccgcct ttttcccgag ggtgggggag aaccgtatat aagtgcagta 420

gtcgccgtga acgttctttt tcgcaacggg tttgccgcca gaacacagct gaagcttcga 480

ggggctcgca tctctccttc acgcgcccgc cgccctacct gaggccgcca tccacgccgg 540

ttgagtcgcg ttctgccgcc tcccgcctgt ggtgcctcct gaactgcgtc cgccgtctag 600

gtaagtttaa agctcaggtc gagaccgggc ctttgtccgg cgctcccttg gagcctacct 660

agactcagcc ggctctccac gctttgcctg accctgcttg ctcaactcta cgtctttgtt 720

tcgttttctg ttctgcgccg ttacagatcc aagctgtgac cggcgcctac gaattcggat 780

cctgcactag tgctgtcgac cagacatgat aagatacatt gatgagtttg gacaaaccac 840

aactagaatg cagtgaaaaa aatgctttat ttgtgaaatt tgtgatgcta ttgctttatt 900

tgtaaccatt ataagctgca ataaacaagt taacaacaac aattgcattc attttatgtt 960

tcaggttcag ggggaggtgt gggaggtttt ttaaagcaag taaaacctct acaaatgtgg 1020

tagcatgcgt caattttacg cagactatct ttctagggtt aaatcgatcc cgctagcccc 1080

gatatcccct taattaagag ggggagacca aagggcgaga cgttaaggcc tcacgtgaca 1140

tgtgagcaaa aggccagcaa aaggccagga accgtaaaaa ggccgcgttg ctggcgtttt 1200

tccataggct ccgcccccct gacgagcatc acaaaaatcg acgctcaagt cagaggtggc 1260

gaaacccgac aggactataa agataccagg cgtttccccc tggaagctcc ctcgtgcgct 1320

ctcctgttcc gaccctgccg cttacgggat acctgtccgc ctttctccct tcgggaagcg 1380

tggcgctttc tcatagctca cgctgtaggt atctcagttc ggtgtaggtc gttcgctcca 1440

agctgggctg tgtgcacgaa ccccccgttc agcccgaccg ctgcgcctta tccggtaact 1500

atcgtcttga gtccaacccg gtaagacacg acttatcgcc actggcagca gccactggta 1560

acaggattag cagagcgagg tatgtaggcg gtgctacaga gttcttgaag tggtggccta 1620

actacggcta cactagaaga acagtatttg gtatctgcgc tctgctgaag ccagttacct 1680

tcggaaaaag agttggtagc tcttgatccg gcaaacaaac caccgctggt agcggtggtt 1740

tttttgtttg caagcagcag attacgcgca gaaaaaaagg atctcaagaa gatcctttga 1800

tcttttctac ggggtctgac gctcagtgga acgaaaactc acgttaaggg attttggtca 1860

tgccgtctca gaagaactcg tcaagaaggc gatagaaggc gatgcgctgc gaatcgggag 1920

cggcgatacc gtaaagcacg aggaagcggt cagcccattc gccgccaagc tcttcagcaa 1980

tatcacgggt agccaacgct atgtcctgat agcggtccgc cacacccagc cggccacagt 2040

cgatgaatcc agaaaagcgg ccattttcca ccatgatatt cggcaagcag gcatcgccat 2100

gggtcacgac gagatcctcg ccgtcgggca tgctcgcctt gagcctggcg aacagttcgg 2160

ctggcgcgag cccctgatgc tcttcgtcca gatcatcctg atcgacaaga ccggcttcca 2220

tccgagtacg tgctctctcg atgcgatgtt tcgcttggtg gtcgaatggg caggtagccg 2280

gatcaagcgt atgcagccgc cgcattgcat cagccatgat ggatactttc tcggcaggag 2340

caaggtgaga tgacaggaga tcctgccccg gcacttcgcc caatagcagc cagtcccttc 2400

ccgcttcagt gacaacgtcg agtacagctg cgcaaggaac gcccgtcgtg gccagccacg 2460

atagccgcgc tgcctcgtct tgcagttcat tcagggcacc ggacaggtcg gtcttgacaa 2520

aaagaaccgg gcgcccctgc gctgacagcc ggaacacggc ggcatcagag cagccgattg 2580

tctgttgtgc ccagtcatag ccgaatagcc tctccaccca agcggccgga gaacctgcgt 2640

gcaatccatc ttgttcaatc ataatattat tgaagcattt atcagggttc gtctcgtccc     2700

ggtctcctcc catgcatg                                                   2718

<210>  28
<211>  18
<212>  DNA
<213>  Artificial Sequence

<220>
<223>  Nucleotide sequence of primer CD137-F

<400>  28
cgagtcacca tatcagta                                                   18

<210>  29
<211>  20
<212>  DNA
<213>  Artificial Sequence

<220>
<223>  Nucleotide sequence of primer CD137-R

<400>  29
cgaagtacca gtcataattc                                                 20

<210>  30
<211>  24
<212>  DNA
<213>  Artificial Sequence

<220>
<223>  Nucleotide sequence in Taqman

<400>  30
cctcgcacag taatatacag ccgt                                            24

<210>  31
<211>  243
<212>  PRT
<213>  Artificial Sequence

<220>
<223>  Amino acid sequence of CD28 extracellular agonistic single-chain
antibody

<400>  31

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Ile
        35                  40                  45

Gly Cys Ile Tyr Pro Gly Asn Val Asn Thr Asn Tyr Asn Glu Lys Phe
    50                  55                  60

Lys Asp Arg Ala Thr Leu Thr Val Asp Thr Ser Ile Ser Thr Ala Tyr
65                  70                  75                  80

Met Glu Leu Ser Arg Leu Arg Ser Asp Asp Thr Ala Val Tyr Phe Cys
                85                  90                  95

Thr Arg Ser His Tyr Gly Leu Asp Trp Asn Phe Asp Val Trp Gly Gln
            100                 105                 110

Gly Thr Thr Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys His Ala
145                 150                 155                 160

Ser Gln Asn Ile Tyr Val Trp Leu Asn Trp Tyr Gln Gln Lys Pro Gly
                165                 170                 175

Lys Ala Pro Lys Leu Leu Ile Tyr Lys Ala Ser Asn Leu His Thr Gly
            180                 185                 190

Val Pro Ser Arg Phe Ser Gly Ser Gly Ser Gly Thr Asp Phe Thr Leu
        195                 200                 205

Thr Ile Ser Ser Leu Gln Pro Glu Asp Phe Ala Thr Tyr Tyr Cys Gln
    210                 215                 220

Gln Gly Gln Thr Tyr Pro Tyr Thr Phe Gly Gly Gly Thr Lys Val Glu
225                 230                 235                 240

Ile Lys Glu

<210>  32
<211>  384
<212>  PRT
<213>  Artificial Sequence

<220>
<223>  Amino acid sequence of 28DCR1 (including signal peptide)

<400>  32

Met Gly Asn Ser Cys Tyr Asn Ile Val Ala Thr Leu Leu Leu Val Leu
1               5                   10                  15

Asn Phe Glu Arg Thr Arg Ser Gln Val Gln Leu Val Gln Ser Gly Ala
            20                  25                  30

Glu Val Lys Lys Pro Gly Ala Ser Val Lys Val Ser Cys Lys Ala Ser
        35                  40                  45

Gly Tyr Thr Phe Thr Ser Tyr Tyr Ile His Trp Val Arg Gln Ala Pro
    50                  55                  60

Gly Gln Gly Leu Glu Trp Ile Gly Cys Ile Tyr Pro Gly Asn Val Asn
65                  70                  75                  80

Thr Asn Tyr Asn Glu Lys Phe Lys Asp Arg Ala Thr Leu Thr Val Asp
                85                  90                  95

Thr Ser Ile Ser Thr Ala Tyr Met Glu Leu Ser Arg Leu Arg Ser Asp
            100                 105                 110

Asp Thr Ala Val Tyr Phe Cys Thr Arg Ser His Tyr Gly Leu Asp Trp
        115                 120                 125

Asn Phe Asp Val Trp Gly Gln Gly Thr Thr Val Thr Val Ser Ser Gly
    130                 135                 140

Gly Gly Gly Ser Gly Gly Gly Gly Ser Gly Gly Gly Gly Ser Asp Ile
145                 150                 155                 160

Gln Met Thr Gln Ser Pro Ser Ser Leu Ser Ala Ser Val Gly Asp Arg
                165                 170                 175

Val Thr Ile Thr Cys His Ala Ser Gln Asn Ile Tyr Val Trp Leu Asn
            180                 185                 190

Trp Tyr Gln Gln Lys Pro Gly Lys Ala Pro Lys Leu Leu Ile Tyr Lys
        195                 200                 205

Ala Ser Asn Leu His Thr Gly Val Pro Ser Arg Phe Ser Gly Ser Gly
    210                 215                 220

Ser Gly Thr Asp Phe Thr Leu Thr Ile Ser Ser Leu Gln Pro Glu Asp
225                 230                 235                 240

Phe Ala Thr Tyr Tyr Cys Gln Gln Gly Gln Thr Tyr Pro Tyr Thr Phe
                245                 250                 255

Gly Gly Gly Thr Lys Val Glu Ile Lys Glu Ser Lys Tyr Gly Pro Pro
            260                 265                 270

Cys Pro Pro Cys Pro Ile Glu Val Met Tyr Pro Pro Pro Tyr Leu Asp
        275                 280                 285

Asn Glu Lys Ser Asn Gly Thr Ile Ile His Val Lys Gly Lys His Leu
    290                 295                 300

Cys Pro Ser Pro Leu Phe Pro Gly Pro Ser Lys Pro Phe Trp Val Leu
305                 310                 315                 320

Val Val Val Gly Gly Val Leu Ala Cys Tyr Ser Leu Leu Val Thr Val
                325                 330                 335

Ala Phe Ile Ile Phe Trp Val Arg Ser Lys Arg Ser Arg Leu Leu His
            340                 345                 350

Ser Asp Tyr Met Asn Met Thr Pro Arg Arg Pro Gly Pro Thr Arg Lys
        355                 360                 365

His Tyr Gln Pro Tyr Ala Pro Pro Arg Asp Phe Ala Ala Tyr Arg Ser
    370                 375                 380

<210>  33
<211>  385
<212>  PRT
<213>  Artificial Sequence

<220>
<223>  Amino acid sequence of 28DCR2 (including signal peptide)

<400>  33

Met Gly Asn Ser Cys Tyr Asn Ile Val Ala Thr Leu Leu Leu Val Leu
1               5                   10                  15

Asn Phe Glu Arg Thr Arg Ser Gln Val Gln Leu Val Gln Ser Gly Ala
            20                  25                  30

Glu Val Lys Lys Pro Gly Ala Ser Val Lys Val Ser Cys Lys Ala Ser
        35                  40                  45

Gly Tyr Thr Phe Thr Ser Tyr Tyr Ile His Trp Val Arg Gln Ala Pro
    50                  55                  60

Gly Gln Gly Leu Glu Trp Ile Gly Cys Ile Tyr Pro Gly Asn Val Asn
65                  70                  75                  80

Thr Asn Tyr Asn Glu Lys Phe Lys Asp Arg Ala Thr Leu Thr Val Asp
                85                  90                  95

Thr Ser Ile Ser Thr Ala Tyr Met Glu Leu Ser Arg Leu Arg Ser Asp
            100                 105                 110

Asp Thr Ala Val Tyr Phe Cys Thr Arg Ser His Tyr Gly Leu Asp Trp
        115                 120                 125

Asn Phe Asp Val Trp Gly Gln Gly Thr Thr Val Thr Val Ser Ser Gly
    130                 135                 140

Gly Gly Gly Ser Gly Gly Gly Gly Ser Gly Gly Gly Gly Ser Asp Ile
145                 150                 155                 160

Gln Met Thr Gln Ser Pro Ser Ser Leu Ser Ala Ser Val Gly Asp Arg
                165                 170                 175

Val Thr Ile Thr Cys His Ala Ser Gln Asn Ile Tyr Val Trp Leu Asn
            180                 185                 190

Trp Tyr Gln Gln Lys Pro Gly Lys Ala Pro Lys Leu Leu Ile Tyr Lys
        195                 200                 205

Ala Ser Asn Leu His Thr Gly Val Pro Ser Arg Phe Ser Gly Ser Gly
    210                 215                 220

Ser Gly Thr Asp Phe Thr Leu Thr Ile Ser Ser Leu Gln Pro Glu Asp
225                 230                 235                 240

Phe Ala Thr Tyr Tyr Cys Gln Gln Gly Gln Thr Tyr Pro Tyr Thr Phe
                245                 250                 255

Gly Gly Gly Thr Lys Val Glu Ile Lys Glu Ser Lys Tyr Gly Pro Pro
            260                 265                 270

Cys Pro Pro Cys Pro Ile Glu Val Met Tyr Pro Pro Pro Tyr Leu Asp
        275                 280                 285

Asn Glu Lys Ser Asn Gly Thr Ile Ile His Val Lys Gly Lys His Leu
    290                 295                 300

Cys Pro Ser Pro Leu Phe Pro Gly Pro Ser Lys Pro Phe Trp Val Leu
305                 310                 315                 320

Val Val Val Gly Gly Val Leu Ala Cys Tyr Ser Leu Leu Val Thr Val
                325                 330                 335

Ala Phe Ile Ile Phe Trp Val Lys Arg Gly Arg Lys Lys Leu Leu Tyr
            340                 345                 350

Ile Phe Lys Gln Pro Phe Met Arg Pro Val Gln Thr Thr Gln Glu Glu
        355                 360                 365

Asp Gly Cys Ser Cys Arg Phe Pro Glu Glu Glu Glu Gly Gly Cys Glu
    370                 375                 380

Leu
385

<210>  34
<211>  426
<212>  PRT
<213>  Artificial Sequence

<220>
<223>  Amino acid sequence of 28DCR3 (including signal peptide)

<400>  34

Met Gly Asn Ser Cys Tyr Asn Ile Val Ala Thr Leu Leu Leu Val Leu
1               5                   10                  15

Asn Phe Glu Arg Thr Arg Ser Gln Val Gln Leu Val Gln Ser Gly Ala
            20                  25                  30

Glu Val Lys Lys Pro Gly Ala Ser Val Lys Val Ser Cys Lys Ala Ser
        35                  40                  45

Gly Tyr Thr Phe Thr Ser Tyr Tyr Ile His Trp Val Arg Gln Ala Pro
    50                  55                  60

Gly Gln Gly Leu Glu Trp Ile Gly Cys Ile Tyr Pro Gly Asn Val Asn
65                  70                  75                  80

Thr Asn Tyr Asn Glu Lys Phe Lys Asp Arg Ala Thr Leu Thr Val Asp
                85                  90                  95

Thr Ser Ile Ser Thr Ala Tyr Met Glu Leu Ser Arg Leu Arg Ser Asp
            100                 105                 110

Asp Thr Ala Val Tyr Phe Cys Thr Arg Ser His Tyr Gly Leu Asp Trp
        115                 120                 125

Asn Phe Asp Val Trp Gly Gln Gly Thr Thr Val Thr Val Ser Ser Gly
    130                 135                 140

Gly Gly Gly Ser Gly Gly Gly Gly Ser Gly Gly Gly Gly Ser Asp Ile
145                 150                 155                 160

Gln Met Thr Gln Ser Pro Ser Ser Leu Ser Ala Ser Val Gly Asp Arg
                165                 170                 175

Val Thr Ile Thr Cys His Ala Ser Gln Asn Ile Tyr Val Trp Leu Asn
            180                 185                 190

Trp Tyr Gln Gln Lys Pro Gly Lys Ala Pro Lys Leu Leu Ile Tyr Lys
        195                 200                 205

Ala Ser Asn Leu His Thr Gly Val Pro Ser Arg Phe Ser Gly Ser Gly
    210                 215                 220

Ser Gly Thr Asp Phe Thr Leu Thr Ile Ser Ser Leu Gln Pro Glu Asp
225                 230                 235                 240

Phe Ala Thr Tyr Tyr Cys Gln Gln Gly Gln Thr Tyr Pro Tyr Thr Phe
                245                 250                 255

Gly Gly Gly Thr Lys Val Glu Ile Lys Glu Ser Lys Tyr Gly Pro Pro
            260                 265                 270

Cys Pro Pro Cys Pro Ile Glu Val Met Tyr Pro Pro Pro Tyr Leu Asp
        275                 280                 285

Asn Glu Lys Ser Asn Gly Thr Ile Ile His Val Lys Gly Lys His Leu
    290                 295                 300

Cys Pro Ser Pro Leu Phe Pro Gly Pro Ser Lys Pro Phe Trp Val Leu
305                 310                 315                 320

Val Val Val Gly Gly Val Leu Ala Cys Tyr Ser Leu Leu Val Thr Val
                325                 330                 335

Ala Phe Ile Ile Phe Trp Val Arg Ser Lys Arg Ser Arg Leu Leu His
            340                 345                 350

Ser Asp Tyr Met Asn Met Thr Pro Arg Arg Pro Gly Pro Thr Arg Lys
        355                 360                 365

His Tyr Gln Pro Tyr Ala Pro Pro Arg Asp Phe Ala Ala Tyr Arg Ser
    370                 375                 380

Lys Arg Gly Arg Lys Lys Leu Leu Tyr Ile Phe Lys Gln Pro Phe Met
385                 390                 395                 400

Arg Pro Val Gln Thr Thr Gln Glu Glu Asp Gly Cys Ser Cys Arg Phe
                405                 410                 415

Pro Glu Glu Glu Glu Gly Gly Cys Glu Leu
            420                 425

<210>  35
<211>  426
<212>  PRT
<213>  Artificial Sequence

<220>
<223>  Amino acid sequence of 28DCR4 (including signal peptide)

<400>  35

Met Gly Asn Ser Cys Tyr Asn Ile Val Ala Thr Leu Leu Leu Val Leu
1               5                   10                  15

Asn Phe Glu Arg Thr Arg Ser Gln Val Gln Leu Val Gln Ser Gly Ala
            20                  25                  30

Glu Val Lys Lys Pro Gly Ala Ser Val Lys Val Ser Cys Lys Ala Ser
        35                  40                  45

Gly Tyr Thr Phe Thr Ser Tyr Tyr Ile His Trp Val Arg Gln Ala Pro
    50                  55                  60

Gly Gln Gly Leu Glu Trp Ile Gly Cys Ile Tyr Pro Gly Asn Val Asn
65                  70                  75                  80

Thr Asn Tyr Asn Glu Lys Phe Lys Asp Arg Ala Thr Leu Thr Val Asp
                85                  90                  95

Thr Ser Ile Ser Thr Ala Tyr Met Glu Leu Ser Arg Leu Arg Ser Asp
            100                 105                 110

Asp Thr Ala Val Tyr Phe Cys Thr Arg Ser His Tyr Gly Leu Asp Trp
        115                 120                 125

Asn Phe Asp Val Trp Gly Gln Gly Thr Thr Val Thr Val Ser Ser Gly
    130                 135                 140

Gly Gly Gly Ser Gly Gly Gly Gly Ser Gly Gly Gly Gly Ser Asp Ile
145                 150                 155                 160

Gln Met Thr Gln Ser Pro Ser Ser Leu Ser Ala Ser Val Gly Asp Arg
                165                 170                 175

Val Thr Ile Thr Cys His Ala Ser Gln Asn Ile Tyr Val Trp Leu Asn
        180                 185                 190

Trp Tyr Gln Gln Lys Pro Gly Lys Ala Pro Lys Leu Leu Ile Tyr Lys
    195                 200                 205

Ala Ser Asn Leu His Thr Gly Val Pro Ser Arg Phe Ser Gly Ser Gly
    210                 215                 220

Ser Gly Thr Asp Phe Thr Leu Thr Ile Ser Ser Leu Gln Pro Glu Asp
225                 230                 235                 240

Phe Ala Thr Tyr Tyr Cys Gln Gln Gly Gln Thr Tyr Pro Tyr Thr Phe
                245                 250                 255

Gly Gly Gly Thr Lys Val Glu Ile Lys Glu Ser Lys Tyr Gly Pro Pro
            260                 265                 270

Cys Pro Pro Cys Pro Ile Glu Val Met Tyr Pro Pro Pro Tyr Leu Asp
        275                 280                 285

Asn Glu Lys Ser Asn Gly Thr Ile Ile His Val Lys Gly Lys His Leu
    290                 295                 300

Cys Pro Ser Pro Leu Phe Pro Gly Pro Ser Lys Pro Phe Trp Val Leu
305                 310                 315                 320

Val Val Val Gly Gly Val Leu Ala Cys Tyr Ser Leu Leu Val Thr Val
                325                 330                 335

Ala Phe Ile Ile Phe Trp Val Lys Arg Gly Arg Lys Lys Leu Leu Tyr
            340                 345                 350

Ile Phe Lys Gln Pro Phe Met Arg Pro Val Gln Thr Thr Gln Glu Glu
        355                 360                 365

Asp Gly Cys Ser Cys Arg Phe Pro Glu Glu Glu Glu Gly Gly Cys Glu
    370                 375                 380

Leu Arg Ser Lys Arg Ser Arg Leu Leu His Ser Asp Tyr Met Asn Met
385                 390                 395                 400

Thr Pro Arg Arg Pro Gly Pro Thr Arg Lys His Tyr Gln Pro Tyr Ala
                405                 410                 415

Pro Pro Arg Asp Phe Ala Ala Tyr Arg Ser
            420                 425

<210>  36
<211>  361
<212>  PRT
<213>  Artificial Sequence

<220>
<223>  Amino acid sequence of 28DCR1 (without signal peptide)

<400>  36

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Ile
        35                  40                  45

Gly Cys Ile Tyr Pro Gly Asn Val Asn Thr Asn Tyr Asn Glu Lys Phe
    50                  55                  60

Lys Asp Arg Ala Thr Leu Thr Val Asp Thr Ser Ile Ser Thr Ala Tyr
65                  70                  75                  80

Met Glu Leu Ser Arg Leu Arg Ser Asp Asp Thr Ala Val Tyr Phe Cys
                85                  90                  95

Thr Arg Ser His Tyr Gly Leu Asp Trp Asn Phe Asp Val Trp Gly Gln
            100                 105                 110

Gly Thr Thr Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys His Ala
145                 150                 155                 160

Ser Gln Asn Ile Tyr Val Trp Leu Asn Trp Tyr Gln Gln Lys Pro Gly
                165                 170                 175

Lys Ala Pro Lys Leu Leu Ile Tyr Lys Ala Ser Asn Leu His Thr Gly
            180                 185                 190

Val Pro Ser Arg Phe Ser Gly Ser Gly Ser Gly Thr Asp Phe Thr Leu
        195                 200                 205

Thr Ile Ser Ser Leu Gln Pro Glu Asp Phe Ala Thr Tyr Tyr Cys Gln
    210                 215                 220

Gln Gly Gln Thr Tyr Pro Tyr Thr Phe Gly Gly Gly Thr Lys Val Glu
225                 230                 235                 240

Ile Lys Glu Ser Lys Tyr Gly Pro Pro Cys Pro Pro Cys Pro Ile Glu
                245                 250                 255

Val Met Tyr Pro Pro Pro Tyr Leu Asp Asn Glu Lys Ser Asn Gly Thr
            260                 265                 270

Ile Ile His Val Lys Gly Lys His Leu Cys Pro Ser Pro Leu Phe Pro
        275                 280                 285

Gly Pro Ser Lys Pro Phe Trp Val Leu Val Val Val Gly Gly Val Leu
    290                 295                 300

Ala Cys Tyr Ser Leu Leu Val Thr Val Ala Phe Ile Ile Phe Trp Val
305                 310                 315                 320

Arg Ser Lys Arg Ser Arg Leu Leu His Ser Asp Tyr Met Asn Met Thr
                325                 330                 335

Pro Arg Arg Pro Gly Pro Thr Arg Lys His Tyr Gln Pro Tyr Ala Pro
            340                 345                 350

Pro Arg Asp Phe Ala Ala Tyr Arg Ser
        355                 360

<210>  37
<211>  362
<212>  PRT
<213>  Artificial Sequence

<220>
<223>  Amino acid sequence of 28DCR2 (without signal peptide)

<400>  37

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Ile
        35                  40                  45

Gly Cys Ile Tyr Pro Gly Asn Val Asn Thr Asn Tyr Asn Glu Lys Phe

50                  55                  60

Lys Asp Arg Ala Thr Leu Thr Val Asp Thr Ser Ile Ser Thr Ala Tyr
65                  70                  75                  80

Met Glu Leu Ser Arg Leu Arg Ser Asp Asp Thr Ala Val Tyr Phe Cys
                85                  90                  95

Thr Arg Ser His Tyr Gly Leu Asp Trp Asn Phe Asp Val Trp Gly Gln
            100                 105                 110

Gly Thr Thr Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys His Ala
145                 150                 155                 160

Ser Gln Asn Ile Tyr Val Trp Leu Asn Trp Tyr Gln Gln Lys Pro Gly
                165                 170                 175

Lys Ala Pro Lys Leu Leu Ile Tyr Lys Ala Ser Asn Leu His Thr Gly
            180                 185                 190

Val Pro Ser Arg Phe Ser Gly Ser Gly Ser Gly Thr Asp Phe Thr Leu
        195                 200                 205

Thr Ile Ser Ser Leu Gln Pro Glu Asp Phe Ala Thr Tyr Tyr Cys Gln
    210                 215                 220

Gln Gly Gln Thr Tyr Pro Tyr Thr Phe Gly Gly Gly Thr Lys Val Glu
225                 230                 235                 240

Ile Lys Glu Ser Lys Tyr Gly Pro Pro Cys Pro Pro Cys Pro Ile Glu
                245                 250                 255

Val Met Tyr Pro Pro Pro Tyr Leu Asp Asn Glu Lys Ser Asn Gly Thr
            260                 265                 270

Ile Ile His Val Lys Gly Lys His Leu Cys Pro Ser Pro Leu Phe Pro
        275                 280                 285

Gly Pro Ser Lys Pro Phe Trp Val Leu Val Val Val Gly Gly Val Leu
    290                 295                 300

Ala Cys Tyr Ser Leu Leu Val Thr Val Ala Phe Ile Ile Phe Trp Val
305                 310                 315                 320

Lys Arg Gly Arg Lys Lys Leu Leu Tyr Ile Phe Lys Gln Pro Phe Met
                325                 330                 335

Arg Pro Val Gln Thr Thr Gln Glu Glu Asp Gly Cys Ser Cys Arg Phe
            340                 345                 350

Pro Glu Glu Glu Glu Gly Gly Cys Glu Leu
        355                 360

<210>  38
<211>  403
<212>  PRT
<213>  Artificial Sequence

<220>
<223>  Amino acid sequence of 28DCR3 (without signal peptide)

<400>  38

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Ile
        35                  40                  45

Gly Cys Ile Tyr Pro Gly Asn Val Asn Thr Asn Tyr Asn Glu Lys Phe
    50                  55                  60

Lys Asp Arg Ala Thr Leu Thr Val Asp Thr Ser Ile Ser Thr Ala Tyr
65                  70                  75                  80

Met Glu Leu Ser Arg Leu Arg Ser Asp Asp Thr Ala Val Tyr Phe Cys
                85                  90                  95

Thr Arg Ser His Tyr Gly Leu Asp Trp Asn Phe Asp Val Trp Gly Gln
            100                 105                 110

Gly Thr Thr Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys His Ala

145                 150                 155                 160

Ser Gln Asn Ile Tyr Val Trp Leu Asn Trp Tyr Gln Gln Lys Pro Gly
                165                 170                 175

Lys Ala Pro Lys Leu Leu Ile Tyr Lys Ala Ser Asn Leu His Thr Gly
            180                 185                 190

Val Pro Ser Arg Phe Ser Gly Ser Gly Ser Gly Thr Asp Phe Thr Leu
        195                 200                 205

Thr Ile Ser Ser Leu Gln Pro Glu Asp Phe Ala Thr Tyr Tyr Cys Gln
    210                 215                 220

Gln Gly Gln Thr Tyr Pro Tyr Thr Phe Gly Gly Gly Thr Lys Val Glu
225                 230                 235                 240

Ile Lys Glu Ser Lys Tyr Gly Pro Pro Cys Pro Pro Cys Pro Ile Glu
                245                 250                 255

Val Met Tyr Pro Pro Pro Tyr Leu Asp Asn Glu Lys Ser Asn Gly Thr
            260                 265                 270

Ile Ile His Val Lys Gly Lys His Leu Cys Pro Ser Pro Leu Phe Pro
        275                 280                 285

Gly Pro Ser Lys Pro Phe Trp Val Leu Val Val Val Gly Gly Val Leu
    290                 295                 300

Ala Cys Tyr Ser Leu Leu Val Thr Val Ala Phe Ile Ile Phe Trp Val
305                 310                 315                 320

Arg Ser Lys Arg Ser Arg Leu Leu His Ser Asp Tyr Met Asn Met Thr
                325                 330                 335

Pro Arg Arg Pro Gly Pro Thr Arg Lys His Tyr Gln Pro Tyr Ala Pro
            340                 345                 350

Pro Arg Asp Phe Ala Ala Tyr Arg Ser Lys Arg Gly Arg Lys Lys Leu
        355                 360                 365

Leu Tyr Ile Phe Lys Gln Pro Phe Met Arg Pro Val Gln Thr Thr Gln
    370                 375                 380

Glu Glu Asp Gly Cys Ser Cys Arg Phe Pro Glu Glu Glu Glu Gly Gly
385                 390                 395                 400

Cys Glu Leu

<210> 39
<211> 403
<212> PRT
<213> Artificial Sequence

<220>
<223> Amino acid sequence of 28DCR4 (without signal peptide)

<400> 39

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Tyr Ile His Trp Val Arg Gln Ala Pro Gly Gln Gly Leu Glu Trp Ile
        35                  40                  45

Gly Cys Ile Tyr Pro Gly Asn Val Asn Thr Asn Tyr Asn Glu Lys Phe
    50                  55                  60

Lys Asp Arg Ala Thr Leu Thr Val Asp Thr Ser Ile Ser Thr Ala Tyr
65                  70                  75                  80

Met Glu Leu Ser Arg Leu Arg Ser Asp Asp Thr Ala Val Tyr Phe Cys
                85                  90                  95

Thr Arg Ser His Tyr Gly Leu Asp Trp Asn Phe Asp Val Trp Gly Gln
            100                 105                 110

Gly Thr Thr Val Thr Val Ser Ser Gly Gly Gly Gly Ser Gly Gly Gly
        115                 120                 125

Gly Ser Gly Gly Gly Gly Ser Asp Ile Gln Met Thr Gln Ser Pro Ser
    130                 135                 140

Ser Leu Ser Ala Ser Val Gly Asp Arg Val Thr Ile Thr Cys His Ala
145                 150                 155                 160

Ser Gln Asn Ile Tyr Val Trp Leu Asn Trp Tyr Gln Gln Lys Pro Gly
                165                 170                 175

Lys Ala Pro Lys Leu Leu Ile Tyr Lys Ala Ser Asn Leu His Thr Gly
            180                 185                 190

Val Pro Ser Arg Phe Ser Gly Ser Gly Ser Gly Thr Asp Phe Thr Leu

195                 200                 205

Thr Ile Ser Ser Leu Gln Pro Glu Asp Phe Ala Thr Tyr Tyr Cys Gln
    210                 215                 220

Gln Gly Gln Thr Tyr Pro Tyr Thr Phe Gly Gly Gly Thr Lys Val Glu
225                 230                 235                 240

Ile Lys Glu Ser Lys Tyr Gly Pro Pro Cys Pro Pro Cys Pro Ile Glu
                245                 250                 255

Val Met Tyr Pro Pro Pro Tyr Leu Asp Asn Glu Lys Ser Asn Gly Thr
            260                 265                 270

Ile Ile His Val Lys Gly Lys His Leu Cys Pro Ser Pro Leu Phe Pro
        275                 280                 285

Gly Pro Ser Lys Pro Phe Trp Val Leu Val Val Val Gly Gly Val Leu
    290                 295                 300

Ala Cys Tyr Ser Leu Leu Val Thr Val Ala Phe Ile Ile Phe Trp Val
305                 310                 315                 320

Lys Arg Gly Arg Lys Lys Leu Leu Tyr Ile Phe Lys Gln Pro Phe Met
                325                 330                 335

Arg Pro Val Gln Thr Thr Gln Glu Glu Asp Gly Cys Ser Cys Arg Phe
            340                 345                 350

Pro Glu Glu Glu Glu Gly Gly Cys Glu Leu Arg Ser Lys Arg Ser Arg
        355                 360                 365

Leu Leu His Ser Asp Tyr Met Asn Met Thr Pro Arg Arg Pro Gly Pro
    370                 375                 380

Thr Arg Lys His Tyr Gln Pro Tyr Ala Pro Pro Arg Asp Phe Ala Ala
385                 390                 395                 400

Tyr Arg Ser

<210>  40
<211>  1158
<212>  DNA
<213>  Artificial Sequence

<220>
<223>  Nucleic acid sequence of 28DCR1 (including signal peptide)

<400> 40
atgggaaaca gctgttacaa catagtagcc actctgttgc tggtcctcaa ctttgagagg 60

acaagatcac aggtgcagct ggtgcagtct ggggctgagg tgaagaagcc tggggcctca 120

gtgaaggtct cctgcaaggc ttctggatac accttcacca gctactatat acactgggtg 180

cgacaggccc ctggacaagg gcttgagtgg attggatgta tttatcctgg aaatgtcaat 240

actaactata atgagaagtt caaggacagg gccaccctga ccgtagacac gtccatcagc 300

acagcctaca tggagctgag caggctgaga tctgacgaca cggccgtgta tttctgtaca 360

agatcacact acggcctcga ctggaacttc gatgtctggg gccaagggac cacggtcacc 420

gtctcctcag gtggaggcgg ttcaggcgga ggtggcagcg gcggtggcgg gtcggacatc 480

cagatgaccc agtctccatc ctccctgtct gcatctgtag gagacagagt caccatcact 540

tgccatgcca gtcaaaacat ttatgtttgg ttaaactggt atcagcagaa accagggaaa 600

gcccctaagc tcctgatcta taaggcttcc aacctgcaca caggggtccc atcaaggttc 660

agtggcagtg gatctgggac agatttcact ctcaccatca gcagtctgca acctgaagat 720

tttgcaactt actactgtca acagggtcaa acttatccgt acacgttcgg cggagggacc 780

aaggtggaga tcaaagagtc caaatatggt cccccatgcc caccatgccc aattgaagtt 840

atgtatcctc ctccttacct agacaatgag aagagcaatg gaaccattat ccatgtgaaa 900

gggaaacacc tttgtccaag tcccctattt cccggacctt ctaagccctt ttgggtgctg 960

gtggtggttg gtggagtcct ggcttgctat agcttgctag taacagtggc ctttattatt 1020

ttctgggtga ggagtaagag gagcaggctc ctgcacagtg actacatgaa catgactccc 1080

cgccgccccg ggcccacccg caagcattac cagccctatg ccccaccacg cgacttcgca 1140

gcctatcgct cctgataa 1158

<210> 41
<211> 1161
<212> DNA
<213> Artificial Sequence

<220>
<223> Nucleic acid sequence of 28DCR2 (including signal peptide)

<400> 41
atgggaaaca gctgttacaa catagtagcc actctgttgc tggtcctcaa ctttgagagg 60

acaagatcac aggtgcagct ggtgcagtct ggggctgagg tgaagaagcc tggggcctca 120

gtgaaggtct cctgcaaggc ttctggatac accttcacca gctactatat acactgggtg 180

cgacaggccc ctggacaagg gcttgagtgg attggatgta tttatcctgg aaatgtcaat 240

actaactata atgagaagtt caaggacagg gccaccctga ccgtagacac gtccatcagc 300

acagcctaca tggagctgag caggctgaga tctgacgaca cggccgtgta tttctgtaca 360

agatcacact acggcctcga ctggaacttc gatgtctggg gccaagggac cacggtcacc 420

gtctcctcag gtggaggcgg ttcaggcgga ggtggcagcg gcggtggcgg gtcggacatc 480

cagatgaccc agtctccatc ctccctgtct gcatctgtag gagacagagt caccatcact 540

tgccatgcca gtcaaaacat ttatgtttgg ttaaactggt atcagcagaa accagggaaa 600

gcccctaagc tcctgatcta taaggcttcc aacctgcaca caggggtccc atcaaggttc 660

agtggcagtg gatctgggac agatttcact ctcaccatca gcagtctgca acctgaagat 720

tttgcaactt actactgtca acagggtcaa acttatccgt acacgttcgg cggagggacc 780

aaggtggaga tcaaagagtc caaatatggt cccccatgcc caccatgccc aattgaagtt 840

atgtatcctc ctccttacct agacaatgag aagagcaatg gaaccattat ccatgtgaaa 900

gggaaacacc tttgtccaag tcccctattt cccggacctt ctaagccctt ttgggtgctg 960

gtggtggttg gtggagtcct ggcttgctat agcttgctag taacagtggc ctttattatt 1020

ttctgggtga aacggggcag aaagaagctc ctgtatatat tcaaacaacc atttatgaga 1080

ccagtacaaa ctactcaaga ggaagatggc tgtagctgcc gatttccaga agaagaagaa 1140

ggaggatgtg aactgtgata a 1161

<210> 42
<211> 1284
<212> DNA
<213> Artificial Sequence

<220>
<223> Nucleic acid sequence of 28DCR3 (including signal peptide)

<400> 42
atgggaaaca gctgttacaa catagtagcc actctgttgc tggtcctcaa ctttgagagg 60

acaagatcac aggtgcagct ggtgcagtct ggggctgagg tgaagaagcc tggggcctca 120

gtgaaggtct cctgcaaggc ttctggatac accttcacca gctactatat acactgggtg 180

cgacaggccc ctggacaagg gcttgagtgg attggatgta tttatcctgg aaatgtcaat 240

actaactata atgagaagtt caaggacagg gccaccctga ccgtagacac gtccatcagc 300

acagcctaca tggagctgag caggctgaga tctgacgaca cggccgtgta tttctgtaca 360

agatcacact acggcctcga ctggaacttc gatgtctggg gccaagggac cacggtcacc 420

gtctcctcag gtggaggcgg ttcaggcgga ggtggcagcg gcggtggcgg gtcggacatc 480

cagatgaccc agtctccatc ctccctgtct gcatctgtag gagacagagt caccatcact 540

tgccatgcca gtcaaaacat ttatgtttgg ttaaactggt atcagcagaa accagggaaa 600

gcccctaagc tcctgatcta taaggcttcc aacctgcaca caggggtccc atcaaggttc 660

agtggcagtg gatctgggac agatttcact ctcaccatca gcagtctgca acctgaagat 720

tttgcaactt actactgtca acagggtcaa acttatccgt acacgttcgg cggagggacc 780

aaggtggaga tcaaagagtc caaatatggt cccccatgcc caccatgccc aattgaagtt 840

atgtatcctc ctccttacct agacaatgag aagagcaatg gaaccattat ccatgtgaaa 900

gggaaacacc tttgtccaag tcccctattt cccggacctt ctaagccctt ttgggtgctg 960

gtggtggttg gtggagtcct ggcttgctat agcttgctag taacagtggc ctttattatt 1020

ttctgggtga ggagtaagag gagcaggctc ctgcacagtg actacatgaa catgactccc 1080

cgccgccccg ggcccacccg caagcattac cagccctatg ccccaccacg cgacttcgca 1140

gcctatcgct ccaaacgggg cagaaagaag ctcctgtata tattcaaaca accatttatg 1200

agaccagtac aaactactca agaggaagat ggctgtagct gccgatttcc agaagaagaa 1260

gaaggaggat gtgaactgtg ataa 1284

<210> 43
<211> 1284
<212> DNA
<213> Artificial Sequence

<220>
<223> Nucleic acid sequence of 28DCR4 (including signal peptide)

<400> 43
atgggaaaca gctgttacaa catagtagcc actctgttgc tggtcctcaa ctttgagagg 60

acaagatcac aggtgcagct ggtgcagtct ggggctgagg tgaagaagcc tggggcctca 120

gtgaaggtct cctgcaaggc ttctggatac accttcacca gctactatat acactgggtg 180

cgacaggccc ctggacaagg gcttgagtgg attggatgta tttatcctgg aaatgtcaat 240

actaactata atgagaagtt caaggacagg gccaccctga ccgtagacac gtccatcagc 300

acagcctaca tggagctgag caggctgaga tctgacgaca cggccgtgta tttctgtaca 360

agatcacact acggcctcga ctggaacttc gatgtctggg gccaagggac cacggtcacc 420

gtctcctcag gtggaggcgg ttcaggcgga ggtggcagcg gcggtggcgg gtcggacatc 480

cagatgaccc agtctccatc ctccctgtct gcatctgtag gagacagagt caccatcact 540

tgccatgcca gtcaaaacat ttatgtttgg ttaaactggt atcagcagaa accagggaaa 600

gcccctaagc tcctgatcta taaggcttcc aacctgcaca caggggtccc atcaaggttc 660

agtggcagtg gatctgggac agatttcact ctcaccatca gcagtctgca acctgaagat 720

tttgcaactt actactgtca acagggtcaa acttatccgt acacgttcgg cggagggacc 780

aaggtggaga tcaaagagtc caaatatggt cccccatgcc caccatgccc aattgaagtt 840

atgtatcctc ctccttacct agacaatgag aagagcaatg gaaccattat ccatgtgaaa 900

gggaaacacc tttgtccaag tcccctattt cccggacctt ctaagccctt ttgggtgctg 960

gtggtggttg gtggagtcct ggcttgctat agcttgctag taacagtggc ctttattatt 1020

ttctgggtga aacggggcag aaagaagctc ctgtatatat tcaaacaacc atttatgaga 1080

ccagtacaaa ctactcaaga ggaagatggc tgtagctgcc gatttccaga agaagaagaa 1140

ggaggatgtg aactgaggag taagaggagc aggctcctgc acagtgacta catgaacatg 1200

actccccgcc gccccgggcc cacccgcaag cattaccagc cctatgcccc accacgcgac 1260

ttcgcagcct atcgctcctg ataa 1284

<210> 44
<211> 1089
<212> DNA
<213> Artificial Sequence

<220>
<223> Nucleic acid sequence of 28DCR1 (without signal peptide)

<400> 44
caggtgcagc tggtgcagtc tggggctgag gtgaagaagc ctggggcctc agtgaaggtc 60

tcctgcaagg cttctggata caccttcacc agctactata tacactgggt gcgacaggcc 120

cctggacaag ggcttgagtg gattggatgt atttatcctg gaaatgtcaa tactaactat 180

aatgagaagt tcaaggacag ggccaccctg accgtagaca cgtccatcag cacagcctac 240

atggagctga gcaggctgag atctgacgac acggccgtgt atttctgtac aagatcacac 300

tacggcctcg actggaactt cgatgtctgg ggccaaggga ccacggtcac cgtctcctca 360

ggtggaggcg gttcaggcgg aggtggcagc ggcggtggcg ggtcggacat ccagatgacc 420

cagtctccat cctccctgtc tgcatctgta ggagacagag tcaccatcac ttgccatgcc 480

agtcaaaaca tttatgtttg gttaaactgg tatcagcaga aaccagggaa agcccctaag 540

ctcctgatct ataaggcttc caacctgcac acaggggtcc catcaaggtt cagtggcagt 600

ggatctggga cagatttcac tctcaccatc agcagtctgc aacctgaaga ttttgcaact 660

tactactgtc aacaggtcaa aacttatccg tacacgttcg gcggagggac caaggtggag 720

atcaaagagt ccaaatatgg tcccccatgc ccaccatgcc caattgaagt tatgtatcct 780

cctccttacc tagacaatga gaagagcaat ggaaccatta tccatgtgaa agggaaacac 840

ctttgtccaa gtcccctatt tcccggacct tctaagccct tttgggtgct ggtggtggtt 900

ggtggagtcc tggcttgcta tagcttgcta gtaacagtgg cctttattat tttctgggtg 960

aggagtaaga ggagcaggct cctgcacagt gactacatga acatgactcc ccgccgcccc 1020

gggcccaccc gcaagcatta ccagccctat gccccaccac gcgacttcgc agcctatcgc 1080

tcctgataa 1089

<210> 45
<211> 1092
<212> DNA
<213> Artificial Sequence

<220>
<223>  Nucleic acid sequence of 28DCR2 (without signal peptide)

<400>  45
caggtgcagc tggtgcagtc tggggctgag gtgaagaagc ctggggcctc agtgaaggtc      60

tcctgcaagg cttctggata caccttcacc agctactata tacactgggt gcgacaggcc     120

cctggacaag ggcttgagtg gattggatgt atttatcctg gaaatgtcaa tactaactat     180

aatgagaagt tcaaggacag ggccaccctg accgtagaca cgtccatcag cacagcctac     240

atggagctga gcaggctgag atctgacgac acggccgtgt atttctgtac aagatcacac     300

tacggcctcg actggaactt cgatgtctgg ggccaaggga ccacggtcac cgtctcctca     360

ggtggaggcg gttcaggcgg aggtggcagc ggcggtggcg ggtcggacat ccagatgacc     420

cagtctccat cctccctgtc tgcatctgta ggagacagag tcaccatcac ttgccatgcc     480

agtcaaaaca tttatgtttg gttaaactgg tatcagcaga aaccagggaa agcccctaag     540

ctcctgatct ataaggcttc caacctgcac acaggggtcc catcaaggtt cagtggcagt     600

ggatctggga cagatttcac tctcaccatc agcagtctgc aacctgaaga ttttgcaact     660

tactactgtc aacagggtca aacttatccg tacacgttcg gcggagggac caaggtggag     720

atcaaagagt ccaaatatgg tcccccatgc ccaccatgcc caattgaagt tatgtatcct     780

cctccttacc tagacaatga gaagagcaat ggaaccatta tccatgtgaa agggaaacac     840

ctttgtccaa gtcccctatt tcccggacct tctaagccct tttgggtgct ggtggtggtt     900

ggtggagtcc tggcttgcta tagcttgcta gtaacagtgg cctttattat tttctgggtg     960

aaacggggca gaaagaagct cctgtatata ttcaaacaac catttatgag accagtacaa    1020

actactcaag aggaagatgg ctgtagctgc cgatttccag aagaagaaga aggaggatgt    1080

gaactgtgat aa                                                        1092


<210>  46
<211>  1215
<212>  DNA
<213>  Artificial Sequence

<220>
<223>  Nucleic acid sequence of 28DCR3 (without signal peptide)

<400>  46
caggtgcagc tggtgcagtc tggggctgag gtgaagaagc ctggggcctc agtgaaggtc      60

tcctgcaagg cttctggata caccttcacc agctactata tacactgggt gcgacaggcc     120

cctggacaag ggcttgagtg gattggatgt atttatcctg gaaatgtcaa tactaactat     180

aatgagaagt tcaaggacag ggccaccctg accgtagaca cgtccatcag cacagcctac     240

atggagctga gcaggctgag atctgacgac acggccgtgt atttctgtac aagatcacac     300

tacggcctcg actggaactt cgatgtctgg ggccaaggga ccacggtcac cgtctcctca 360

ggtggaggcg gttcaggcgg aggtggcagc ggcggtggcg ggtcggacat ccagatgacc 420

cagtctccat cctccctgtc tgcatctgta ggagacagag tcaccatcac ttgccatgcc 480

agtcaaaaca tttatgtttg gttaaactgg tatcagcaga aaccagggaa agcccctaag 540

ctcctgatct ataaggcttc caacctgcac acaggggtcc catcaaggtt cagtggcagt 600

ggatctggga cagatttcac tctcaccatc agcagtctgc aacctgaaga ttttgcaact 660

tactactgtc aacaggtcaa aacttatccg tacacgttcg gcggagggac caaggtggag 720

atcaaagagt ccaaatatgg tcccccatgc ccaccatgcc caattgaagt tatgtatcct 780

cctccttacc tagacaatga gaagagcaat ggaaccatta tccatgtgaa agggaaacac 840

ctttgtccaa gtcccctatt tcccggacct tctaagccct tttgggtgct ggtggtggtt 900

ggtggagtcc tggcttgcta tagcttgcta gtaacagtgg cctttattat tttctgggtg 960

aggagtaaga ggagcaggct cctgcacagt gactacatga acatgactcc ccgccgcccc 1020

gggcccaccc gcaagcatta ccagccctat gccccaccac gcgacttcgc agcctatcgc 1080

tccaaacggg gcagaaagaa gctcctgtat atattcaaac aaccatttat gagaccagta 1140

caaactactc aagaggaaga tggctgtagc tgccgatttc cagaagaaga agaaggagga 1200

tgtgaactgt gataa 1215

<210> 47
<211> 1215
<212> DNA
<213> Artificial Sequence

<220>
<223> Nucleic acid sequence of 28DCR4 (without signal peptide)

<400> 47
caggtgcagc tggtgcagtc tggggctgag gtgaagaagc ctggggcctc agtgaaggtc 60

tcctgcaagg cttctggata caccttcacc agctactata tacactgggt gcgacaggcc 120

cctggacaag ggcttgagtg gattggatgt atttatcctg gaaatgtcaa tactaactat 180

aatgagaagt tcaaggacag ggccaccctg accgtagaca cgtccatcag cacagcctac 240

atggagctga gcaggctgag atctgacgac acggccgtgt atttctgtac aagatcacac 300

tacggcctcg actggaactt cgatgtctgg ggccaaggga ccacggtcac cgtctcctca 360

ggtggaggcg gttcaggcgg aggtggcagc ggcggtggcg ggtcggacat ccagatgacc 420

cagtctccat cctccctgtc tgcatctgta ggagacagag tcaccatcac ttgccatgcc 480

agtcaaaaca tttatgtttg gttaaactgg tatcagcaga aaccagggaa agcccctaag 540

ctcctgatct ataaggcttc caacctgcac acaggggtcc catcaaggtt cagtggcagt 600

ggatctggga cagatttcac tctcaccatc agcagtctgc aacctgaaga ttttgcaact 660

tactactgtc aacagggtca aacttatccg tacacgttcg gcggagggac caaggtggag 720

atcaaagagt ccaaatatgg tcccccatgc ccaccatgcc caattgaagt tatgtatcct 780

cctccttacc tagacaatga gaagagcaat ggaaccatta tccatgtgaa agggaaacac 840

ctttgtccaa gtcccctatt tcccggacct tctaagccct tttgggtgct ggtggtggtt 900

ggtggagtcc tggcttgcta tagcttgcta gtaacagtgg cctttattat tttctgggtg 960

aaacggggca gaaagaagct cctgtatata ttcaaacaac catttatgag accagtacaa 1020

actactcaag aggaagatgg ctgtagctgc cgatttccag aagaagaaga aggaggatgt 1080

gaactgagga gtaagaggag caggctcctg cacagtgact acatgaacat gactccccgc 1140

cgccccgggc ccacccgcaa gcattaccag ccctatgccc caccacgcga cttcgcagcc 1200

tatcgctcct gataa 1215

<210> 48
<211> 729
<212> PRT
<213> Artificial Sequence

<220>
<223> Amino acid sequence of Muc1 G1 CAR

<400> 48

Met Ala Leu Pro Val Thr Ala Leu Leu Leu Pro Leu Ala Leu Leu Leu
1               5                   10                  15

His Ala Ala Arg Pro Ser Glu Val Gln Leu Gln Gln Ser Gly Gly Gly
            20                  25                  30

Leu Val Gln Pro Gly Gly Ser Met Lys Leu Ser Cys Val Ala Ser Gly
        35                  40                  45

Phe Thr Phe Ser Asn Tyr Trp Met Asn Trp Val Arg Gln Ser Pro Glu
    50                  55                  60

Lys Gly Leu Glu Trp Val Ala Glu Ile Arg Leu Lys Ser Asn Asn Tyr
65                  70                  75                  80

Ala Thr His Tyr Ala Glu Ser Val Lys Gly Arg Phe Thr Ile Ser Arg
                85                  90                  95

Asp Asp Ser Lys Ser Ser Val Tyr Leu Gln Met Asn Asn Leu Arg Ala
            100                 105                 110

Glu Asp Thr Gly Ile Tyr Tyr Cys Thr Phe Gly Asn Ser Phe Ala Tyr
        115                 120                 125

Trp Gly Gln Gly Thr Thr Val Thr Val Ser Ser Gly Gly Ser Gly Ser
    130                 135                 140

Gly Gly Ser Gly Ser Gly Gly Ser Gly Ser Asp Ile Val Val Thr Gln
145                 150                 155                 160

Glu Ser Ala Leu Thr Thr Ser Pro Gly Glu Thr Val Thr Leu Thr Cys
                165                 170                 175

Arg Ser Ser Thr Gly Ala Val Thr Thr Ser Asn Tyr Ala Asn Trp Val
            180                 185                 190

Gln Glu Lys Pro Asp His Leu Phe Thr Gly Leu Ile Gly Gly Thr Asn
        195                 200                 205

Asn Arg Ala Pro Gly Val Pro Ala Arg Phe Ser Gly Ser Leu Ile Gly
    210                 215                 220

Asp Lys Ala Ala Leu Thr Ile Thr Gly Ala Gln Thr Glu Asp Glu Ala
225                 230                 235                 240

Ile Tyr Phe Cys Ala Leu Trp Tyr Ser Asn His Trp Val Phe Gly Gly
                245                 250                 255

Gly Thr Lys Leu Thr Val Leu Gly Ser Glu Glu Ser Lys Tyr Gly Pro
            260                 265                 270

Pro Cys Pro Pro Cys Pro Ala Pro Pro Val Ala Gly Pro Ser Val Phe
        275                 280                 285

Leu Phe Pro Pro Lys Pro Lys Asp Thr Leu Met Ile Ser Arg Thr Pro
    290                 295                 300

Glu Val Thr Cys Val Val Val Asp Val Ser Gln Glu Asp Pro Glu Val
305                 310                 315                 320

Gln Phe Asn Trp Tyr Val Asp Gly Val Glu Val His Asn Ala Lys Thr
                325                 330                 335

Lys Pro Arg Glu Glu Gln Phe Gln Ser Thr Tyr Arg Val Val Ser Val
            340                 345                 350

Leu Thr Val Leu His Gln Asp Trp Leu Asn Gly Lys Glu Tyr Lys Cys
        355                 360                 365

Lys Val Ser Asn Lys Gly Leu Pro Ser Ser Ile Glu Lys Thr Ile Ser
    370                 375                 380

Lys Ala Lys Gly Gln Pro Arg Glu Pro Gln Val Tyr Thr Leu Pro Pro
385                 390                 395                 400

Ser Gln Glu Glu Met Thr Lys Asn Gln Val Ser Leu Thr Cys Leu Val
                405                 410                 415

Lys Gly Phe Tyr Pro Ser Asp Ile Ala Val Glu Trp Glu Ser Asn Gly
            420                 425                 430

Gln Pro Glu Asn Asn Tyr Lys Thr Thr Pro Pro Val Leu Asp Ser Asp
        435                 440                 445

Gly Ser Phe Phe Leu Tyr Ser Arg Leu Thr Val Asp Lys Ser Arg Trp
    450                 455                 460

Gln Glu Gly Asn Val Phe Ser Cys Ser Val Met His Glu Ala Leu His
465                 470                 475                 480

Asn His Tyr Thr Gln Lys Ser Leu Ser Leu Ser Leu Gly Lys Phe Val
                485                 490                 495

Pro Val Phe Leu Pro Ala Lys Pro Thr Thr Thr Pro Ala Pro Arg Pro
            500                 505                 510

Pro Thr Pro Ala Pro Thr Ile Ala Ser Gln Pro Leu Ser Leu Arg Pro
        515                 520                 525

Glu Ala Cys Arg Pro Ala Ala Gly Gly Ala Val His Thr Arg Gly Leu
    530                 535                 540

Asp Phe Ala Cys Asp Ile Tyr Ile Trp Ala Pro Leu Ala Gly Thr Cys
545                 550                 555                 560

Gly Val Leu Leu Leu Ser Leu Val Ile Thr Leu Tyr Cys Asn His Lys
                565                 570                 575

Arg Gly Arg Lys Lys Leu Leu Tyr Ile Phe Lys Gln Pro Phe Met Arg
            580                 585                 590

Pro Val Gln Thr Thr Gln Glu Glu Asp Gly Cys Ser Cys Arg Phe Pro
        595                 600                 605

Glu Glu Glu Glu Gly Gly Cys Glu Leu Arg Val Lys Phe Ser Arg Ser
    610                 615                 620

Ala Asp Ala Pro Ala Tyr Gln Gln Gly Gln Asn Gln Leu Tyr Asn Glu

625                 630                 635                 640

Leu Asn Leu Gly Arg Arg Glu Glu Tyr Asp Val Leu Asp Lys Arg Arg
                645                 650                 655

Gly Arg Asp Pro Glu Met Gly Gly Lys Pro Arg Arg Lys Asn Pro Gln
            660                 665                 670

Glu Gly Leu Tyr Asn Glu Leu Gln Lys Asp Lys Met Ala Glu Ala Tyr
        675                 680                 685

Ser Glu Ile Gly Met Lys Gly Glu Arg Arg Arg Gly Lys Gly His Asp
    690                 695                 700

Gly Leu Tyr Gln Gly Leu Ser Thr Ala Thr Lys Asp Thr Tyr Asp Ala
705                 710                 715                 720

Leu His Met Gln Ala Leu Pro Pro Arg
                725

<210>  49
<211>  1371
<212>  DNA
<213>  Artificial Sequence

<220>
<223>  Nucleic acid sequence of Muc1 G1 CAR

<400>  49
ccatgcccac catgcccagc acctcccgtg gccggaccat cagtcttcct gttcccccca      60

aaacccaagg acactctcat gatctcccgg acccctgagg tcacgtgcgt ggtggtggac     120

gtgagccagg aagaccccga ggtccagttc aactggtacg tggatggcgt ggaggtgcat     180

aatgccaaga caaagccgcg ggaggagcag ttccagagca cgtaccgtgt ggtcagcgtc     240

ctcaccgtcc tgcaccagga ctggctgaac ggcaaggagt acaagtgcaa ggtctccaac     300

aaaggcctcc cgtcctccat cgagaaaacc atctccaaag ccaaagggca gccccgagag     360

ccacaggtgt acaccctgcc cccatcccag gaggagatga ccaagaacca ggtcagcctg     420

acctgcctgg tcaaaggctt ctaccccagc gacatcgccg tggagtggga gagcaatggg     480

cagccggaga acaactacaa gaccacgcct cccgtgctgg actccgacgg ctccttcttc     540

ctctacagca ggctaaccgt ggacaagagc aggtggcagg aggggaatgt cttctcatgc     600

tccgtgatgc atgaggctct gcacaaccac tacacacaga agagcctctc cctgtctctg     660

ggtaaattcg tgccggtctt cctgccagcg aagcccacca cgacgccagc gccgcgacca     720

ccaacaccgg cgcccaccat cgcgtcgcag cccctgtccc tgcgcccaga ggcgtgccgg     780

ccagcggcgg ggggcgcagt gcacacgagg gggctggact tcgcctgtga tatctacatc     840

tgggcgcccc tggccgggac ttgtggggtc cttctcctgt cactggttat caccctttac 900

tgcaaccaca aacggggcag aaagaagctc ctgtatatat tcaaacaacc atttatgaga 960

ccagtacaaa ctactcaaga ggaagatggc tgtagctgcc gatttccaga agaagaagaa 1020

ggaggatgtg aactgagagt gaagttcagc aggagcgcag acgcccccgc gtaccagcag 1080

ggccagaacc agctctataa cgagctcaat ctaggacgaa gagaggagta cgatgttttg 1140

gacaagagac gtggccggga ccctgagatg gggggaaagc cgagaaggaa gaaccctcag 1200

gaaggcctgt acaatgaact gcagaaagat aagatggcgg aggcctacag tgagattggg 1260

atgaaaggcg agcgccggag gggcaagggg cacgatggcc tttaccaggg tctcagtaca 1320

gccaccaagg acacctacga cgcccttcac atgcaggccc tgccccctcg c 1371

<210> 50
<211> 2187
<212> DNA
<213> Artificial Sequence

<220>
<223> Nucleotide sequence of Muc1 G2 CAR

<400> 50
atggccttac cagtgaccgc cttgctcctg ccgctggcct tgctgctcca cgccgccagg 60

ccgagcgagg tccagctgca gcagtcagga ggaggcttgg tgcaacctgg aggatccatg 120

aaactctcct gtgttgcctc tggattcact ttcagtaact actggatgaa ctgggtccgc 180

cagtctccag agaaggggct tgagtgggtt gctgaaatta gattgaaatc taataattat 240

gcaacacatt atgcggagtc tgtgaaaggg aggttcacca tctcaagaga tgattccaaa 300

agtagtgtct acctgcaaat gaacaactta agagctgaag acactggcat ttattactgt 360

acctttggta actcctttgc ttactggggc caaggaacca cggtcaccgt ctcctcaggt 420

ggttctggtt ctggcggctc cggttccggt ggatccggct ctgatatcgt tgtgactcag 480

gaatctgcac tcaccacatc acctggtgaa acagtcacac tcacttgtcg ctcaagtact 540

ggggctgtta caactagtaa ctatgccaac tgggtccaag aaaaaccaga tcatttattc 600

actggtctaa taggtggtac caacaaccga gcaccaggtg ttcctgccag attctcaggc 660

tccctgattg gagacaaggc tgccctcacc atcacagggg cacagactga ggatgaggca 720

atatatttct gtgctctatg gtacagcaac cattgggtgt tcggtggagg aaccaaactg 780

actgtcctag gatccgagga gtccaaatat ggtcccccat gcccaccatg cccagcacct 840

cccgtggccg gaccatcagt cttcctgttc cccccaaaac ccaaggacac tctcatgatc 900

tcccggaccc ctgaggtcac gtgcgtggtg gtggacgtga gccaggaaga ccccgaggtc 960

cagttcaact ggtacgtgga tggcgtggag gtgcataatg ccaagacaaa gccgcgggag 1020

gagcagttcc agagcacgta ccgtgtggtc agcgtcctca ccgtcctgca ccaggactgg 1080

ctgaacggca aggagtacaa gtgcaaggtc tccaacaaag gcctcccgtc ctccatcgag 1140

aaaaccatct ccaaagccaa agggcagccc cgagagccac aggtgtacac cctgccccca 1200

tcccaggagg agatgaccaa gaaccaggtc agcctgacct gcctggtcaa aggcttctac 1260

cccagcgaca tcgccgtgga gtgggagagc aatgggcagc cggagaacaa ctacaagacc 1320

acgcctcccg tgctggactc cgacggctcc ttcttcctct acagcaggct aaccgtggac 1380

aagagcaggt ggcaggaggg gaatgtcttc tcatgctccg tgatgcatga ggctctgcac 1440

aaccactaca cacagaagag cctctccctg tctctgggta aattcgtgcc ggtcttcctg 1500

ccagcgaagc ccaccacgac gccagcgccg cgaccaccaa caccggcgcc caccatcgcg 1560

tcgcagcccc tgtccctgcg cccagaggcg tgccggccag cggcgggggg cgcagtgcac 1620

acgagggggc tggacttcgc ctgtgatatc tacatctggg cgcccctggc cgggacttgt 1680

ggggtccttc tcctgtcact ggttatcacc ctttactgca accacaaacg gggcagaaag 1740

aagctcctgt atatattcaa acaaccattt atgagaccag tacaaactac tcaagaggaa 1800

gatggctgta gctgccgatt tccagaagaa gaagaaggag gatgtgaact gagagtgaag 1860

ttcagcagga gcgcagacgc ccccgcgtac cagcagggcc agaaccagct ctataacgag 1920

ctcaatctag gacgaagaga ggagtacgat gttttggaca agagacgtgg ccgggaccct 1980

gagatggggg gaaagccgag aaggaagaac cctcaggaag gcctgtacaa tgaactgcag 2040

aaagataaga tggcggaggc ctacagtgag attgggatga aaggcgagcg ccggaggggc 2100

aaggggcacg atggccttta ccagggtctc agtacagcca ccaaggacac ctacgacgcc 2160

cttcacatgc aggccctgcc ccctcgc 2187

<210> 51
<211> 18
<212> DNA
<213> Artificial Sequence

<220>
<223> Nucleotide sequence of primer CD28-F

<400> 51
gcttctggat acaccttc 18

<210> 52
<211> 24
<212> DNA
<213> Artificial Sequence

<220>
<223> Nucleotide sequence of primer CD28-R

<400> 52
ccttgaactt ctcattatag ttag 24

<210>  53
<211>  23
<212>  DNA
<213>  Artificial Sequence

<220>
<223>  Nucleotide sequence in Taqman

<400>  53
aatacatcca atccactcaa gcc                                               23


<210>  54
<211>  22
<212>  PRT
<213>  Artificial Sequence

<220>
<223>  Amino acid sequence of CD8 signal peptide

<400>  54

Met Ala Leu Pro Val Thr Ala Leu Leu Leu Pro Leu Ala Leu Leu Leu
1               5                   10                  15


His Ala Ala Arg Pro Ser
            20


<210>  55
<211>  248
<212>  PRT
<213>  Artificial Sequence

<220>
<223>  Amino acid sequence of CD40 extracellular agonistic single-chain
antibody

<400>  55

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Gly Tyr
            20                  25                  30


Tyr Met His Trp Val Arg Gln Ala Pro Gly Gln Gly Leu Glu Trp Met
        35                  40                  45


Gly Trp Ile Asn Pro Asp Ser Gly Gly Thr Asn Tyr Ala Gln Lys Phe
    50                  55                  60


Gln Gly Arg Val Thr Met Thr Arg Asp Thr Ser Ile Ser Thr Ala Tyr
65                  70                  75                  80

Met Glu Leu Asn Arg Leu Arg Ser Asp Asp Thr Ala Val Tyr Tyr Cys
                85                  90                  95

Ala Arg Asp Gln Pro Leu Gly Tyr Cys Thr Asn Gly Val Cys Ser Tyr
            100                 105                 110

Phe Asp Tyr Trp Gly Gln Gly Thr Leu Val Thr Val Ser Ser Gly Gly
        115                 120                 125

Gly Gly Ser Gly Gly Gly Gly Ser Gly Gly Gly Gly Ser Asp Ile Gln
    130                 135                 140

Met Thr Gln Ser Pro Ser Ser Val Ser Ala Ser Val Gly Asp Arg Val
145                 150                 155                 160

Thr Ile Thr Cys Arg Ala Ser Gln Gly Ile Tyr Ser Trp Leu Ala Trp
                165                 170                 175

Tyr Gln Gln Lys Pro Gly Lys Ala Pro Asn Leu Leu Ile Tyr Thr Ala
            180                 185                 190

Ser Thr Leu Gln Ser Gly Val Pro Ser Arg Phe Ser Gly Ser Gly Ser
        195                 200                 205

Gly Thr Asp Phe Thr Leu Thr Ile Ser Ser Leu Gln Pro Glu Asp Phe
    210                 215                 220

Ala Thr Tyr Tyr Cys Gln Gln Ala Asn Ile Phe Pro Leu Thr Phe Gly
225                 230                 235                 240

Gly Gly Thr Lys Val Glu Ile Lys
                245

<210>  56
<211>  229
<212>  PRT
<213>  Artificial Sequence

<220>
<223>  Amino acid sequence of IgG4Fc CH2CH3 hinge region

<400>  56

Glu Ser Lys Tyr Gly Pro Pro Cys Pro Pro Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val

35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210>  57
<211>  568
<212>  PRT
<213>  Artificial Sequence

<220>
<223>  Amino acid sequence of 40DCR1 (including signal peptide)

<400>  57

Met Ala Leu Pro Val Thr Ala Leu Leu Leu Pro Leu Ala Leu Leu Leu
1               5                   10                  15

His Ala Ala Arg Pro Ser Gln Val Gln Leu Val Gln Ser Gly Ala Glu
            20                  25                  30

Val Lys Lys Pro Gly Ala Ser Val Lys Val Ser Cys Lys Ala Ser Gly
        35                  40                  45

Tyr Thr Phe Thr Gly Tyr Tyr Met His Trp Val Arg Gln Ala Pro Gly
    50                  55                  60

Gln Gly Leu Glu Trp Met Gly Trp Ile Asn Pro Asp Ser Gly Gly Thr
65                  70                  75                  80

Asn Tyr Ala Gln Lys Phe Gln Gly Arg Val Thr Met Thr Arg Asp Thr
                85                  90                  95

Ser Ile Ser Thr Ala Tyr Met Glu Leu Asn Arg Leu Arg Ser Asp Asp
            100                 105                 110

Thr Ala Val Tyr Tyr Cys Ala Arg Asp Gln Pro Leu Gly Tyr Cys Thr
        115                 120                 125

Asn Gly Val Cys Ser Tyr Phe Asp Tyr Trp Gly Gln Gly Thr Leu Val
    130                 135                 140

Thr Val Ser Ser Gly Gly Gly Gly Ser Gly Gly Gly Gly Ser Gly Gly
145                 150                 155                 160

Gly Gly Ser Asp Ile Gln Met Thr Gln Ser Pro Ser Ser Val Ser Ala
                165                 170                 175

Ser Val Gly Asp Arg Val Thr Ile Thr Cys Arg Ala Ser Gln Gly Ile
            180                 185                 190

Tyr Ser Trp Leu Ala Trp Tyr Gln Gln Lys Pro Gly Lys Ala Pro Asn
        195                 200                 205

Leu Leu Ile Tyr Thr Ala Ser Thr Leu Gln Ser Gly Val Pro Ser Arg
    210                 215                 220

Phe Ser Gly Ser Gly Ser Gly Thr Asp Phe Thr Leu Thr Ile Ser Ser
225                 230                 235                 240

Leu Gln Pro Glu Asp Phe Ala Thr Tyr Tyr Cys Gln Gln Ala Asn Ile
                245                 250                 255

Phe Pro Leu Thr Phe Gly Gly Gly Thr Lys Val Glu Ile Lys Glu Ser

            260                 265                 270

Lys Tyr Gly Pro Pro Cys Pro Pro Cys Pro Ala Pro Glu Phe Leu Gly
        275                 280                 285

Gly Pro Ser Val Phe Leu Phe Pro Pro Lys Pro Lys Asp Thr Leu Met
    290                 295                 300

Ile Ser Arg Thr Pro Glu Val Thr Cys Val Val Val Asp Val Ser Gln
305                 310                 315                 320

Glu Asp Pro Glu Val Gln Phe Asn Trp Tyr Val Asp Gly Val Glu Val
                325                 330                 335

His Asn Ala Lys Thr Lys Pro Arg Glu Glu Gln Phe Asn Ser Thr Tyr
            340                 345                 350

Arg Val Val Ser Val Leu Thr Val Leu His Gln Asp Trp Leu Asn Gly
        355                 360                 365

Lys Glu Tyr Lys Cys Lys Val Ser Asn Lys Gly Leu Pro Ser Ser Ile
    370                 375                 380

Glu Lys Thr Ile Ser Lys Ala Lys Gly Gln Pro Arg Glu Pro Gln Val
385                 390                 395                 400

Tyr Thr Leu Pro Pro Ser Gln Glu Glu Met Thr Lys Asn Gln Val Ser
                405                 410                 415

Leu Thr Cys Leu Val Lys Gly Phe Tyr Pro Ser Asp Ile Ala Val Glu
            420                 425                 430

Trp Glu Ser Asn Gly Gln Pro Glu Asn Asn Tyr Lys Thr Thr Pro Pro
        435                 440                 445

Val Leu Asp Ser Asp Gly Ser Phe Phe Leu Tyr Ser Arg Leu Thr Val
    450                 455                 460

Asp Lys Ser Arg Trp Gln Glu Gly Asn Val Phe Ser Cys Ser Val Met
465                 470                 475                 480

His Glu Ala Leu His Asn His Tyr Thr Gln Lys Ser Leu Ser Leu Ser
                485                 490                 495

Leu Gly Lys Pro Phe Trp Val Leu Val Val Val Gly Gly Val Leu Ala
            500                 505                 510

Cys Tyr Ser Leu Leu Val Thr Val Ala Phe Ile Ile Phe Trp Val Arg
        515                 520                 525

Ser Lys Arg Ser Arg Leu Leu His Ser Asp Tyr Met Asn Met Thr Pro
    530                 535                 540

Arg Arg Pro Gly Pro Thr Arg Lys His Tyr Gln Pro Tyr Ala Pro Pro
545                 550                 555                 560

Arg Asp Phe Ala Ala Tyr Arg Ser
                565

<210>  58
<211>  569
<212>  PRT
<213>  Artificial Sequence

<220>
<223>  Amino acid sequence of 40DCR2 (including signal peptide)

<400>  58

Met Ala Leu Pro Val Thr Ala Leu Leu Leu Pro Leu Ala Leu Leu Leu
1               5                   10                  15

His Ala Ala Arg Pro Ser Gln Val Gln Leu Val Gln Ser Gly Ala Glu
            20                  25                  30

Val Lys Lys Pro Gly Ala Ser Val Lys Val Ser Cys Lys Ala Ser Gly
        35                  40                  45

Tyr Thr Phe Thr Gly Tyr Tyr Met His Trp Val Arg Gln Ala Pro Gly
    50                  55                  60

Gln Gly Leu Glu Trp Met Gly Trp Ile Asn Pro Asp Ser Gly Gly Thr
65                  70                  75                  80

Asn Tyr Ala Gln Lys Phe Gln Gly Arg Val Thr Met Thr Arg Asp Thr
                85                  90                  95

Ser Ile Ser Thr Ala Tyr Met Glu Leu Asn Arg Leu Arg Ser Asp Asp
            100                 105                 110

Thr Ala Val Tyr Tyr Cys Ala Arg Asp Gln Pro Leu Gly Tyr Cys Thr
        115                 120                 125

Asn Gly Val Cys Ser Tyr Phe Asp Tyr Trp Gly Gln Gly Thr Leu Val
    130                 135                 140

Thr Val Ser Ser Gly Gly Gly Gly Ser Gly Gly Gly Gly Ser Gly Gly

145                 150                 155                 160

Gly Gly Ser Asp Ile Gln Met Thr Gln Ser Pro Ser Ser Val Ser Ala
                165                 170                 175

Ser Val Gly Asp Arg Val Thr Ile Thr Cys Arg Ala Ser Gln Gly Ile
            180                 185                 190

Tyr Ser Trp Leu Ala Trp Tyr Gln Gln Lys Pro Gly Lys Ala Pro Asn
        195                 200                 205

Leu Leu Ile Tyr Thr Ala Ser Thr Leu Gln Ser Gly Val Pro Ser Arg
    210                 215                 220

Phe Ser Gly Ser Gly Ser Gly Thr Asp Phe Thr Leu Thr Ile Ser Ser
225                 230                 235                 240

Leu Gln Pro Glu Asp Phe Ala Thr Tyr Tyr Cys Gln Gln Ala Asn Ile
                245                 250                 255

Phe Pro Leu Thr Phe Gly Gly Gly Thr Lys Val Glu Ile Lys Glu Ser
            260                 265                 270

Lys Tyr Gly Pro Pro Cys Pro Pro Cys Pro Ala Pro Glu Phe Leu Gly
        275                 280                 285

Gly Pro Ser Val Phe Leu Phe Pro Pro Lys Pro Lys Asp Thr Leu Met
    290                 295                 300

Ile Ser Arg Thr Pro Glu Val Thr Cys Val Val Val Asp Val Ser Gln
305                 310                 315                 320

Glu Asp Pro Glu Val Gln Phe Asn Trp Tyr Val Asp Gly Val Glu Val
                325                 330                 335

His Asn Ala Lys Thr Lys Pro Arg Glu Glu Gln Phe Asn Ser Thr Tyr
            340                 345                 350

Arg Val Val Ser Val Leu Thr Val Leu His Gln Asp Trp Leu Asn Gly
        355                 360                 365

Lys Glu Tyr Lys Cys Lys Val Ser Asn Lys Gly Leu Pro Ser Ser Ile
    370                 375                 380

Glu Lys Thr Ile Ser Lys Ala Lys Gly Gln Pro Arg Glu Pro Gln Val
385                 390                 395                 400

Tyr Thr Leu Pro Pro Ser Gln Glu Glu Met Thr Lys Asn Gln Val Ser
                405                 410                 415

Leu Thr Cys Leu Val Lys Gly Phe Tyr Pro Ser Asp Ile Ala Val Glu
            420                 425                 430

Trp Glu Ser Asn Gly Gln Pro Glu Asn Asn Tyr Lys Thr Thr Pro Pro
        435                 440                 445

Val Leu Asp Ser Asp Gly Ser Phe Phe Leu Tyr Ser Arg Leu Thr Val
    450                 455                 460

Asp Lys Ser Arg Trp Gln Glu Gly Asn Val Phe Ser Cys Ser Val Met
465                 470                 475                 480

His Glu Ala Leu His Asn His Tyr Thr Gln Lys Ser Leu Ser Leu Ser
                485                 490                 495

Leu Gly Lys Pro Phe Trp Val Leu Val Val Val Gly Gly Val Leu Ala
            500                 505                 510

Cys Tyr Ser Leu Leu Val Thr Val Ala Phe Ile Ile Phe Trp Val Lys
        515                 520                 525

Arg Gly Arg Lys Lys Leu Leu Tyr Ile Phe Lys Gln Pro Phe Met Arg
    530                 535                 540

Pro Val Gln Thr Thr Gln Glu Glu Asp Gly Cys Ser Cys Arg Phe Pro
545                 550                 555                 560

Glu Glu Glu Glu Gly Gly Cys Glu Leu
                565

<210>  59
<211>  610
<212>  PRT
<213>  Artificial Sequence

<220>
<223>  Amino acid sequence of 40DCR3 (including signal peptide)

<400>  59

Met Ala Leu Pro Val Thr Ala Leu Leu Leu Pro Leu Ala Leu Leu Leu
1               5                   10                  15

His Ala Ala Arg Pro Ser Gln Val Gln Leu Val Gln Ser Gly Ala Glu
            20                  25                  30

Val Lys Lys Pro Gly Ala Ser Val Lys Val Ser Cys Lys Ala Ser Gly

35                  40                  45

Tyr Thr Phe Thr Gly Tyr Tyr Met His Trp Val Arg Gln Ala Pro Gly
    50                  55                  60

Gln Gly Leu Glu Trp Met Gly Trp Ile Asn Pro Asp Ser Gly Gly Thr
65                  70                  75                  80

Asn Tyr Ala Gln Lys Phe Gln Gly Arg Val Thr Met Thr Arg Asp Thr
                85                  90                  95

Ser Ile Ser Thr Ala Tyr Met Glu Leu Asn Arg Leu Arg Ser Asp Asp
            100                 105                 110

Thr Ala Val Tyr Tyr Cys Ala Arg Asp Gln Pro Leu Gly Tyr Cys Thr
        115                 120                 125

Asn Gly Val Cys Ser Tyr Phe Asp Tyr Trp Gly Gln Gly Thr Leu Val
    130                 135                 140

Thr Val Ser Ser Gly Gly Gly Gly Ser Gly Gly Gly Gly Ser Gly Gly
145                 150                 155                 160

Gly Gly Ser Asp Ile Gln Met Thr Gln Ser Pro Ser Ser Val Ser Ala
                165                 170                 175

Ser Val Gly Asp Arg Val Thr Ile Thr Cys Arg Ala Ser Gln Gly Ile
            180                 185                 190

Tyr Ser Trp Leu Ala Trp Tyr Gln Gln Lys Pro Gly Lys Ala Pro Asn
        195                 200                 205

Leu Leu Ile Tyr Thr Ala Ser Thr Leu Gln Ser Gly Val Pro Ser Arg
    210                 215                 220

Phe Ser Gly Ser Gly Ser Gly Thr Asp Phe Thr Leu Thr Ile Ser Ser
225                 230                 235                 240

Leu Gln Pro Glu Asp Phe Ala Thr Tyr Tyr Cys Gln Gln Ala Asn Ile
                245                 250                 255

Phe Pro Leu Thr Phe Gly Gly Gly Thr Lys Val Glu Ile Lys Glu Ser
            260                 265                 270

Lys Tyr Gly Pro Pro Cys Pro Pro Cys Pro Ala Pro Glu Phe Leu Gly
        275                 280                 285

Gly Pro Ser Val Phe Leu Phe Pro Pro Lys Pro Lys Asp Thr Leu Met
    290                 295                 300

Ile Ser Arg Thr Pro Glu Val Thr Cys Val Val Val Asp Val Ser Gln
305                 310                 315                 320

Glu Asp Pro Glu Val Gln Phe Asn Trp Tyr Val Asp Gly Val Glu Val
                325                 330                 335

His Asn Ala Lys Thr Lys Pro Arg Glu Glu Gln Phe Asn Ser Thr Tyr
            340                 345                 350

Arg Val Val Ser Val Leu Thr Val Leu His Gln Asp Trp Leu Asn Gly
        355                 360                 365

Lys Glu Tyr Lys Cys Lys Val Ser Asn Lys Gly Leu Pro Ser Ser Ile
    370                 375                 380

Glu Lys Thr Ile Ser Lys Ala Lys Gly Gln Pro Arg Glu Pro Gln Val
385                 390                 395                 400

Tyr Thr Leu Pro Pro Ser Gln Glu Glu Met Thr Lys Asn Gln Val Ser
                405                 410                 415

Leu Thr Cys Leu Val Lys Gly Phe Tyr Pro Ser Asp Ile Ala Val Glu
            420                 425                 430

Trp Glu Ser Asn Gly Gln Pro Glu Asn Asn Tyr Lys Thr Thr Pro Pro
        435                 440                 445

Val Leu Asp Ser Asp Gly Ser Phe Phe Leu Tyr Ser Arg Leu Thr Val
    450                 455                 460

Asp Lys Ser Arg Trp Gln Glu Gly Asn Val Phe Ser Cys Ser Val Met
465                 470                 475                 480

His Glu Ala Leu His Asn His Tyr Thr Gln Lys Ser Leu Ser Leu Ser
                485                 490                 495

Leu Gly Lys Pro Phe Trp Val Leu Val Val Val Gly Gly Val Leu Ala
            500                 505                 510

Cys Tyr Ser Leu Leu Val Thr Val Ala Phe Ile Ile Phe Trp Val Arg
        515                 520                 525

Ser Lys Arg Ser Arg Leu Leu His Ser Asp Tyr Met Asn Met Thr Pro
    530                 535                 540

Arg Arg Pro Gly Pro Thr Arg Lys His Tyr Gln Pro Tyr Ala Pro Pro
545                 550                 555                 560

Arg Asp Phe Ala Ala Tyr Arg Ser Lys Arg Gly Arg Lys Lys Leu Leu
                565                 570                 575

Tyr Ile Phe Lys Gln Pro Phe Met Arg Pro Val Gln Thr Thr Gln Glu
            580                 585                 590

Glu Asp Gly Cys Ser Cys Arg Phe Pro Glu Glu Glu Glu Gly Gly Cys
        595                 600                 605

Glu Leu
    610

<210>  60
<211>  610
<212>  PRT
<213>  Artificial Sequence

<220>
<223>  Amino acid sequence of 40DCR4 (including signal peptide)

<400>  60

Met Ala Leu Pro Val Thr Ala Leu Leu Leu Pro Leu Ala Leu Leu Leu
1               5                   10                  15

His Ala Ala Arg Pro Ser Gln Val Gln Leu Val Gln Ser Gly Ala Glu
            20                  25                  30

Val Lys Lys Pro Gly Ala Ser Val Lys Val Ser Cys Lys Ala Ser Gly
        35                  40                  45

Tyr Thr Phe Thr Gly Tyr Tyr Met His Trp Val Arg Gln Ala Pro Gly
    50                  55                  60

Gln Gly Leu Glu Trp Met Gly Trp Ile Asn Pro Asp Ser Gly Gly Thr
65                  70                  75                  80

Asn Tyr Ala Gln Lys Phe Gln Gly Arg Val Thr Met Thr Arg Asp Thr
                85                  90                  95

Ser Ile Ser Thr Ala Tyr Met Glu Leu Asn Arg Leu Arg Ser Asp Asp
            100                 105                 110

Thr Ala Val Tyr Tyr Cys Ala Arg Asp Gln Pro Leu Gly Tyr Cys Thr
        115                 120                 125

Asn Gly Val Cys Ser Tyr Phe Asp Tyr Trp Gly Gln Gly Thr Leu Val
    130                 135                 140

Thr Val Ser Ser Gly Gly Gly Gly Ser Gly Gly Gly Gly Ser Gly Gly
145                 150                 155                 160

Gly Gly Ser Asp Ile Gln Met Thr Gln Ser Pro Ser Ser Val Ser Ala
                165                 170                 175

Ser Val Gly Asp Arg Val Thr Ile Thr Cys Arg Ala Ser Gln Gly Ile
            180                 185                 190

Tyr Ser Trp Leu Ala Trp Tyr Gln Gln Lys Pro Gly Lys Ala Pro Asn
        195                 200                 205

Leu Leu Ile Tyr Thr Ala Ser Thr Leu Gln Ser Gly Val Pro Ser Arg
    210                 215                 220

Phe Ser Gly Ser Gly Ser Gly Thr Asp Phe Thr Leu Thr Ile Ser Ser
225                 230                 235                 240

Leu Gln Pro Glu Asp Phe Ala Thr Tyr Tyr Cys Gln Gln Ala Asn Ile
                245                 250                 255

Phe Pro Leu Thr Phe Gly Gly Gly Thr Lys Val Glu Ile Lys Glu Ser
            260                 265                 270

Lys Tyr Gly Pro Pro Cys Pro Pro Cys Pro Ala Pro Glu Phe Leu Gly
        275                 280                 285

Gly Pro Ser Val Phe Leu Phe Pro Pro Lys Pro Lys Asp Thr Leu Met
    290                 295                 300

Ile Ser Arg Thr Pro Glu Val Thr Cys Val Val Val Asp Val Ser Gln
305                 310                 315                 320

Glu Asp Pro Glu Val Gln Phe Asn Trp Tyr Val Asp Gly Val Glu Val
                325                 330                 335

His Asn Ala Lys Thr Lys Pro Arg Glu Glu Gln Phe Asn Ser Thr Tyr
            340                 345                 350

Arg Val Val Ser Val Leu Thr Val Leu His Gln Asp Trp Leu Asn Gly
        355                 360                 365

Lys Glu Tyr Lys Cys Lys Val Ser Asn Lys Gly Leu Pro Ser Ser Ile
    370                 375                 380

Glu Lys Thr Ile Ser Lys Ala Lys Gly Gln Pro Arg Glu Pro Gln Val
385                 390                 395                 400

Tyr Thr Leu Pro Pro Ser Gln Glu Glu Met Thr Lys Asn Gln Val Ser
                405                 410                 415

Leu Thr Cys Leu Val Lys Gly Phe Tyr Pro Ser Asp Ile Ala Val Glu
            420                 425                 430

Trp Glu Ser Asn Gly Gln Pro Glu Asn Asn Tyr Lys Thr Thr Pro Pro
        435                 440                 445

Val Leu Asp Ser Asp Gly Ser Phe Phe Leu Tyr Ser Arg Leu Thr Val
    450                 455                 460

Asp Lys Ser Arg Trp Gln Glu Gly Asn Val Phe Ser Cys Ser Val Met
465                 470                 475                 480

His Glu Ala Leu His Asn His Tyr Thr Gln Lys Ser Leu Ser Leu Ser
                485                 490                 495

Leu Gly Lys Pro Phe Trp Val Leu Val Val Val Gly Gly Val Leu Ala
            500                 505                 510

Cys Tyr Ser Leu Leu Val Thr Val Ala Phe Ile Ile Phe Trp Val Lys
        515                 520                 525

Arg Gly Arg Lys Lys Leu Leu Tyr Ile Phe Lys Gln Pro Phe Met Arg
    530                 535                 540

Pro Val Gln Thr Thr Gln Glu Glu Asp Gly Cys Ser Cys Arg Phe Pro
545                 550                 555                 560

Glu Glu Glu Glu Gly Gly Cys Glu Leu Arg Ser Lys Arg Ser Arg Leu
                565                 570                 575

Leu His Ser Asp Tyr Met Asn Met Thr Pro Arg Arg Pro Gly Pro Thr
            580                 585                 590

Arg Lys His Tyr Gln Pro Tyr Ala Pro Pro Arg Asp Phe Ala Ala Tyr
        595                 600                 605

Arg Ser
    610

<210>  61
<211>  546

<212>  PRT
<213>  Artificial Sequence

<220>
<223>  Amino acid sequence of 40DCR1 (without signal peptide)

<400>  61

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asp Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Asn Arg Leu Arg Ser Asp Asp Thr Ala Val Tyr Tyr Cys
                85                  90                  95

Ala Arg Asp Gln Pro Leu Gly Tyr Cys Thr Asn Gly Val Cys Ser Tyr
            100                 105                 110

Phe Asp Tyr Trp Gly Gln Gly Thr Leu Val Thr Val Ser Ser Gly Gly
        115                 120                 125

Gly Gly Ser Gly Gly Gly Gly Ser Gly Gly Gly Gly Ser Asp Ile Gln
    130                 135                 140

Met Thr Gln Ser Pro Ser Ser Val Ser Ala Ser Val Gly Asp Arg Val
145                 150                 155                 160

Thr Ile Thr Cys Arg Ala Ser Gln Gly Ile Tyr Ser Trp Leu Ala Trp
                165                 170                 175

Tyr Gln Gln Lys Pro Gly Lys Ala Pro Asn Leu Leu Ile Tyr Thr Ala
            180                 185                 190

Ser Thr Leu Gln Ser Gly Val Pro Ser Arg Phe Ser Gly Ser Gly Ser
        195                 200                 205

Gly Thr Asp Phe Thr Leu Thr Ile Ser Ser Leu Gln Pro Glu Asp Phe
    210                 215                 220

Ala Thr Tyr Tyr Cys Gln Gln Ala Asn Ile Phe Pro Leu Thr Phe Gly
225                 230                 235                 240

Gly Gly Thr Lys Val Glu Ile Lys Glu Ser Lys Tyr Gly Pro Pro Cys
                245                 250                 255

Pro Pro Cys Pro Ala Pro Glu Phe Leu Gly Gly Pro Ser Val Phe Leu
            260                 265                 270

Phe Pro Pro Lys Pro Lys Asp Thr Leu Met Ile Ser Arg Thr Pro Glu
        275                 280                 285

Val Thr Cys Val Val Val Asp Val Ser Gln Glu Asp Pro Glu Val Gln
    290                 295                 300

Phe Asn Trp Tyr Val Asp Gly Val Glu Val His Asn Ala Lys Thr Lys
305                 310                 315                 320

Pro Arg Glu Glu Gln Phe Asn Ser Thr Tyr Arg Val Val Ser Val Leu
                325                 330                 335

Thr Val Leu His Gln Asp Trp Leu Asn Gly Lys Glu Tyr Lys Cys Lys
            340                 345                 350

Val Ser Asn Lys Gly Leu Pro Ser Ser Ile Glu Lys Thr Ile Ser Lys
        355                 360                 365

Ala Lys Gly Gln Pro Arg Glu Pro Gln Val Tyr Thr Leu Pro Pro Ser
    370                 375                 380

Gln Glu Glu Met Thr Lys Asn Gln Val Ser Leu Thr Cys Leu Val Lys
385                 390                 395                 400

Gly Phe Tyr Pro Ser Asp Ile Ala Val Glu Trp Glu Ser Asn Gly Gln
                405                 410                 415

Pro Glu Asn Asn Tyr Lys Thr Thr Pro Pro Val Leu Asp Ser Asp Gly
            420                 425                 430

Ser Phe Phe Leu Tyr Ser Arg Leu Thr Val Asp Lys Ser Arg Trp Gln
        435                 440                 445

Glu Gly Asn Val Phe Ser Cys Ser Val Met His Glu Ala Leu His Asn
    450                 455                 460

His Tyr Thr Gln Lys Ser Leu Ser Leu Ser Leu Gly Lys Pro Phe Trp
465                 470                 475                 480

Val Leu Val Val Val Gly Gly Val Leu Ala Cys Tyr Ser Leu Leu Val
                485                 490                 495

Thr Val Ala Phe Ile Ile Phe Trp Val Arg Ser Lys Arg Ser Arg Leu
            500                 505                 510

Leu His Ser Asp Tyr Met Asn Met Thr Pro Arg Arg Pro Gly Pro Thr
        515                 520                 525

Arg Lys His Tyr Gln Pro Tyr Ala Pro Pro Arg Asp Phe Ala Ala Tyr
    530                 535                 540

Arg Ser
545

<210>  62
<211>  547
<212>  PRT
<213>  Artificial Sequence

<220>
<223>  Amino acid sequence of 40DCR2 (without signal peptide)

<400>  62
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Gly Tyr
               20                  25                  30
   Tyr Met His Trp Val Arg Gln Ala Pro Gly Gln Gly Leu Glu Trp Met
           35                  40                  45
   Gly Trp Ile Asn Pro Asp Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Asn Arg Leu Arg Ser Asp Asp Thr Ala Val Tyr Tyr Cys
                   85                  90                  95
   Ala Arg Asp Gln Pro Leu Gly Tyr Cys Thr Asn Gly Val Cys Ser Tyr
               100                 105                 110
   Phe Asp Tyr Trp Gly Gln Gly Thr Leu Val Thr Val Ser Ser Gly Gly
           115                 120                 125
   Gly Gly Ser Gly Gly Gly Gly Ser Gly Gly Gly Gly Ser Asp Ile Gln
       130                 135                 140
   Met Thr Gln Ser Pro Ser Ser Val Ser Ala Ser Val Gly Asp Arg Val
   145                 150                 155                 160
   Thr Ile Thr Cys Arg Ala Ser Gln Gly Ile Tyr Ser Trp Leu Ala Trp
                   165                 170                 175
   Tyr Gln Gln Lys Pro Gly Lys Ala Pro Asn Leu Leu Ile Tyr Thr Ala
               180                 185                 190
   Ser Thr Leu Gln Ser Gly Val Pro Ser Arg Phe Ser Gly Ser Gly Ser
           195                 200                 205
   Gly Thr Asp Phe Thr Leu Thr Ile Ser Ser Leu Gln Pro Glu Asp Phe
       210                 215                 220
   Ala Thr Tyr Tyr Cys Gln Gln Ala Asn Ile Phe Pro Leu Thr Phe Gly
   225                 230                 235                 240
   Gly Gly Thr Lys Val Glu Ile Lys Glu Ser Lys Tyr Gly Pro Pro Cys
                   245                 250                 255
   Pro Pro Cys Pro Ala Pro Glu Phe Leu Gly Gly Pro Ser Val Phe Leu
               260                 265                 270
   Phe Pro Pro Lys Pro Lys Asp Thr Leu Met Ile Ser Arg Thr Pro Glu
           275                 280                 285
   Val Thr Cys Val Val Val Asp Val Ser Gln Glu Asp Pro Glu Val Gln
       290                 295                 300
   Phe Asn Trp Tyr Val Asp Gly Val Glu Val His Asn Ala Lys Thr Lys
   305                 310                 315                 320
   Pro Arg Glu Glu Gln Phe Asn Ser Thr Tyr Arg Val Val Ser Val Leu
                   325                 330                 335
   Thr Val Leu His Gln Asp Trp Leu Asn Gly Lys Glu Tyr Lys Cys Lys
               340                 345                 350
   Val Ser Asn Lys Gly Leu Pro Ser Ser Ile Glu Lys Thr Ile Ser Lys
           355                 360                 365
   Ala Lys Gly Gln Pro Arg Glu Pro Gln Val Tyr Thr Leu Pro Pro Ser
       370                 375                 380
   Gln Glu Glu Met Thr Lys Asn Gln Val Ser Leu Thr Cys Leu Val Lys
   385                 390                 395                 400
   Gly Phe Tyr Pro Ser Asp Ile Ala Val Glu Trp Glu Ser Asn Gly Gln
                   405                 410                 415
   Pro Glu Asn Asn Tyr Lys Thr Thr Pro Pro Val Leu Asp Ser Asp Gly
               420                 425                 430
   Ser Phe Phe Leu Tyr Ser Arg Leu Thr Val Asp Lys Ser Arg Trp Gln
           435                 440                 445
   Glu Gly Asn Val Phe Ser Cys Ser Val Met His Glu Ala Leu His Asn
       450                 455                 460
   His Tyr Thr Gln Lys Ser Leu Ser Leu Ser Leu Gly Lys Pro Phe Trp
   465                 470                 475                 480
   Val Leu Val Val Val Gly Gly Val Leu Ala Cys Tyr Ser Leu Leu Val
                   485                 490                 495
   Thr Val Ala Phe Ile Ile Phe Trp Val Lys Arg Gly Arg Lys Lys Leu
               500                 505                 510
   Leu Tyr Ile Phe Lys Gln Pro Phe Met Arg Pro Val Gln Thr Thr Gln
           515                 520                 525
   Glu Glu Asp Gly Cys Ser Cys Arg Phe Pro Glu Glu Glu Glu Gly Gly
       530                 535                 540
   Cys Glu Leu
   545

<210>  63
<211>  588
<212>  PRT
<213>  Artificial Sequence

<220>
<223>  Amino acid sequence of 40DCR3 (without signal peptide)

<400>  63

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asp Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Asn Arg Leu Arg Ser Asp Asp Thr Ala Val Tyr Tyr Cys
                85                  90                  95

Ala Arg Asp Gln Pro Leu Gly Tyr Cys Thr Asn Gly Val Cys Ser Tyr
            100                 105                 110

Phe Asp Tyr Trp Gly Gln Gly Thr Leu Val Thr Val Ser Ser Gly Gly
        115                 120                 125

Gly Gly Ser Gly Gly Gly Gly Ser Gly Gly Gly Gly Ser Asp Ile Gln
    130                 135                 140

Met Thr Gln Ser Pro Ser Ser Val Ser Ala Ser Val Gly Asp Arg Val
145                 150                 155                 160

Thr Ile Thr Cys Arg Ala Ser Gln Gly Ile Tyr Ser Trp Leu Ala Trp
                165                 170                 175

Tyr Gln Gln Lys Pro Gly Lys Ala Pro Asn Leu Leu Ile Tyr Thr Ala
            180                 185                 190

Ser Thr Leu Gln Ser Gly Val Pro Ser Arg Phe Ser Gly Ser Gly Ser
        195                 200                 205

Gly Thr Asp Phe Thr Leu Thr Ile Ser Ser Leu Gln Pro Glu Asp Phe
    210                 215                 220

Ala Thr Tyr Tyr Cys Gln Gln Ala Asn Ile Phe Pro Leu Thr Phe Gly
225                 230                 235                 240

Gly Gly Thr Lys Val Glu Ile Lys Glu Ser Lys Tyr Gly Pro Pro Cys
                245                 250                 255

Pro Pro Cys Pro Ala Pro Glu Phe Leu Gly Gly Pro Ser Val Phe Leu
            260                 265                 270

Phe Pro Pro Lys Pro Lys Asp Thr Leu Met Ile Ser Arg Thr Pro Glu
        275                 280                 285

Val Thr Cys Val Val Val Asp Val Ser Gln Glu Asp Pro Glu Val Gln
    290                 295                 300

Phe Asn Trp Tyr Val Asp Gly Val Glu Val His Asn Ala Lys Thr Lys
305                 310                 315                 320

Pro Arg Glu Glu Gln Phe Asn Ser Thr Tyr Arg Val Val Ser Val Leu
                325                 330                 335

Thr Val Leu His Gln Asp Trp Leu Asn Gly Lys Glu Tyr Lys Cys Lys
            340                 345                 350

Val Ser Asn Lys Gly Leu Pro Ser Ser Ile Glu Lys Thr Ile Ser Lys
        355                 360                 365

Ala Lys Gly Gln Pro Arg Glu Pro Gln Val Tyr Thr Leu Pro Pro Ser
    370                 375                 380

Gln Glu Glu Met Thr Lys Asn Gln Val Ser Leu Thr Cys Leu Val Lys
385                 390                 395                 400

Gly Phe Tyr Pro Ser Asp Ile Ala Val Glu Trp Glu Ser Asn Gly Gln
                405                 410                 415

Pro Glu Asn Asn Tyr Lys Thr Thr Pro Pro Val Leu Asp Ser Asp Gly
            420                 425                 430

Ser Phe Phe Leu Tyr Ser Arg Leu Thr Val Asp Lys Ser Arg Trp Gln
        435                 440                 445

Glu Gly Asn Val Phe Ser Cys Ser Val Met His Glu Ala Leu His Asn
    450                 455                 460

His Tyr Thr Gln Lys Ser Leu Ser Leu Ser Leu Gly Lys Pro Phe Trp
465                 470                 475                 480

Val Leu Val Val Val Gly Gly Val Leu Ala Cys Tyr Ser Leu Leu Val
                485                 490                 495

Thr Val Ala Phe Ile Ile Phe Trp Val Arg Ser Lys Arg Ser Arg Leu
            500                 505                 510

Leu His Ser Asp Tyr Met Asn Met Thr Pro Arg Arg Pro Gly Pro Thr
        515                 520                 525

Arg Lys His Tyr Gln Pro Tyr Ala Pro Pro Arg Asp Phe Ala Ala Tyr

530                 535                 540

Arg Ser Lys Arg Gly Arg Lys Lys Leu Leu Tyr Ile Phe Lys Gln Pro
545                 550                 555                 560

Phe Met Arg Pro Val Gln Thr Thr Gln Glu Glu Asp Gly Cys Ser Cys
                565                 570                 575

Arg Phe Pro Glu Glu Glu Glu Gly Gly Cys Glu Leu
            580                 585

<210>  64
<211>  588
<212>  PRT
<213>  Artificial Sequence

<220>
<223>  Amino acid sequence of 40DCR4 (without signal peptide)

<400>  64

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asp Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Asn Arg Leu Arg Ser Asp Asp Thr Ala Val Tyr Tyr Cys
                85                  90                  95

Ala Arg Asp Gln Pro Leu Gly Tyr Cys Thr Asn Gly Val Cys Ser Tyr
            100                 105                 110

Phe Asp Tyr Trp Gly Gln Gly Thr Leu Val Thr Val Ser Ser Gly Gly
        115                 120                 125

Gly Gly Ser Gly Gly Gly Gly Ser Gly Gly Gly Gly Ser Asp Ile Gln
    130                 135                 140

Met Thr Gln Ser Pro Ser Ser Val Ser Ala Ser Val Gly Asp Arg Val
145                 150                 155                 160

Thr Ile Thr Cys Arg Ala Ser Gln Gly Ile Tyr Ser Trp Leu Ala Trp
                165                 170                 175

Tyr Gln Gln Lys Pro Gly Lys Ala Pro Asn Leu Leu Ile Tyr Thr Ala
            180                 185                 190

Ser Thr Leu Gln Ser Gly Val Pro Ser Arg Phe Ser Gly Ser Gly Ser
        195                 200                 205

Gly Thr Asp Phe Thr Leu Thr Ile Ser Ser Leu Gln Pro Glu Asp Phe
    210                 215                 220

Ala Thr Tyr Tyr Cys Gln Gln Ala Asn Ile Phe Pro Leu Thr Phe Gly
225                 230                 235                 240

Gly Gly Thr Lys Val Glu Ile Lys Glu Ser Lys Tyr Gly Pro Pro Cys
                245                 250                 255

Pro Pro Cys Pro Ala Pro Glu Phe Leu Gly Gly Pro Ser Val Phe Leu
            260                 265                 270

Phe Pro Pro Lys Pro Lys Asp Thr Leu Met Ile Ser Arg Thr Pro Glu
        275                 280                 285

Val Thr Cys Val Val Val Asp Val Ser Gln Glu Asp Pro Glu Val Gln
    290                 295                 300

Phe Asn Trp Tyr Val Asp Gly Val Glu Val His Asn Ala Lys Thr Lys
305                 310                 315                 320

Pro Arg Glu Glu Gln Phe Asn Ser Thr Tyr Arg Val Val Ser Val Leu
                325                 330                 335

Thr Val Leu His Gln Asp Trp Leu Asn Gly Lys Glu Tyr Lys Cys Lys
            340                 345                 350

Val Ser Asn Lys Gly Leu Pro Ser Ser Ile Glu Lys Thr Ile Ser Lys
        355                 360                 365

Ala Lys Gly Gln Pro Arg Glu Pro Gln Val Tyr Thr Leu Pro Pro Ser
    370                 375                 380

Gln Glu Glu Met Thr Lys Asn Gln Val Ser Leu Thr Cys Leu Val Lys
385                 390                 395                 400

Gly Phe Tyr Pro Ser Asp Ile Ala Val Glu Trp Glu Ser Asn Gly Gln

405                 410                 415

Pro Glu Asn Asn Tyr Lys Thr Thr Pro Pro Val Leu Asp Ser Asp Gly
            420                 425                 430

Ser Phe Phe Leu Tyr Ser Arg Leu Thr Val Asp Lys Ser Arg Trp Gln
        435                 440                 445

Glu Gly Asn Val Phe Ser Cys Ser Val Met His Glu Ala Leu His Asn
    450                 455                 460

His Tyr Thr Gln Lys Ser Leu Ser Leu Ser Leu Gly Lys Pro Phe Trp
465                 470                 475                 480

Val Leu Val Val Val Gly Gly Val Leu Ala Cys Tyr Ser Leu Leu Val
                485                 490                 495

Thr Val Ala Phe Ile Ile Phe Trp Val Lys Arg Gly Arg Lys Lys Leu
            500                 505                 510

Leu Tyr Ile Phe Lys Gln Pro Phe Met Arg Pro Val Gln Thr Thr Gln
        515                 520                 525

Glu Glu Asp Gly Cys Ser Cys Arg Phe Pro Glu Glu Glu Glu Gly Gly
    530                 535                 540

Cys Glu Leu Arg Ser Lys Arg Ser Arg Leu Leu His Ser Asp Tyr Met
545                 550                 555                 560

Asn Met Thr Pro Arg Arg Pro Gly Pro Thr Arg Lys His Tyr Gln Pro
                565                 570                 575

Tyr Ala Pro Pro Arg Asp Phe Ala Ala Tyr Arg Ser
            580                 585

<210>  65
<211>  1704
<212>  DNA
<213>  Artificial Sequence

<220>
<223>  Nucleic acid sequence of 40DCR1 (including signal peptide)

<400>  65
atggccttac cagtgaccgc cttgctcctg ccgctggcct tgctgctcca cgccgccagg      60

ccgagccagg tgcagctggt gcagtctggg gctgaggtga agaagcctgg ggcctcagtg     120

aaggtctcct gcaaggcttc tggatacacc ttcaccggct actatatgca ctgggtgcga     180

caggcccctg gacaagggct tgagtggatg ggatggatca accctgacag tggtggcaca     240

aactatgcac agaagtttca gggcagggtc accatgacca gggacacgtc catcagcaca 300

gcctacatgg agctgaacag gctgagatct gacgacacgg ccgtgtatta ctgtgcgaga 360

gatcagcccc taggatattg tactaatggt gtatgctcct actttgacta ctggggccag 420

ggaaccctgg tcaccgtctc ctcaggtgga ggcggttcag gcggaggtgg cagcggcggt 480

ggcgggtcgg acatccagat gacccagtct ccatcttccg tgtctgcatc tgtaggagac 540

agagtcacca tcacttgtcg ggcgagtcag ggtatttaca gctggttagc ctggtatcag 600

cagaaaccag ggaaagcccc taacctcctg atctatactg catccacttt acaaagtggg 660

gtcccatcaa ggttcagcgg cagtggatct gggacagatt tcactctcac catcagcagc 720

ctgcaacctg aagattttgc aacttactat tgtcaacagg ctaacatttt cccgctcact 780

ttcggcggag ggaccaaggt ggagatcaaa gagtccaaat atggtccccc atgcccacca 840

tgcccagcac ctgagttcct ggggggacca tcagtcttcc tgttcccccc aaaacccaag 900

gacactctca tgatctcccg gacccctgag gtcacgtgcg tggtggtgga cgtgagccag 960

gaagaccccg aggtccagtt caactggtac gtggatggcg tggaggtgca taatgccaag 1020

acaaagccgc gggaggagca gttcaacagc acgtaccgtg tggtcagcgt cctcaccgtc 1080

ctgcaccagg actggctgaa cggcaaggag tacaagtgca aggtctccaa caaaggcctc 1140

ccgtcctcca tcgagaaaac catctccaaa gccaaagggc agccccgaga gccacaggtg 1200

tacaccctgc ccccatccca ggaggagatg accaagaacc aggtcagcct gacctgcctg 1260

gtcaaaggct tctaccccag cgacatcgcc gtggagtggg agagcaatgg gcagccggag 1320

aacaactaca agaccacgcc tcccgtgctg gactccgacg gctccttctt cctctacagc 1380

aggctaaccg tggacaagag caggtggcag gaggggaatg tcttctcatg ctccgtgatg 1440

catgaggctc tgcacaacca ctacacacag aagagcctct ccctgtctct gggtaaaccc 1500

ttttgggtgc tggtggtggt tggtggagtc ctggcttgct atagcttgct agtaacagtg 1560

gcctttatta ttttctgggt gaggagtaag aggagcaggc tcctgcacag tgactacatg 1620

aacatgactc cccgccgccc cgggcccacc cgcaagcatt accagcccta tgccccacca 1680

cgcgacttcg cagcctatcg ctcc 1704

<210> 66
<211> 1704
<212> DNA
<213> Artificial Sequence

<220>
<223> Nucleic acid sequence of 40DCR2 (including signal peptide)

<400> 66
atggccttac cagtgaccgc cttgctcctg ccgctggcct tgctgctcca cgccgccagg 60

ccgagccagg tgcagctggt gcagtctggg gctgaggtga agaagcctgg ggcctcagtg 120

aaggtctcct gcaaggcttc tggatacacc ttcaccggct actatatgca ctgggtgcga 180

caggcccctg gacaagggct tgagtggatg ggatggatca accctgacag tggtggcaca 240

aactatgcac agaagtttca gggcagggtc accatgacca gggacacgtc catcagcaca 300

gcctacatgg agctgaacag gctgagatct gacgacacgg ccgtgtatta ctgtgcgaga 360

gatcagcccc taggatattg tactaatggt gtatgctcct actttgacta ctggggccag 420

ggaaccctgg tcaccgtctc ctcaggtgga ggcggttcag gcggaggtgg cagcggcggt 480

ggcgggtcgg acatccagat gacccagtct ccatcttccg tgtctgcatc tgtaggagac 540

agagtcacca tcacttgtcg ggcgagtcag ggtatttaca gctggttagc ctggtatcag 600

cagaaaccag ggaaagcccc taacctcctg atctatactg catccacttt acaaagtggg 660

gtcccatcaa ggttcagcgg cagtggatct gggacagatt tcactctcac catcagcagc 720

ctgcaacctg aagattttgc aacttactat tgtcaacagg ctaacatttt cccgctcact 780

ttcggcggag ggaccaaggt ggagatcaaa gagtccaaat atggtccccc atgcccacca 840

tgcccagcac ctgagttcct ggggggacca tcagtcttcc tgttcccccc aaaacccaag 900

gacactctca tgatctcccg gacccctgag gtcacgtgcg tggtggtgga cgtgagccag 960

gaagaccccg aggtccagtt caactggtac gtggatggcg tggaggtgca taatgccaag 1020

acaaagccgc gggaggagca gttcaacagc acgtaccgtg tggtcagcgt cctcaccgtc 1080

ctgcaccagg actggctgaa cggcaaggag tacaagtgca aggtctccaa caaaggcctc 1140

ccgtcctcca tcgagaaaac catctccaaa gccaaagggc agccccgaga gccacaggtg 1200

tacaccctgc ccccatccca ggaggagatg accaagaacc aggtcagcct gacctgcctg 1260

gtcaaaggct tctaccccag cgacatcgcc gtggagtggg agagcaatgg gcagccggag 1320

aacaactaca agaccacgcc tcccgtgctg gactccgacg gctccttctt cctctacagc 1380

aggctaaccg tggacaagag caggtggcag gaggggaatg tcttctcatg ctccgtgatg 1440

catgaggctc tgcacaacca ctacacacag aagagcctct ccctgtctct gggtaaaccc 1500

ttttgggtgc tggtggtggt tggtggagtc ctggcttgct atagcttgct agtaacagtg 1560

gcctttatta ttttctgggt gaggagtaag aggagcaggc tcctgcacag tgactacatg 1620

aacatgactc cccgccgccc cgggcccacc cgcaagcatt accagcccta tgccccacca 1680

cgcgacttcg cagcctatcg ctcc 1704

<210> 67
<211> 1830
<212> DNA
<213> Artificial Sequence

<220>

<223>  Nucleic acid sequence of 40DCR3 (including signal peptide)

<400>  67
atggccttac cagtgaccgc cttgctcctg ccgctggcct tgctgctcca cgccgccagg      60

ccgagccagg tgcagctggt gcagtctggg gctgaggtga agaagcctgg ggcctcagtg     120

aaggtctcct gcaaggcttc tggatacacc ttcaccggct actatatgca ctgggtgcga     180

caggcccctg gacaagggct tgagtggatg ggatggatca accctgacag tggtggcaca     240

aactatgcac agaagtttca gggcagggtc accatgacca gggacacgtc catcagcaca     300

gcctacatgg agctgaacag gctgagatct gacgacacgg ccgtgtatta ctgtgcgaga     360

gatcagcccc taggatattg tactaatggt gtatgctcct actttgacta ctggggccag     420

ggaaccctgg tcaccgtctc ctcaggtgga ggcggttcag gcggaggtgg cagcggcggt     480

ggcgggtcgg acatccagat gacccagtct ccatcttccg tgtctgcatc tgtaggagac     540

agagtcacca tcacttgtcg ggcgagtcag ggtatttaca gctggttagc ctggtatcag     600

cagaaaccag ggaaagcccc taacctcctg atctatactg catccacttt acaaagtggg     660

gtcccatcaa ggttcagcgg cagtggatct gggacagatt tcactctcac catcagcagc     720

ctgcaacctg aagattttgc aacttactat tgtcaacagg ctaacatttt cccgctcact     780

ttcggcggag ggaccaaggt ggagatcaaa gagtccaaat atggtccccc atgcccacca     840

tgcccagcac ctgagttcct ggggggacca tcagtcttcc tgttcccccc aaaacccaag     900

gacactctca tgatctcccg gacccctgag gtcacgtgcg tggtggtgga cgtgagccag     960

gaagaccccg aggtccagtt caactggtac gtggatggcg tggaggtgca taatgccaag    1020

acaaagccgc gggaggagca gttcaacagc acgtaccgtg tggtcagcgt cctcaccgtc    1080

ctgcaccagg actggctgaa cggcaaggag tacaagtgca aggtctccaa caaaggcctc    1140

ccgtcctcca tcgagaaaac catctccaaa gccaaagggc agccccgaga gccacaggtg    1200

tacaccctgc ccccatccca ggaggagatg accaagaacc aggtcagcct gacctgcctg    1260

gtcaaaggct tctaccccag cgacatcgcc gtggagtggg agagcaatgg gcagccggag    1320

aacaactaca agaccacgcc tcccgtgctg gactccgacg gctccttctt cctctacagc    1380

aggctaaccg tggacaagag caggtggcag gaggggaatg tcttctcatg ctccgtgatg    1440

catgaggctc tgcacaacca ctacacacag aagagcctct ccctgtctct gggtaaaccc    1500

ttttgggtgc tggtggtggt tggtggagtc ctggcttgct atagcttgct agtaacagtg    1560

gcctttatta ttttctgggt gaggagtaag aggagcaggc tcctgcacag tgactacatg    1620

aacatgactc cccgccgccc cgggcccacc cgcaagcatt accagcccta tgccccacca    1680

cgcgacttcg cagcctatcg ctccaaacgg ggcagaaaga agctcctgta tatattcaaa    1740

caaccattta tgagaccagt acaaactact caagaggaag atggctgtag ctgccgattt    1800

ccagaagaag aagaaggagg atgtgaactg 1830

<210> 68
<211> 1830
<212> DNA
<213> Artificial Sequence

<220>
<223> Nucleic acid sequence of 40DCR4 (including signal peptide)

<400> 68
atggccttac cagtgaccgc cttgctcctg ccgctggcct tgctgctcca cgccgccagg 60

ccgagccagg tgcagctggt gcagtctggg gctgaggtga agaagcctgg ggcctcagtg 120

aaggtctcct gcaaggcttc tggatacacc ttcaccggct actatatgca ctgggtgcga 180

caggcccctg gacaagggct tgagtggatg ggatggatca accctgacag tggtggcaca 240

aactatgcac agaagtttca gggcagggtc accatgacca gggacacgtc catcagcaca 300

gcctacatgg agctgaacag gctgagatct gacgacacgg ccgtgtatta ctgtgcgaga 360

gatcagcccc taggatattg tactaatggt gtatgctcct actttgacta ctggggccag 420

ggaaccctgg tcaccgtctc ctcaggtgga ggcggttcag gcggaggtgg cagcggcggt 480

ggcgggtcgg acatccagat gacccagtct ccatcttccg tgtctgcatc tgtaggagac 540

agagtcacca tcacttgtcg ggcgagtcag ggtatttaca gctggttagc ctggtatcag 600

cagaaaccag ggaaagcccc taacctcctg atctatactg catccacttt acaaagtggg 660

gtcccatcaa ggttcagcgg cagtggatct gggacagatt tcactctcac catcagcagc 720

ctgcaacctg aagattttgc aacttactat tgtcaacagg ctaacatttt cccgctcact 780

ttcggcggag ggaccaaggt ggagatcaaa gagtccaaat atggtccccc atgcccacca 840

tgcccagcac ctgagttcct ggggggacca tcagtcttcc tgttcccccc aaaacccaag 900

gacactctca tgatctcccg gacccctgag gtcacgtgcg tggtggtgga cgtgagccag 960

gaagaccccg aggtccagtt caactggtac gtggatggcg tggaggtgca taatgccaag 1020

acaaagccgc gggaggagca gttcaacagc acgtaccgtg tggtcagcgt cctcaccgtc 1080

ctgcaccagg actggctgaa cggcaaggag tacaagtgca aggtctccaa caaaggcctc 1140

ccgtcctcca tcgagaaaac catctccaaa gccaaagggc agccccgaga gccacaggtg 1200

tacaccctgc ccccatccca ggaggagatg accaagaacc aggtcagcct gacctgcctg 1260

gtcaaaggct tctaccccag cgacatcgcc gtggagtggg agagcaatgg gcagccggag 1320

aacaactaca agaccacgcc tcccgtgctg gactccgacg gctccttctt cctctacagc 1380

aggctaaccg tggacaagag caggtggcag gaggggaatg tcttctcatg ctccgtgatg 1440

catgaggctc tgcacaacca ctacacacag aagagcctct ccctgtctct gggtaaaccc 1500

ttttgggtgc tggtggtggt tggtggagtc ctggcttgct atagcttgct agtaacagtg 1560

gcctttatta ttttctgggt gaaacggggc agaaagaagc tcctgtatat attcaaacaa 1620

ccatttatga gaccagtaca aactactcaa gaggaagatg gctgtagctg ccgatttcca 1680

gaagaagaag aaggaggatg tgaactgagg agtaagagga gcaggctcct gcacagtgac 1740

tacatgaaca tgactccccg ccgccccggg cccacccgca agcattacca gccctatgcc 1800

ccaccacgcg acttcgcagc ctatcgctcc 1830

<210> 69
<211> 1638
<212> DNA
<213> Artificial Sequence

<220>
<223> Nucleic acid sequence of 40DCR1 (without signal peptide)

<400> 69
caggtgcagc tggtgcagtc tggggctgag gtgaagaagc ctggggcctc agtgaaggtc 60

tcctgcaagg cttctggata caccttcacc ggctactata tgcactgggt gcgacaggcc 120

cctggacaag ggcttgagtg gatgggatgg atcaaccctg acagtggtgg cacaaactat 180

gcacagaagt ttcagggcag ggtcaccatg accagggaca cgtccatcag cacagcctac 240

atggagctga acaggctgag atctgacgac acggccgtgt attactgtgc gagagatcag 300

cccctaggat attgtactaa tggtgtatgc tcctactttg actactgggg ccagggaacc 360

ctggtcaccg tctcctcagg tggaggcggt tcaggcggag gtggcagcgg cggtggcggg 420

tcggacatcc agatgaccca gtctccatct tccgtgtctg catctgtagg agacagagtc 480

accatcactt gtcgggcgag tcagggtatt tacagctggt tagcctggta tcagcagaaa 540

ccagggaaag cccctaacct cctgatctat actgcatcca ctttacaaag tggggtccca 600

tcaaggttca gcggcagtgg atctgggaca gatttcactc tcaccatcag cagcctgcaa 660

cctgaagatt ttgcaactta ctattgtcaa caggctaaca ttttcccgct cactttcggc 720

ggagggacca aggtggagat caaagagtcc aaatatggtc ccccatgccc accatgccca 780

gcacctgagt tcctgggggg accatcagtc ttcctgttcc ccccaaaacc caaggacact 840

ctcatgatct cccggacccc tgaggtcacg tgcgtggtgg tggacgtgag ccaggaagac 900

cccgaggtcc agttcaactg gtacgtggat ggcgtggagg tgcataatgc caagacaaag 960

ccgcgggagg agcagttcaa cagcacgtac cgtgtggtca gcgtcctcac cgtcctgcac 1020

caggactggc tgaacggcaa ggagtacaag tgcaaggtct ccaacaaagg cctcccgtcc 1080

tccatcgaga aaaccatctc caaagccaaa gggcagcccc gagagccaca ggtgtacacc 1140

ctgcccccat cccaggagga gatgaccaag aaccaggtca gcctgacctg cctggtcaaa 1200

ggcttctacc ccagcgacat cgccgtggag tgggagagca atgggcagcc ggagaacaac 1260

tacaagacca cgcctcccgt gctggactcc gacggctcct tcttcctcta cagcaggcta 1320

accgtggaca agagcaggtg gcaggagggg aatgtcttct catgctccgt gatgcatgag 1380

gctctgcaca accactacac acagaagagc ctctccctgt ctctgggtaa acccttttgg 1440

gtgctggtgg tggttggtgg agtcctggct tgctatagct tgctagtaac agtggccttt 1500

attattttct gggtgaggag taagaggagc aggctcctgc acagtgacta catgaacatg 1560

actccccgcc gccccgggcc cacccgcaag cattaccagc cctatgcccc accacgcgac 1620

ttcgcagcct atcgctcc 1638

<210> 70
<211> 1638
<212> DNA
<213> Artificial Sequence

<220>
<223> Nucleic acid sequence of 28DCR2 (without signal peptide)

<400> 70
caggtgcagc tggtgcagtc tggggctgag gtgaagaagc ctggggcctc agtgaaggtc 60

tcctgcaagg cttctggata caccttcacc ggctactata tgcactgggt gcgacaggcc 120

cctggacaag ggcttgagtg gatgggatgg atcaaccctg acagtggtgg cacaaactat 180

gcacagaagt ttcagggcag ggtcaccatg accagggaca cgtccatcag cacagcctac 240

atggagctga acaggctgag atctgacgac acggccgtgt attactgtgc gagagatcag 300

cccctaggat attgtactaa tggtgtatgc tcctactttg actactgggg ccagggaacc 360

ctggtcaccg tctcctcagg tggaggcggt tcaggcggag gtggcagcgg cggtggcggg 420

tcggacatcc agatgaccca gtctccatct tccgtgtctg catctgtagg agacagagtc 480

accatcactt gtcgggcgag tcagggtatt tacagctggt tagcctggta tcagcagaaa 540

ccagggaaag cccctaacct cctgatctat actgcatcca ctttacaaag tggggtccca 600

tcaaggttca gcggcagtgg atctgggaca gatttcactc tcaccatcag cagcctgcaa 660

cctgaagatt ttgcaactta ctattgtcaa caggctaaca ttttcccgct cactttcggc 720

ggagggacca aggtggagat caaagagtcc aaatatggtc ccccatgccc accatgccca 780

gcacctgagt tcctgggggg accatcagtc ttcctgttcc ccccaaaacc caaggacact 840

ctcatgatct cccggacccc tgaggtcacg tgcgtggtgg tggacgtgag ccaggaagac 900

cccgaggtcc agttcaactg gtacgtggat ggcgtggagg tgcataatgc caagacaaag 960

ccgcgggagg agcagttcaa cagcacgtac cgtgtggtca gcgtcctcac cgtcctgcac 1020

caggactggc tgaacggcaa ggagtacaag tgcaaggtct ccaacaaagg cctcccgtcc 1080

tccatcgaga aaaccatctc caaagccaaa gggcagcccc gagagccaca ggtgtacacc 1140

ctgcccccat cccaggagga gatgaccaag aaccaggtca gcctgacctg cctggtcaaa 1200

ggcttctacc ccagcgacat cgccgtggag tgggagagca atgggcagcc ggagaacaac 1260

tacaagacca cgcctcccgt gctggactcc gacggctcct tcttcctcta cagcaggcta 1320

accgtggaca agagcaggtg gcaggagggg aatgtcttct catgctccgt gatgcatgag 1380

gctctgcaca accactacac acagaagagc ctctccctgt ctctgggtaa acccttttgg 1440

gtgctggtgg tggttggtgg agtcctggct tgctatagct tgctagtaac agtggccttt 1500

attattttct gggtgaggag taagaggagc aggctcctgc acagtgacta catgaacatg 1560

actccccgcc gccccgggcc cacccgcaag cattaccagc cctatgcccc accacgcgac 1620

ttcgcagcct atcgctcc 1638

<210> 71
<211> 1764
<212> DNA
<213> Artificial Sequence

<220>
<223> Nucleic acid sequence of 40DCR3 (without signal peptide)

<400> 71
caggtgcagc tggtgcagtc tggggctgag gtgaagaagc ctggggcctc agtgaaggtc 60

tcctgcaagg cttctggata caccttcacc ggctactata tgcactgggt gcgacaggcc 120

cctggacaag ggcttgagtg gatgggatgg atcaaccctg acagtggtgg cacaaactat 180

gcacagaagt ttcagggcag ggtcaccatg accagggaca cgtccatcag cacagcctac 240

atggagctga acaggctgag atctgacgac acggccgtgt attactgtgc gagagatcag 300

cccctaggat attgtactaa tggtgtatgc tcctactttg actactgggg ccagggaacc 360

ctggtcaccg tctcctcagg tggaggcggt tcaggcggag gtggcagcgg cggtggcggg 420

tcggacatcc agatgaccca gtctccatct tccgtgtctg catctgtagg agacagagtc 480

accatcactt gtcgggcgag tcagggtatt tacagctggt tagcctggta tcagcagaaa 540

ccagggaaag cccctaacct cctgatctat actgcatcca ctttacaaag tggggtccca 600

tcaaggttca gcggcagtgg atctgggaca gatttcactc tcaccatcag cagcctgcaa 660

cctgaagatt ttgcaactta ctattgtcaa caggctaaca ttttcccgct cactttcggc 720

ggagggacca aggtggagat caaagagtcc aaatatggtc ccccatgccc accatgccca 780

gcacctgagt tcctgggggg accatcagtc ttcctgttcc ccccaaaacc caaggacact 840

ctcatgatct cccggacccc tgaggtcacg tgcgtggtgg tggacgtgag ccaggaagac 900

cccgaggtcc agttcaactg gtacgtggat ggcgtggagg tgcataatgc caagacaaag 960

ccgcgggagg agcagttcaa cagcacgtac cgtgtggtca gcgtcctcac cgtcctgcac 1020

caggactggc tgaacggcaa ggagtacaag tgcaaggtct ccaacaaagg cctcccgtcc 1080

tccatcgaga aaaccatctc caaagccaaa gggcagcccc gagagccaca ggtgtacacc 1140

ctgcccccat cccaggagga gatgaccaag aaccaggtca gcctgacctg cctggtcaaa 1200

ggcttctacc ccagcgacat cgccgtggag tgggagagca atgggcagcc ggagaacaac 1260

tacaagacca cgcctcccgt gctggactcc gacggctcct tcttcctcta cagcaggcta 1320

accgtggaca agagcaggtg gcaggagggg aatgtcttct catgctccgt gatgcatgag 1380

gctctgcaca accactacac acagaagagc ctctccctgt ctctgggtaa acccttttgg 1440

gtgctggtgg tggttggtgg agtcctggct tgctatagct tgctagtaac agtggccttt 1500

attattttct gggtgaggag taagaggagc aggctcctgc acagtgacta catgaacatg 1560

actccccgcc gccccgggcc cacccgcaag cattaccagc cctatgcccc accacgcgac 1620

ttcgcagcct atcgctccaa acggggcaga aagaagctcc tgtatatatt caaacaacca 1680

tttatgagac cagtacaaac tactcaagag gaagatggct gtagctgccg atttccagaa 1740

gaagaagaag gaggatgtga actg 1764

<210> 72
<211> 1764
<212> DNA
<213> Artificial Sequence

<220>
<223> Nucleic acid sequence of 40DCR4 (without signal peptide)

<400> 72
caggtgcagc tggtgcagtc tggggctgag gtgaagaagc ctggggcctc agtgaaggtc 60

tcctgcaagg cttctggata caccttcacc ggctactata tgcactgggt gcgacaggcc 120

cctggacaag ggcttgagtg gatgggatgg atcaaccctg acagtggtgg cacaaactat 180

gcacagaagt ttcagggcag ggtcaccatg accagggaca cgtccatcag cacagcctac 240

atggagctga acaggctgag atctgacgac acggccgtgt attactgtgc gagagatcag 300

cccctaggat attgtactaa tggtgtatgc tcctactttg actactgggg ccagggaacc 360

ctggtcaccg tctcctcagg tggaggcggt tcaggcggag gtggcagcgg cggtggcggg 420

tcggacatcc agatgaccca gtctccatct tccgtgtctg catctgtagg agacagagtc 480

accatcactt gtcgggcgag tcagggtatt tacagctggt tagcctggta tcagcagaaa 540

ccagggaaag cccctaacct cctgatctat actgcatcca ctttacaaag tggggtccca 600

tcaaggttca gcggcagtgg atctgggaca gatttcactc tcaccatcag cagcctgcaa 660

cctgaagatt ttgcaactta ctattgtcaa caggctaaca ttttcccgct cactttcggc 720

ggagggacca aggtggagat caaagagtcc aaatatggtc ccccatgccc accatgccca 780

gcacctgagt tcctgggggg accatcagtc ttcctgttcc ccccaaaacc caaggacact 840

ctcatgatct cccggacccc tgaggtcacg tgcgtggtgg tggacgtgag ccaggaagac 900

cccgaggtcc agttcaactg gtacgtggat ggcgtggagg tgcataatgc caagacaaag 960

ccgcgggagg agcagttcaa cagcacgtac cgtgtggtca gcgtcctcac cgtcctgcac 1020

caggactggc tgaacggcaa ggagtacaag tgcaaggtct ccaacaaagg cctcccgtcc 1080

tccatcgaga aaaccatctc caaagccaaa gggcagcccc gagagccaca ggtgtacacc 1140

ctgcccccat cccaggagga gatgaccaag aaccaggtca gcctgacctg cctggtcaaa 1200

ggcttctacc ccagcgacat cgccgtggag tgggagagca atgggcagcc ggagaacaac 1260

tacaagacca cgcctcccgt gctggactcc gacggctcct tcttcctcta cagcaggcta 1320

accgtggaca agagcaggtg gcaggagggg aatgtcttct catgctccgt gatgcatgag 1380

gctctgcaca accactacac acagaagagc ctctccctgt ctctgggtaa acccttttgg 1440

gtgctggtgg tggttggtgg agtcctggct tgctatagct tgctagtaac agtggccttt 1500

attattttct gggtgaaacg gggcagaaag aagctcctgt atatattcaa acaaccattt 1560

atgagaccag tacaaactac tcaagaggaa gatggctgta gctgccgatt tccagaagaa 1620

gaagaaggag gatgtgaact gaggagtaag aggagcaggc tcctgcacag tgactacatg 1680

aacatgactc cccgccgccc cgggcccacc cgcaagcatt accagcccta tgccccacca 1740

cgcgacttcg cagcctatcg ctcc 1764

<210> 73
<211> 499
<212> PRT
<213> Artificial Sequence

<220>
<223> Amino acid sequence of EGFR G1 CAR

<400> 73

Ala Thr Met Ala Leu Pro Val Thr Ala Leu Leu Leu Pro Leu Ala Leu
1               5                   10                  15

Leu Leu His Ala Ala Arg Pro Ser Asp Ile Leu Leu Thr Gln Ser Pro
            20                  25                  30

Val Ile Leu Ser Val Ser Pro Gly Glu Arg Val Ser Phe Ser Cys Arg
        35                  40                  45

Ala Ser Gln Ser Ile Gly Thr Asn Ile His Trp Tyr Gln Gln Arg Thr
    50                  55                  60

Asn Gly Ser Pro Arg Leu Leu Ile Lys Tyr Ala Ser Glu Ser Ile Ser
65                  70                  75                  80

Gly Ile Pro Ser Arg Phe Ser Gly Ser Gly Ser Gly Thr Asp Phe Thr

85                  90                  95

Leu Ser Ile Asn Ser Val Glu Ser Glu Asp Ile Ala Asp Tyr Tyr Cys
            100                 105                 110

Gln Gln Asn Asn Asn Trp Pro Thr Thr Phe Gly Ala Gly Thr Lys Leu
        115                 120                 125

Glu Leu Lys Gly Gly Gly Gly Ser Gly Gly Gly Gly Ser Gly Gly Gly
    130                 135                 140

Gly Ser Gln Val Gln Leu Lys Gln Ser Gly Pro Gly Leu Val Gln Pro
145                 150                 155                 160

Ser Gln Ser Leu Ser Ile Thr Cys Thr Val Ser Gly Phe Ser Leu Thr
                165                 170                 175

Asn Tyr Gly Val His Trp Val Arg Gln Ser Pro Gly Lys Gly Leu Glu
            180                 185                 190

Trp Leu Gly Val Ile Trp Ser Gly Gly Asn Thr Asp Tyr Asn Thr Pro
        195                 200                 205

Phe Thr Ser Arg Leu Ser Ile Asn Lys Asp Asn Ser Lys Ser Gln Val
    210                 215                 220

Phe Phe Lys Met Asn Ser Leu Gln Ser Asn Asp Thr Ala Ile Tyr Tyr
225                 230                 235                 240

Cys Ala Arg Ala Leu Thr Tyr Tyr Asp Tyr Glu Phe Ala Tyr Trp Gly
                245                 250                 255

Gln Gly Thr Leu Val Thr Val Ser Ser Phe Val Pro Val Phe Leu Pro
            260                 265                 270

Ala Lys Pro Thr Thr Thr Pro Ala Pro Arg Pro Pro Thr Pro Ala Pro
        275                 280                 285

Thr Ile Ala Ser Gln Pro Leu Ser Leu Arg Pro Glu Ala Cys Arg Pro
    290                 295                 300

Ala Ala Gly Gly Ala Val His Thr Arg Gly Leu Asp Phe Ala Cys Asp
305                 310                 315                 320

Ile Tyr Ile Trp Ala Pro Leu Ala Gly Thr Cys Gly Val Leu Leu Leu
                325                 330                 335

Ser Leu Val Ile Thr Leu Tyr Cys Asn His Arg Ser Lys Arg Ser Arg
            340                 345                 350

Leu Leu His Ser Asp Tyr Met Asn Met Thr Pro Arg Arg Pro Gly Pro
        355                 360                 365

Thr Arg Lys His Tyr Gln Pro Tyr Ala Pro Pro Arg Asp Phe Ala Ala
    370                 375                 380

Tyr Arg Ser Arg Val Lys Phe Ser Arg Ser Ala Asp Ala Pro Ala Tyr
385                 390                 395                 400

Gln Gln Gly Gln Asn Gln Leu Tyr Asn Glu Leu Asn Leu Gly Arg Arg
                405                 410                 415

Glu Glu Tyr Asp Val Leu Asp Lys Arg Arg Gly Arg Asp Pro Glu Met
            420                 425                 430

Gly Gly Lys Pro Arg Arg Lys Asn Pro Gln Glu Gly Leu Tyr Asn Glu
        435                 440                 445

Leu Gln Lys Asp Lys Met Ala Glu Ala Tyr Ser Glu Ile Gly Met Lys
    450                 455                 460

Gly Glu Arg Arg Arg Gly Lys Gly His Asp Gly Leu Tyr Gln Gly Leu
465                 470                 475                 480

Ser Thr Ala Thr Lys Asp Thr Tyr Asp Ala Leu His Met Gln Ala Leu
                485                 490                 495

Pro Pro Arg

<210>  74
<211>  1503
<212>  DNA
<213>  Artificial Sequence

<220>
<223>  Nucleic acid sequence of EGFR G1 CAR

<400>  74
gccaccatgg ccttaccagt gaccgccttg ctcctgccgc tggccttgct gctccacgcc      60

gccaggccga gcgacatctt gctgactcag tctccagtca tcctgtctgt gagtccagga     120

gaaagagtca gtttctcctg cagggccagt cagagtattg gcacaaacat acactggtat     180

cagcaaagaa caaatggttc tccaaggctt ctcataaagt atgcttctga gtctatctct     240

gggatccctt ccaggtttag tggcagtgga tcagggacag attttactct tagcatcaac     300

agtgtggagt ctgaagatat tgcagattat tactgtcaac aaaataataa ctggccaacc 360

acgttcggtg ctgggaccaa gctggagctg aaaggtggag gcggttcagg cggaggtggc 420

agcggcggtg gcgggtcgca ggtgcagctg aagcagtcag gacctggcct agtgcagccc 480

tcacagagcc tgtccatcac ctgcacagtc tctggtttct cattaactaa ctatggtgta 540

cactgggttc gccagtctcc aggaaagggt ctggagtggc tgggagtgat atggagtggt 600

ggaaacacag actataatac acctttcaca tccagactga gcatcaacaa ggacaattcc 660

aagagccaag ttttctttaa aatgaacagt ctgcaatcta atgacacagc catatattac 720

tgtgccagag ccctcaccta ctatgattac gagtttgctt actggggcca agggactctg 780

gtcactgtct cttcgttcgt gccggtcttc ctgccagcga agcccaccac gacgccagcg 840

ccgcgaccac caacaccggc gcccaccatc gcgtcgcagc ccctgtccct gcgcccagag 900

gcgtgccggc cagcggcggg gggcgcagtg cacacgaggg ggctggactt cgcctgtgat 960

atctacatct gggcgcccct ggccgggact tgtggggtcc ttctcctgtc actggttatc 1020

accctttact gcaaccacag gagtaagagg agcaggctcc tgcacagtga ctacatgaac 1080

atgactcccc gccgccccgg gcccacccgc aagcattacc agccctatgc cccaccacgc 1140

gacttcgcag cctatcgctc cagagtgaag ttcagcagga gcgcagacgc ccccgcgtac 1200

cagcagggcc agaaccagct ctataacgag ctcaatctag gacgaagaga ggagtacgat 1260

gttttggaca agagacgtgg ccgggaccct gagatggggg gaaagccgag aaggaagaac 1320

cctcaggaag gcctgtacaa tgaactgcag aaagataaga tggcggaggc ctacagtgag 1380

attgggatga aaggcgagcg ccggaggggc aaggggcacg atggccttta ccagggtctc 1440

agtacagcca ccaaggacac ctacgacgcc cttcacatgc aggccctgcc ccctcgctga 1500

taa 1503

<210> 75
<211> 1503
<212> DNA
<213> Artificial Sequence

<220>
<223> Nucleotide sequence of EGFR G2 CAR

<400> 75
gccaccatgg ccttaccagt gaccgccttg ctcctgccgc tggccttgct gctccacgcc 60

gccaggccga gcgacatctt gctgactcag tctccagtca tcctgtctgt gagtccagga 120

gaaagagtca gtttctcctg cagggccagt cagagtattg gcacaaacat acactggtat 180

cagcaaagaa caaatggttc tccaaggctt ctcataaagt atgcttctga gtctatctct 240

gggatccctt ccaggtttag tggcagtgga tcagggacag attttactct tagcatcaac 300

agtgtggagt ctgaagatat tgcagattat tactgtcaac aaaataataa ctggccaacc 360

acgttcggtg ctgggaccaa gctggagctg aaaggtggag gcggttcagg cggaggtggc 420

agcggcggtg gcgggtcgca ggtgcagctg aagcagtcag gacctggcct agtgcagccc 480

tcacagagcc tgtccatcac ctgcacagtc tctggtttct cattaactaa ctatggtgta 540

cactgggttc gccagtctcc aggaaagggt ctggagtggc tgggagtgat atggagtggt 600

ggaaacacag actataatac acctttcaca tccagactga gcatcaacaa ggacaattcc 660

aagagccaag ttttctttaa aatgaacagt ctgcaatcta atgacacagc catatattac 720

tgtgccagag ccctcaccta ctatgattac gagtttgctt actggggcca agggactctg 780

gtcactgtct cttcgttcgt gccggtcttc ctgccagcga agcccaccac gacgccagcg 840

ccgcgaccac caacaccggc gcccaccatc gcgtcgcagc ccctgtccct gcgcccagag 900

gcgtgccggc cagcggcggg gggcgcagtg cacacgaggg ggctggactt cgcctgtgat 960

atctacatct gggcgcccct ggccgggact tgtggggtcc ttctcctgtc actggttatc 1020

accctttact gcaaccacag gagtaagagg agcaggctcc tgcacagtga ctacatgaac 1080

atgactcccc gccgccccgg gcccacccgc aagcattacc agccctatgc cccaccacgc 1140

gacttcgcag cctatcgctc cagagtgaag ttcagcagga gcgcagacgc ccccgcgtac 1200

cagcagggcc agaaccagct ctataacgag ctcaatctag gacgaagaga ggagtacgat 1260

gttttggaca agagacgtgg ccgggaccct gagatggggg gaaagccgag aaggaagaac 1320

cctcaggaag gcctgtacaa tgaactgcag aaagataaga tggcggaggc ctacagtgag 1380

attgggatga aaggcgagcg ccggaggggc aaggggcacg atggccttta ccagggtctc 1440

agtacagcca ccaaggacac ctacgacgcc cttcacatgc aggccctgcc ccctcgctga 1500

taa 1503

<210> 76
<211> 18
<212> DNA
<213> Artificial Sequence

<220>
<223> Nucleotide sequence of primer CD40-F

<400> 76
acctcctgat ctatactg 18

<210> 77
<211> 18
<212> DNA
<213> Artificial Sequence

<220>
<223> Nucleotide sequence of primer CD40-R

<400> 77

gatggtgaga gtgaaatc                                                    18

<210>  78
<211>  21
<212>  DNA
<213>  Artificial Sequence

<220>
<223>  Nucleotide sequence in Taqman

<400>  78
cactgccgct gaaccttgat g                                               21